(19) Europäisches Patentamt
European Patent Office
Office européen des brevets

(11) EP 3 754 028 A1

(12) **EUROPEAN PATENT APPLICATION**

(43) Date of publication:
**23.12.2020 Bulletin 2020/52**

(51) Int Cl.:
***C12Q 1/6813*** *(2018.01)*

(21) Application number: **19181051.4**

(22) Date of filing: **18.06.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Apollo Life Sciences GmbH**
**40474 Düsseldorf (DE)**

(72) Inventors:
- **Geipel, Andreas**
  **40724 Hilden (DE)**
- **Reinecke, Frank**
  **40724 Hilden (DE)**
- **Korfhage, Christian**
  **40724 Hilden (DE)**
- **Jagemann, Nadine**
  **40724 Hilden (DE)**
- **Möllering, Vanessa**
  **40233 Düsseldorf (DE)**

(74) Representative: **Witte, Weller & Partner**
**Patentanwälte mbB**
**Postfach 10 54 62**
**70047 Stuttgart (DE)**

# (54) METHOD OF SIGNAL ENCODING OF ANALYTES IN A SAMPLE

(57) The present invention is directed to a method of sequential signal-encoding of analytes in a sample, a use of a set of decoding oligonucleotides to sequentially signal-encode analytes in a sample, and to a kit for sequentially signal-encoding of analytes in a sample.

EP 3 754 028 A1

**Description**

The present invention is directed to a method of sequential signal-encoding of analytes in a sample, a use of a set of decoding oligonucleotides to sequentially signal-encode analytes in a sample, and to a kit for sequentially signal-encoding of analytes in a sample.

FIELD OF THE INVENTION

The present invention relates to the field of molecular biology, more particularly to the detection of analytes in a sample, preferably the detection of biomolecules such as nucleic acid molecules and/or proteins in a biological sample.

BACKGROUND OF THE INVENTION

The analysis and detection of small quantities of analytes in biological and non-biological samples has become a routine practice in the clinical and analytical environment. Numerous analytical methods have been established for this purpose. Some of them use encoding techniques assigning a particular readable code to a specific first analyte which differs from a code assigned to a specific second analyte.

One of the prior art techniques in this field is the so-called 'single molecule fluorescence in situ hybridization' (smFISH) essentially developed to detect mRNA molecules in a sample. In Lubeck et al. (2014), Single-cell in situ RNA profiling by sequential hybridization, Nat. Methods11(4), p. 360-361, the mRNAs of interest are detected via specific directly labeled probe sets. After one round of hybridization and detection, the set of mRNA specific probes is eluted from the mRNAs and the same set of probes with other (or the same) fluorescent labels is used in the next round of hybridization and imaging to generate gene specific color-code schemes over several rounds. The technology needs several differently tagged probe sets per transcript and needs to denature these probe sets after every detection round.

A further development of this technology does not use directly labeled probe sets. Instead, the oligonucleotides of the probe sets provide nucleic acid sequences that serve as initiator for hybridization chain reactions (HCR), a technology that enables signal amplification; see Shah et al. (2016), In situ transcription profiling of single cells reveals spatial organization of cells in the mouse hippocampus, Neuron 92(2), p. 342-357.

Another technique referred to as 'multiplexed error robust fluorescence in situ hybridization' (merFISH) is described by Chen et al. (2015), RNA imaging. Spatially resolved, highly multiplexed RNA profiling in single cells, Science 348(6233):aaa6090. There, the mRNAs of interest are detected via specific probe sets that provide additional sequence elements for the subsequent specific hybridization of fluorescently labeled oligonucleotides. Each probe set provides four different sequence elements out of a total of 16 sequence elements. After hybridization of the specific probe sets to the mRNAs of interest, the so-called readout hybridizations are performed. In each readout hybridization one out of the 16 fluorescently labeled oligonucleotides complementary to one of the sequence elements is hybridized. All readout oligonucleotides use the same fluorescent color. After imaging, the fluorescent signals are destroyed via illumination and the next round of readout hybridization takes place without a denaturing step. As a result, a binary code is generated for each mRNA species. A unique signal signature of 4 signals in 16 rounds is created using only a single hybridization round for binding of specific probe sets to the mRNAs of interest, followed by 16 rounds of hybridization of readout oligonucleotides labeled by a single fluorescence color.

A further development of this technology improves the throughput by using two different fluorescent colors, eliminating the signals via disulfide cleavage between the readout-oligonucleotides and the fluorescent label and an alternative hybridization buffer; see Moffitt et al. (2016), High-throughput single-cell gene-expression profiling with multiplexed error-robust fluorescence in situ hybridization, Proc. Natl. Acad. Sci. U S A. 113(39), p. 11046-11051.

A technology referred to as 'intron seqFISH' is described in Shah et al. (2018), Dynamics and spatial genomics of the nascent transcriptome by intron seqFISH, Cell 117(2), p. 363-376. There, the mRNAs of interest are detected via specific probe sets that provide additional sequence elements for the subsequent specific hybridization of fluorescently labeled oligonucleotides. Each probe set provides one out of 12 possible sequence elements (representing the 12 'pseudocolors' used) per color-coding round. Each color-coding round consists of four serial hybridizations. In each of these serial hybridizations, three readout probes, each labeled with a different fluorophore, are hybridized to the corresponding elements of the mRNA-specific probe sets. After imaging, the readout probes are stripped off by a 55% formamide buffer and the next hybridization follows. After 5 color-coding rounds with 4 serial hybridizations each, the color-codes are completed.

EP 0 611 828 discloses the use of a bridging element to recruit a signal generating element to probes that specifically bind to an analyte. A more specific statement describes the detection of nucleic acids via specific probes that recruit a bridging nucleic acid molecule. This bridging nucleic acids eventually recruit signal generating nucleic acids. This document also describes the use of a bridging element with more than one binding site for the signal generating element for signal amplification like branched DNA.

Player et al. (2001), Single-copy gene detection using branched DNA (bDNA) in situ hybridization, J. Histochem. Cytochem. 49(5), p. 603-611, describe a method where the nucleic acids of interest are detected via specific probe sets providing an additional sequence element. In a second step, a preamplifier oligonucleotide is hybridized to this sequence element. This preamplifier oligonucleotide comprises multiple binding sites for amplifier oligonucleotides that are hybridized in a subsequent step. These amplifier oligonucleotides provide multiple sequence elements for the labeled oligonucleotides. This way a branched oligonucleotide tree is build up that leads to an amplification of the signal.

A further development of this method referred to as is described by Wang et al. (2012), RNAscope: a novel in situ RNA analysis platform for formalin-fixed, paraffin-embedded tissues, J. Mol. Diagn. 14(1), p.22-29, which uses another design of the mRNA-specific probes. Here two of the mRNA-specific oligonucleotides have to hybridize in close proximity to provide a sequence that can recruit the preamplifier oligonucleotide. This way the specificity of the method is increased by reducing the number of false positive signals.

Choi et al. (2010), Programmable in situ amplification for multiplexed imaging of mRNA expression, Nat. Biotechnol. 28(11), p. 1208-1212, disclose a method known as 'HCR-hybridization chain reaction'. The mRNAs of interest are detected via specific probe sets that provide an additional sequence element. The additional sequence element is an initiator sequence to start the hybridization chain reaction. Basically, the hybridization chain reaction is based on metastable oligonucleotide hairpins that self-assemble into polymers after a first hairpin is opened via the initiator sequence.

A further development of the technology uses so called split initiator probes that have to hybridize in close proximity to form the initiator sequence for HCR, similarly to the RNAscope technology, this reduces the number of false positive signals; see Choi et al. (2018), Third-generation in situ hybridization chain reaction: multiplexed, quantitative, sensitive, versatile, robust. Development 145(12).

The methods known in the art, however, have numerous disadvantages. In particular, they are inflexible, expensive, complex, time consuming and quite often provide non-accurate results. In particular, the encoding capacities of the existing methods are low and do not meet the requirements of modern molecular biology and medicine.

Against this background, it is an object underlying the present invention to provide a method by means of which the disadvantages of the prior art methods can be reduced or even avoided.

The present invention satisfies these and other needs.

SUMMARY OF THE INVENTION

The present invention provides a method of sequential signal-encoding of analytes in a sample, the method comprising the steps:

(1) providing a set of analyte-specific probes, each analyte-specific probe comprising:

- a binding element (S) that specifically interacts with one of the analytes to be encoded, and
- an identifier element (T) comprising a nucleotide sequence which is unique to said set of analyte-specific probes (unique identifier sequence);

(2) incubating the set of analyte-specific probes with the sample, thereby allowing a specific binding of the analyte-specific probes to the analyte to be encoded;

(3) removing non-bound probes from the sample;

(4) providing a set of decoding oligonucleotides, each decoding oligonucleotide comprising:

- a first connector element (t) comprising a nucleotide sequence which is essentially complementary to at least a section of the unique identifier sequence, and
- a translator element (c) comprising a nucleotide sequence allowing a specific hybridization of a signal oligonucleotide;

(5) incubating the set of decoding oligonucleotides with the sample, thereby allowing a specific hybridization of the decoding oligonucleotides to the unique identifier sequence;

(6) removing non-bound decoding oligonucleotides from the sample;

(7) providing a set of signal oligonucleotides, each signal oligonucleotide comprising:

- a second connector element (C) comprising a nucleotide sequence which is essentially complementary to at least a section of the nucleotide sequence of the translator element (c), and
- a signal element; and

(8) incubating the set of signal oligonucleotides with the sample, thereby allowing a specific hybridization of the signal oligonucleotides to the translator element (c).

The inventors have realized that this novel method provides the essential steps required to set up a process allowing the specific quantitative and/or spatial detection of different analytes in a sample in parallel via specific hybridization. The technology allows distinguishing a higher number of analytes than different signals available. In contrast to other state-of-the-art methods the oligonucleotides providing the detectable signal are not directly interacting with sample-specific nucleic acid sequences but are mediated by so called 'decoding-oligonucleotides'. This mechanism decouples the dependency between the analyte-specific oligonucleotides and the signal oligonucleotides and, therefore, results in a dramatical increase of the encoding capacity.

Another subject-matter of the invention is the use of a set of decoding oligonucleotides to sequentially signal-encode analytes in a sample, each decoding oligonucleotide comprising:

- a first connector element (t) comprising a nucleotide sequence which is essentially complementary to at least a section of a nucleotide sequence which is unique to a set of analyte-specific probes (unique identifier sequence), and
- a translator element (c) comprising a nucleotide sequence allowing a specific hybridization of a signal oligonucleotide.

A still further subject-matter of the present invention is a kit for sequentially signal-encoding of analytes in a sample, comprising

- a set of analyte-specific probes, each analyte-specific probe comprising:
- a binding element (S) that specifically interacts with one of the analytes to be encoded, and
- an identifier element (T) comprising a nucleotide sequence which is unique to said set of analyte-specific probes (unique identifier sequence); and
- a set of decoding oligonucleotides, each decoding oligonucleotide comprising:
- a first connector element (t) comprising a nucleotide sequence which is essentially complementary to at least a section of the unique identifier sequence, and
- a translator element (c) comprising a nucleotide sequence allowing a specific hybridization of a signal oligonucleotide; and, preferably,
- a set of signal oligonucleotides, each signal oligonucleotide comprising:
- a second connector element (C) comprising a nucleotide sequence which is essentially complementary to at least a section of the nucleotide sequence of the translator element (c), and
- a signal element.

The use of decoding-oligonucleotides allows a much higher flexibility while dramatically decreasing the number of different signal oligonucleotides needed which in turn increases the encoding capacity achieved. The utilization of decoding-oligonucleotides leads to a sequential signal-coding technology that is more flexible, cheaper, simpler, faster and/or more accurate than other methods. In particular, the invention results in a significant increase of the encoding capacity in comparison to the prior art methods.

The use of decoding oligonucleotides breaks the dependencies between the target specific probes and the signal oligonucleotides. Without decoupling target specific probes and signal generation as in the methods of the state of the art, two different signals can only be generated for a certain target if using two different molecular tags. Each of these molecular tags can only be used once. Multiple readouts of the same molecular tag do not increase the information about the target. In order to create an encoding scheme, a change of the target specific probe set after each round is required (SeqFISH) or multiple molecular tags must be present on the same probe set (like merFISH, intronSeqFISH). These restrictions in the art are very relevant and reduce the flexibility, coding capacity, accuracy, reproducibility and increase the costs of the experiment.

According to the invention an "analyte" is the subject to be specifically detected as being present or absent in a sample and, in case of its presence, to encode it. It can be any kind of entity, including a protein or a nucleic acid molecule of interest. The analyte provides at least one site for specific binding with analyte-specific probes. Sometimes herein the term "analyte" is replaced by "target".

A "sample" as referred to herein is a composition in liquid or solid form suspected of comprising the analytes to be encoded.

An "oligonucleotide" as used herein, refers to s short nucleic acid molecule, such as DNA, PNA, LNA or RNA. The length of the oligonucleotides is within the range 4-200 nucleotides (nt), preferably 6-80 nt, more preferably 8-60 nt, more preferably 10-50 nt, more preferably 12 to 35 depending on the number of consecutive sequence elements. The nucleic acid molecule can be fully or partially single-stranded. The oligonucleotides may be linear or may comprise hairpin or loop structures. The oligonucleotides may comprise modifications such as biotin, labeling moieties, blocking moieties, or other modifications.

The "analyte-specific probe" consists of at least two elements, namely the so-called binding element (S) which specifically interacts with one of the analytes, and a so-called identifier element (T) comprising the 'unique identifier sequence'. The binding element may be a nucleic acid such as a hybridization sequence or an aptamer, or a peptidic structure such as an antibody. The "unique identifier sequence" as comprised by the analyte-specific probe is unique in its sequence compared to other unique identifiers. "Unique" in this context means that it specifically identifies only one analyte, such as Cyclin A, Cyclin D, Cyclin E etc., or, alternatively, it specifically identifies only a group of analytes, independently whether the group of analytes comprises a gene family or not. Therefore, the analyte or a group of analytes to be encoded by this unique identifier can be distinguished from all other analytes or groups of analytes that are to be encoded based on the sequence of the element. The length of the unique identifier sequence is within the range 8-60 nt, preferably 12-40 nt, more preferably 14-20 nt, depending on the number of analytes encoded in parallel and the stability of interaction needed. A unique identifier may be a sequence element of the analyte-specific probe, attached directly or by a linker, a covalent bond or high affinity binding modes, e.g. antibody-antigen interaction, streptavidin-biotin interaction etc.

A "decoding oligonucleotide" consists of at least two sequence elements. One sequence element that can specifically bind to a unique identifier sequence, referred to as "first connector element" (t), and a second sequence element specifically binding to a signal oligonucleotide, referred to as "translator element" (c). The length of the sequence elements is within the range 8-60 nt, preferably 12-40 nt, more preferably 14-20 nt, depending on the number of analytes to be encoded in parallel, the stability of interaction needed and the number of different signal oligonucleotides used. The length of the two sequence elements may or may not be the same.

A "signal oligonucleotide" as used herein comprises at least two elements, a so-called "second connector element" (C) having a nucleotide sequence specifically hybridizable to at least a section of the nucleotide sequence of the translator element (c) of the decoding oligonucleotide, and a "signal element" which provides a detectable signal. This element can either actively generate a detectable signal or provide such a signal via manipulation, e.g. fluorescent excitation. Typical signal elements are, for example, enzymes that catalyze a detectable reaction, fluorophores, radioactive elements or dyes.

A "set" refers to a plurality of moieties or subjects, e.g. analyte-specific probes or decoding oligonucleotides, whether the individual members of said plurality are identical or different from each other. In an embodiment of the invention a single set refers to a plurality of oligonucleotides

An "analyte specific probe set" refers to a plurality of moieties or subjects, e.g. analyte-specific probes that are different from each other and bind to independent regions of the analyte. A single analyte specific probe set is further characterized by the same unique identifier.

A "decoding oligonucleotide set" refers to a plurality of decoding oligonucleotides specific for a certain unique identifier needed to realize the encoding independent of the length of the code word.

In the "incubation" steps as understood herein the respective moieties or subjects such as probes or oligonucleotide, are brought into contact with each other under conditions well known to the skilled person allowing a specific binding or hybridization reaction, e.g. pH, temperature, salt conditions etc. Such steps may therefore, be preferably carried out in a liquid environment such as a buffer system which is well known in the art.

The "removing" steps according to the invention may include the washing away of the moieties or subjects to be removed such as the probes or oligonucleotides by certain conditions, e.g. pH, temperature, salt conditions etc., as known in the art.

A "kit" is a combination of individual elements useful for carrying out the use and/or method of the invention, wherein the elements are optimized for use together in the methods. The kits may also contain additional reagents, chemicals, buffers, reaction vials etc. which may be useful for carrying out the method according to the invention. Such kits unify all essential elements required to work the method according to the invention, thus minimizing the risk of errors. Therefore, such kits also allow semi-skilled laboratory staff to perform the method according to the invention.

The features, characteristics, advantages and embodiments specified herein apply to the method, use, and kit according to the invention, even if not specifically indicated.

In an embodiment of the invention the sample is a biological sample, preferably comprising biological tissue, further preferably comprising biological cells.

The method is particularly qualified to encode or identify analytes in a biological sample, i.e. such as a sample which contains nucleic acids or proteins as said analytes. It is understood that the biological sample may be in a form as it is in its natural environment (i.e. liquid, semi-liquid, solid etc.), or processed, e.g. as a dried film on the surface of a device which may be re-liquefied before the method is carried out.

In another embodiment of the invention prior to step (2) the biological tissue and/or biological cells are fixed.

This measure has the advantage that the analytes to be encoded, e.g. the nuclei acids or proteins, are immobilized and cannot escape. In doing so, the analytes then prepared for a better detection or encoding by the method according to the invention. The fixation of the sample can be, e.g., carried out by means of formaline, ethanol, methanol or other components well known to the skilled person.

In yet a further embodiment within the set of analyte-specific probes the individual analyte-specific probes comprise binding elements (S1, S2, S3, S4, S5) which specifically interact with different sub-structures of one of the analytes to be encoded.

By this measure the method becomes even more robust and reliable because the signal intensity obtained at the end of the method or a cycle, respectively, is increased. It is understood, that the individual probes of a set while binding to the same analyte differ in their binding position or binding site at or on the analyte. The binding elements S1, S2, S3, S4, S5 etc. of the first, second, third fourth, fifth etc. analyte-specific probes therefore bind to or at a different position which, however, may or may not overlap.

in another embodiment of the method according to the invention it comprises the following additional steps:

(9) removing non-bound signal oligonucleotides from the sample; and

(10) detecting the signal.

By this measure the method is further developed to such an extent that the encoded analytes can be detected by any means which is adapted to visualize the signal element. Examples of detectable physical features include e.g. light, chemical reactions, molecular mass, radioactivity, etc.

In a further embodiment of the method according to the invention the following additional step is carried out: (11) selectively removing the decoding oligonucleotides and signal oligonucleotides from the sample, thereby essentially maintaining the specific binding of the analyte-specific probes to the analyte to be encoded.

By this measure the requirements for another round of binding further decoding oligonucleotides to the same analyte-specific probes are established, thus finally resulting in a code or encoding scheme comprising more than one signal. This step is realized by applying conditions and factors well known to the skilled person, e.g. pH, temperature, salt conditions, oligonucleotide concentration, polymers etc.

In another embodiment of the invention, the method comprises the following step:
(12) repeating steps (4)-(11) at least once [$(4_2)$-$(11_2)$] to generate an encoding scheme consisting of at least two signals.

With this measure a code of more than one signal is set up, i.e. of two, three, four, five etc. signals in case of two [$(4_2)$-$(11_2)$], three [$(4_3)$-$(11_3)$], four [$(4_4)$-$(11_4)$], five [$(4_5)$-$(11_5)$], etc. [$(4_n)$-$(11_n)$] rounds which are carried out by the user, where 'n' is an integer representing the number of rounds. The encoding capacity of the method according to the invention is herewith increased depending on the nature of the analyte and the needs of the operator.

In an embodiment of the invention said encoding scheme is predetermined and allocated to the analyte to be encoded.

This measure enables a precise experimental set-up by providing the appropriate sequential order of the employed decoding and signal oligonucleotides and, therefore, allows the correct allocation of a specific analyte to a respective encoding scheme.

The decoding oligonucleotides which are used in repeated steps $(4_2)$-$(11_2)$ may comprise a translator element (c2) which is identical with the translator element (c1) of the decoding oligonucleotides used in previous steps (4)-(11). In another embodiment of the invention decoding oligonucleotides are used in repeated steps $(4_2)$-$(11_2)$ comprising a translator element (c2) which differs from the translator element (c1) of the decoding oligonucleotides used in previous steps (4)-(11).

It is understood that the decoding elements may or may not be changed from round to round, i.e. in the second

round ($4_2$)-($11_2$) comprising the translator element c2, in the third round ($4_3$)-($11_3$) comprising the translator element c3, in the fourth round ($4_4$)-($11_4$) comprising the translator element c4, in the fifths round comprising the translator element c5, in the 'n' round ($4_n$)-($11_n$) comprising the translator element cn, etc., wherein 'n' is an integer representing the number of rounds.

The signal oligonucleotides which are used in repeated steps ($4_2$)-($11_2$) may comprise a signal element which is identical with the signal element of the decoding oligonucleotides used in previous steps (4)-(11). In a further embodiment of the invention signal oligonucleotides are used in repeated steps ($4_2$)-($11_2$) comprising a signal element which differs from the signal element of the decoding oligonucleotides used in previous steps (4)-(11).

By this measure each round the same or a different signal is provided resulting in an encoding scheme characterized by a signal sequence consisting of numerous different signals. This measure allows the creation of a unique code or code word which differs from all other code words of the encoding scheme.

In another embodiment of the invention the binding element (S) of the analyte-specific probe comprises a nucleic acid comprising a nucleotide sequence allowing a specific binding to the analyte to be encoded, preferably a specific hybridization to the analyte to be encoded.

This measure creates the condition for encoding a nucleic acid analyte, such as an mRNA, e.g. such an mRNA coding for a particular protein.

In an alternative embodiment of the invention the binding element (S) of the analyte-specific probe comprises an amino acid sequence allowing a specific binding to the analyte to be encoded, preferably the binding element is an antibody.

This measure creates the condition for encoding a nucleic acid analyte, such as an mRNA, e.g. such an mRNA coding for a particular protein.

In another embodiment the analyte to be encoded or detected is a nucleic acid, preferably DNA or RNA, further preferably mRNA, and/or, alternatively the analyte to be decoded is a peptide or a protein.

By this measure the invention is adapted to the detection of such kinds of analytes which are of upmost importance in the clinical routine or the focus of biological questions.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention. The features mentioned in the specific embodiments are general features of the invention which are not only applicable in the specific embodiment but also in an isolated manner in the context of any embodiment of the invention.

The invention is now described and explained in further detail by referring to the following non-limiting examples and figures.

Fig. 1: Embodiment where the analyte is a nucleic acid and the probe set comprises oligonucleotides specifically binding to the analyte. The probes comprise a unique identifier sequence allowing hybridization of decoding oligonucleotides;

Fig. 2: Embodiment where the analyte is a protein and the probe set comprises proteins (here: antibodies) specifically binding to the analyte. The probes comprise a unique identifier sequence allowing hybridization of decoding oligonucleotides;

Fig. 3: Flowchart of the method according to the invention;

Fig. 4: Alternative options for the application of decoding and signal oligonucleotides.

Fig. 5: Example for signal encoding of three different nucleic acid sequences by two different signal types and three detection rounds; in this example, the encoding scheme includes error detection;

Fig. 6: Number of generated code words (logarithmic scale) against number of detection cycles;

Fig. 7: Calculated total efficiency of a 5-round encoding scheme based on single step efficiencies;

Fig. 8: Comparison of relative transcript abundances between different experiments;

Fig. 9: Correlation of relative transcript abundances between different experiments;

Fig. 10 Comparison of intercellular distribution of signals;

Fig. 11: Comparison of intracellular distribution of signals;

Fig. 12: Distribution pattern of different cell cycle dependent transcripts.

EXAMPLES

1. Introduction

The method disclosed herein is used for specific detection of many different analytes in parallel. The technology allows distinguishing a higher number of analytes than different signals are available. The process preferably includes at least two consecutive rounds of specific binding, signal detection and selective denaturation (if a next round is required), eventually producing a signal code. To decouple the dependency between the analyte specific binding and the oligonucleotides providing the detectable signal, a so called "decoding" oligonucleotide is introduced. The decoding oligonucleotide transcribes the information of the analyte specific probe set to the signal oligonucleotides.

In a first application variant, the analyte or target is nucleic acid, e.g. DNA or RNA, and the probe set comprises oligonucleotides that are partially or completely complementary to the whole sequence or a subsequence of the nucleic acid sequence to be detected (Figure 1). The nucleic acid sequence specific oligonucleotide probe sets comprising analyte-specific probes (1) including a binding element (S) that specifically hybridizes to the target nucleic acid sequence to be detected, and an identifier element (T) comprising a nucleotide sequence which is unique to said set of analyte-specific probes (unique identifier sequence).

In a second application variant, the analyte or target is a protein and the probe set comprises one or more proteins, e.g. antibodies (Figure 2). The protein specific probe set comprising analyte-specific probes (1) including a binding element (T) such as the (hyper-)variable region of an antibody, that specifically interacts with the target protein to be detected, and the identifier element (T).

In a third application variant, at least one analyte is a nucleic acid and at least a second analyte is a protein and at least the first probe set binds to the nucleic acid sequence and at least the second probe set binds specifically to the protein analyte. Other combinations are possible as well.

2. General method according to the invention

In order to elucidate the workflow in more depth, the following workflow is restricted on the first application variant. A well-trained person can easily adapt the exemplary workflow to other applications. The method steps are depicted in the flowchart of Figure 3.

Step 1: Applying the analyte- or target-specific probe set. The target nucleic acid sequence is incubated with a probe set consisting of oligonucleotides with sequences complementary to the target nucleic acid. In this example, a probe set of 5 different probes is shown, each comprising a sequence element complementary to an individual subsequence of the target nucleic acid sequence (S1 to S5). In this example, the regions do not overlap. Each of the oligonucleotides targeting the same nucleic acid sequence comprises the identifier element or unique identifier sequence (T), respectively.

Step 2: Hybridization of the probe set. The probe set is hybridized to the target nucleic acid sequence under conditions allowing a specific hybridization. After the incubation, the probes are hybridized to their corresponding target sequences and provide the identifier element (T) for the next steps.

Step 3: Eliminating non-bound probes. After hybridization, the unbound oligonucleotides are eliminated, e.g. by washing steps.

Step 4: Applying the decoding oligonucleotides. The decoding oligonucleotides consisting of at least two sequence elements (t) and (c) are applied. While sequence element (t) is complementary to the unique identifier sequence (T), the sequence element (c) provides a region for the subsequent hybridization of signal oligonucleotides (translator element).

Step 5: Hybridization of decoding oligonucleotides. The decoding oligonucleotides are hybridized with the unique identifier sequences of the probes (T) via their complementary first sequence elements (t). After incubation, the decoding oligonucleotides provide the translator sequence element (c) for a subsequent hybridization step.

Step 6: Eliminating the excess of decoding oligonucleotides. After hybridization, the unbound decoding oligonucleotides are eliminated, e.g. by washing steps.

Step 7: Applying the signal oligonucleotide. The signal oligonucleotides are applied. The signal oligonucleotides comprise at least one second connector element (C) that is essentially complementary to the translator sequence element (c) and at least one signal element that provides a detectable signal (F).

Step 8: Hybridization of the signal oligonucleotides. The signal oligonucleotides are hybridized via the complementary sequence connector element (C) to the translator element (c) of decoding oligonucleotide. After incubation, the signal oligonucleotides are hybridized to their corresponding decoding oligonucleotides and provide a signal (F) that can be detected.

Step 9: Eliminating the excess of signal oligonucleotides. After hybridization, the unbound signal oligonucleotides are eliminated, e.g. by washing steps.

Step 10: Signal detection. The signals provided by the signal oligonucleotides are detected.

The following steps (steps 11 and 12) are unnecessary for the last detection round.

Step 11: Selective denaturation. The hybridization between the unique identifier sequence (T) and the first sequence element (t) of the decoding oligonucleotides is dissolved. The destabilization can be achieved via different mechanisms well known to the trained person like for example: increased temperature, denaturing agents, etc. The target- or analyte-specific probes are not affected by this step.

Step 12: Eliminating the denatured decoding oligonucleotides. The denatured decoding oligonucleotides and signal oligonucleotides are eliminated (e.g. by washing steps) leaving the specific probe sets with free unique identifier sequences, reusable in a next round of hybridization and detection (steps 4 to 10). This detection cycle (steps 4 to 12) is repeated ’n’ times until the planed encoding scheme is completed.

Note that in every round of detection, the type of signal provided by a certain unique identifier is controlled by the use of a certain decoding oligonucleotide. As a result, the sequence of decoding oligonucleotides applied in the detection cycles transcribes the binding specificity of the probe set into a unique signal sequence.

3. <u>Alternative options for the application of decoding- and signal oligonucleotides</u>

The steps of decoding oligonucleotide hybridization (steps 4 to 6) and signal oligonucleotide hybridization (steps 7 to 9) can also be combined in two alternative ways as shown in Figure 4.

Opt. 1: Simultaneous hybridization. Instead of the steps 4 to 9 of Figure 3, specific hybridization of decoding oligonucleotides and signal oligonucleotides can also be done simultaneously leading to the same result as shown in step 9 of Figure 3, after eliminating the excess decoding- and signal oligonucleotides.

Opt. 2: Preincubation. Additionally to option 1 of Figure 3, decoding- and signal oligonucleotides can be preincubated in a separate reaction before being applied to the target nucleic acid with the already bound specific probe set.

4. <u>Example for signal encoding of three different nucleic acid sequences by two different signal types and three detection rounds</u>

Figure 3 shows the general concept of generation and detection of specific signals mediated by decoding oligonucleotides. It does not show the general concept of encoding that can be achieved by this procedure. To illustrate the use of the process shown in Figure 3 for the generation of an encoding scheme, Figure 5 shows a general example for a multiple round encoding experiment with three different nucleic acid sequences. In this example, the encoding scheme includes error detection.

Step 1: Target nucleic acids. In this example three different target nucleic acids (A), (B) and (C) have to be detected and differentiated by using only two different types of signal. Before starting the experiment, a certain encoding scheme is set. In this example, the three different nucleic acid sequences are encoded by three rounds of detection with two different signals (1) and (2) and a resulting hamming distance of 2 to allow for error detection. The planed code words are:

sequence A: (1) - (2) - (2);

sequence B: (1) - (1) - (1);

sequence C: (2) - (1) - (2).

Step 2: Hybridization of the probe sets. For each target nucleic acid, an own probe set is applied, specifically hybridizing to the corresponding nucleic acid sequence of interest. Each probe set provides a unique identifier sequence (T1), (T2) or (T3). This way each different target nucleic acid is uniquely labeled. In this example sequence (T) is labeled with (T1), sequence (B) with (T2) and sequence (C) with (T3). The illustration summarizes steps 1 to 3 of Figure 3.

Step 3: Hybridization of the decoding oligonucleotides. For each unique identifier present, a certain decoding oligonucleotide is applied specifically hybridizing to the corresponding unique identifier sequence by its first sequence element (here (t1) to (T1), (t2) to (T2) and (t3) to (T3)). Each of the decoding oligonucleotides provides a translator element that defines the signal that will be generated after hybridization of signal oligonucleotides. Here nucleic acid sequences (A) and (B) are labeled with the translator element (c1) and sequence (C) is labeled with (c2). The illustration summarizes steps 4 to 6 of Figure 3.

Step 4: Hybridization of signal oligonucleotides. For each type of translator element, a signal oligonucleotide with a certain signal (2), differentiable from signals of other signal oligonucleotides, is applied. This signal oligonucleotide can specifically hybridize to the corresponding translator element. The illustration summarizes steps 7 to 9 of Figure 3.

Step 5: Signal detection for the encoding scheme. The different signals are detected. Note that in this example the nucleic acid sequence (C) can be distinguished from the other sequences by the unique signal (2) it provides, while sequences (A) and (B) provide the same kind of signal (1) and cannot be distinguished after the first cycle of detection. This is due to the fact, that the number of different nucleic acid sequences to be detected exceeds the number of different signals available. The illustration corresponds to step 10 of Figure 3.

Step 6: Selective denaturation. The decoding (and signal) oligonucleotides of all nucleic acid sequences to be detected are selectively denatured and eliminated as described in steps 11 and 12 of Figure 3. Afterwards the unique identifier sequences of the different probe sets can be used for the next round of hybridization and detection.

Step 7: Second round of detection. A next round of hybridization and detection is done as described in steps 3 to 5. Note that in this new round the mix of different decoding oligonucleotides is changed. For example, decoding oligonucleotide of nucleic acid sequence (A) used in the first round comprised of sequence elements (t1) and (c1) while the new decoding oligonucleotide comprises of the sequence elements (t1) and (c2). Note that now all three sequences can clearly be distinguished due to the unique combination of first and second round signals.

Step 8: Third round of detection. Again, a new combination of decoding oligonucleotides is used leading to new signal combinations. After signal detection, the resulting code words for the three different nucleic acid sequences are not only unique and therefore distinguishable but comprise a hamming distance of 2 to other code words. Due to the hamming distance, an error in the detection of the signals (signal exchange) would not result in a valid code word and therefore could be detected. By this way three different nucleic acids can be distinguished in three detection rounds with two different signals, allowing error detection.

## 5. Advantages over prior art technologies

### *Coding strategy*

Compared to state-of-the-art methods, one particular advantage of the method according to the invention is the use of decoding oligonucleotides breaking the dependencies between the target specific probes and the signal oligonucleotides.

Without decoupling target specific probes and signal generation, two different signals can only be generated for a certain target if using two different molecular tags. Each of these molecular tags can only be used once. Multiple readouts of the same molecular tag do not increase the information about the target. In order to create an encoding scheme, a change of the target specific probe set after each round is required (SeqFISH) or multiple molecular tags must be present on the same probe set (like merFISH, intronSeqFISH).

Following the method according to the invention, different signals are achieved by using different decoding oligonucleotides reusing the same unique identifier (molecular tag) and a small number of different, mostly cost-intensive signal oligonucleotides. This leads to several advantages in contrast to the other methods.

(1) The encoding scheme is not defined by the target specific probe set as it is the case for all other methods of prior art. Here the encoding scheme is transcribed by the decoding oligonucleotides. This leads to a much higher flexibility concerning the number of rounds and the freedom in signal choice for the codewords. Looking on the methods of prior art (e.g. merFISH or intronSeqFISH), the encoding scheme (number, type and sequence of detectable signals) for all target sequences is predefined by the presence of the different tag sequences on the specific probe sets (4 of 16 different tags per probe set in the case of merFISH and 5 of 60 different tags in the case of intron FISH). In order to produce a sufficient number of different tags per probe set, the methods use rather complex oligonucleotide designs with several tags present on one target specific oligonucleotide. In order to change the encoding scheme for a certain target nucleic acid, the specific probe set has to be replaced. The method according to the invention describes the use of a single unique tag sequence (unique identifier) per analyte, because it can be reused in every detection round to produce a new information. The encoding scheme is defined by the order of decoding oligonucleotides that are used in the detection rounds. Therefore, the encoding scheme is not predefined by the specific probes (or the unique tag sequence) but can be adjusted to different needs, even during the experiment. This is achieved by simply changing the decoding oligonucleotides used in the detection rounds or adding additional detection rounds.

(2) The number of different signal oligonucleotides must match the number of different tag sequences with methods of prior art (16 in the case of merFISH and 60 in the case of intronSeqFISH). Using the method according to the invention, the number of different signal oligonucleotides matches the number of different signals used. Due to this, the number of signal oligonucleotides stays constant for the method described here and never exceeds the number of different signals but increases with the complexity of the encoding scheme in the methods of prior art (more detection rounds more different signal oligonucleotides needed). As a result, the method described here leads to a much lower complexity (unintended interactions of signal oligonucleotides with environment or with each other) and dramatically reduces the cost of the assay since the major cost factor are the signal oligonucleotides.

(3) In the methods of prior art, the number of different signals generated by a target specific probe set is restricted by the number of different tag sequences the probe set can provide. Since each additional tag sequence increases the total size of the target specific probe, there is a limitation to the number of different tags a single probe can provide. This limitation is given by the size dependent increase of several problems (unintended inter- and intramolecular interactions, costs, diffusion rate, stability, errors during synthesis etc.). Additionally, there is a limitation of the total number of target specific probes that can be applied to a certain analyte. In case of nucleic acids, this limitation is given by the length of the target sequence and the proportion of suitable binding sites. These factors lead to severe limitations in the number of different signals a probe set can provide (4 signals in the case of merFISH and 5 signals in the case of intronSeqFISH). This limitation substantially affects the number of different code words that can be produced with a certain number of detection rounds. In the approach of the invention only one tag is needed and can be freely reused in every detection round. This leads to a low oligonucleotide complexity/length and at the same time to the maximum encoding efficiency possible (number of $\text{colors}^{\text{number of rounds}}$). The vast differences of coding capacity of our method compared to the other methods is shown in Figures 1 and 5. Due to this in approach of the invention a much lower number of detection rounds is needed to produce the same amount of information. A lower number of detection rounds is connected to lower cost, lower experimental time, lower complexity, higher stability and success rate, lower amount of data to be collected and analyzed and a higher accuracy of the results.

*Coding capacity*

All three methods compared in the Table 1 below use specific probe sets that are not denatured between different rounds of detection. For intronSeqFISH there are four detection rounds needed to produce the pseudo colors of one coding round, therefore data is only given for rounds 4, 8,12,16 and 20. The merFISH-method uses a constant number of 4 signals, therefore the data starts with the smallest number of rounds possible. After 8 detection rounds our method exceeds the maximum coding capacity reached with 20 rounds of merFISH (depicted with one asterisk) and after 12 rounds of detection the maximum coding capacity of intron FISH is exceeded (depicted with two asterisks). For the method according to the invention usage of 3 different signals is assumed (as is with intronSeqFISH).

Table 1: Comparison of coding capacity

| NUMBER OF ROUNDS: | CODING CAPACITY | | |
|---|---|---|---|
| | invention | intron FISH | merFISH |
| 1 | 3 | - | - |
| 2 | 9 | - | - |
| 3 | 27 | - | - |
| 4 | 81 | 12 | 1 |
| 5 | 243 | - | 5 |
| 6 | 729 | - | 15 |
| 7 | 2187 | - | 35 |
| 8* | 6561 | 144 | 70 |
| 9 | 19683 | - | 126 |
| 10 | 59049 | - | 210 |
| 11 | 177147 | - | 330 |
| 12** | 531441 | 1728 | 495 |

(continued)

| NUMBER OF ROUNDS: | CODING CAPACITY | | |
|---|---|---|---|
| | **invention** | **intron FISH** | **merFISH** |
| 13 | 1594323 | - | 715 |
| 14 | 4782969 | - | 1001 |
| 15 | 14348907 | - | 1365 |
| 16 | 43046721 | 20736 | 1820 |
| 17 | 129140163 | - | 2380 |
| 18 | 387420489 | - | 3060 |
| 19 | 1162261467 | - | 3876 |
| 20 | 3486784401 | 248832 | 4845 |

As shown in Figure 6 the number of codewords for merFISH does not exponentially increase with the number of detection cycles but gets less effective with each added round. In contrast, the number of codewords for intronSeqFISH in the method according to the invention increases exponentially. The slope of the curve for the proposed method is much higher than that of intron FISH, leading to more than 10,000 times more code words usable after 20 rounds of detection.

Note that this maximum efficiency of coding capacity is also reached in case of seqFISH, where specific probes are denatured after every detection round and a new probe set is specifically hybridized to the target sequence for each detection round. However, this method has major downsides to technologies using only one specific hybridization for their encoding scheme (all other methods):

(1) For the efficient denaturation of the specific probes, rather crude conditions must be used (high temperatures, high concentrations of denaturing agent, long incubation times) leading to much higher probability for the loss or the damage of the analyte.

(2) For each round of detection an own probe set has to be used for every target nucleic acid sequence. Therefore, the number of specific probes needed for the experiment scales with the number of different signals needed for the encoding scheme. This dramatically increases the complexity and the cost of the assay.

(3) Because the hybridization efficiency of every target nucleic acid molecule is subject to some probabilistic effects, the fluctuations of signal intensity between the different detection rounds is much higher than in methods using only one specific hybridization event, reducing the proportion of complete codes.

(4) The time needed for the specific hybridization is much longer than for the hybridization of signal oligonucleotides or decoding oligonucleotides (as can be seen in the method parts of the intronSeqFISH, merFISH and seqFISH publications), which dramatically increases the time needed to complete an experiment.

Due to these reasons all other methods use a single specific hybridization event and accept the major downside of lower code complexity and therefore the need of more detection rounds and a higher oligonucleotide design complexity.

The method according to the invention combines the advantages of seqFISH (mainly complete freedom concerning the encoding scheme) with all advantages of methods using only one specific hybridization event while eliminating the major problems of such methods.

Note that the high numbers of code words produced after 20 rounds can also be used to introduce higher hamming distances (differences) between different codewords, allowing error detection of 1, 2 or even more errors and even error corrections. Therefore, even very high coding capacities are still practically relevant.

6. <u>Selective denaturation, oligonucleotide assembly and reuse of unique identifiers are surprisingly efficient</u>

A key factor of the method according to the invention is the consecutive process of decoding oligonucleotide binding, signal oligonucleotide binding, signal detection and selective denaturation. In order to generate an encoding scheme, this process has to be repeated several times (depending on the length of the code word). Because the same unique identifier is reused in every detection cycle, all events from the first to the last detection cycle are depending on each other. Additionally, the selective denaturation depends on two different events: While the decoding oligonucleotide has to be dissolved from the unique identifier with highest efficiency, specific probes have to stay hybridized with highest efficiency.

Due to this the efficiency E of the whole encoding process can be described by the following equation:

$$E = B_{sp} \text{ x } (B_{de} \text{ x } B_{si} \text{ x } E_{de} \text{ x } S_{sp})^n$$

E = total efficiency
$B_{sp}$= binding of specific probes
$B_{de}$= binding of decoding oligonucleotides
$B_{si}$= binding of signal oligonucleotides
$E_{de}$= elimination of decoding oligonucleotides
$S_{sp}$= stability of specific probes during elimination process
n = number of detection cycles

Based on this equation the efficiency of each single step can be estimated for a given total efficiency of the method. The calculation is hereby based on the assumption, that each process has the same efficiency. The total efficiency describes the portion of successfully decodable signals of the total signals present.

The total efficiency of the method is dependent on the efficiency of each single step of the different factors described by the equation. Under the assumption of an equally distributed efficiency the total efficiency can be plotted against the single step efficiency as shown in Figure 7. As can be seen, a practically relevant total efficiency for an encoding scheme with 5 detection cycles can only be achieved with single step efficiencies clearly above 90%. For example, to achieve a total efficiency of 50% an average efficiency within each single step of 97.8% is needed. These calculations are even based on the assumption of a 100% signal detection and analysis efficiency. Due to broad DNA melting curves of oligonucleotides of a variety of sequences, the inventors assumed prior to experiments that the selective denaturation would work less efficient for denaturation of decoding oligonucleotides and that sequence specific binding probes are not stable enough. In contrast to this assumption, we found a surprising effectiveness of all steps and a high stability of sequence specific probes during selective denaturation.

Experimentally, the inventors achieved a total decoding efficiency of about 30% to 65% based on 5 detection cycles. A calculation of the efficiency of each single step (Bsp, Bde, Bsi, Ede, Ssp) by the formula given above revealed an average efficiency of about 94.4% to 98%. These high efficiencies are very surprising and cannot easily be anticipated by a well-trained person in this field.

## 7. Experimental data

### Background

The experiment shows the specific detection of 10 to 50 different mRNAs species in parallel with single molecule resolution. It is based on 5 detection cycles, 3 different fluorescent signals and an encoding scheme without signal gaps and a hamming distance of 2 (error detection). The experiment proofs the enablement and functionality of the method according to the invention.

### Oligonucleotides and their sequences

All oligonucleotide sequences used in the experiment (target specific probes, decoding oligonucleotides, signal oligonucleotides) are listed in the sequence listing of the appendix. The signal oligonucleotide R:ST05*O_Atto594 was ordered from biomers.net GmbH. All other oligonucleotides were ordered from Integrated DNA Technologies. Oligonucleotides were dissolved in water. The stock solutions (100 µM) were stored at -20°C.

### Experimental overview

The 50 different target specific probe sets are divided into 5 groups. The name of the transcript to be detected and the name of the target specific probe set are the same (transcript variant names of www.ensemble.org). The term "new" indicates a revised probe design. All oligonucleotide sequences of the probe sets can be found in the sequence listing. The table lists the unique identifier name of the probe set as well as the names of the decoding oligonucleotides used in the different detection cycles. The resulting code shows the sequence of fluorescent signals generated during the 5 detection cycles (G(reen)=Alexa Fluor 488, O(range)= Atto 594, Y(ellow)= Alexa Fluor 546).

Table 2: Experimental overview

| | | Decoding oligonucleotides in detection cycle: | | | | | |
|---|---|---|---|---|---|---|---|
| **Target transcript** | **unique identifier** | **1** | **2** | **3** | **4** | **5** | **resulting code** |
| **Groupe 1** | | | | | | | |
| DDX5-201 | ST21 | ST21-ST07 | ST21-ST05 | ST21-ST07 | ST21-ST05 | ST21-ST06 | GOGOY |
| RAD17-208 | ST02 | ST02-ST06 | ST02-ST07 | ST02-ST06 | ST02-ST06 | ST02-ST07 | YGYYG |
| SPOCK1-202 | ST03 | ST03-ST06 | ST03-ST06 | ST03-ST07 | ST03-ST05 | ST03-ST05 | YYGOO |
| FBXO32-203 | ST04 | ST04-ST07 | ST04-ST06 | ST04-ST06 | ST04-ST06 | ST04-ST05 | GYYYO |
| THRAP3-203 | ST14 | ST14-ST07 | ST14-ST05 | ST14-ST05 | ST14-ST07 | ST14-ST05 | GOOGO |
| GART-203 | ST11 | ST11-ST06 | ST11-ST07 | ST11-ST06 | ST11-ST05 | ST11-ST05 | YGYOO |
| KAT2A-201 | ST13 | ST13-ST06 | ST13-ST06 | ST13-ST07 | ST13-ST06 | ST13-ST07 | YYGYG |
| HPRT1-201 | ST12 | ST12-ST06 | ST12-ST07 | ST12-ST07 | ST12-ST06 | ST12-ST06 | YGGYY |
| CCNA2-201 | ST22 | ST22-ST05 | ST22-ST07 | ST22-ST06 | ST22-ST06 | ST22-ST05 | OGYYO |
| NKRF-201 | ST23 | ST23-ST05 | ST23-ST06 | ST23-ST07 | ST23-ST06 | ST23-ST05 | OYGYO |
| **Groupe 2** | | | | | | | |
| CCNE1-201-new | NT01 | NT01-ST07 | NT01-ST07 | NT01-ST06 | NT01-ST06 | NT01-ST06 | GGYYY |
| COG5-201 | NT03 | NT03-ST06 | NT03-ST06 | NT03-ST05 | NT03-ST05 | NT03-ST06 | YYOOY |
| FBN1-201 | NT04 | NT04-ST05 | NT04-ST07 | NT04-ST06 | NT04-ST07 | NT04-ST07 | OGYGG |
| DYNC1H1-201 | NT05 | NT05-ST07 | NT05-ST06 | NT05-ST07 | NT05-ST06 | NT05-ST06 | GYGYY |
| CKAP5-202 | NT06 | NT06-ST05 | NT06-ST06 | NT06-ST07 | NT06-ST05 | NT06-ST06 | OYGOY |
| KRAS-202 | NT07 | NT07-ST06 | NT07-ST05 | NT07-ST06 | NT07-ST06 | NT07-ST05 | YOYYO |
| EGFR-207 | NT08 | NT08-ST07 | NT08-ST06 | NT08-ST05 | NT08-ST05 | NT08-ST05 | GYOOO |
| TP53-205 | NT09 | NT09-ST06 | NT09-ST05 | NT09-ST06 | NT09-ST07 | NT09-ST07 | YOYGG |
| NF1-204 | XT01 | XT01-ST06 | XT01-ST06 | XT01-ST07 | XT01-ST07 | XT01-ST06 | YYGGY |

(continued)

| | | | | | | | |
|---|---|---|---|---|---|---|---|
| **Groupe 2** | | | | | | | |
| NF2-204 | XT02 | XT02-ST07 | XT02-ST06 | XT02-ST05 | XT02-ST06 | XT02-ST07 | GYOYG |
| **Groupe 3** | | | | | | | |
| ACO2-201 | XT03 | XT03-ST06 | XT03-ST06 | XT03-ST06 | XT03-ST07 | XT03-ST05 | YYYGO |
| AKT1-211 | XT04 | XT04-ST07 | XT04-ST06 | XT04-ST07 | XT04-ST07 | XT04-ST05 | GYGGO |
| LYPLAL1-202 | XT05 | XT05-ST07 | XT05-ST05 | XT05-ST06 | XT05-ST05 | XT05-ST05 | GOYOO |
| PKD2-201 | XT06 | XT06-ST06 | XT06-ST07 | XT06-ST05 | XT06-ST05 | XT06-ST07 | YGOOG |
| ENG-204 | XT09 | XT09-ST05 | XT09-ST05 | XT09-ST06 | XT09-ST06 | XT09-ST06 | OOYYY |
| FANCE-201 | XT10 | XT10-ST05 | XT10-ST07 | XT10-ST06 | XT10-ST05 | XT10-ST06 | OGYOY |
| MET-201 | XT12 | XT12-ST05 | XT12-ST06 | XT12-ST05 | XT12-ST06 | XT12-ST06 | OYOYY |
| NOTCH2-201 | XT13 | XT13-ST05 | XT13-ST05 | XT13-ST06 | XT13-ST07 | XT13-ST05 | OOYGO |
| SPOP-206 | XT14 | XT14-ST05 | XT14-ST07 | XT14-ST05 | XT14-ST07 | XT14-ST06 | OGOGY |
| ABL1-202 | XT16 | XT16-ST05 | XT16-ST06 | XT16-ST06 | XT16-ST05 | XT16-ST05 | OYYOO |
| **Groupe 4** | | | | | | | |
| ATP11C-202 | XT17 | XT17-ST07 | XT17-ST06 | XT17-ST06 | XT17-ST05 | XT17-ST06 | GYYOY |
| BCR-202 | XT18 | XT18-ST05 | XT18-ST06 | XT18-ST06 | XT18-ST07 | XT18-ST06 | OYYGY |
| CAV1-205 | XT19 | XT19-ST07 | XT19-ST05 | XT19-ST06 | XT19-ST07 | XT19-ST06 | GOYGY |
| CDK2-201 | XT20 | XT20-ST05 | XT20-ST05 | XT20-ST07 | XT20-ST07 | XT20-ST06 | OOGGY |
| DCAF1-202 | XT201 | XT201-ST06 | XT201-ST06 | XT201-ST05 | XT201-ST07 | XT201-ST07 | YYOGG |
| FHOD1-201 | XT202 | XT202-ST05 | XT202-ST07 | XT202-ST07 | XT202-ST05 | XT202-ST07 | OGGOG |
| GMDS-202 | XT203 | XT203-ST07 | XT203-ST05 | XT203-ST07 | XT203-ST06 | XT203-ST05 | GOGYO |
| IFNAR1-201 | XT204 | XT204-ST06 | XT204-ST07 | XT204-ST05 | XT204-ST07 | XT204-ST05 | YGOGO |
| NSMF-203 | XT206 | XT206-ST07 | XT206-ST06 | XT206-ST07 | XT206-ST05 | XT206-ST07 | GYGOG |
| POLA2-201 | XT208 | XT208-ST06 | XT208-ST06 | XT208-ST06 | XT208-ST05 | XT208-ST07 | YYYOG |

(continued)

| Groupe 5 | | | | | | | |
|---|---|---|---|---|---|---|---|
| BRCA1-210new | NT10 | NT10-ST07 | NT10-ST05 | NT10-ST06 | NT10-ST06 | NT10-ST07 | GOYYG |
| JAK1-201new | XT11 | XT11-ST05 | XT11-ST05 | XT11-ST07 | XT11-ST06 | XT11-ST07 | OOGYG |
| STRAP-202 | XT207 | XT207-ST07 | XT207-ST07 | XT207-ST06 | XT207-ST05 | XT207-ST07 | GGYOG |
| SERPINB5-201 | XT209 | XT209-ST07 | XT209-ST05 | XT209-ST05 | XT209-ST05 | XT209-ST07 | GOOOG |
| SETX-201 | XT210 | XT210-ST06 | XT210-ST07 | XT210-ST06 | XT210-ST07 | XT210-ST06 | YGYGY |
| WDFY1-201 | XT212 | XT212-ST05 | XT212-ST07 | XT212-ST05 | XT212-ST06 | XT212-ST07 | OGOYG |
| TACC1-201 | XT213 | XT213-ST05 | XT213-ST07 | XT213-ST07 | XT213-ST07 | XT213-ST05 | OGGGO |
| KIF2A-203 | XT214 | XT214-ST07 | XT214-ST07 | XT214-ST06 | XT214-ST07 | XT214-ST05 | GGYGO |
| CDT1-201 | XT215 | XT215-ST07 | XT215-ST07 | XT215-ST07 | XT215-ST05 | XT215-ST05 | GGGOO |
| CENPE-202 | XT216 | XT216-ST07 | XT216-ST06 | XT216-ST05 | XT216-ST07 | XT216-ST06 | GYOGY |

Variations of the experiment

Some Variations of the experiment have been performed. Experiments 1 to 4 mainly differ in the number of transcripts detected in parallel. The groups listed as target specific probe sets refer to table 6. Experiments 5 to 8 are single round, single target controls for comparison with the decoded signals.

Table 3: Variations of the experiment

| Experiment | Target specific probe sets used | Nr. of detection cycles | Imaging with trolox |
|---|---|---|---|
| 1. 50 transcripts_T+ | Groups 1 to 5 of table 6 | 5 | + |
| 2. 50 transcripts_T- | Groups 1 to 5 of table 6 | 5 | - |
| 3. 30 transcripts_T+ | Groups 2 to 4 of table 6 | 5 | + |
| 4. 10 transcripts_T+ | Group 1 of table 6 | 5 | + |
| 5. DDX5 | DDX5-ST21 | 1 | - |
| 6. RAD17 | RAD17-ST02 | 1 | - |
| 7. SPOCK1 | SPOCK1-ST03 | 1 | - |
| 8. THRAP3 | THRAP3-ST14 | 1 | - |

Experimental details

*A. Seeding and cultivation of cells*

HeLa cells were grown in HeLa cell culture medium to nearly 100% confluency. The HeLa cell culture medium comprises DMEM (Thermo Fisher, Cat.: 31885) with 10% FCS (Biochrom, Cat.: S0415), 1% Penicilliin-Streptomycin (Sigma-Adrich, Cat.: P0781) and 1% MEM Non-Essential Amino Acids Solution (Thermo Fisher, Cat.: 11140035). After aspiration of cell culture medium, cells were trypsinized by incubation with trypsin EDTA solution (Sigma-Aldrich, Cat.: T3924) for 5 min at 37°C after a washing step with PBS (1,424 g/l $Na_2HPO_4$*$2H_2O$, 0,276 g/l, $NaH_2PO_4$*$2H_2O$, 8,19 g/l NaCl in water, pH 7,4). Cells were then seeded on the wells of a μ-Slide 8 Well ibidiTreat (Ibidi, Cat.: 80826). The number of cells per well was adjusted to reach about 50% confluency after adhesion of the cells. Cells were incubated over night with 200 μl HeLa cell culture medium per well.

*B. Fixation of cells*

After aspiration of cell culture medium and two washing steps with 200 μl 37°C warm PBS per well, cells were fixed with 200 μl precooled methanol (-20°C, Roth, Cat.: 0082.1) for 10 min at -20°C.

*C. Counterstaining with Sudan Black*

Methanol was aspirated and 150 μl of 0.2% Sudan Black-solution diluted in 70% ethanol were added to each well. Wells were incubated for 5 min in the dark at room temperature. After incubation cells were washed three times with 400 μl 70% ethanol per well to eliminate the excess of Sudan Black-solution.

*D. Hybridization of analyte/target-specific probes*

Before hybridization, cells were equilibrated with 200 μl sm-wash-buffer. The sm-wash-buffer comprises 30 mM $Na_3$Citrate, 300 mM NaCl, pH7, 10% formamide (Roth, Cat.:P040.1) and 5mM Ribonucleoside Vanadyl Complex (NEB, Cat.: S1402S). For each target-specific probe set 1 μl of a 100 μM oligonucleotide stock solution was added to the mixture. The oligonucleotide stock solution comprises equimolar amounts of all target specific oligonucleotides of the corresponding target specific probe set. The total volume of the mixture was adjusted to 100 μl with water and mixed with 100 μl of a 2x concentrated hybridization buffer solution. The 2x concentrated hybridization buffer comprises 120 mM $Na_3$Citrate, 1200 mM NaCl, pH7, 20% formamide and 20 mM Ribonucleoside Vanadyl Complex. The resulting 200 μl hybridization mixture was added to the corresponding well and incubated at 37°C for 2 h. Afterwards cells were washed three times with 200 μl per well for 10 min with target probe wash buffer at 37°C. The target probe wash buffer comprises 30 mM $Na_3$Citrate, 300 mM NaCl, pH7, 20% formamide and 5 mM Ribonucleoside Vanadyl Complex.

*E. Hybridization of decoding oligonucleotides*

Before hybridization, cells were equilibrated with 200 μl sm-wash-buffer. For each decoding oligonucleotide 1,5 μl of a 5 μM stock solution were added to the mixture. The total volume of the mixture was adjusted to 75 μl with water and mixed with 75 μl of a 2x concentrated hybridization buffer solution. The resulting 150 μl decoding oligonucleotide hybridization mixture was added to the corresponding well and incubated at room temperature for 45 min. Afterwards cells were washed three times with 200 μl per well for 2 min with sm-wash-buffer at room temperature.

*F. Hybridization of signal oligonucleotides*

Before hybridization, cells were equilibrated with 200 μl sm-wash-buffer. The signal oligonucleotide hybridization mixture was the same for all rounds of experiments 1 to 4 and comprised 0,3 μM of each signal oligonucleotide (see table A3) in 1x concentrated hybridization buffer solution. In each round 150 μl of this solution were added per well and incubated at room temperature for 45 min. The procedure was the same for experiments 5 to 8 with the exception that the final concentration of each signal oligonucleotide was 0,15 μM. Afterwards cells were washed three times with 200 μl per well for 2 min with sm-wash-buffer at room temperature.

*G. Fluorescence and white light imaging*

Cells were washed once with 200 μl of imaging buffer per well at room temperature. In experiments without Trolox (see table 7, last column) imaging buffer comprises 30 mM $Na_3$Citrate, 300 mM NaCl, pH7 and 5mM Ribonucleoside Vanadyl Complex. In experiments with Trolox, imaging buffer additionally contains 10 % VectaCell Trolox Antifade Reagent (Vector laboratories, Cat.: CB-1000), resulting in a final Trolox concentration of 10 mM.

A Zeiss Axiovert 200M microscope with a 63x immersion oil objective (Zeiss, apochromat) with numerical aperture of 1.4, a pco.edge 4.2 CMOS camera (PCO AG) and an LED-light source (Zeiss, colibri 7) was used for imaging of the regions. Filter sets and LED-wavelengths were adjusted to the different optima of the fluorophores used. Illumination times per image were 1000 ms for Alexa Fluor 546 and Atto 594 and 400 ms for Alexa Fluor 488.

In each experiment, three regions were randomly chosen for imaging. For each region, a z-stack of 32 images was detected with a z-step size of 350 nm. Additionally, one white light image was taken from the regions. In experiments with more than one detection cycle, the regions of the first detection round were found back and imaged in every subsequent round.

*H. Selective denaturation*

For selective denaturation, every well was incubated with 200 μl of sm-wash-buffer at 42°C for 6 min. This procedure was repeated six times.

Steps (E) to (H) were repeated 5 times in experiments 1 to 4. Step (H) was omitted for the $5^{th}$ detection cycle.

*I. Analysis*

Based on custom ImageJ-plugins a semi-automated analysis of the raw data was performed to distinguish the specific fluorescent signals from the background. The resulting 3D-point clouds of all three fluorescent channels were combined in silico with a custom VBA script. The resulting combined 3D-point clouds of the 5 detection cycles were aligned to each other on the basis of a VBA script. The resulting alignments revealed the code words for each unique signal detected. Successfully decoded signals were used for quantitative and spatial analysis of the experiments based on custom VBA-scripts and ImageJ-plugins.

## Results

*1. Absolute numbers of decoded signals*

The absolute numbers of successfully decoded signals for all transcripts are listed for each region of each experiment in the following Table 4. In summary, the sum of correct codes depicts the total number of decoded signals that were assigned to transcripts detectable in the corresponding experiment, while the sum of incorrect codes it the total number of decoded signals not detectable in the corresponding experiment. The total number of signals comprises successfully decoded as well as unsuccessfully decoded signals.

Table 4: Absolute numbers of decoded signals

| | Experiment 1: region: | | | Experiment 2: region: | | | Experiment 3: region: | | | Experiment 4: region: | | |
|---|---|---|---|---|---|---|---|---|---|---|---|---|
| **transcript name:** | **1** | **2** | **3** | **1** | **2** | **3** | **1** | **2** | **3** | **1** | **2** | **3** |
| **Groupe 1** | | | | | | | | | | | | |
| **DDX5-201** | 1214 | 1136 | 927 | 1144 | 1509 | 1176 | 2964 | 2034 | 2141 | 8 | 2 | 2 |
| **RAD17-208** | 40 | 126 | 50 | 26 | 55 | 62 | 22 | 30 | 33 | 7 | 9 | 10 |
| **SPOCK1-202** | 581 | 655 | 301 | 483 | 875 | 149 | 1349 | 621 | 539 | 2 | 10 | 8 |
| **FBXO32-203** | 153 | 175 | 68 | 113 | 106 | 78 | 301 | 160 | 269 | 5 | 13 | 9 |
| **THRAP3-203** | 1079 | 2180 | 1035 | 810 | 1179 | 1047 | 2318 | 1609 | 1609 | 6 | 8 | 16 |
| **GART-203** | 422 | 397 | 346 | 350 | 333 | 202 | 766 | 658 | 569 | 5 | 3 | 2 |
| **KAT2A-201** | 141 | 310 | 166 | 174 | 307 | 186 | 382 | 315 | 340 | 5 | 1 | 3 |
| **HPRT1-201** | 63 | 79 | 34 | 44 | 116 | 71 | 85 | 88 | 112 | 1 | 0 | 6 |
| **CCNA2-201** | 91 | 248 | 205 | 95 | 134 | 138 | 241 | 151 | 238 | 10 | 20 | 9 |
| **NKRF-201** | 162 | 318 | 153 | 101 | 99 | 135 | 313 | 254 | 209 | 7 | 5 | 12 |
| **Group 2** | | | | | | | | | | | | |
| **CCNE1-201-new** | 75 | 180 | 38 | 57 | 110 | 97 | 0 | 0 | 1 | 122 | 104 | 120 |
| **COG5-201** | 57 | 21 | 38 | 26 | 45 | 23 | 0 | 0 | 1 | 56 | 86 | 60 |
| **FBN1-201** | 202 | 1338 | 499 | 456 | 513 | 571 | 0 | 1 | 3 | 450 | 778 | 895 |
| **DYNC1H1-201** | 554 | 892 | 398 | 664 | 1026 | 666 | 4 | 3 | 7 | 823 | 1148 | 1248 |
| **CKAP5-202** | 43 | 23 | 77 | 51 | 98 | 74 | 1 | 2 | 1 | 94 | 190 | 212 |
| **KRAS-202** | 331 | 417 | 355 | 302 | 333 | 230 | 0 | 0 | 1 | 279 | 637 | 371 |
| **EGFR-207** | 527 | 252 | 372 | 293 | 519 | 322 | 0 | 1 | 0 | 411 | 719 | 818 |
| **TP53-205** | 116 | 324 | 194 | 138 | 198 | 169 | 0 | 1 | 4 | 182 | 280 | 181 |
| **NF1-204** | 381 | 676 | 320 | 347 | 522 | 416 | 3 | 2 | 0 | 507 | 642 | 659 |
| **NF2-204** | 434 | 638 | 361 | 336 | 468 | 401 | 0 | 0 | 2 | 508 | 523 | 636 |
| **Group 3** | | | | | | | | | | | | |
| **ACO2-201** | 456 | 759 | 444 | 333 | 367 | 345 | 0 | 0 | 0 | 556 | 681 | 681 |

(continued)

| | | | | | | | | | | | | |
|---|---|---|---|---|---|---|---|---|---|---|---|---|
| **Group 3** | | | | | | | | | | | | |
| **AKT1-211** | 351 | 710 | 301 | 230 | 437 | 297 | 0 | 0 | 3 | 558 | 614 | 690 |
| **LYPLAL1-202** | 65 | 62 | 51 | 33 | 58 | 51 | 7 | 6 | 8 | 28 | 34 | 42 |
| **PKD2-201** | 72 | 194 | 124 | 95 | 129 | 69 | 1 | 0 | 0 | 122 | 164 | 169 |
| **ENG-204** | 472 | 890 | 458 | 446 | 494 | 558 | 0 | 2 | 0 | 1145 | 799 | 1119 |
| **FANCE-201** | 24 | 82 | 61 | 56 | 67 | 56 | 0 | 0 | 2 | 119 | 131 | 153 |
| **MET-201** | 268 | 744 | 333 | 426 | 275 | 462 | 0 | 0 | 0 | 790 | 662 | 782 |
| **NOTCH2-201** | 344 | 823 | 404 | 172 | 256 | 208 | 4 | 0 | 3 | 402 | 779 | 772 |
| **SPOP-206** | 43 | 377 | 139 | 68 | 117 | 100 | 0 | 0 | 0 | 215 | 289 | 300 |
| **ABL1-202** | 224 | 72 | 218 | 107 | 122 | 153 | 4 | 1 | 1 | 302 | 393 | 480 |
| **Group 4** | | | | | | | | | | | | |
| **ATP11C-202** | 170 | 116 | 121 | 86 | 165 | 128 | 2 | 1 | 1 | 130 | 206 | 234 |
| **BCR-202** | 180 | 401 | 185 | 177 | 149 | 169 | 1 | 0 | 0 | 321 | 372 | 485 |
| **CAV1-205** | 728 | 777 | 644 | 328 | 653 | 567 | 2 | 0 | 5 | 613 | 997 | 852 |
| **CDK2-201** | 306 | 937 | 367 | 297 | 385 | 358 | 4 | 1 | 3 | 568 | 742 | 888 |
| **DCAF1-202** | 119 | 292 | 187 | 59 | 67 | 65 | 0 | 1 | 0 | 108 | 171 | 131 |
| **FHOD1-201** | 60 | 233 | 143 | 132 | 185 | 157 | 1 | 0 | 1 | 194 | 294 | 300 |
| **GMDS-202** | 67 | 124 | 49 | 56 | 80 | 63 | 7 | 2 | 3 | 59 | 73 | 114 |
| **IFNAR1-201** | 81 | 221 | 135 | 99 | 104 | 55 | 1 | 0 | 1 | 159 | 266 | 238 |
| **NSMF-203** | 448 | 583 | 386 | 293 | 453 | 331 | 8 | 4 | 11 | 525 | 608 | 616 |
| **POLA2-201** | 74 | 111 | 62 | 45 | 72 | 67 | 2 | 4 | 2 | 41 | 75 | 57 |
| **Group 5** | | | | | | | | | | | | |
| **BRCA1-210new** | 230 | 704 | 248 | 324 | 439 | 374 | 2 | 1 | 0 | 2 | 3 | 3 |
| **JAK1-201 new** | 157 | 554 | 223 | 185 | 259 | 263 | 0 | 1 | 0 | 5 | 4 | 2 |
| **STRAP-202** | 42 | 75 | 31 | 18 | 52 | 40 | 3 | 1 | 0 | 6 | 4 | 4 |
| **SERPINB5-201** | 324 | 598 | 343 | 286 | 344 | 364 | 6 | 13 | 9 | 0 | 1 | 2 |

(continued)

| | | | | | | | | | | | | |
|---|---|---|---|---|---|---|---|---|---|---|---|---|
| **Group 5** | | | | | | | | | | | | |
| **SETX-201** | 212 | 540 | 254 | 291 | 439 | 336 | 2 | 2 | 2 | 12 | 9 | 5 |
| **WDFY1-201** | 43 | 516 | 218 | 176 | 206 | 147 | 0 | 0 | 0 | 4 | 2 | 8 |
| **TACC1-201** | 69 | 373 | 131 | 80 | 156 | 118 | 1 | 0 | 0 | 21 | 29 | 32 |
| **KIF2A-203** | 442 | 879 | 445 | 298 | 519 | 430 | 5 | 1 | 2 | 6 | 11 | 9 |
| **CDT1-201** | 31 | 27 | 33 | 19 | 61 | 28 | 15 | 8 | 9 | 0 | 8 | 1 |
| **CENPE-202** | 192 | 246 | 215 | 94 | 262 | 225 | 0 | 1 | 0 | 8 | 9 | 9 |
| **Summary** | | | | | | | | | | | | |
| **sum of correct codes:** | 12960 | 23405 | 12890 | 11319 | 15917 | 12797 | 8741 | 5920 | 6059 | 10387 | 13457 | 14303 |
| **sum of incorrect codes:** | 0 | 0 | 0 | 0 | 0 | 0 | 86 | 60 | 86 | 120 | 151 | 152 |
| **total number of signals:** | 42959 | 72157 | 32185 | 32037 | 58793 | 35470 | 13549 | 9182 | 10109 | 26701 | 32966 | 35451 |
| **% successfully decoded:** | 30,2 | 32,4 | 40,0 | 35,3 | 27,1 | 36,1 | 64,5 | 64,5 | 59,9 | 38,9 | 40,8 | 40,3 |

Table 4 shows a very low number of incorrectly decoded signals compared to the number of correctly decoded signals. The absolute values for decoded signals of a certain transcript are very similar between different regions of one experiment. The fraction of the total number of signals that can be successfully decoded is between 27.1 % and 64.5 %. This fraction depends on the number of transcripts and/or the total number of signals present in the respective region/experiment.

*Conclusion*

The method according to the invention produces a low amount of incorrectly assigned code words and can therefore be considered specific. The fraction of successfully decodable signals is very high, even with very high numbers of signals per region and very high numbers of transcripts detected in parallel. The high fraction of assignable signals and the high specificity make the method practically useful.

*2. Comparison of relative transcript abundancies between different experiments*

As shown in Figure 8 for both comparisons (A and B) the overlap of detected transcripts between the experiments is used for the analysis. Each bar represents the mean abundance of all three regions of an experiment. The standard deviation between these regions is also indicated.

*3. Correlation of relative transcript abundancies between different experiments*

As can be seen in Figure 9 the mean relative abundances of transcripts from experiment 1 are correlated to the abundances of the overlapping transcripts of experiment 3, 4 and 2. The correlation coefficient as well as the formula for the linear regression are indicated for each correlation.

Figure 8 shows low standard deviations, indicating low variations of relative abundances between different regions of one experiment. The differences of relative abundances between transcripts from different experiments are also very low. This is the case for the comparison of transcripts from group 1 (Fig. 8A), that were detected in experiments 1, 2 and 3. It is also the case for the comparison of the transcripts from groups 2, 3 and 4 that were overlapping between experiments 1, 2 and 4. The very high correlation of these abundances can also be seen in Figure 9. The abundances of transcripts from experiment 1 correlate very well with the abundances of the other multi round experiments. The correlation factors are between 0.88 and 0.91, while the slope of the linear regressions is between 0.97 and 1.05.

*Conclusion*

The relative abundancies of transcripts correlate very well between different regions of one experiment but also between different experiments. This can be clearly seen by the comparisons of Figures 3 and 4. The main difference between the experiments is the number of different targets and hence the total number of signals detected. Therefore, the number of transcripts detected as well as the number and density of signals does not interfere with the ability of the method to accurately quantify the number of transcripts. The very good correlations further support the specificity and stability of the method, even with very high numbers of signals.

*4. Comparison of intercellular distribution of signals*

In Figure 10 the maximum projections of image stacks are shown. A: region 1 of experiment 7 (single round, single transcript experiment detecting SPOCK1), B: 2D-projection of all selected signals from experiment 1, region 1 assigned to SPOCK1, C: region 1 of experiment 8 (single round, single transcript experiment detecting THRAP3), D: 2D-projection of all selected signals from experiment 1, region 1 assigned to THRAP3.

*5. Comparison of intracellular distribution of signals*

In Figure 11 the maximum projections of image stacks are shown. Magnified sub regions of the corresponding regions are shown. A: region 1 of experiment 8 (single round, single transcript experiment detecting THRAP3), B: 2D-projection of selected signals from experiment 1, region 1 assigned to THRAP3, C: region 1 of experiment 5 (single round, single transcript experiment detecting DDX5), D: 2D-projection of all selected signals from experiment 1, region 1 assigned to DDX5.

Figure 10 shows huge differences of intercellular distributions between different transcripts. SPOCK1 seems to be highly abundant in some cells but nearly absent in other cells (Figure 10 A). THRAP3 shows a more uniform distribution over all cells of a region (Figure 10 C). These spatial distribution patterns can also clearly be observed with the point clouds assigned to the corresponding transcripts from experiment 1 (Figure 10 B and D).

Figure 11 shows huge differences of intracellular distributions between different transcripts. THRAP3 can be mainly observed in the periphery (cytoplasm) of the cells (Figure 11 A), while DDX5 shows a higher abundance in the center (nucleus) of the cells (Figure 11 C). These intracellular distributions can also be observed with the point clouds of experiment 1 assigned to THRAP3 and DDX5 (Figure 11 B and D).

*Conclusion*

Next to the reliability of quantification, the point clouds of multi round experiments also show the same intracellular and intercellular distribution patterns of transcripts. This is clearly proven by the direct comparison of the assigned point clouds with signals from single round experiments detecting only one characteristic mRNA-species.

*6. Distribution pattern of different cell cycle dependent transcripts*

All images of Figure 12 show region 1 of experiment 1. In each image, a point cloud is shown, that is assigned to a certain transcript, A: CCNA2, B: CENPE, C: CCNE1, D: all transcripts. Figure 12 shows the transcripts of three different cell cycle dependent proteins. CENPE (Figure 12 B) is also known as Centromere protein E and accumulates during G2 phase. It is proposed to be responsible for spindle elongation and for chromosome movement. It is not present during interphase. CCNA2 (Figure 12 A) is also known as Cyclin A2. It regulates the cell cycle progression by interacting with CDK1 during transition from G2 to M-phase. Interestingly there is an obvious colocalization of both mRNA-species. They are mainly present in the three central cells of region 1. CCNE1 (Figure 12 C) is also known as Cyclin E1. This cyclin interacts with CDK2 and is responsible for the transition from G1 to S-phase. Figure 12 shows clearly, that the transcripts of this gene are not present in the three central cells, but quite equally distributed over the other cells. It therefore shows an anti-localization to the other two transcripts. The data for the corresponding point-clouds are derived from a point cloud with a very high number of points and a very high point density (Figure 12 D gives an impression).

*Conclusion*

The three decoded point clouds of cell cycle dependent proteins shown in Figure 12, show distribution patterns that can be explained by their corresponding function. These data strongly suggest, that our method reliably produces biological relevant data, even with a low number of signals per cell (Figure 12 C) and with very high signal densities (Figure 12 D).

<u>Sequence listing</u>

In the accompanying sequence listing SEQ ID Nos. 1-1247 refer to nucleotide sequences of exemplary target-specific oligonucleotides. The oligonucleotides listed consist of a target specific binding site (5'-end) a spacer/linker sequence (gtaac or tagac) and the unique identifier sequence, which is the same for all oligonucleotides of one probe set.

In the accompanying sequence listing SEQ ID Nos. 1248-1397 refer to nucleotide sequences of exemplary decoding oligonucleotides.

In the accompanying sequence listing SEQ ID Nos. 1398-1400 refer to the nucleotide sequences of exemplary signal oligonucleotides. For each signal oligonucleotide the corresponding fluorophore is present twice. One fluorophore is covalently linked to the 5'-end and one fluorophore is covalently linked to the 3'-end. SEQ ID No. 1398 comprises at its 5' terminus "5Alex488N", and at its 3' terminus "3AlexF488N". SEQ ID No. 1399 comprises at its 5' terminus "5Alex546", and at its 3' terminus 3Alex546N. SEQ ID No. 1400 comprises at its 5' terminus and at its 3' terminus "Atto594".

## Claims

1. A method of sequential signal-encoding of analytes in a sample, the method comprising the steps:

   (1) providing a set of analyte-specific probes, each analyte-specific probe comprising:

   - a binding element (S) that specifically interacts with one of the analytes to be encoded, and
   - an identifier element (T) comprising a nucleotide sequence which is unique to said set of analyte-specific probes (unique identifier sequence);

   (2) incubating the set of analyte-specific probes with the sample, thereby allowing a specific binding of the analyte-specific probes to the analyte to be encoded;
   (3) removing non-bound probes from the sample;
   (4) providing a set of decoding oligonucleotides, each decoding oligonucleotide comprising:

   - a first connector element (t) comprising a nucleotide sequence which is essentially complementary to at least a section of the unique identifier sequence, and
   - a translator element (c) comprising a nucleotide sequence allowing a specific hybridization of a signal oligonucleotide;

   (5) incubating the set of decoding oligonucleotides with the sample, thereby allowing a specific hybridization of the decoding oligonucleotides to the unique identifier sequence;
   (6) removing non-bound decoding oligonucleotides from the sample;
   (7) providing a set of signal oligonucleotides, each signal oligonucleotide comprising:

   - a second connector element (C) comprising a nucleotide sequence which is essentially complementary to at least a section of the nucleotide sequence of the translator element (c), and
   - a signal element; and

   (8) incubating the set of signal oligonucleotides with the sample, thereby allowing a specific hybridization of the signal oligonucleotides to the translator element (c).

2. The method of claim 1, wherein the sample is a biological sample, preferably comprising biological tissue, further preferably comprising biological cells.

3. The method of claim 2, wherein prior to step (2) the biological tissue and/or biological cells are fixed.

4. The method of any of claims 1-3, wherein within the set of analyte-specific probes the individual analyte-specific probes comprise binding elements (S1, S2, S3, S4, S5) which specifically interact with different sub-structures of one of the analytes to be encoded.

5. The method of any of claims 1-4, further comprising:

   (9) removing non-bound signal oligonucleotides from the sample; and
   (10) detecting the signal, and, optionally,

   further comprising:
   (11) selectively removing the decoding oligonucleotides and signal oligonucleotides from the sample, thereby essentially maintaining the specific binding of the analyte-specific probes to the analyte to be encoded, and, optionally, further comprising:
   (12) repeating steps (4)-(11) at least once [($4_2$)-($11_2$)] to generate an encoding scheme consisting of at least two signals.

**6.** The method of claim 5, wherein said encoding scheme is predetermined and allocated to the analyte to be encoded.

**7.** The method of claim 5 and 6, wherein decoding oligonucleotides are used in repeated steps ($4_2$)-($11_2$) comprising a translator element (c2) which is identical with or differs from the translator element (c1) of the decoding oligonucleotides used in previous steps (4)-(11).

**8.** The method of claims 5-7, wherein signal oligonucleotides are used in repeated steps ($4_2$)-($11_2$) comprising a signal element which is identical with or differs from the signal element of the decoding oligonucleotides used in previous steps (4)-(11).

**9.** The method of any of claims 1-8, wherein the binding element (S) comprise a nucleic acid comprising a nucleotide sequence allowing a specific binding to the analyte to be encoded, preferably a specific hybridization to the analyte to be encoded.

**10.** The method of any of claims 1-9, wherein the binding element (S) comprise an amino acid sequence allowing a specific binding to the analyte to be encoded, preferably the binding element is an antibody.

**11.** The method of any of claims 1-10, wherein the analyte to be encoded is a nucleic acid, preferably DNA or RNA, further preferably mRNA.

**12.** The method of any of claims 1-11, wherein the analyte to be encoded is a peptide or a protein.

**13.** Use of a set of decoding oligonucleotides to sequentially signal-encode analytes in a sample, each decoding oligonucleotide comprising:

- a first connector element (t) comprising a nucleotide sequence which is essentially complementary to at least a section of a nucleotide sequence which is unique to a set of analyte-specific probes (unique identifier sequence), and
- a translator element (c) comprising a nucleotide sequence allowing a specific hybridization of a signal oligonucleotide.

**14.** A kit for sequentially signal-encoding of analytes in a sample, comprising

- a set of analyte-specific probes, each analyte-specific probe comprising:
    - a binding element (S) that specifically interacts with one of the analytes to be encoded, and
    - an identifier element (T) comprising a nucleotide sequence which is unique to said set of analyte-specific probes (unique identifier sequence); and
- a set of decoding oligonucleotides, each decoding oligonucleotide comprising:
    - a first connector element (t) comprising a nucleotide sequence which is essentially complementary to at least a section of the unique identifier sequence, and
    - a translator element (c) comprising a nucleotide sequence allowing a specific hybridization of a signal oligonucleotide.

**15.** The kit of claim 14, further comprising:

- a set of signal oligonucleotides, each signal oligonucleotide comprising:
    - a second connector element (C) comprising a nucleotide sequence which is essentially complementary to at least a section of the nucleotide sequence of the translator element (c), and
    - a signal element.

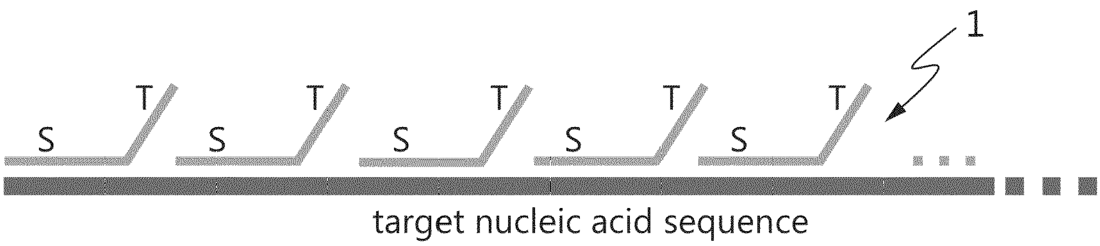
1
T
S
T
S
T
S
T
S
T
S
target nucleic acid sequence

Fig. 1

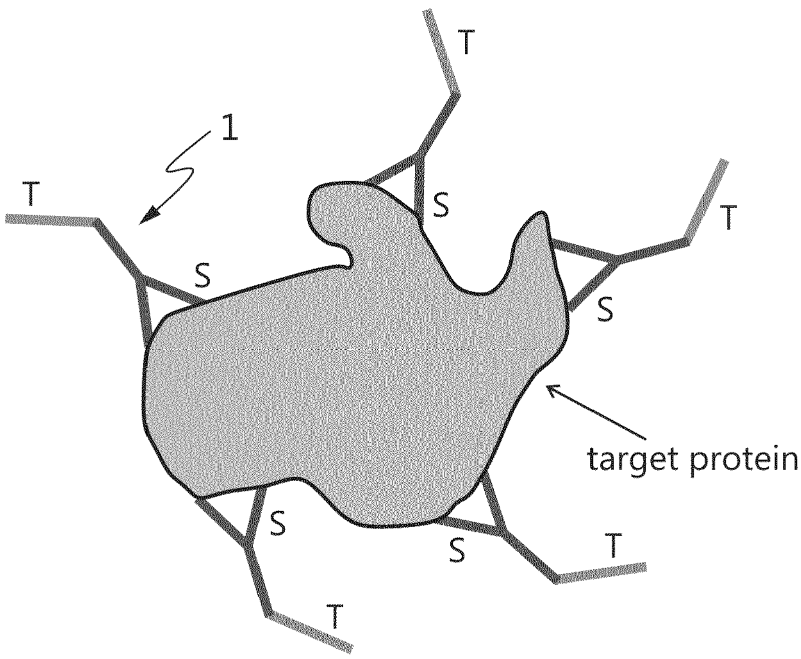
T
1
T
S
S
T
S
target protein
S
S
T
T

Fig. 2

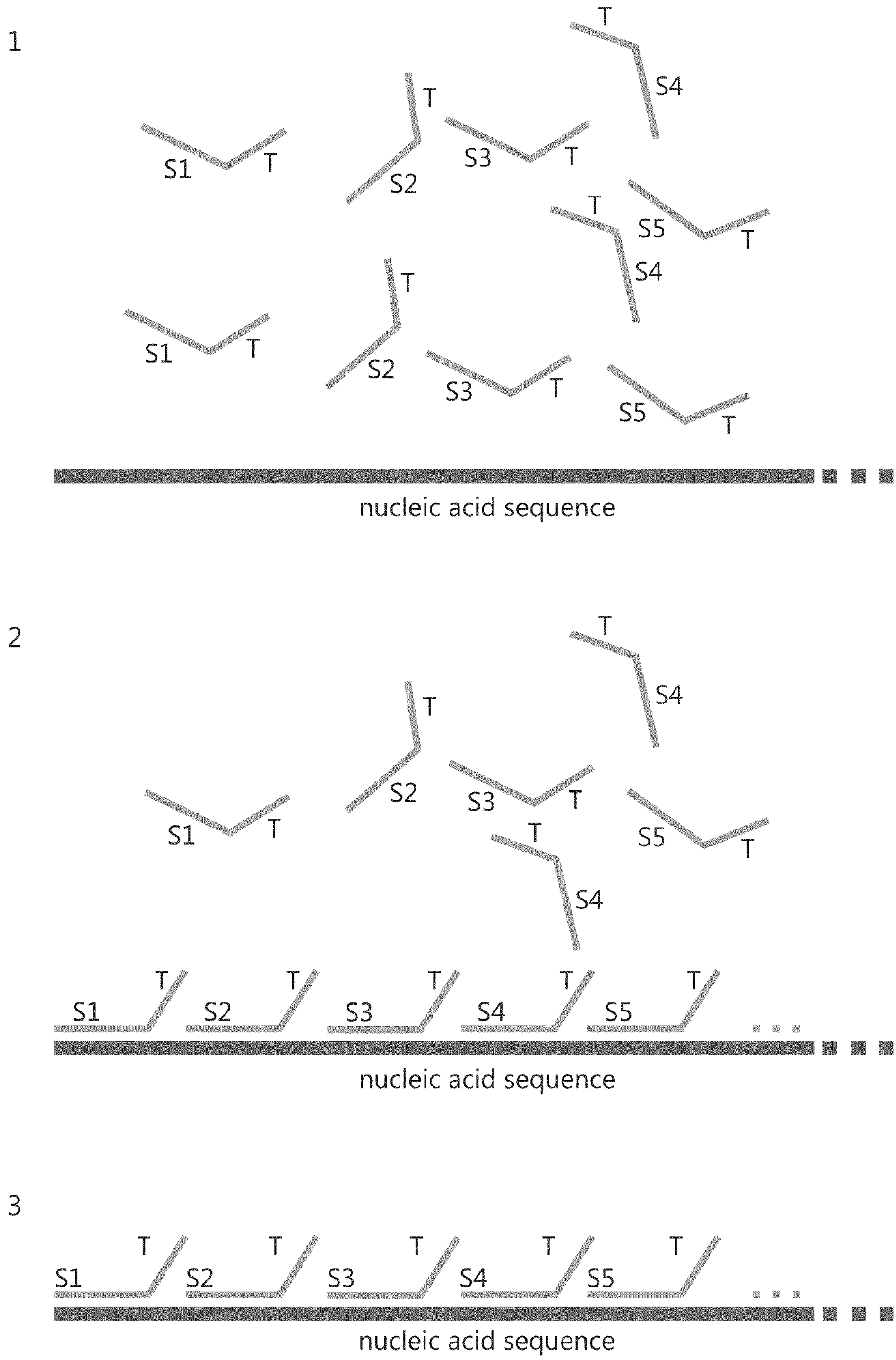
1
S1
T
S2
S3
S4
S5
nucleic acid sequence
2
S1
T
S2
S3
S4
S5
nucleic acid sequence
3
S1
T
S2
S3
S4
S5
nucleic acid sequence

Fig. 3

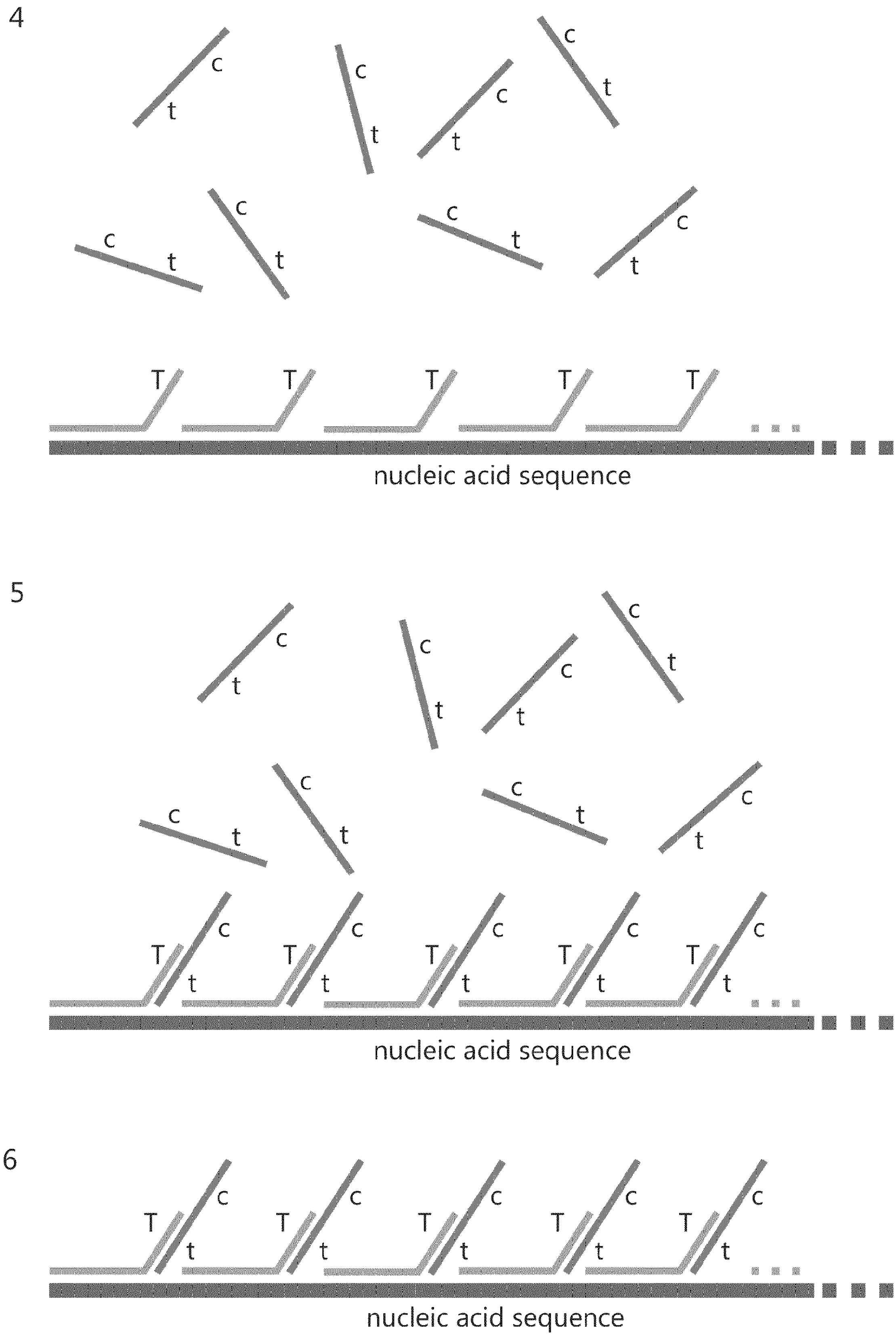
4
c
t
T
nucleic acid sequence
5
c
t
T
nucleic acid sequence
6
c
t
T
nucleic acid sequence

Fig. 3

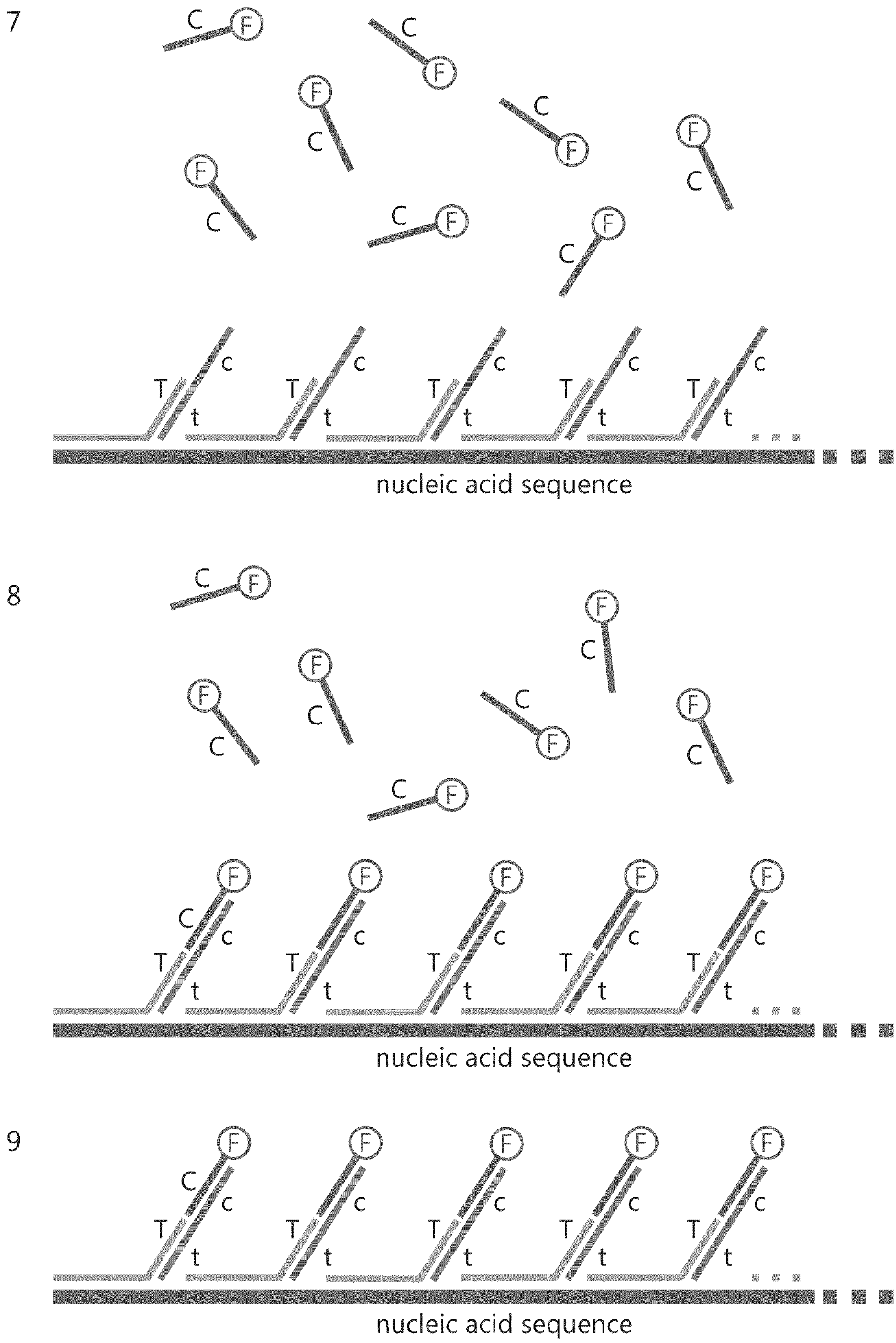
7
C
F
c
T
t
nucleic acid sequence
8
C
F
c
T
t
nucleic acid sequence
9
C
F
c
T
t
nucleic acid sequence

Fig. 3

10

11

12

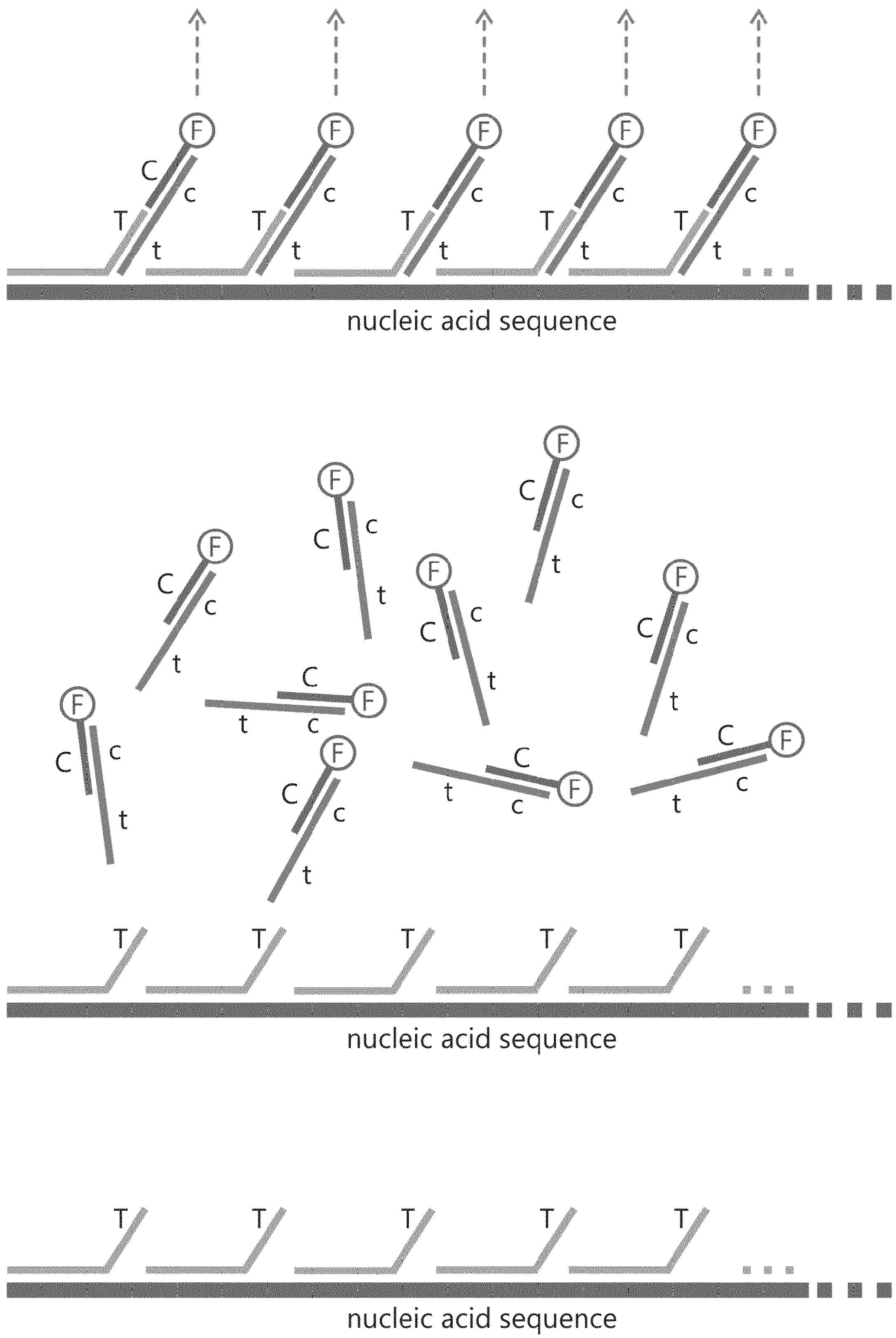
F
C
c
T
t
nucleic acid sequence
nucleic acid sequence
nucleic acid sequence

Fig. 3

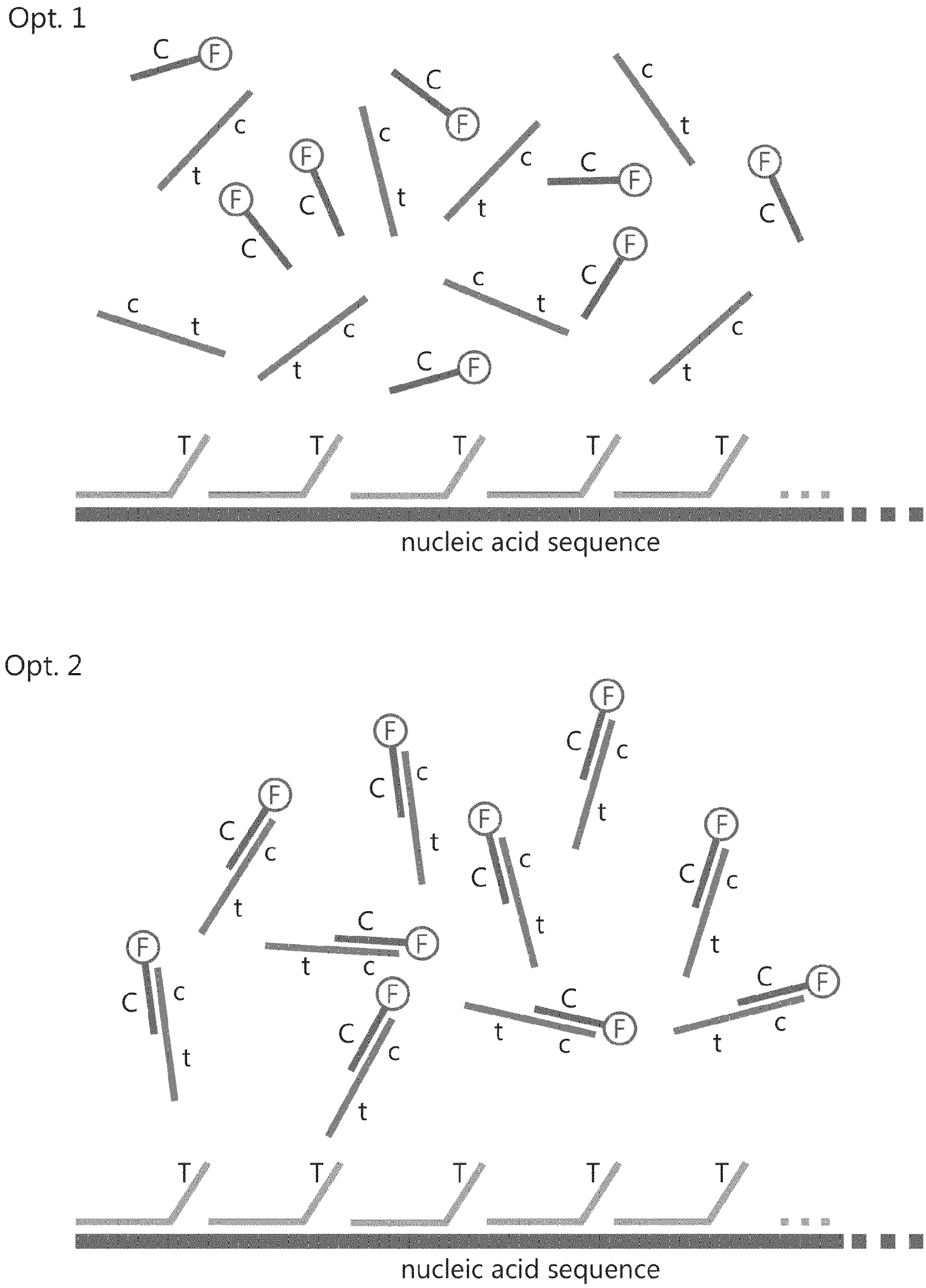
Opt. 1
C
F
c
t
T
nucleic acid sequence
Opt. 2
C
F
c
t
T
nucleic acid sequence

Fig. 4

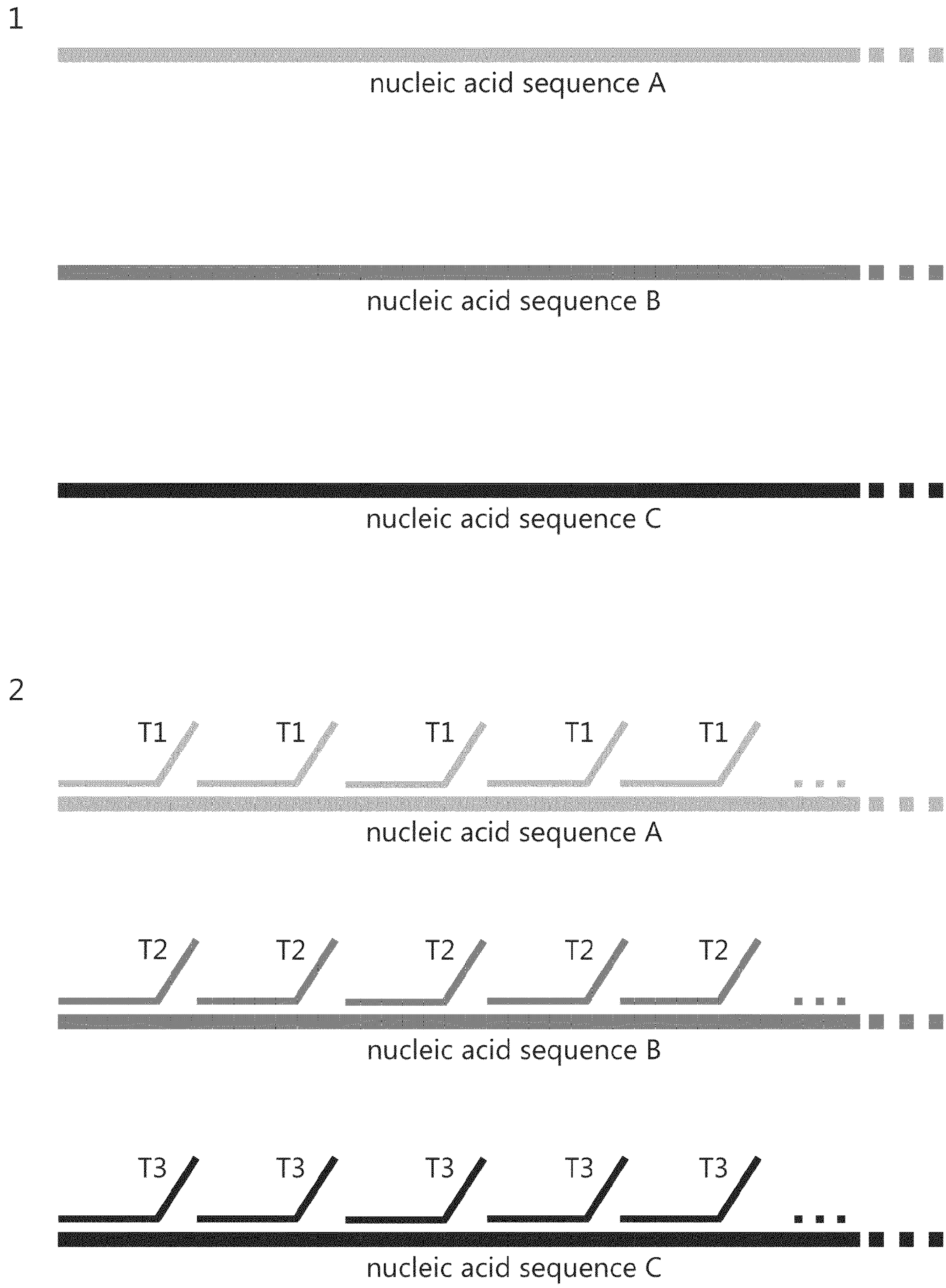
1
nucleic acid sequence A
nucleic acid sequence B
nucleic acid sequence C
2
T1
T1
T1
T1
T1
nucleic acid sequence A
T2
T2
T2
T2
T2
nucleic acid sequence B
T3
T3
T3
T3
T3
nucleic acid sequence C

Fig. 5

3

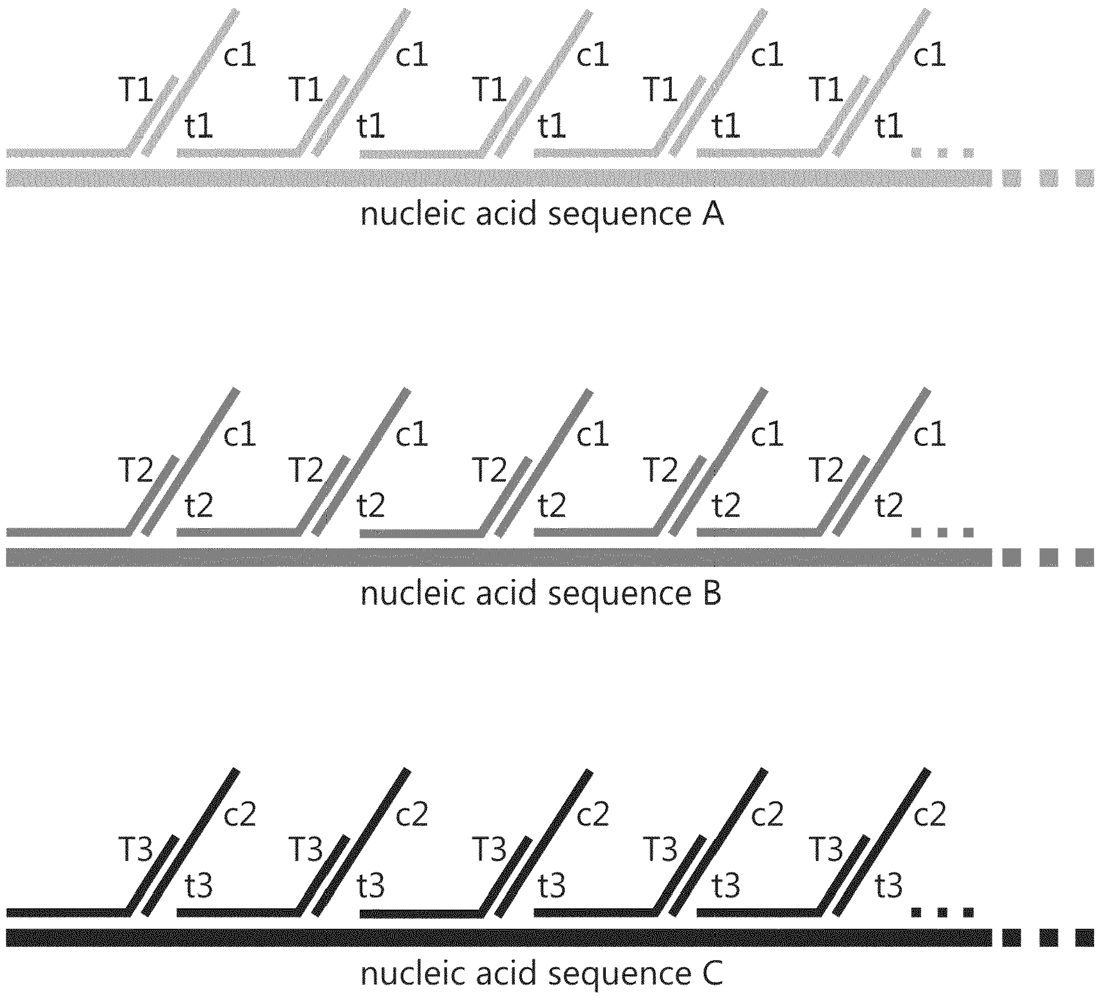
c1
T1
t1
nucleic acid sequence A
c1
T2
t2
nucleic acid sequence B
c2
T3
t3
nucleic acid sequence C

Fig. 5

4

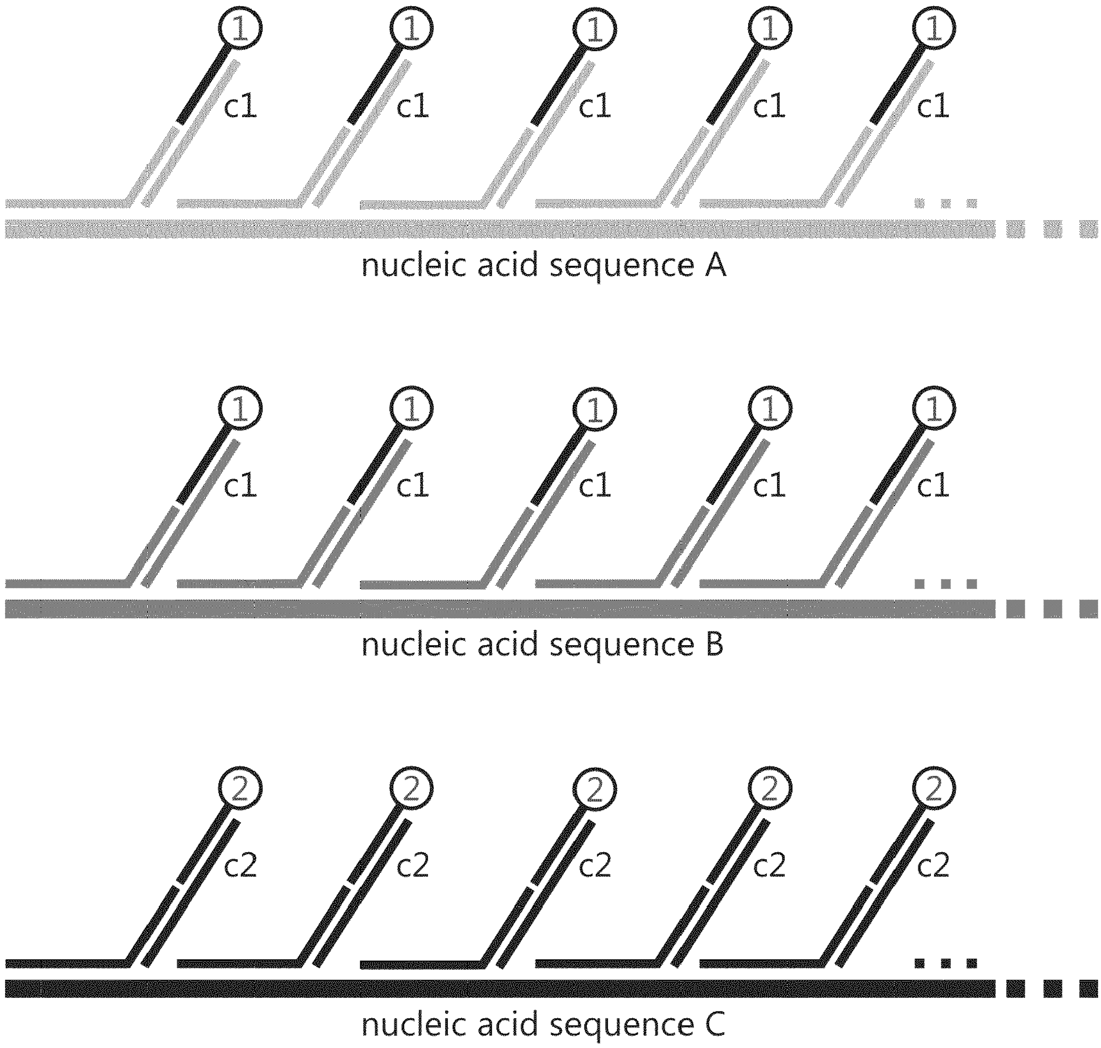
1
c1
nucleic acid sequence A
1
c1
nucleic acid sequence B
2
c2
nucleic acid sequence C

Fig. 5

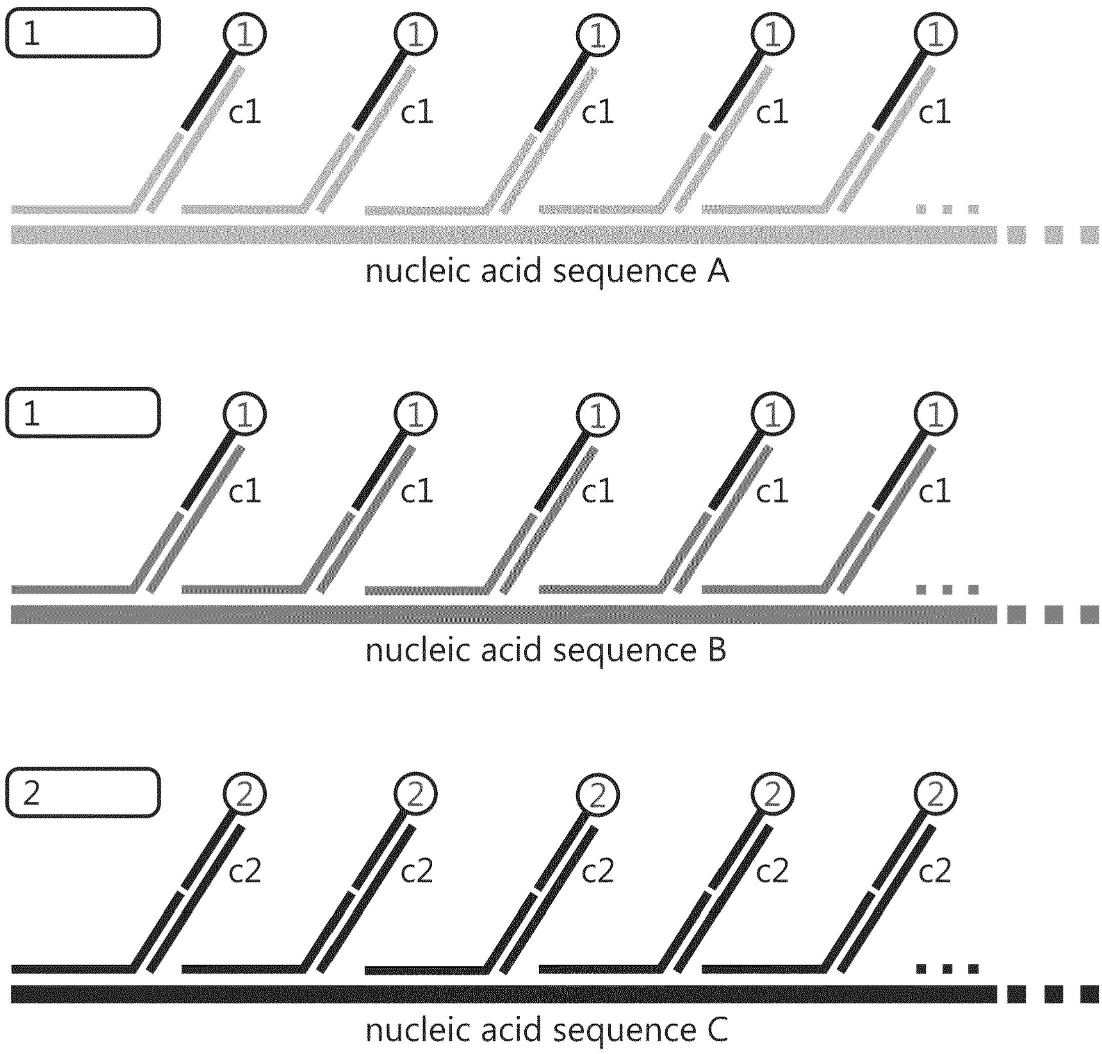
1
1
1
1
1
1
c1
c1
c1
c1
c1
nucleic acid sequence A
1
1
1
1
1
1
c1
c1
c1
c1
c1
nucleic acid sequence B
2
2
2
2
2
2
c2
c2
c2
c2
c2
nucleic acid sequence C

Fig. 5

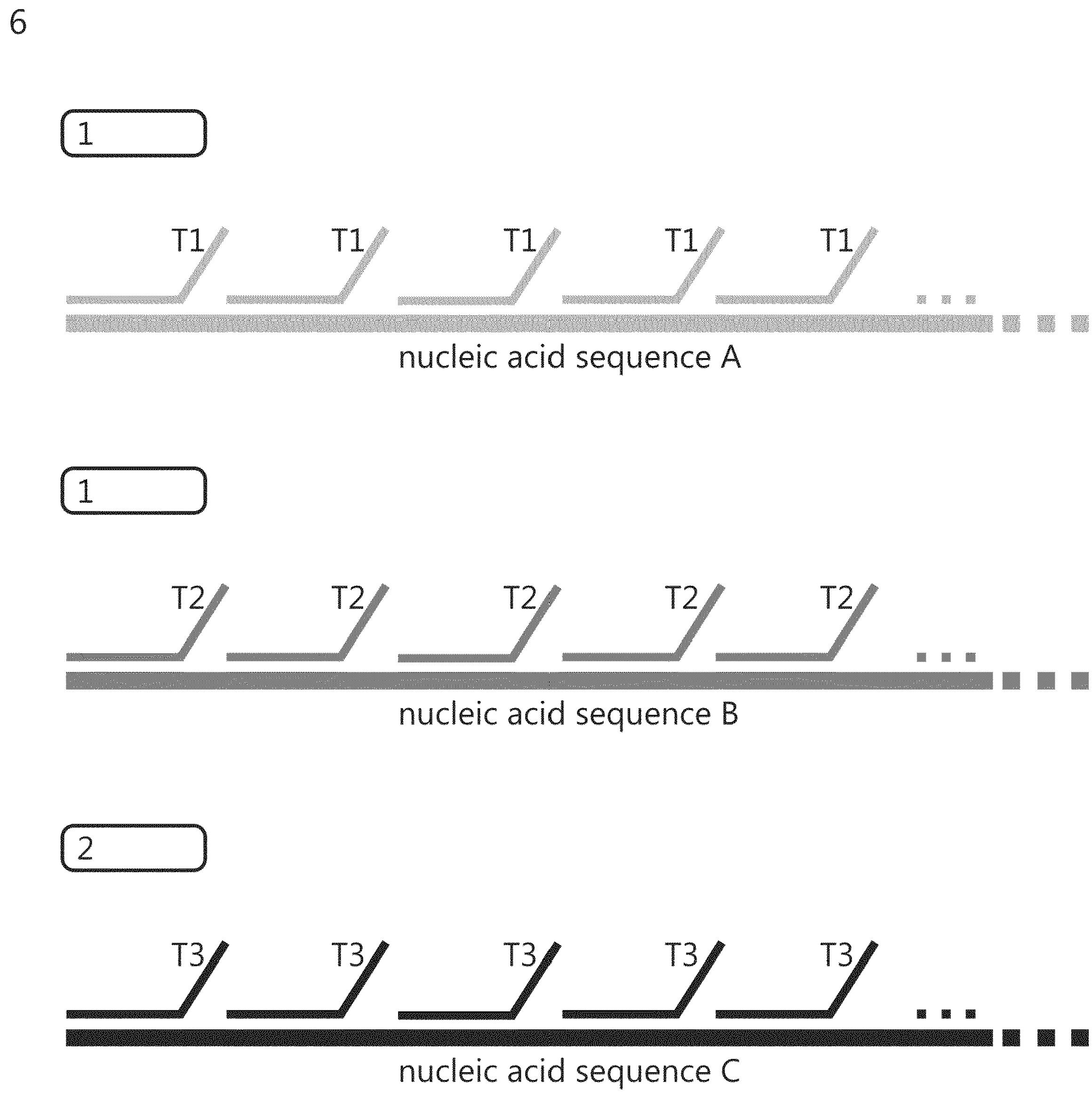
6
1
T1
T1
T1
T1
T1
nucleic acid sequence A
1
T2
T2
T2
T2
T2
nucleic acid sequence B
2
T3
T3
T3
T3
T3
nucleic acid sequence C

Fig. 5

7

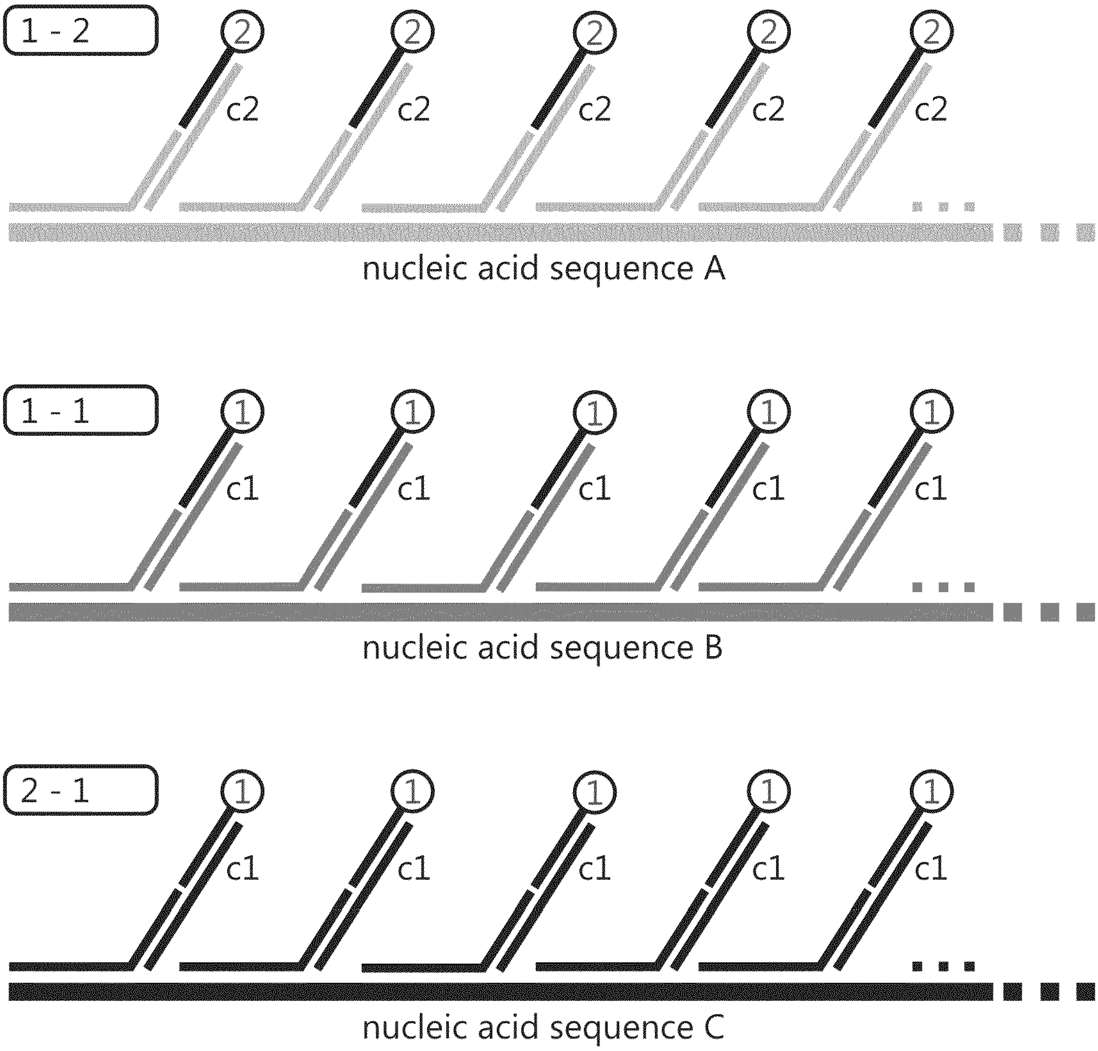
1 - 2
2
c2
nucleic acid sequence A
1 - 1
1
c1
nucleic acid sequence B
2 - 1
1
c1
nucleic acid sequence C

Fig. 5

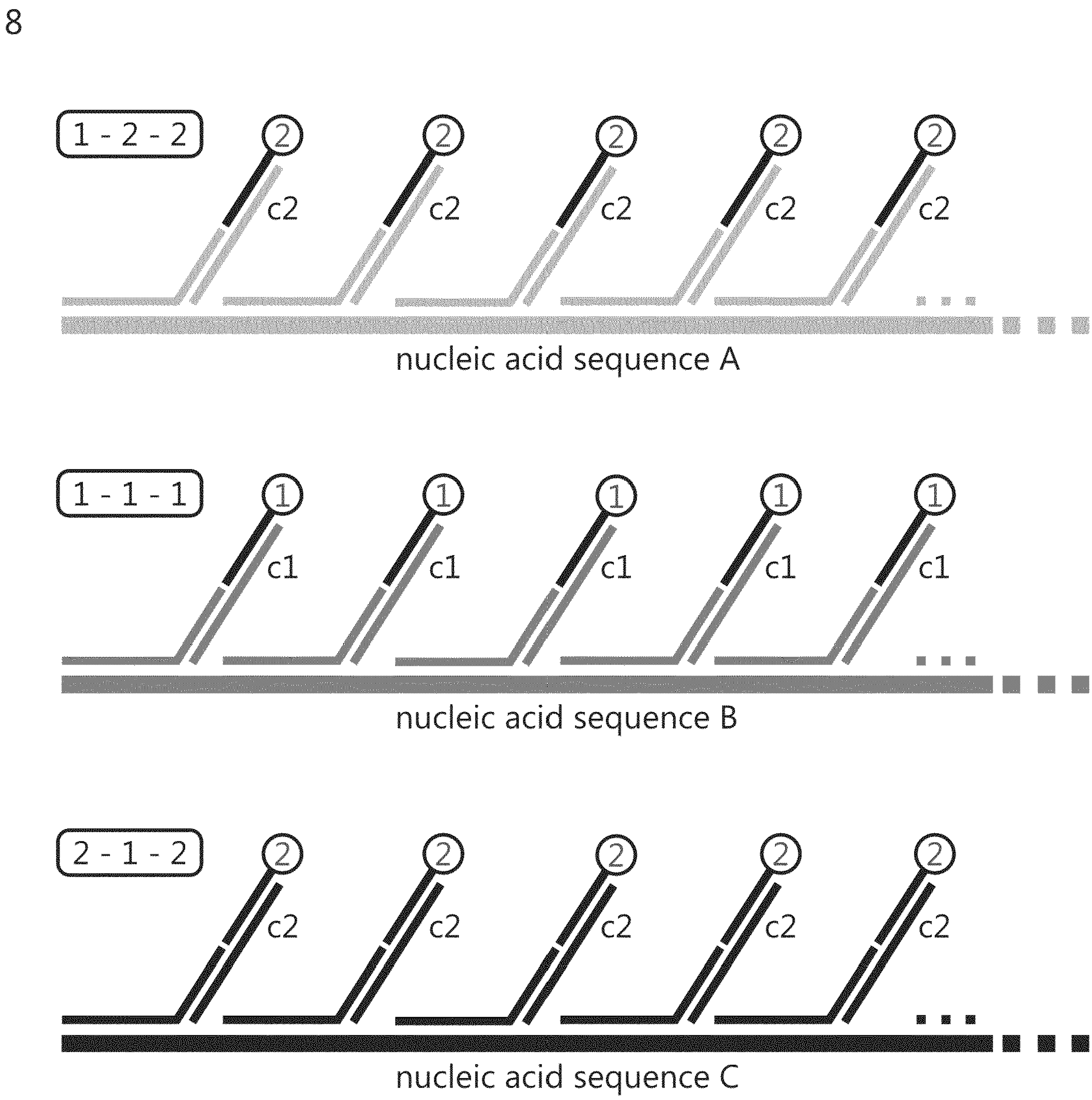
8
1 - 2 - 2
2
c2
nucleic acid sequence A
1 - 1 - 1
1
c1
nucleic acid sequence B
2 - 1 - 2
2
c2
nucleic acid sequence C

Fig. 5

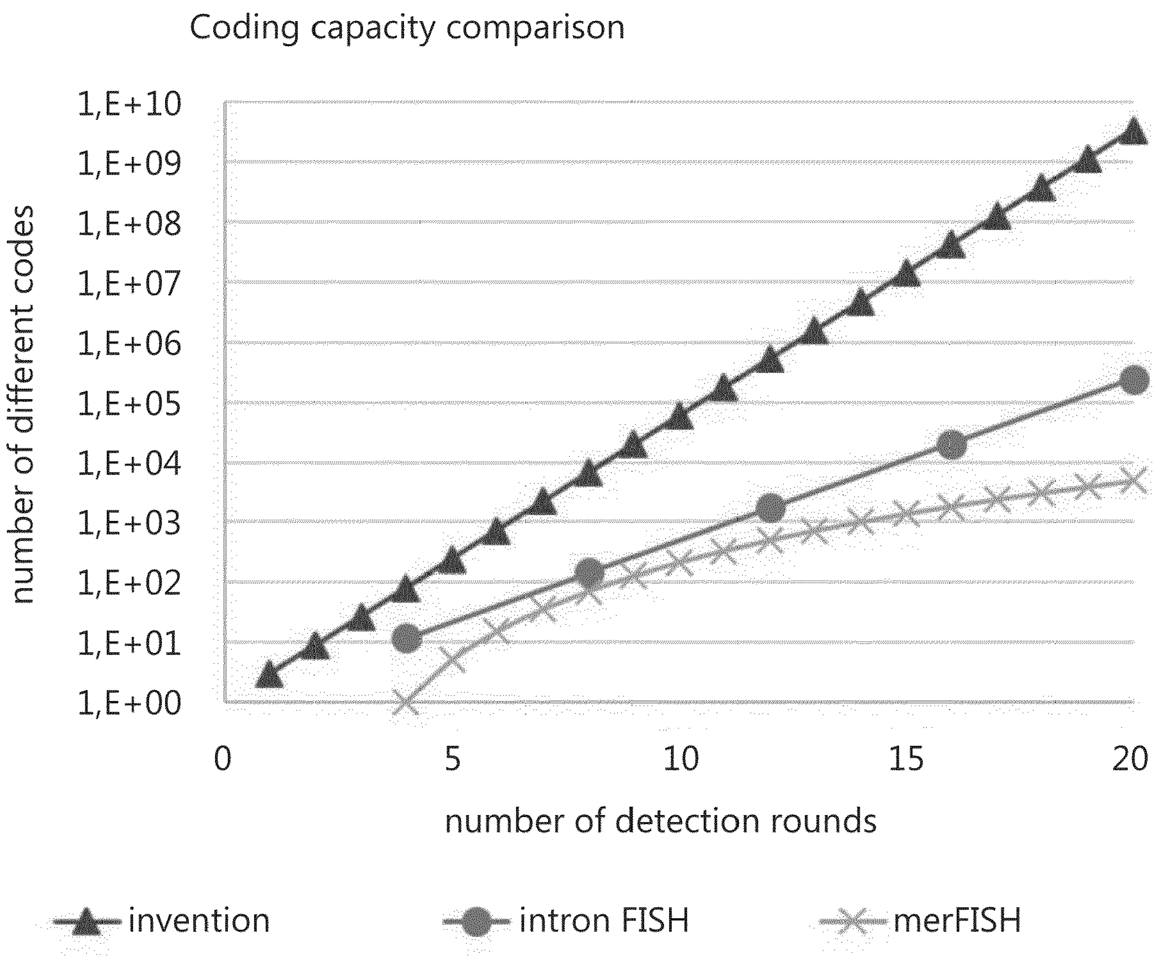
Coding capacity comparison
1,E+10
1,E+09
1,E+08
1,E+07
1,E+06
1,E+05
1,E+04
1,E+03
1,E+02
1,E+01
1,E+00
number of different codes
0
5
10
15
20
number of detection rounds
invention
intron FISH
merFISH

Fig. 6

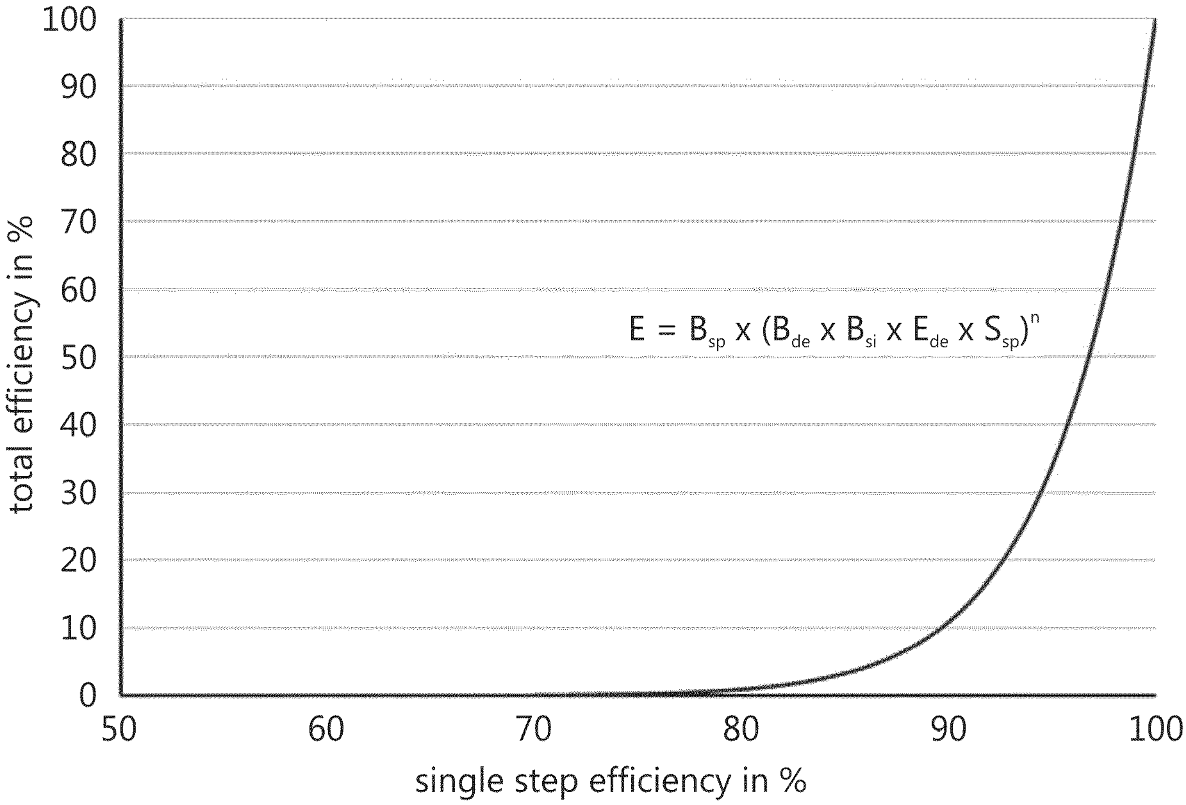
100
90
80
70
60
50
40
30
20
10
0
total efficiency in %
$E = B_{sp} \times (B_{de} \times B_{si} \times E_{de} \times S_{sp})^n$
50
60
70
80
90
100
single step efficiency in %

Fig. 7

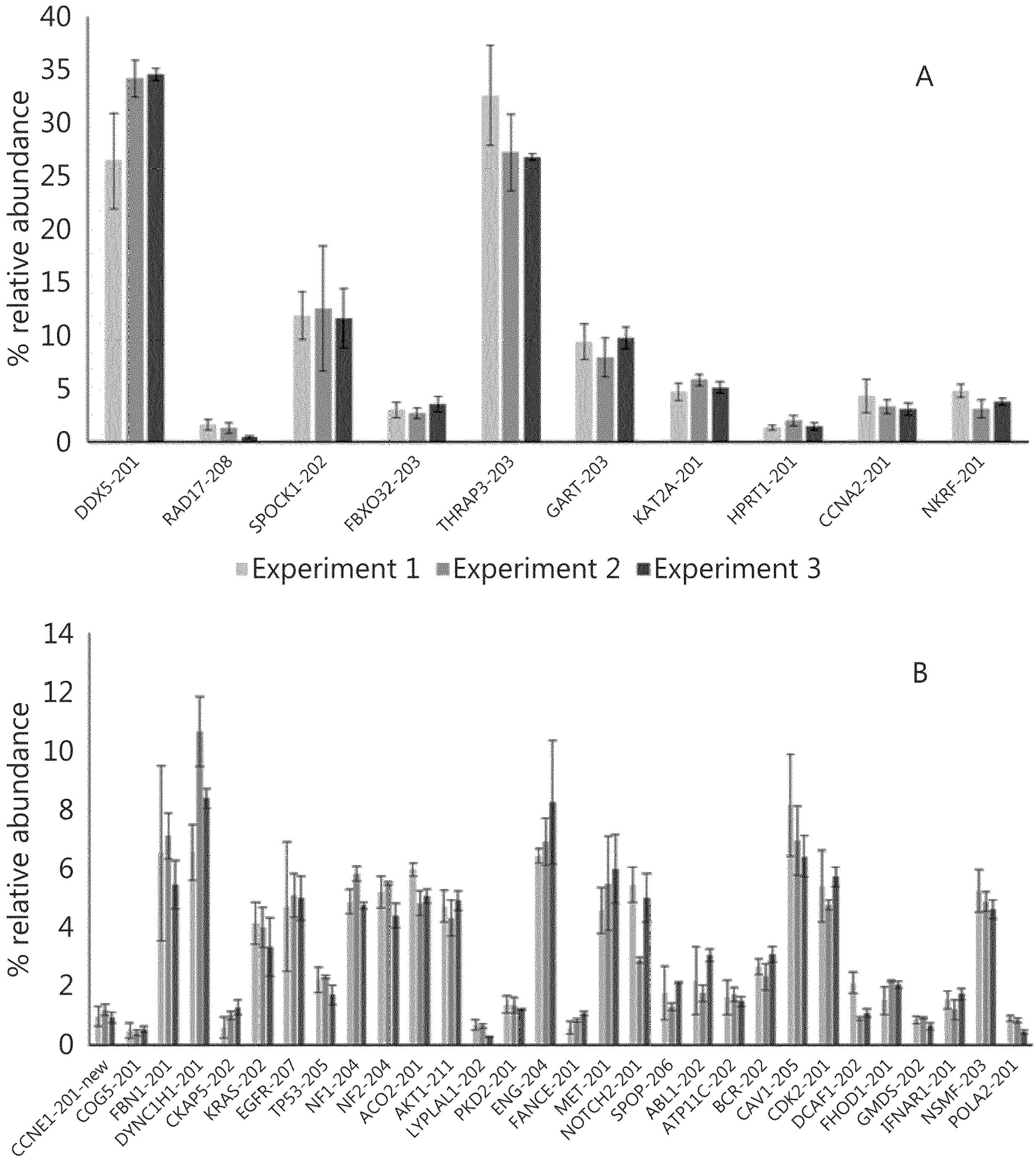
A
% relative abundance
40
35
30
25
20
15
10
5
0
DDX5-201
RAD17-208
SPOCK1-202
FBXO32-203
THRAP3-203
GART-203
KAT2A-201
HPRT1-201
CCNA2-201
NKRF-201
Experiment 1
Experiment 2
Experiment 3
B
% relative abundance
14
12
10
8
6
4
2
0
CCNE1-201-new
COG5-201
FBN1-201
DYNC1H1-201
CKAP5-202
KRAS-202
EGFR-207
TP53-205
NF1-204
NF2-204
ACO2-201
AKT1-211
LYPLAL1-202
PKD2-201
ENG-204
FANCE-201
MET-201
NOTCH2-201
SPOP-206
ABL1-202
ATP11C-202
BCR-202
CAV1-205
CDK2-201
DCAF1-202
FHOD1-201
GMDS-202
IFNAR1-201
NSMF-203
POLA2-201
Experiment 1
Experiment 2
Experiment 3

Fig. 8

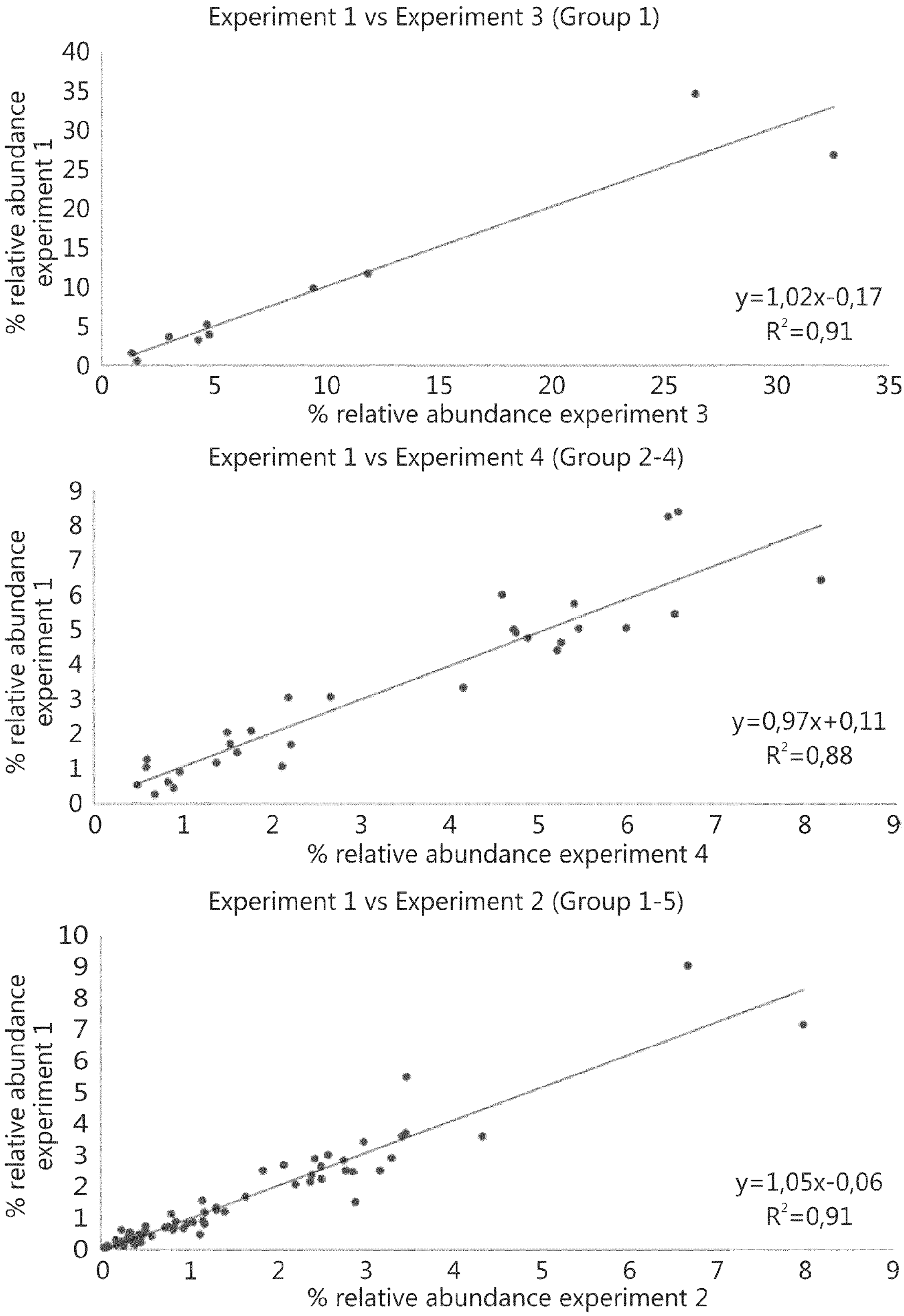
Experiment 1 vs Experiment 3 (Group 1)
% relative abundance experiment 1
% relative abundance experiment 3
y=1,02x-0,17
R²=0,91
Experiment 1 vs Experiment 4 (Group 2-4)
% relative abundance experiment 1
% relative abundance experiment 4
y=0,97x+0,11
R²=0,88
Experiment 1 vs Experiment 2 (Group 1-5)
% relative abundance experiment 1
% relative abundance experiment 2
y=1,05x-0,06
R²=0,91

Fig. 8

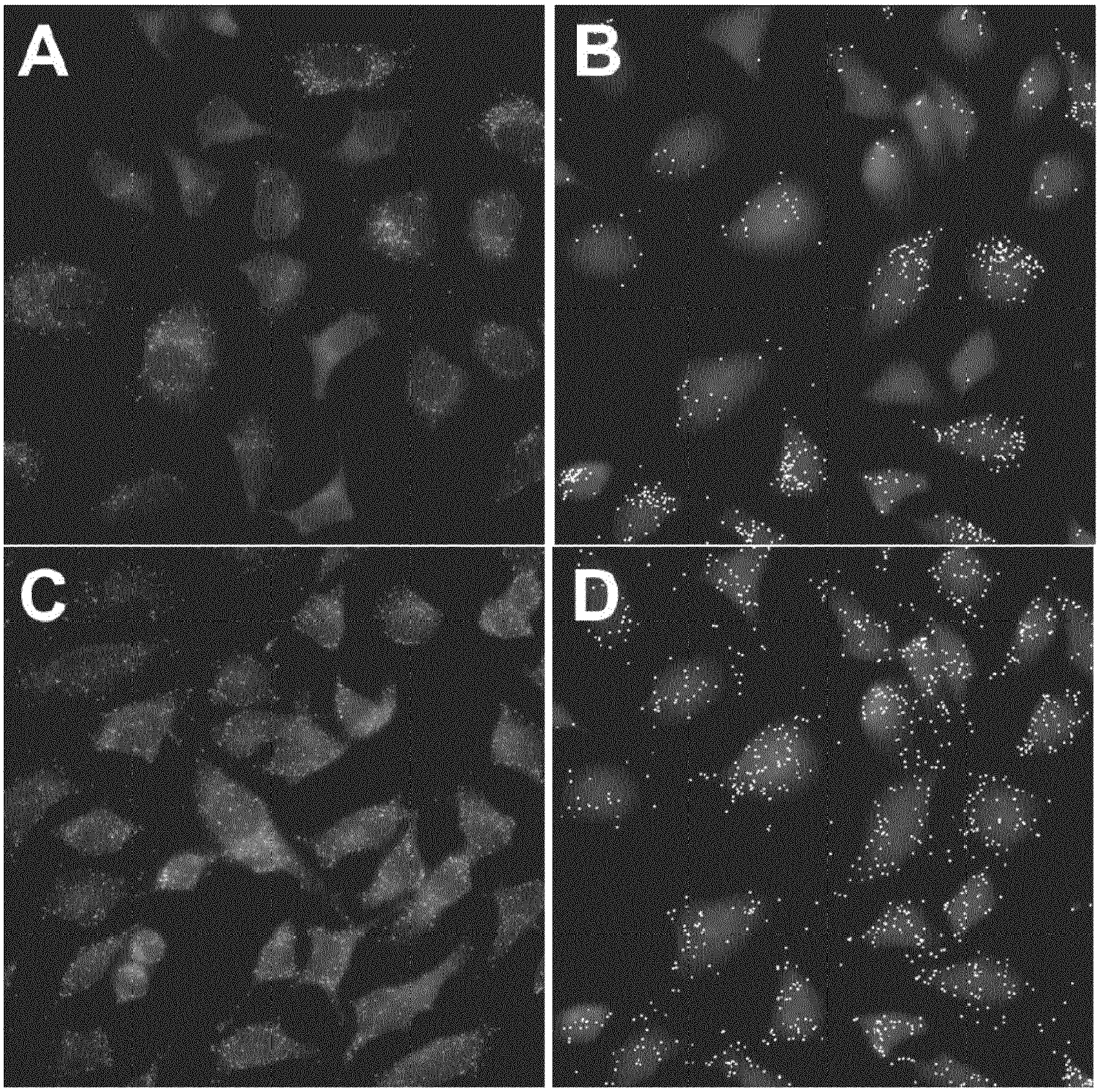


Fig. 10

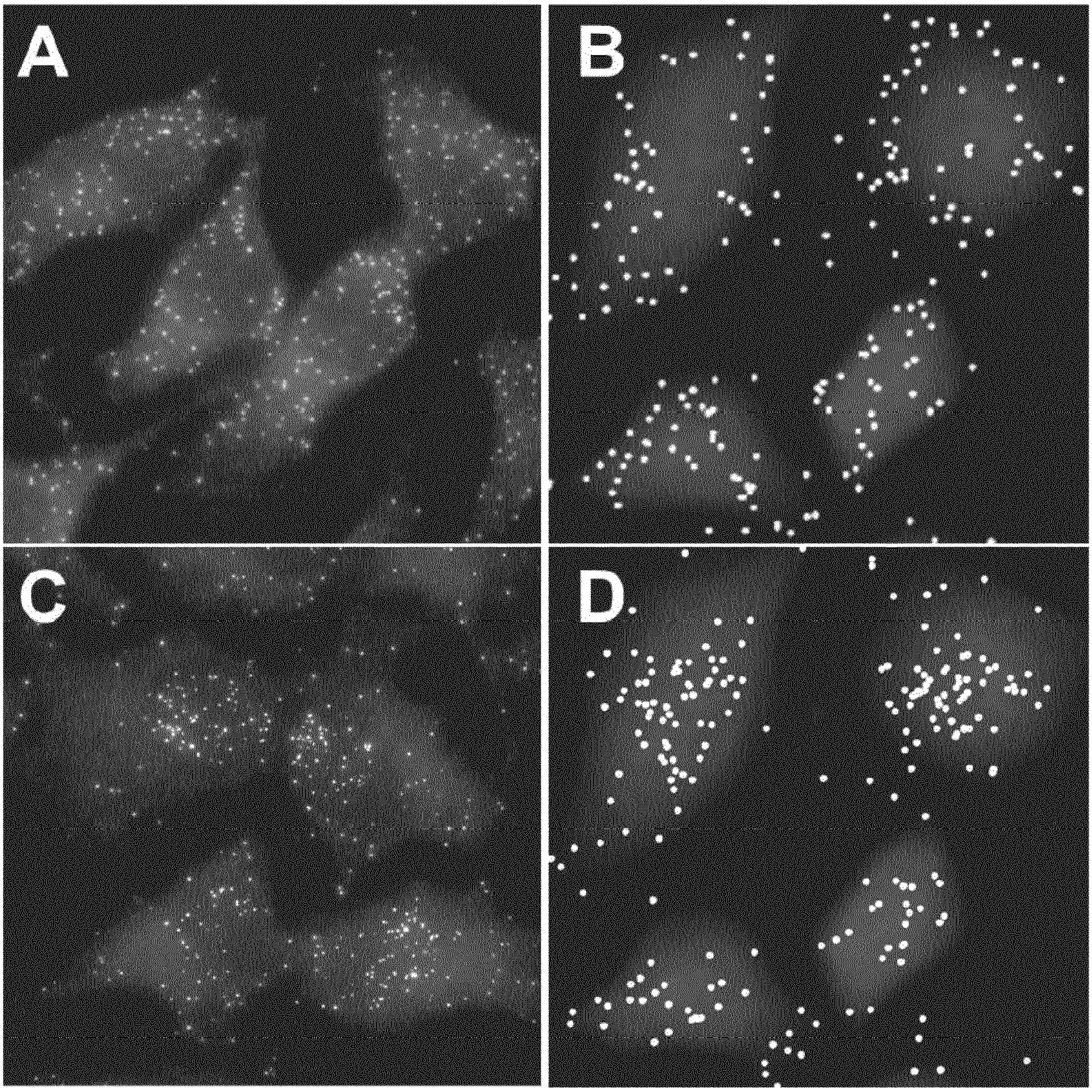


Fig. 11

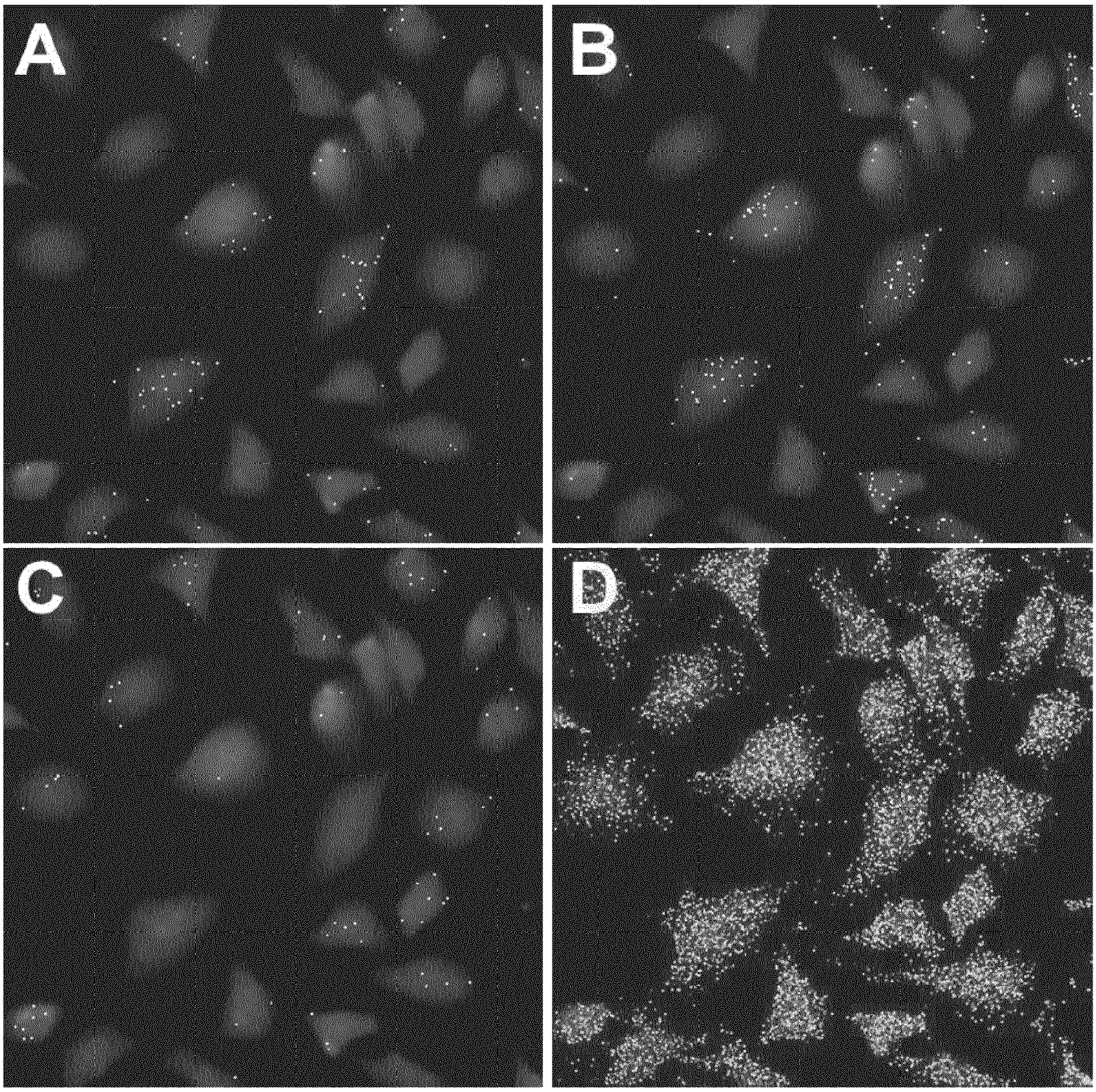


Fig. 12

Europäisches Patentamt
European Patent Office
Office européen des brevets

# EUROPEAN SEARCH REPORT

Application Number
EP 19 18 1051

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KARUNANAYAKE MUDIYANSELAGE ARUNI P K K ET AL: ""Second-generation" fluorogenic RNA-based sensors", METHODS, vol. 161, 17 January 2019 (2019-01-17), pages 24-34, XP085713798, ISSN: 1046-2023, DOI: 10.1016/J.YMETH.2019.01.008 | 13-15 | INV. C12Q1/6813 |
| Y | * the whole document * ----- | 1-12 | |
| X | HARRY M. T. CHOI ET AL: "Third-generation in situ hybridization chain reaction: multiplexed, quantitative, sensitive, versatile, robust", DEVELOPMENT (SPECIAL ISSUE ON DEVELOPMENT AT THE SINGLE CELL LEVEL), vol. 145, no. 12, 15 June 2018 (2018-06-15), XP055590305, GB ISSN: 0950-1991, DOI: 10.1242/dev.165753 | 13-15 | |
| Y | * the whole document * ----- | 1-12 | TECHNICAL FIELDS SEARCHED (IPC) |
| X | WO 2017/189525 A1 (HARVARD COLLEGE [US]) 2 November 2017 (2017-11-02) | 13-15 | C12Q |
| Y | * the whole document * ----- | 1-12 | |
| X | SUN Z, NGUYEN T, MCAULIFFE K, YOU M: "Intracellular Imaging with Genetically Encoded RNA-based Molecular Sensors.", NANOMATERIALS (BASEL), vol. 9, 8 February 2019 (2019-02-08), XP002795696, | 13-15 | |
| Y | * the whole document * ----- | 1-12 | |

The present search report has been drawn up for all claims

| Place of search | Date of completion of the search | Examiner |
|---|---|---|
| Munich | 26 November 2019 | Young, Craig |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 18 1051

This annex lists the patent family members relating to the patent documents cited in the above-mentioned European search report. The members are as contained in the European Patent Office EDP file on
The European Patent Office is in no way liable for these particulars which are merely given for the purpose of information.

26-11-2019

| Patent document cited in search report | Publication date | Patent family member(s) | Publication date |
|---|---|---|---|
| WO 2017189525 A1 | 02-11-2017 | AU 2017257624 A1 | 22-11-2018 |
| | | CA 3022290 A1 | 02-11-2017 |
| | | CN 109415761 A | 01-03-2019 |
| | | EP 3449016 A1 | 06-03-2019 |
| | | JP 2019518434 A | 04-07-2019 |
| | | US 2019218608 A1 | 18-07-2019 |
| | | WO 2017189525 A1 | 02-11-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0611828 A


### Non-patent literature cited in the description


- **LUBECK et al.** Single-cell in situ RNA profiling by sequential hybridization. *Nat. Methods,* 2014, vol. 11 (4), 360-361
- *Neuron,* vol. 92 (2), 342-357
- **CHEN et al.** *RNA imaging,* 2015
- *Science,* vol. 348 (6233), aaa6090
- **MOFFITT et al.** High-throughput single-cell gene-expression profiling with multiplexed error-robust fluorescence in situ hybridization. *Proc. Natl. Acad. Sci. U S A.,* 2016, vol. 113 (39), 11046-11051
- **SHAH et al.** Dynamics and spatial genomics of the nascent transcriptome by intron seqFISH. *Cell,* 2018, vol. 117 (2), 363-376
- **PLAYER et al.** Single-copy gene detection using branched DNA (bDNA) in situ hybridization. *J. Histochem. Cytochem.,* 2001, vol. 49 (5), 603-611
- **WANG et al.** RNAscope: a novel in situ RNA analysis platform for formalin-fixed, paraffin-embedded tissues. *J. Mol. Diagn.,* 2012, vol. 14 (1), 22-29
- **CHOI et al.** Programmable in situ amplification for multiplexed imaging of mRNA expression. *Nat. Biotechnol.,* 2010, vol. 28 (11), 1208-1212
- **CHOI et al.** Third-generation in situ hybridization chain reaction: multiplexed, quantitative, sensitive, versatile, robust. *Development,* 2018, vol. 145 (12

SEQUENCE LISTING

<110> QIAGEN GmbH

<120> Method of signal encoding of analytes in a sample

<130> 4203P117EP

<160> 1400

<170> BiSSAP 1.3.6

<210> 1
<211> 45
<212> DNA
<213> Artificial Sequence

<220>
<223> Target Specific Oligonucleotide

<400> 1
actactagag accggtagaa atgagtaacg attaccgact tatcc 45

<210> 2
<211> 42
<212> DNA
<213> Artificial Sequence

<220>
<223> Target Specific Oligonucleotide

<400> 2
tggcggggaa cgaagtatat agtaacgatt accgacttat cc 42

<210> 3
<211> 40
<212> DNA
<213> Artificial Sequence

<220>
<223> Target Specific Oligonucleotide

<400> 3
tggcgtcaat ggttgcggtg taacgattac cgacttatcc 40

<210> 4
<211> 41
<212> DNA
<213> Artificial Sequence

<220>
<223> Target Specific Oligonucleotide

<400> 4
tcggtcactc gaataacccg gtaacgatta ccgacttatc c 41

<210> 5
<211> 41
<212> DNA
<213> Artificial Sequence

<220>
<223> Target Specific Oligonucleotide

<400> 5
ctccaaatcg aggtgcacca gtaacgatta ccgacttatc c 41

<210> 6
<211> 40
<212> DNA
<213> Artificial Sequence

<220>
<223> Target Specific Oligonucleotide

<400> 6
ggccagttcc cgagttggtg taacgattac cgacttatcc 40

<210> 7
<211> 42
<212> DNA
<213> Artificial Sequence

<220>
<223> Target Specific Oligonucleotide

<400> 7
agacttcaag cgacatgctc tgtaacgatt accgacttat cc 42

<210> 8
<211> 42
<212> DNA
<213> Artificial Sequence

<220>
<223> Target Specific Oligonucleotide

<400> 8
ggagcaccac cgtagataca agtaacgatt accgacttat cc 42

<210> 9
<211> 44
<212> DNA
<213> Artificial Sequence

<220>
<223> Target Specific Oligonucleotide

<400> 9

ctctccaaat cacgtatttg tgggtaacga ttaccgactt atcc 44

<210> 10
<211> 45
<212> DNA
<213> Artificial Sequence

<220>
<223> Target Specific Oligonucleotide

<400> 10
agagtttgcc tatcaggtct tattgtaacg attaccgact tatcc 45

<210> 11
<211> 40
<212> DNA
<213> Artificial Sequence

<220>
<223> Target Specific Oligonucleotide

<400> 11
ggccaagtcg cactccacag taacgattac cgacttatcc 40

<210> 12
<211> 43
<212> DNA
<213> Artificial Sequence

<220>
<223> Target Specific Oligonucleotide

<400> 12
tcctttteta cgtcatgaca cagtaacgat taccgactta tcc 43

<210> 13
<211> 46
<212> DNA
<213> Artificial Sequence

<220>
<223> Target Specific Oligonucleotide

<400> 13
ttccattaga cgaataagtt tttcagtaac gattaccgac ttatcc 46

<210> 14
<211> 44
<212> DNA
<213> Artificial Sequence

<220>
<223> Target Specific Oligonucleotide

<400> 14
tttctggtaa gctcatcaca tctgtaacga ttaccgactt atcc 44

<210> 15
<211> 46
<212> DNA
<213> Artificial Sequence

<220>
<223> Target Specific Oligonucleotide

<400> 15
gagttagggt agtcataatt gatgagtaac gattaccgac ttatcc 46

<210> 16
<211> 47
<212> DNA
<213> Artificial Sequence

<220>
<223> Target Specific Oligonucleotide

<400> 16
ttcttccaat tcgatgaata taatccgtaa cgattaccga cttatcc 47

<210> 17
<211> 48
<212> DNA
<213> Artificial Sequence

<220>
<223> Target Specific Oligonucleotide

<400> 17
tgttattagg tgtaaagaaa gtgtatggta acgattaccg acttatcc 48

<210> 18
<211> 42
<212> DNA
<213> Artificial Sequence

<220>
<223> Target Specific Oligonucleotide

<400> 18
gagataaggt cgctcacttg cgtaacgatt accgacttat cc 42

<210> 19
<211> 42
<212> DNA
<213> Artificial Sequence

<220>
<223> Target Specific Oligonucleotide

<400> 19
acctctgtct tcgaccaact ggtaacgatt accgacttat cc 42

<210> 20
<211> 41
<212> DNA
<213> Artificial Sequence

<220>
<223> Target Specific Oligonucleotide

<400> 20
acccctggaa cgacctgaac gtaacgatta ccgacttatc c 41

<210> 21
<211> 41
<212> DNA
<213> Artificial Sequence

<220>
<223> Target Specific Oligonucleotide

<400> 21
agtatctgtc ccgacggtca gtaacgatta ccgacttatc c 41

<210> 22
<211> 40
<212> DNA
<213> Artificial Sequence

<220>
<223> Target Specific Oligonucleotide

<400> 22
accccttttg cccgcagagg taacgattac cgacttatcc 40

<210> 23
<211> 44
<212> DNA
<213> Artificial Sequence

<220>
<223> Target Specific Oligonucleotide

<400> 23
tttaagcagg ctagagtaac ctcgtaacga ttaccgactt atcc 44

<210> 24
<211> 45
<212> DNA
<213> Artificial Sequence

<220>
<223> Target Specific Oligonucleotide

<400> 24
gcattggata accaatcata ggtggtaacg attaccgact tatcc 45

<210> 25
<211> 41
<212> DNA
<213> Artificial Sequence

<220>
<223> Target Specific Oligonucleotide

<400> 25
tcccctagtc cgagttgctc gtaaccctaa ttatgggaat c 41

<210> 26
<211> 43
<212> DNA
<213> Artificial Sequence

<220>
<223> Target Specific Oligonucleotide

<400> 26
ccaatttgca cttggatgtg tagtaaccct aattatggga atc 43

<210> 27
<211> 43
<212> DNA
<213> Artificial Sequence

<220>
<223> Target Specific Oligonucleotide

<400> 27
ttttgccagt agaacaagca tggtaaccct aattatggga atc 43

<210> 28
<211> 40
<212> DNA
<213> Artificial Sequence

<220>
<223> Target Specific Oligonucleotide

<400> 28
ggaggcaagg ttccgcaacg taaccctaat tatgggaatc 40

<210> 29
<211> 44

<212> DNA
<213> Artificial Sequence

<220>
<223> Target Specific Oligonucleotide

<400> 29
agaattccac gtaaatcaca tgcgtaaccc taattatggg aatc 44

<210> 30
<211> 44
<212> DNA
<213> Artificial Sequence

<220>
<223> Target Specific Oligonucleotide

<400> 30
tatgcatgta cagggaagta tccgtaaccc taattatggg aatc 44

<210> 31
<211> 43
<212> DNA
<213> Artificial Sequence

<220>
<223> Target Specific Oligonucleotide

<400> 31
gtgtgcatgc cttagaaaaa gcgtaaccct aattatggga atc 43

<210> 32
<211> 43
<212> DNA
<213> Artificial Sequence

<220>
<223> Target Specific Oligonucleotide

<400> 32
agcttttgtg cccttatcat gagtaaccct aattatggga atc 43

<210> 33
<211> 46
<212> DNA
<213> Artificial Sequence

<220>
<223> Target Specific Oligonucleotide

<400> 33
cttcattagt atcttgtcac ttggggtaac cctaattatg ggaatc 46

<210> 34
<211> 45
<212> DNA
<213> Artificial Sequence

<220>
<223> Target Specific Oligonucleotide

<400> 34
agatgatgac cctaccaaat ttgggtaacc ctaattatgg gaatc 45

<210> 35
<211> 44
<212> DNA
<213> Artificial Sequence

<220>
<223> Target Specific Oligonucleotide

<400> 35
aggcaagaga tagatatgtg ggcgtaaccc taattatggg aatc 44

<210> 36
<211> 44
<212> DNA
<213> Artificial Sequence

<220>
<223> Target Specific Oligonucleotide

<400> 36
cagctgccac tataatcatg tttgtaaccc taattatggg aatc 44

<210> 37
<211> 45
<212> DNA
<213> Artificial Sequence

<220>
<223> Target Specific Oligonucleotide

<400> 37
ggaacatgct aatttaaggt gagtgtaacc ctaattatgg gaatc 45

<210> 38
<211> 46
<212> DNA
<213> Artificial Sequence

<220>
<223> Target Specific Oligonucleotide

<400> 38
aggactttgc tatatcaagt agttcgtaac cctaattatg ggaatc 46

<210> 39
<211> 46
<212> DNA
<213> Artificial Sequence

<220>
<223> Target Specific Oligonucleotide

<400> 39
aaaggagata gtaacaatgg ttttcgtaac cctaattatg ggaatc 46

<210> 40
<211> 43
<212> DNA
<213> Artificial Sequence

<220>
<223> Target Specific Oligonucleotide

<400> 40
aaatcagtgg ttcaccctgt tcgtaaccct aattatggga atc 43

<210> 41
<211> 48
<212> DNA
<213> Artificial Sequence

<220>
<223> Target Specific Oligonucleotide

<400> 41
cttgtcataa gataattagg caaattagta accctaatta tgggaatc 48

<210> 42
<211> 47
<212> DNA
<213> Artificial Sequence

<220>
<223> Target Specific Oligonucleotide

<400> 42
tcattaagtt aatgctaagg atctttgtaa ccctaattat gggaatc 47

<210> 43
<211> 43
<212> DNA
<213> Artificial Sequence

<220>
<223> Target Specific Oligonucleotide

<400> 43
tgtcactggt acaagtggac ttgtaaccct aattatggga atc 43

<210> 44
<211> 45
<212> DNA
<213> Artificial Sequence

<220>
<223> Target Specific Oligonucleotide

<400> 44
ttggtcacta tacaagtgac ttctgtaacc ctaattatgg gaatc 45

<210> 45
<211> 43
<212> DNA
<213> Artificial Sequence

<220>
<223> Target Specific Oligonucleotide

<400> 45
aggaaaatgg gcgtaaagga gggtaaccct aattatggga atc 43

<210> 46
<211> 46
<212> DNA
<213> Artificial Sequence

<220>
<223> Target Specific Oligonucleotide

<400> 46
ttcatctaca atagggacaa caaacgtaac cctaattatg ggaatc 46

<210> 47
<211> 44
<212> DNA
<213> Artificial Sequence

<220>
<223> Target Specific Oligonucleotide

<400> 47
gataagtcta cgtggaaaag catgtaaccc taattatggg aatc 44

<210> 48
<211> 45
<212> DNA
<213> Artificial Sequence

<220>

<223> Target Specific Oligonucleotide

<400> 48
caggactaca gttaagcatt tactgtaacc ctaattatgg gaatc 45

<210> 49
<211> 43
<212> DNA
<213> Artificial Sequence

<220>
<223> Target Specific Oligonucleotide

<400> 49
gtctaggaaa ttgccgtggt tggtaaccct aattatggga atc 43

<210> 50
<211> 42
<212> DNA
<213> Artificial Sequence

<220>
<223> Target Specific Oligonucleotide

<400> 50
aatcatcgtc tcgaaagcgg tgtaacccta attatgggaa tc 42

<210> 51
<211> 43
<212> DNA
<213> Artificial Sequence

<220>
<223> Target Specific Oligonucleotide

<400> 51
cttgaccaaa ttcgaaggtc cagtaaccct aattatggga atc 43

<210> 52
<211> 43
<212> DNA
<213> Artificial Sequence

<220>
<223> Target Specific Oligonucleotide

<400> 52
gatgactctg ttcgcatcct cggtaaccct aattatggga atc 43

<210> 53
<211> 42
<212> DNA
<213> Artificial Sequence

<220>
<223> Target Specific Oligonucleotide

<400> 53
gatgctagtg tcaaacctgc cgtaacccta attatgggaa tc 42

<210> 54
<211> 44
<212> DNA
<213> Artificial Sequence

<220>
<223> Target Specific Oligonucleotide

<400> 54
tgatacaggg ctcgtactta tccgtaaccc taattatggg aatc 44

<210> 55
<211> 43
<212> DNA
<213> Artificial Sequence

<220>
<223> Target Specific Oligonucleotide

<400> 55
aggagtcaca cgagttgaaa aggtaaccct aattatggga atc 43

<210> 56
<211> 44
<212> DNA
<213> Artificial Sequence

<220>
<223> Target Specific Oligonucleotide

<400> 56
ctcattacac cgaggtatga agggtaaccc taattatggg aatc 44

<210> 57
<211> 40
<212> DNA
<213> Artificial Sequence

<220>
<223> Target Specific Oligonucleotide

<400> 57
ctctgtcacg gctcgggtgg taaccctaat tatgggaatc 40

<210> 58
<211> 43
<212> DNA

<213> Artificial Sequence

<220>
<223> Target Specific Oligonucleotide

<400> 58
ctaccatatg tacccgacct cagtaaccct aattatggga atc 43

<210> 59
<211> 44
<212> DNA
<213> Artificial Sequence

<220>
<223> Target Specific Oligonucleotide

<400> 59
aaccttaggt cgggtataga gaggtaaccc taattatggg aatc 44

<210> 60
<211> 44
<212> DNA
<213> Artificial Sequence

<220>
<223> Target Specific Oligonucleotide

<400> 60
gaagcactta gacactgtaa ggcgtaaccc taattatggg aatc 44

<210> 61
<211> 42
<212> DNA
<213> Artificial Sequence

<220>
<223> Target Specific Oligonucleotide

<400> 61
aataatcacc ggcagtaacg ggtaacccta attatgggaa tc 42

<210> 62
<211> 43
<212> DNA
<213> Artificial Sequence

<220>
<223> Target Specific Oligonucleotide

<400> 62
aggaagaaac ctatggcaga cagtaaccct aattatggga atc 43

<210> 63

<211> 46
<212> DNA
<213> Artificial Sequence

<220>
<223> Target Specific Oligonucleotide

<400> 63
catgtgttat tacgatgttc tttgtgtaac cctaattatg ggaatc 46

<210> 64
<211> 45
<212> DNA
<213> Artificial Sequence

<220>
<223> Target Specific Oligonucleotide

<400> 64
actactgttt ggtacttgta tctggtaacc ctaattatgg gaatc 45

<210> 65
<211> 42
<212> DNA
<213> Artificial Sequence

<220>
<223> Target Specific Oligonucleotide

<400> 65
aaatcagtgg cggacagtag cgtaacccta attatgggaa tc 42

<210> 66
<211> 41
<212> DNA
<213> Artificial Sequence

<220>
<223> Target Specific Oligonucleotide

<400> 66
caggcaatgc cgactggatt gtaaccctaa ttatgggaat c 41

<210> 67
<211> 48
<212> DNA
<213> Artificial Sequence

<220>
<223> Target Specific Oligonucleotide

<400> 67
tcttatctaa gaccaactat aggtatggta accctaatta tgggaatc 48

<210> 68
<211> 43
<212> DNA
<213> Artificial Sequence

<220>
<223> Target Specific Oligonucleotide

<400> 68
gggaactgtc tattgagcac tcgtaaccct aattatggga atc 43

<210> 69
<211> 47
<212> DNA
<213> Artificial Sequence

<220>
<223> Target Specific Oligonucleotide

<400> 69
attacaatct ttagtactca tggaaagtaa ccctaattat gggaatc 47

<210> 70
<211> 46
<212> DNA
<213> Artificial Sequence

<220>
<223> Target Specific Oligonucleotide

<400> 70
aaggtattct catgcctaga atattgtaac cctaattatg ggaatc 46

<210> 71
<211> 43
<212> DNA
<213> Artificial Sequence

<220>
<223> Target Specific Oligonucleotide

<400> 71
tggacagaca cgttgtcatt tggtaaccct aattatggga atc 43

<210> 72
<211> 48
<212> DNA
<213> Artificial Sequence

<220>
<223> Target Specific Oligonucleotide

<400> 72

ctctcattac aatgctatac atttaacgta accctaatta tgggaatc 48

<210> 73
<211> 41
<212> DNA
<213> Artificial Sequence

<220>
<223> Target Specific Oligonucleotide

<400> 73
tgcagctcgc aacggaacaa gtaacggatt ttacaacttt a 41

<210> 74
<211> 43
<212> DNA
<213> Artificial Sequence

<220>
<223> Target Specific Oligonucleotide

<400> 74
gagtccatcc ggacattgac ctgtaacgga ttttacaact tta 43

<210> 75
<211> 41
<212> DNA
<213> Artificial Sequence

<220>
<223> Target Specific Oligonucleotide

<400> 75
cttgaacggc ctcgacgagg gtaacggatt ttacaacttt a 41

<210> 76
<211> 43
<212> DNA
<213> Artificial Sequence

<220>
<223> Target Specific Oligonucleotide

<400> 76
cctcctcggt tatattggcc ccgtaacgga ttttacaact tta 43

<210> 77
<211> 44
<212> DNA
<213> Artificial Sequence

<220>
<223> Target Specific Oligonucleotide

<400> 77
accgagactt cgaaaatgaa cgagtaacgg attttacaac ttta 44

<210> 78
<211> 43
<212> DNA
<213> Artificial Sequence

<220>
<223> Target Specific Oligonucleotide

<400> 78
cctctggctc gaaactgaaa gggtaacgga ttttacaact tta 43

<210> 79
<211> 44
<212> DNA
<213> Artificial Sequence

<220>
<223> Target Specific Oligonucleotide

<400> 79
tgctatggac tagatctcgg caagtaacgg attttacaac ttta 44

<210> 80
<211> 42
<212> DNA
<213> Artificial Sequence

<220>
<223> Target Specific Oligonucleotide

<400> 80
gccttgaccg gtcagaagac ggtaacggat tttacaactt ta 42

<210> 81
<211> 42
<212> DNA
<213> Artificial Sequence

<220>
<223> Target Specific Oligonucleotide

<400> 81
taccacagag atcgcagctg cgtaacggat tttacaactt ta 42

<210> 82
<211> 40
<212> DNA
<213> Artificial Sequence

<220>
<223> Target Specific Oligonucleotide

<400> 82
accagcccga acctcgcccg taacggattt tacaacttta 40

<210> 83
<211> 44
<212> DNA
<213> Artificial Sequence

<220>
<223> Target Specific Oligonucleotide

<400> 83
actgtatgct tgccggtaat tctgtaacgg attttacaac ttta 44

<210> 84
<211> 41
<212> DNA
<213> Artificial Sequence

<220>
<223> Target Specific Oligonucleotide

<400> 84
gaacatgaac cggccccgac gtaacggatt ttacaacttt a 41

<210> 85
<211> 42
<212> DNA
<213> Artificial Sequence

<220>
<223> Target Specific Oligonucleotide

<400> 85
caggaatccc gtcaagggtg ggtaacggat tttacaactt ta 42

<210> 86
<211> 47
<212> DNA
<213> Artificial Sequence

<220>
<223> Target Specific Oligonucleotide

<400> 86
tctcgattta cctcgtttaa gatctcgtaa cggattttac aacttta 47

<210> 87
<211> 43
<212> DNA
<213> Artificial Sequence

<220>
<223> Target Specific Oligonucleotide

<400> 87
agaaccatgg tcgaactgac ctgtaacgga ttttacaact tta 43

<210> 88
<211> 41
<212> DNA
<213> Artificial Sequence

<220>
<223> Target Specific Oligonucleotide

<400> 88
tctgaggggc tacgctgaga gtaacggatt ttacaacttt a 41

<210> 89
<211> 41
<212> DNA
<213> Artificial Sequence

<220>
<223> Target Specific Oligonucleotide

<400> 89
aagcctctgt tacgcccacg gtaacggatt ttacaacttt a 41

<210> 90
<211> 46
<212> DNA
<213> Artificial Sequence

<220>
<223> Target Specific Oligonucleotide

<400> 90
aatgattcaa ctcgtactgg atcccgtaac ggattttaca acttta 46

<210> 91
<211> 44
<212> DNA
<213> Artificial Sequence

<220>
<223> Target Specific Oligonucleotide

<400> 91
cctcggaaat cccgattctg atagtaacgg attttacaac ttta 44

<210> 92
<211> 41

<212> DNA
<213> Artificial Sequence

<220>
<223> Target Specific Oligonucleotide

<400> 92
ggcccctact cggttgtggg gtaacggatt ttacaacttt a 41

<210> 93
<211> 45
<212> DNA
<213> Artificial Sequence

<220>
<223> Target Specific Oligonucleotide

<400> 93
ggaaaatgga acgaaactcc tgttgtaacg gattttacaa cttta 45

<210> 94
<211> 40
<212> DNA
<213> Artificial Sequence

<220>
<223> Target Specific Oligonucleotide

<400> 94
ctgggtcgtg cgaggtcctg taacggattt tacaacttta 40

<210> 95
<211> 40
<212> DNA
<213> Artificial Sequence

<220>
<223> Target Specific Oligonucleotide

<400> 95
cacggggcta ggacggggtg taacggattt tacaacttta 40

<210> 96
<211> 39
<212> DNA
<213> Artificial Sequence

<220>
<223> Target Specific Oligonucleotide

<400> 96
ccgatggcgt actcgtcggg taacccttat tcggtacta 39

<210> 97
<211> 42
<212> DNA
<213> Artificial Sequence

<220>
<223> Target Specific Oligonucleotide

<400> 97
gatcagatca gaatccgagg tggtaaccct tattcggtac ta 42

<210> 98
<211> 40
<212> DNA
<213> Artificial Sequence

<220>
<223> Target Specific Oligonucleotide

<400> 98
ggttgtactc gcgacagttg gtaaccctta ttcggtacta 40

<210> 99
<211> 40
<212> DNA
<213> Artificial Sequence

<220>
<223> Target Specific Oligonucleotide

<400> 99
gacctgctca ttcgaggtga gtaaccctta ttcggtacta 40

<210> 100
<211> 39
<212> DNA
<213> Artificial Sequence

<220>
<223> Target Specific Oligonucleotide

<400> 100
tcacgtagta gcggcttcgg taacccttat tcggtacta 39

<210> 101
<211> 42
<212> DNA
<213> Artificial Sequence

<220>
<223> Target Specific Oligonucleotide

<400> 101
ccaaagatgg gcctacttgt cagtaaccct tattcggtac ta 42

<210> 102
<211> 39
<212> DNA
<213> Artificial Sequence

<220>
<223> Target Specific Oligonucleotide

<400> 102
gacagagctg cacgcttggg taacccttat tcggtacta 39

<210> 103
<211> 39
<212> DNA
<213> Artificial Sequence

<220>
<223> Target Specific Oligonucleotide

<400> 103
ctgggcctgt ttgcgctcag taacccttat tcggtacta 39

<210> 104
<211> 38
<212> DNA
<213> Artificial Sequence

<220>
<223> Target Specific Oligonucleotide

<400> 104
tccttaggca tgcgcggcgt aacccttatt cggtacta 38

<210> 105
<211> 39
<212> DNA
<213> Artificial Sequence

<220>
<223> Target Specific Oligonucleotide

<400> 105
cagcggcagt actcgctgtg taacccttat tcggtacta 39

<210> 106
<211> 38
<212> DNA
<213> Artificial Sequence

<220>
<223> Target Specific Oligonucleotide

<400> 106
atgccaccga tgacccgcgt aacccttatt cggtacta 38

<210> 107
<211> 38
<212> DNA
<213> Artificial Sequence

<220>
<223> Target Specific Oligonucleotide

<400> 107
atcagcgtcg ctccctcggt aacccttatt cggtacta 38

<210> 108
<211> 41
<212> DNA
<213> Artificial Sequence

<220>
<223> Target Specific Oligonucleotide

<400> 108
ctttgagagc tcgaacatcg tgtaaccctt attcggtact a 41

<210> 109
<211> 42
<212> DNA
<213> Artificial Sequence

<220>
<223> Target Specific Oligonucleotide

<400> 109
ttccagtagt taaggcagag cagtaaccct tattcggtac ta 42

<210> 110
<211> 39
<212> DNA
<213> Artificial Sequence

<220>
<223> Target Specific Oligonucleotide

<400> 110
gagctgcagg atcgggtccg taacccttat tcggtacta 39

<210> 111
<211> 41
<212> DNA
<213> Artificial Sequence

<220>

<223> Target Specific Oligonucleotide

<400> 111
acatgagtgg tttcgtagcg ggtaaccctt attcggtact a 41

<210> 112
<211> 40
<212> DNA
<213> Artificial Sequence

<220>
<223> Target Specific Oligonucleotide

<400> 112
tgccgatgac atggaactcg gtaaccctta ttcggtacta 40

<210> 113
<211> 43
<212> DNA
<213> Artificial Sequence

<220>
<223> Target Specific Oligonucleotide

<400> 113
tggtgtaatt gaccttgtag gtagtaaccc ttattcggta cta 43

<210> 114
<211> 39
<212> DNA
<213> Artificial Sequence

<220>
<223> Target Specific Oligonucleotide

<400> 114
cattggccga aagcaggctg taacccttat tcggtacta 39

<210> 115
<211> 40
<212> DNA
<213> Artificial Sequence

<220>
<223> Target Specific Oligonucleotide

<400> 115
gagacctctg ccgaaactgg gtaaccctta ttcggtacta 40

<210> 116
<211> 41
<212> DNA
<213> Artificial Sequence

<220>
<223> Target Specific Oligonucleotide

<400> 116
agtcttgtgc ttcgggtcaa agtaaccctt attcggtact a 41

<210> 117
<211> 41
<212> DNA
<213> Artificial Sequence

<220>
<223> Target Specific Oligonucleotide

<400> 117
cctcttctcg cctggcatag ggtaaccctt attcggtact a 41

<210> 118
<211> 41
<212> DNA
<213> Artificial Sequence

<220>
<223> Target Specific Oligonucleotide

<400> 118
gtaacggtga aaatggaccg ggtaaccctt attcggtact a 41

<210> 119
<211> 42
<212> DNA
<213> Artificial Sequence

<220>
<223> Target Specific Oligonucleotide

<400> 119
tccctctaag ggattaatgc cagtaaccct tattcggtac ta 42

<210> 120
<211> 43
<212> DNA
<213> Artificial Sequence

<220>
<223> Target Specific Oligonucleotide

<400> 120
agttcaagta tcccgcgact agtaacaatt agagtgaaat acc 43

<210> 121
<211> 43
<212> DNA

<213> Artificial Sequence

<220>
<223> Target Specific Oligonucleotide

<400> 121
gaacgcaggc tgtttactgt tgtaacaatt agagtgaaat acc 43

<210> 122
<211> 44
<212> DNA
<213> Artificial Sequence

<220>
<223> Target Specific Oligonucleotide

<400> 122
ggtccaggta aactaatggc tggtaacaat tagagtgaaa tacc 44

<210> 123
<211> 44
<212> DNA
<213> Artificial Sequence

<220>
<223> Target Specific Oligonucleotide

<400> 123
ctctgttgag tttacctcgc aagtaacaat tagagtgaaa tacc 44

<210> 124
<211> 44
<212> DNA
<213> Artificial Sequence

<220>
<223> Target Specific Oligonucleotide

<400> 124
tacttcaact aaccagtcca cggtaacaat tagagtgaaa tacc 44

<210> 125
<211> 44
<212> DNA
<213> Artificial Sequence

<220>
<223> Target Specific Oligonucleotide

<400> 125
ccatgagaca aggcttaaga ctgtaacaat tagagtgaaa tacc 44

<210> 126

<211> 43
<212> DNA
<213> Artificial Sequence

<220>
<223> Target Specific Oligonucleotide

<400> 126
cggccaaaga atagtcgtag cgtaacaatt agagtgaaat acc 43

<210> 127
<211> 45
<212> DNA
<213> Artificial Sequence

<220>
<223> Target Specific Oligonucleotide

<400> 127
ccatttccct aaggtatgtg tgagtaacaa ttagagtgaa atacc 45

<210> 128
<211> 46
<212> DNA
<213> Artificial Sequence

<220>
<223> Target Specific Oligonucleotide

<400> 128
ctctcatact gttagtgatg tctggtaaca attagagtga aatacc 46

<210> 129
<211> 42
<212> DNA
<213> Artificial Sequence

<220>
<223> Target Specific Oligonucleotide

<400> 129
cagctttgtc ccgtgactgt gtaacaatta gagtgaaata cc 42

<210> 130
<211> 41
<212> DNA
<213> Artificial Sequence

<220>
<223> Target Specific Oligonucleotide

<400> 130
tgctgcaatg ctagcagcgg taacaattag agtgaaatac c 41

<210> 131
<211> 46
<212> DNA
<213> Artificial Sequence

<220>
<223> Target Specific Oligonucleotide

<400> 131
aagacaggaa cctatcaatg tagtgtaaca attagagtga aatacc 46

<210> 132
<211> 46
<212> DNA
<213> Artificial Sequence

<220>
<223> Target Specific Oligonucleotide

<400> 132
tagattagat tatgcccaag tcaggtaaca attagagtga aatacc 46

<210> 133
<211> 46
<212> DNA
<213> Artificial Sequence

<220>
<223> Target Specific Oligonucleotide

<400> 133
catgtaaccc actttaggtt tacagtaaca attagagtga aatacc 46

<210> 134
<211> 47
<212> DNA
<213> Artificial Sequence

<220>
<223> Target Specific Oligonucleotide

<400> 134
ccagtctttc gtattaatga ttcaggtaac aattagagtg aaatacc 47

<210> 135
<211> 42
<212> DNA
<213> Artificial Sequence

<220>
<223> Target Specific Oligonucleotide

<400> 135

tgcaacccgt ctcgtcttcg gtaacaatta gagtgaaata cc 42

<210> 136
<211> 47
<212> DNA
<213> Artificial Sequence

<220>
<223> Target Specific Oligonucleotide

<400> 136
gtgtaggtat catctgtaat gtacagtaac aattagagtg aaatacc 47

<210> 137
<211> 41
<212> DNA
<213> Artificial Sequence

<220>
<223> Target Specific Oligonucleotide

<400> 137
tcccggactt cagtaccgcg taacaattag agtgaaatac c 41

<210> 138
<211> 41
<212> DNA
<213> Artificial Sequence

<220>
<223> Target Specific Oligonucleotide

<400> 138
cgaggaggtt gcgaaaggcg taacaattag agtgaaatac c 41

<210> 139
<211> 49
<212> DNA
<213> Artificial Sequence

<220>
<223> Target Specific Oligonucleotide

<400> 139
tatacatata ctcaacactt atagagggta acaattagag tgaaatacc 49

<210> 140
<211> 49
<212> DNA
<213> Artificial Sequence

<220>
<223> Target Specific Oligonucleotide

<400> 140
gtaatagata ccataatttg tacttgggta acaattagag tgaaatacc 49

<210> 141
<211> 40
<212> DNA
<213> Artificial Sequence

<220>
<223> Target Specific Oligonucleotide

<400> 141
tgctgctgcg ctagacccgt aacaattaga gtgaaatacc 40

<210> 142
<211> 40
<212> DNA
<213> Artificial Sequence

<220>
<223> Target Specific Oligonucleotide

<400> 142
gcgtggcgag ccaaagacgt aacaattaga gtgaaatacc 40

<210> 143
<211> 40
<212> DNA
<213> Artificial Sequence

<220>
<223> Target Specific Oligonucleotide

<400> 143
tccgcggttg ttggacgggt aacaattaga gtgaaatacc 40

<210> 144
<211> 41
<212> DNA
<213> Artificial Sequence

<220>
<223> Target Specific Oligonucleotide

<400> 144
ctgtcacata cgcaaactgg tgtaacatcg ttatagctag a 41

<210> 145
<211> 41
<212> DNA
<213> Artificial Sequence

<220>
<223> Target Specific Oligonucleotide

<400> 145
tcgccatata ccggtcaaag agtaacatcg ttatagctag a 41

<210> 146
<211> 42
<212> DNA
<213> Artificial Sequence

<220>
<223> Target Specific Oligonucleotide

<400> 146
tggatggtgc aataatccga gggtaacatc gttatagcta ga 42

<210> 147
<211> 43
<212> DNA
<213> Artificial Sequence

<220>
<223> Target Specific Oligonucleotide

<400> 147
agtgctgatc ccttaagtat gtcgtaacat cgttatagct aga 43

<210> 148
<211> 43
<212> DNA
<213> Artificial Sequence

<220>
<223> Target Specific Oligonucleotide

<400> 148
acctccatta accaatccag aaggtaacat cgttatagct aga 43

<210> 149
<211> 43
<212> DNA
<213> Artificial Sequence

<220>
<223> Target Specific Oligonucleotide

<400> 149
tggtgtactt gacccactta tttgtaacat cgttatagct aga 43

<210> 150
<211> 41
<212> DNA
<213> Artificial Sequence

<220>
<223> Target Specific Oligonucleotide

<400> 150
cagaagagaa cgtggagcag ggtaacatcg ttatagctag a 41

<210> 151
<211> 44
<212> DNA
<213> Artificial Sequence

<220>
<223> Target Specific Oligonucleotide

<400> 151
tccctgtgaa gtttatagac ttcagtaaca tcgttatagc taga 44

<210> 152
<211> 40
<212> DNA
<213> Artificial Sequence

<220>
<223> Target Specific Oligonucleotide

<400> 152
tgtaaaagca aacgcacgcc gtaacatcgt tatagctaga 40

<210> 153
<211> 44
<212> DNA
<213> Artificial Sequence

<220>
<223> Target Specific Oligonucleotide

<400> 153
attcagatga cgagaaatga tacagtaaca tcgttatagc taga 44

<210> 154
<211> 39
<212> DNA
<213> Artificial Sequence

<220>
<223> Target Specific Oligonucleotide

<400> 154
tggtcacgcc atttccggcg taacatcgtt atagctaga 39

<210> 155
<211> 45

<212> DNA
<213> Artificial Sequence

<220>
<223> Target Specific Oligonucleotide

<400> 155
agcaagtata ccataaggaa attcagtaac atcgttatag ctaga 45

<210> 156
<211> 40
<212> DNA
<213> Artificial Sequence

<220>
<223> Target Specific Oligonucleotide

<400> 156
ctcgccgtcc tgtcgatttt gtaacatcgt tatagctaga 40

<210> 157
<211> 38
<212> DNA
<213> Artificial Sequence

<220>
<223> Target Specific Oligonucleotide

<400> 157
agcgagccga gaactccggt aacatcgtta tagctaga 38

<210> 158
<211> 43
<212> DNA
<213> Artificial Sequence

<220>
<223> Target Specific Oligonucleotide

<400> 158
tattttgttc agacaacatg gctgtaacat cgttatagct aga 43

<210> 159
<211> 40
<212> DNA
<213> Artificial Sequence

<220>
<223> Target Specific Oligonucleotide

<400> 159
ccacttggta caacggagcc gtaacatcgt tatagctaga 40

<210> 160
<211> 40
<212> DNA
<213> Artificial Sequence

<220>
<223> Target Specific Oligonucleotide

<400> 160
cagaacacct gcgaggagag gtaacatcgt tatagctaga 40

<210> 161
<211> 39
<212> DNA
<213> Artificial Sequence

<220>
<223> Target Specific Oligonucleotide

<400> 161
ttcatcagcg acgcccctgg taacatcgtt atagctaga 39

<210> 162
<211> 41
<212> DNA
<213> Artificial Sequence

<220>
<223> Target Specific Oligonucleotide

<400> 162
cctgcaaaaa aacggtcacg tgtaacatcg ttatagctag a 41

<210> 163
<211> 41
<212> DNA
<213> Artificial Sequence

<220>
<223> Target Specific Oligonucleotide

<400> 163
tgtgggagtc ccttaggtca agtaacatcg ttatagctag a 41

<210> 164
<211> 38
<212> DNA
<213> Artificial Sequence

<220>
<223> Target Specific Oligonucleotide

<400> 164
gcatcgggag cacgcactgt aacatcgtta tagctaga 38

<210> 165
<211> 39
<212> DNA
<213> Artificial Sequence

<220>
<223> Target Specific Oligonucleotide

<400> 165
ggctctgcac aacgcttgcg taacatcgtt atagctaga 39

<210> 166
<211> 41
<212> DNA
<213> Artificial Sequence

<220>
<223> Target Specific Oligonucleotide

<400> 166
agccaggaca caatagtcag ggtaacatcg ttatagctag a 41

<210> 167
<211> 41
<212> DNA
<213> Artificial Sequence

<220>
<223> Target Specific Oligonucleotide

<400> 167
agccaggaca caatagtcag ggtaacatcg ttatagctag a 41

<210> 168
<211> 42
<212> DNA
<213> Artificial Sequence

<220>
<223> Target Specific Oligonucleotide

<400> 168
ttgcacatcc tacggtcttc tgtaacttac tacggagtta ac 42

<210> 169
<211> 43
<212> DNA
<213> Artificial Sequence

<220>
<223> Target Specific Oligonucleotide

<400> 169
gtagctcatt cgcaaatctt gggtaactta ctacggagtt aac 43

<210> 170
<211> 41
<212> DNA
<213> Artificial Sequence

<220>
<223> Target Specific Oligonucleotide

<400> 170
cctttaagcg cgtcgtgtcc gtaacttact acggagttaa c 41

<210> 171
<211> 39
<212> DNA
<213> Artificial Sequence

<220>
<223> Target Specific Oligonucleotide

<400> 171
cctcgacgca tgatgccggt aacttactac ggagttaac 39

<210> 172
<211> 40
<212> DNA
<213> Artificial Sequence

<220>
<223> Target Specific Oligonucleotide

<400> 172
gccccatggc tcgtgtaggg taacttacta cggagttaac 40

<210> 173
<211> 42
<212> DNA
<213> Artificial Sequence

<220>
<223> Target Specific Oligonucleotide

<400> 173
ccaattgtgt tccggcaagt tgtaacttac tacggagtta ac 42

<210> 174
<211> 43
<212> DNA
<213> Artificial Sequence

<220>

<223> Target Specific Oligonucleotide

<400> 174
gataggattt ggtcggaaac ctgtaactta ctacggagtt aac 43

<210> 175
<211> 41
<212> DNA
<213> Artificial Sequence

<220>
<223> Target Specific Oligonucleotide

<400> 175
tagttgccaa cggtgttgta gtaacttact acggagttaa c 41

<210> 176
<211> 42
<212> DNA
<213> Artificial Sequence

<220>
<223> Target Specific Oligonucleotide

<400> 176
cttggcctat gcggaagtaa cgtaacttac tacggagtta ac 42

<210> 177
<211> 41
<212> DNA
<213> Artificial Sequence

<220>
<223> Target Specific Oligonucleotide

<400> 177
ctcgcagcga taggaaccat gtaacttact acggagttaa c 41

<210> 178
<211> 44
<212> DNA
<213> Artificial Sequence

<220>
<223> Target Specific Oligonucleotide

<400> 178
tttgcattcg tccatatcaa ctggtaactt actacggagt taac 44

<210> 179
<211> 43
<212> DNA
<213> Artificial Sequence

<220>
<223> Target Specific Oligonucleotide

<400> 179
tcaatatcaa cgggtaaacc gggtaactta ctacggagtt aac 43

<210> 180
<211> 45
<212> DNA
<213> Artificial Sequence

<220>
<223> Target Specific Oligonucleotide

<400> 180
ttgttactga cgtgggaaat attggtaact tactacggag ttaac 45

<210> 181
<211> 40
<212> DNA
<213> Artificial Sequence

<220>
<223> Target Specific Oligonucleotide

<400> 181
tctccgctga tacccgggtg taacttacta cggagttaac 40

<210> 182
<211> 42
<212> DNA
<213> Artificial Sequence

<220>
<223> Target Specific Oligonucleotide

<400> 182
ggccagtcac cgaaatttca tgtaacttac tacggagtta ac 42

<210> 183
<211> 43
<212> DNA
<213> Artificial Sequence

<220>
<223> Target Specific Oligonucleotide

<400> 183
agttcgtagc ctatctcaca ctgtaactta ctacggagtt aac 43

<210> 184
<211> 42
<212> DNA

<213> Artificial Sequence

<220>
<223> Target Specific Oligonucleotide

<400> 184
cacattcccg tacgtttgct ggtaacttac tacggagtta ac 42

<210> 185
<211> 45
<212> DNA
<213> Artificial Sequence

<220>
<223> Target Specific Oligonucleotide

<400> 185
agtgagtccc tatgtatcct ttctgtaact tactacggag ttaac 45

<210> 186
<211> 44
<212> DNA
<213> Artificial Sequence

<220>
<223> Target Specific Oligonucleotide

<400> 186
aagcattata acgtgatcca caggtaactt actacggagt taac 44

<210> 187
<211> 38
<212> DNA
<213> Artificial Sequence

<220>
<223> Target Specific Oligonucleotide

<400> 187
tgcgccctga agcgcacgta acttactacg gagttaac 38

<210> 188
<211> 47
<212> DNA
<213> Artificial Sequence

<220>
<223> Target Specific Oligonucleotide

<400> 188
taaaaaatag cacgattaca gtatacgtaa cttactacgg agttaac 47

<210> 189

<211> 44
<212> DNA
<213> Artificial Sequence

<220>
<223> Target Specific Oligonucleotide

<400> 189
catatcgacg gattgagcct aacgtaactt actacggagt taac 44

<210> 190
<211> 42
<212> DNA
<213> Artificial Sequence

<220>
<223> Target Specific Oligonucleotide

<400> 190
cggtttctcg cgagagaaat agtaacttac tacggagtta ac 42

<210> 191
<211> 43
<212> DNA
<213> Artificial Sequence

<220>
<223> Target Specific Oligonucleotide

<400> 191
cttcccatct cgtgtaacat gagtaactta ctacggagtt aac 43

<210> 192
<211> 41
<212> DNA
<213> Artificial Sequence

<220>
<223> Target Specific Oligonucleotide

<400> 192
gcatttcaca tcggactgta ccgtaacttc gaaacggaaa c 41

<210> 193
<211> 38
<212> DNA
<213> Artificial Sequence

<220>
<223> Target Specific Oligonucleotide

<400> 193
tggaattctc ggcggaccag taacttcgaa acggaaac 38

<210> 194
<211> 41
<212> DNA
<213> Artificial Sequence

<220>
<223> Target Specific Oligonucleotide

<400> 194
gaagtggaaa tacgaccttt gcgtaacttc gaaacggaaa c 41

<210> 195
<211> 39
<212> DNA
<213> Artificial Sequence

<220>
<223> Target Specific Oligonucleotide

<400> 195
gctggtccct tgcgtaacat gtaacttcga aacggaaac 39

<210> 196
<211> 43
<212> DNA
<213> Artificial Sequence

<220>
<223> Target Specific Oligonucleotide

<400> 196
ttttcatcta ttcgagatgc tcccgtaact tcgaaacgga aac 43

<210> 197
<211> 40
<212> DNA
<213> Artificial Sequence

<220>
<223> Target Specific Oligonucleotide

<400> 197
tattcatccc gtggggtagt agtaacttcg aaacggaaac 40

<210> 198
<211> 42
<212> DNA
<213> Artificial Sequence

<220>
<223> Target Specific Oligonucleotide

<400> 198

taaaaatagg cccggatttg tctgtaactt cgaaacggaa ac 42

<210> 199
<211> 42
<212> DNA
<213> Artificial Sequence

<220>
<223> Target Specific Oligonucleotide

<400> 199
tccaaccttg acgacaagat agggtaactt cgaaacggaa ac 42

<210> 200
<211> 40
<212> DNA
<213> Artificial Sequence

<220>
<223> Target Specific Oligonucleotide

<400> 200
aggcaacagt cgaaccattc tgtaacttcg aaacggaaac 40

<210> 201
<211> 40
<212> DNA
<213> Artificial Sequence

<220>
<223> Target Specific Oligonucleotide

<400> 201
gcttcatttg cgactgctct tgtaacttcg aaacggaaac 40

<210> 202
<211> 41
<212> DNA
<213> Artificial Sequence

<220>
<223> Target Specific Oligonucleotide

<400> 202
catcaggacg cttgtattgg tggtaacttc gaaacggaaa c 41

<210> 203
<211> 41
<212> DNA
<213> Artificial Sequence

<220>
<223> Target Specific Oligonucleotide

<400> 203
atcttcaatc cgagaatcca gcgtaacttc gaaacggaaa c 41

<210> 204
<211> 42
<212> DNA
<213> Artificial Sequence

<220>
<223> Target Specific Oligonucleotide

<400> 204
ctgtagtgtg gtagtaagga agagtaactt cgaaacggaa ac 42

<210> 205
<211> 42
<212> DNA
<213> Artificial Sequence

<220>
<223> Target Specific Oligonucleotide

<400> 205
gaacacctta cttacaacac ctggtaactt cgaaacggaa ac 42

<210> 206
<211> 43
<212> DNA
<213> Artificial Sequence

<220>
<223> Target Specific Oligonucleotide

<400> 206
gtacattgta caccgttaca atgggtaact tcgaaacgga aac 43

<210> 207
<211> 42
<212> DNA
<213> Artificial Sequence

<220>
<223> Target Specific Oligonucleotide

<400> 207
tttcaagtcg tctatgttag ctggtaactt cgaaacggaa ac 42

<210> 208
<211> 44
<212> DNA
<213> Artificial Sequence

<220>
<223> Target Specific Oligonucleotide

<400> 208
caaagcaact atatgaagct tcattgtaac ttcgaaacgg aaac 44

<210> 209
<211> 42
<212> DNA
<213> Artificial Sequence

<220>
<223> Target Specific Oligonucleotide

<400> 209
catacttcta tcgagagctc agggtaactt cgaaacggaa ac 42

<210> 210
<211> 43
<212> DNA
<213> Artificial Sequence

<220>
<223> Target Specific Oligonucleotide

<400> 210
acgaagaaat cgagtaggtc tagggtaact tcgaaacgga aac 43

<210> 211
<211> 41
<212> DNA
<213> Artificial Sequence

<220>
<223> Target Specific Oligonucleotide

<400> 211
gaatcattga gtcgaccctt cagtaacttc gaaacggaaa c 41

<210> 212
<211> 41
<212> DNA
<213> Artificial Sequence

<220>
<223> Target Specific Oligonucleotide

<400> 212
tgatgtgctt aagtagtgca gcgtaacttc gaaacggaaa c 41

<210> 213
<211> 41
<212> DNA
<213> Artificial Sequence

<220>
<223> Target Specific Oligonucleotide

<400> 213
tccacggcat gatacataca acgtaacttc gaaacggaaa c 41

<210> 214
<211> 40
<212> DNA
<213> Artificial Sequence

<220>
<223> Target Specific Oligonucleotide

<400> 214
tctcagagca tcccgaatcc agtaacttcg aaacggaaac 40

<210> 215
<211> 42
<212> DNA
<213> Artificial Sequence

<220>
<223> Target Specific Oligonucleotide

<400> 215
gtggaacgaa gagtaggtag tttgtaactt cgaaacggaa ac 42

<210> 216
<211> 39
<212> DNA
<213> Artificial Sequence

<220>
<223> Target Specific Oligonucleotide

<400> 216
gatcaatact ggacggagtc aggtaacctc gtcggatca 39

<210> 217
<211> 38
<212> DNA
<213> Artificial Sequence

<220>
<223> Target Specific Oligonucleotide

<400> 217
gagcttgtta ctcgtgcctt ggtaacctcg tcggatca 38

<210> 218
<211> 37

<212> DNA
<213> Artificial Sequence

<220>
<223> Target Specific Oligonucleotide

<400> 218
cttcttacac ttgcggacgc gtaacctcgt cggatca 37

<210> 219
<211> 41
<212> DNA
<213> Artificial Sequence

<220>
<223> Target Specific Oligonucleotide

<400> 219
caatacctat tccgttacac acttgtaacc tcgtcggatc a 41

<210> 220
<211> 39
<212> DNA
<213> Artificial Sequence

<220>
<223> Target Specific Oligonucleotide

<400> 220
gctccttcag tccggtttta ttgtaacctc gtcggatca 39

<210> 221
<211> 36
<212> DNA
<213> Artificial Sequence

<220>
<223> Target Specific Oligonucleotide

<400> 221
gacgcacgag ccgtgatctg taacctcgtc ggatca 36

<210> 222
<211> 41
<212> DNA
<213> Artificial Sequence

<220>
<223> Target Specific Oligonucleotide

<400> 222
gactgctaag gcataggaat tttcgtaacc tcgtcggatc a 41

<210> 223
<211> 39
<212> DNA
<213> Artificial Sequence

<220>
<223> Target Specific Oligonucleotide

<400> 223
caccacataa ttacggggac acgtaacctc gtcggatca 39

<210> 224
<211> 34
<212> DNA
<213> Artificial Sequence

<220>
<223> Target Specific Oligonucleotide

<400> 224
cggcaaggcc cttcgcagta acctcgtcgg atca 34

<210> 225
<211> 37
<212> DNA
<213> Artificial Sequence

<220>
<223> Target Specific Oligonucleotide

<400> 225
tccatctggt acgtggtggg gtaacctcgt cggatca 37

<210> 226
<211> 38
<212> DNA
<213> Artificial Sequence

<220>
<223> Target Specific Oligonucleotide

<400> 226
gtcctgccgc gtatgatttc tgtaacctcg tcggatca 38

<210> 227
<211> 37
<212> DNA
<213> Artificial Sequence

<220>
<223> Target Specific Oligonucleotide

<400> 227
tgttcaggct gacgactgca gtaacctcgt cggatca 37

<210> 228
<211> 36
<212> DNA
<213> Artificial Sequence

<220>
<223> Target Specific Oligonucleotide

<400> 228
gcatggaggt ccgtcctgtg taacctcgtc ggatca 36

<210> 229
<211> 38
<212> DNA
<213> Artificial Sequence

<220>
<223> Target Specific Oligonucleotide

<400> 229
ttgagggagc gtaatcccaa ggtaacctcg tcggatca 38

<210> 230
<211> 36
<212> DNA
<213> Artificial Sequence

<220>
<223> Target Specific Oligonucleotide

<400> 230
ccggcgtctg cgtacttccg taacctcgtc ggatca 36

<210> 231
<211> 39
<212> DNA
<213> Artificial Sequence

<220>
<223> Target Specific Oligonucleotide

<400> 231
cgactatctg cgtctatcat ccgtaacctc gtcggatca 39

<210> 232
<211> 40
<212> DNA
<213> Artificial Sequence

<220>
<223> Target Specific Oligonucleotide

<400> 232
gagaattcga tgatcaactc acggtaacct cgtcggatca 40

<210> 233
<211> 35
<212> DNA
<213> Artificial Sequence

<220>
<223> Target Specific Oligonucleotide

<400> 233
tggcctgtcg tccggtctgt aacctcgtcg gatca 35

<210> 234
<211> 38
<212> DNA
<213> Artificial Sequence

<220>
<223> Target Specific Oligonucleotide

<400> 234
cagggattcc gtcatatggc tgtaacctcg tcggatca 38

<210> 235
<211> 40
<212> DNA
<213> Artificial Sequence

<220>
<223> Target Specific Oligonucleotide

<400> 235
agacatcgat ggtacatatg ggtgtaacct cgtcggatca 40

<210> 236
<211> 37
<212> DNA
<213> Artificial Sequence

<220>
<223> Target Specific Oligonucleotide

<400> 236
tctgagctgt atcgctgcaa gtaacctcgt cggatca 37

<210> 237
<211> 37
<212> DNA
<213> Artificial Sequence

<220>

<223> Target Specific Oligonucleotide

<400> 237
tcttaggccc attcgttgga gtaacctcgt cggatca 37

<210> 238
<211> 37
<212> DNA
<213> Artificial Sequence

<220>
<223> Target Specific Oligonucleotide

<400> 238
ttatacaccg tgccgaacgc gtaacctcgt cggatca 37

<210> 239
<211> 37
<212> DNA
<213> Artificial Sequence

<220>
<223> Target Specific Oligonucleotide

<400> 239
atgccctttg cgatctgcac gtaacctcgt cggatca 37

<210> 240
<211> 42
<212> DNA
<213> Artificial Sequence

<220>
<223> Target Specific Oligonucleotide

<400> 240
ggcagaattc cgaagttcag cgtaacataa tcgtagtttc gg 42

<210> 241
<211> 42
<212> DNA
<213> Artificial Sequence

<220>
<223> Target Specific Oligonucleotide

<400> 241
gtttccttca cgacaggtgt ggtaacataa tcgtagtttc gg 42

<210> 242
<211> 44
<212> DNA
<213> Artificial Sequence

<220>
<223> Target Specific Oligonucleotide

<400> 242
ctggtagaaa tgcgactaaa gacgtaacat aatcgtagtt tcgg 44

<210> 243
<211> 44
<212> DNA
<213> Artificial Sequence

<220>
<223> Target Specific Oligonucleotide

<400> 243
tgaccagcat cgtttcatct aatgtaacat aatcgtagtt tcgg 44

<210> 244
<211> 42
<212> DNA
<213> Artificial Sequence

<220>
<223> Target Specific Oligonucleotide

<400> 244
ttcgaagttc aaccgagtga cgtaacataa tcgtagtttc gg 42

<210> 245
<211> 42
<212> DNA
<213> Artificial Sequence

<220>
<223> Target Specific Oligonucleotide

<400> 245
ccaccaatcc aatgcggaat tgtaacataa tcgtagtttc gg 42

<210> 246
<211> 42
<212> DNA
<213> Artificial Sequence

<220>
<223> Target Specific Oligonucleotide

<400> 246
agactcggtg ccattcgtat tgtaacataa tcgtagtttc gg 42

<210> 247
<211> 42
<212> DNA

<213> Artificial Sequence

<220>
<223> Target Specific Oligonucleotide

<400> 247
ataactgcta actgcgcaac cgtaacataa tcgtagtttc gg 42

<210> 248
<211> 43
<212> DNA
<213> Artificial Sequence

<220>
<223> Target Specific Oligonucleotide

<400> 248
gtagtgaggc cgcttataac cagtaacata atcgtagttt cgg 43

<210> 249
<211> 43
<212> DNA
<213> Artificial Sequence

<220>
<223> Target Specific Oligonucleotide

<400> 249
ggtgatgatt cgatggagtg aagtaacata atcgtagttt cgg 43

<210> 250
<211> 42
<212> DNA
<213> Artificial Sequence

<220>
<223> Target Specific Oligonucleotide

<400> 250
ctgctgcttt acgtttggtg cgtaacataa tcgtagtttc gg 42

<210> 251
<211> 43
<212> DNA
<213> Artificial Sequence

<220>
<223> Target Specific Oligonucleotide

<400> 251
tcatcaacca cgtctttgga tagtaacata atcgtagttt cgg 43

<210> 252

<211> 44
<212> DNA
<213> Artificial Sequence

<220>
<223> Target Specific Oligonucleotide

<400> 252
aggcagatct taaagtgttg gttgtaacat aatcgtagtt tcgg 44

<210> 253
<211> 43
<212> DNA
<213> Artificial Sequence

<220>
<223> Target Specific Oligonucleotide

<400> 253
tgagggctta tacgaaagca aggtaacata atcgtagttt cgg 43

<210> 254
<211> 44
<212> DNA
<213> Artificial Sequence

<220>
<223> Target Specific Oligonucleotide

<400> 254
gctcagtact gactttggta tgtgtaacat aatcgtagtt tcgg 44

<210> 255
<211> 45
<212> DNA
<213> Artificial Sequence

<220>
<223> Target Specific Oligonucleotide

<400> 255
cagtcaatca ttagatccac atctgtaaca taatcgtagt ttcgg 45

<210> 256
<211> 40
<212> DNA
<213> Artificial Sequence

<220>
<223> Target Specific Oligonucleotide

<400> 256
ggaagctgct cgtcgaagcg taacataatc gtagtttcgg 40

<210> 257
<211> 47
<212> DNA
<213> Artificial Sequence

<220>
<223> Target Specific Oligonucleotide

<400> 257
gagaagatac ttatagcttc ttgtctgtaa cataatcgta gtttcgg 47

<210> 258
<211> 45
<212> DNA
<213> Artificial Sequence

<220>
<223> Target Specific Oligonucleotide

<400> 258
tcaatgctat ctaactgatg aagagtaaca taatcgtagt ttcgg 45

<210> 259
<211> 46
<212> DNA
<213> Artificial Sequence

<220>
<223> Target Specific Oligonucleotide

<400> 259
atcttcccaa ttgatgtaag tacttgtaac ataatcgtag tttcgg 46

<210> 260
<211> 40
<212> DNA
<213> Artificial Sequence

<220>
<223> Target Specific Oligonucleotide

<400> 260
catcacagtc cgagacgccg taacataatc gtagtttcgg 40

<210> 261
<211> 40
<212> DNA
<213> Artificial Sequence

<220>
<223> Target Specific Oligonucleotide

<400> 261

cattccaccg gcctgtgcgg taacataatc gtagtttcgg 40

<210> 262
<211> 44
<212> DNA
<213> Artificial Sequence

<220>
<223> Target Specific Oligonucleotide

<400> 262
ggacagcact actctagagt aaggtaacat aatcgtagtt tcgg 44

<210> 263
<211> 43
<212> DNA
<213> Artificial Sequence

<220>
<223> Target Specific Oligonucleotide

<400> 263
tgccaagtaa cttagcacac ccgtaacata atcgtagttt cgg 43

<210> 264
<211> 38
<212> DNA
<213> Artificial Sequence

<220>
<223> Target Specific Oligonucleotide

<400> 264
gtgccacctt tcaccgtgag taaccggtag aattacgg 38

<210> 265
<211> 38
<212> DNA
<213> Artificial Sequence

<220>
<223> Target Specific Oligonucleotide

<400> 265
ccgcaggtac gacttgcctg taaccggtag aattacgg 38

<210> 266
<211> 40
<212> DNA
<213> Artificial Sequence

<220>
<223> Target Specific Oligonucleotide

<400> 266
tgggcttcaa tcagatggtc agtaaccggt agaattacgg 40

<210> 267
<211> 41
<212> DNA
<213> Artificial Sequence

<220>
<223> Target Specific Oligonucleotide

<400> 267
agaacaccag ggtacgcata gtgtaaccgg tagaattacg g 41

<210> 268
<211> 38
<212> DNA
<213> Artificial Sequence

<220>
<223> Target Specific Oligonucleotide

<400> 268
tctccgagag tgtcagcggg taaccggtag aattacgg 38

<210> 269
<211> 38
<212> DNA
<213> Artificial Sequence

<220>
<223> Target Specific Oligonucleotide

<400> 269
gtcttcccgg ccggtcttgg taaccggtag aattacgg 38

<210> 270
<211> 39
<212> DNA
<213> Artificial Sequence

<220>
<223> Target Specific Oligonucleotide

<400> 270
catgttgcag attgtcgcca gtaaccggta gaattacgg 39

<210> 271
<211> 39
<212> DNA
<213> Artificial Sequence

<220>
<223> Target Specific Oligonucleotide

<400> 271
ggttcagtcg tttgcgaaca gtaaccggta gaattacgg 39

<210> 272
<211> 41
<212> DNA
<213> Artificial Sequence

<220>
<223> Target Specific Oligonucleotide

<400> 272
agctaccaat tagacccact cggtaaccgg tagaattacg g 41

<210> 273
<211> 40
<212> DNA
<213> Artificial Sequence

<220>
<223> Target Specific Oligonucleotide

<400> 273
cgtagttctc gtctccgatc agtaaccggt agaattacgg 40

<210> 274
<211> 39
<212> DNA
<213> Artificial Sequence

<220>
<223> Target Specific Oligonucleotide

<400> 274
ggagctgaac ttgacgccag gtaaccggta gaattacgg 39

<210> 275
<211> 40
<212> DNA
<213> Artificial Sequence

<220>
<223> Target Specific Oligonucleotide

<400> 275
taatggatgt actcgttggg cgtaaccggt agaattacgg 40

<210> 276
<211> 38
<212> DNA
<213> Artificial Sequence

<220>
<223> Target Specific Oligonucleotide

<400> 276
tctgtgcata gccgtcccgg taaccggtag aattacgg 38

<210> 277
<211> 38
<212> DNA
<213> Artificial Sequence

<220>
<223> Target Specific Oligonucleotide

<400> 277
tggctggacg gatcggatcg taaccggtag aattacgg 38

<210> 278
<211> 37
<212> DNA
<213> Artificial Sequence

<220>
<223> Target Specific Oligonucleotide

<400> 278
ggtttgcgtc gttgcggcgt aaccggtaga attacgg 37

<210> 279
<211> 39
<212> DNA
<213> Artificial Sequence

<220>
<223> Target Specific Oligonucleotide

<400> 279
tctctgggga cgtgacaaag gtaaccggta gaattacgg 39

<210> 280
<211> 42
<212> DNA
<213> Artificial Sequence

<220>
<223> Target Specific Oligonucleotide

<400> 280
gaattcatca gctagattgg caagtaaccg gtagaattac gg 42

<210> 281
<211> 38

<212> DNA
<213> Artificial Sequence

<220>
<223> Target Specific Oligonucleotide

<400> 281
tcactccgtg ggctaagcgg taaccggtag aattacgg 38

<210> 282
<211> 40
<212> DNA
<213> Artificial Sequence

<220>
<223> Target Specific Oligonucleotide

<400> 282
tgtaagggaa cacggaagtg ggtaaccggt agaattacgg 40

<210> 283
<211> 38
<212> DNA
<213> Artificial Sequence

<220>
<223> Target Specific Oligonucleotide

<400> 283
ctcaatggtg gcgcggatcg taaccggtag aattacgg 38

<210> 284
<211> 40
<212> DNA
<213> Artificial Sequence

<220>
<223> Target Specific Oligonucleotide

<400> 284
aaccactcaa tctgcgtctc ggtaaccggt agaattacgg 40

<210> 285
<211> 39
<212> DNA
<213> Artificial Sequence

<220>
<223> Target Specific Oligonucleotide

<400> 285
gctgtagggc gccattttgt gtaaccggta gaattacgg 39

<210> 286
<211> 38
<212> DNA
<213> Artificial Sequence

<220>
<223> Target Specific Oligonucleotide

<400> 286
tgagtgacgg cattgcgcag taaccggtag aattacgg 38

<210> 287
<211> 39
<212> DNA
<213> Artificial Sequence

<220>
<223> Target Specific Oligonucleotide

<400> 287
caaagtggct catcgccacc gtaaccggta gaattacgg 39

<210> 288
<211> 45
<212> DNA
<213> Artificial Sequence

<220>
<223> Target Specific Oligonucleotide

<400> 288
tctatatgct aaatgtattg ccatggtaac gcttctcata acact 45

<210> 289
<211> 43
<212> DNA
<213> Artificial Sequence

<220>
<223> Target Specific Oligonucleotide

<400> 289
ctatgaaact tcgtggtcac tccgtaacgc ttctcataac act 43

<210> 290
<211> 43
<212> DNA
<213> Artificial Sequence

<220>
<223> Target Specific Oligonucleotide

<400> 290
aactaactca tctgcagtac catgtaacgc ttctcataac act 43

<210> 291
<211> 43
<212> DNA
<213> Artificial Sequence

<220>
<223> Target Specific Oligonucleotide

<400> 291
aaaacctgct tgatccacat tctgtaacgc ttctcataac act 43

<210> 292
<211> 45
<212> DNA
<213> Artificial Sequence

<220>
<223> Target Specific Oligonucleotide

<400> 292
acattaacaa atcactcttg attcagtaac gcttctcata acact 45

<210> 293
<211> 43
<212> DNA
<213> Artificial Sequence

<220>
<223> Target Specific Oligonucleotide

<400> 293
ttttgcttag ctcatggtaa acagtaacgc ttctcataac act 43

<210> 294
<211> 45
<212> DNA
<213> Artificial Sequence

<220>
<223> Target Specific Oligonucleotide

<400> 294
gaggagctgt tggataaata attttgtaac gcttctcata acact 45

<210> 295
<211> 43
<212> DNA
<213> Artificial Sequence

<220>
<223> Target Specific Oligonucleotide

<400> 295
aagcaggcag gtattgtatg attgtaacgc ttctcataac act 43

<210> 296
<211> 45
<212> DNA
<213> Artificial Sequence

<220>
<223> Target Specific Oligonucleotide

<400> 296
gcaacttata tatcagtggt gaatggtaac gcttctcata acact 45

<210> 297
<211> 45
<212> DNA
<213> Artificial Sequence

<220>
<223> Target Specific Oligonucleotide

<400> 297
agattatgta tgcatgagaa ccaacgtaac gcttctcata acact 45

<210> 298
<211> 45
<212> DNA
<213> Artificial Sequence

<220>
<223> Target Specific Oligonucleotide

<400> 298
gatcatgact ctccttatgg ttaatgtaac gcttctcata acact 45

<210> 299
<211> 43
<212> DNA
<213> Artificial Sequence

<220>
<223> Target Specific Oligonucleotide

<400> 299
tgtgaaaccg gtttataaac ctagtaacgc ttctcataac act 43

<210> 300
<211> 46
<212> DNA
<213> Artificial Sequence

<220>

<223> Target Specific Oligonucleotide

<400> 300
aataagatta gctactgtct acagtggtaa cgcttctcat aacact 46

<210> 301
<211> 45
<212> DNA
<213> Artificial Sequence

<220>
<223> Target Specific Oligonucleotide

<400> 301
taaacaaata gtgatacatc cacacgtaac gcttctcata acact 45

<210> 302
<211> 45
<212> DNA
<213> Artificial Sequence

<220>
<223> Target Specific Oligonucleotide

<400> 302
ttcagtcatc taacaatgta ttcctgtaac gcttctcata acact 45

<210> 303
<211> 43
<212> DNA
<213> Artificial Sequence

<220>
<223> Target Specific Oligonucleotide

<400> 303
agctgtggga aagtctttaa ctcgtaacgc ttctcataac act 43

<210> 304
<211> 46
<212> DNA
<213> Artificial Sequence

<220>
<223> Target Specific Oligonucleotide

<400> 304
atagatgaac tattgatgta cacaacgtaa cgcttctcat aacact 46

<210> 305
<211> 45
<212> DNA
<213> Artificial Sequence

<220>
<223> Target Specific Oligonucleotide

<400> 305
tacagatgaa attgagacct aaaacgtaac gcttctcata acact 45

<210> 306
<211> 45
<212> DNA
<213> Artificial Sequence

<220>
<223> Target Specific Oligonucleotide

<400> 306
agcaatttgg gttaacagaa atagagtaac gcttctcata acact 45

<210> 307
<211> 46
<212> DNA
<213> Artificial Sequence

<220>
<223> Target Specific Oligonucleotide

<400> 307
aatccaatgg gataagtact attagtgtaa cgcttctcat aacact 46

<210> 308
<211> 45
<212> DNA
<213> Artificial Sequence

<220>
<223> Target Specific Oligonucleotide

<400> 308
aatgttattc cgtggaaaat tacatgtaac gcttctcata acact 45

<210> 309
<211> 47
<212> DNA
<213> Artificial Sequence

<220>
<223> Target Specific Oligonucleotide

<400> 309
ttacagaatt gtgtctgaaa aattatggta acgcttctca taacact 47

<210> 310
<211> 40
<212> DNA

<213> Artificial Sequence

<220>
<223> Target Specific Oligonucleotide

<400> 310
atcagagagg cgctatgcct gtaacgcttc tcataacact 40

<210> 311
<211> 38
<212> DNA
<213> Artificial Sequence

<220>
<223> Target Specific Oligonucleotide

<400> 311
cggcgacacg atacagcggt aacgcttctc ataacact 38

<210> 312
<211> 40
<212> DNA
<213> Artificial Sequence

<220>
<223> Target Specific Oligonucleotide

<400> 312
cacttcaaat gcgcaacaag cgtaacgtcg ctgaaaaatc 40

<210> 313
<211> 39
<212> DNA
<213> Artificial Sequence

<220>
<223> Target Specific Oligonucleotide

<400> 313
gtaagcactg cgcaagacaa gtaacgtcgc tgaaaaatc 39

<210> 314
<211> 41
<212> DNA
<213> Artificial Sequence

<220>
<223> Target Specific Oligonucleotide

<400> 314
gtgatgagct cgacaggata ttgtaacgtc gctgaaaaat c 41

<210> 315

<211> 37
<212> DNA
<213> Artificial Sequence

<220>
<223> Target Specific Oligonucleotide

<400> 315
atggcgtcgt cggcacacgt aacgtcgctg aaaaatc 37

<210> 316
<211> 40
<212> DNA
<213> Artificial Sequence

<220>
<223> Target Specific Oligonucleotide

<400> 316
tttacaacag cgtggcaagt ggtaacgtcg ctgaaaaatc 40

<210> 317
<211> 40
<212> DNA
<213> Artificial Sequence

<220>
<223> Target Specific Oligonucleotide

<400> 317
aggcatgaag tgagacaatg cgtaacgtcg ctgaaaaatc 40

<210> 318
<211> 41
<212> DNA
<213> Artificial Sequence

<220>
<223> Target Specific Oligonucleotide

<400> 318
cctggctagt ggtatatgtc acgtaacgtc gctgaaaaat c 41

<210> 319
<211> 39
<212> DNA
<213> Artificial Sequence

<220>
<223> Target Specific Oligonucleotide

<400> 319
gctgatgatg ttcaagcgca gtaacgtcgc tgaaaaatc 39

<210> 320
<211> 41
<212> DNA
<213> Artificial Sequence

<220>
<223> Target Specific Oligonucleotide

<400> 320
ctgccagtta gttaggcaag ttgtaacgtc gctgaaaaat c 41

<210> 321
<211> 38
<212> DNA
<213> Artificial Sequence

<220>
<223> Target Specific Oligonucleotide

<400> 321
gcagctccgg gctacaagtg taacgtcgct gaaaaatc 38

<210> 322
<211> 40
<212> DNA
<213> Artificial Sequence

<220>
<223> Target Specific Oligonucleotide

<400> 322
tggcagctgt ctaactggag cgtaacgtcg ctgaaaaatc 40

<210> 323
<211> 38
<212> DNA
<213> Artificial Sequence

<220>
<223> Target Specific Oligonucleotide

<400> 323
acgcgtgtgc gagtagatgg taacgtcgct gaaaaatc 38

<210> 324
<211> 38
<212> DNA
<213> Artificial Sequence

<220>
<223> Target Specific Oligonucleotide

<400> 324

agctccacga aggatgccag taacgtcgct gaaaaatc 38

<210> 325
<211> 39
<212> DNA
<213> Artificial Sequence

<220>
<223> Target Specific Oligonucleotide

<400> 325
tgaggacggc atcgagatcc gtaacgtcgc tgaaaaatc 39

<210> 326
<211> 39
<212> DNA
<213> Artificial Sequence

<220>
<223> Target Specific Oligonucleotide

<400> 326
gtcttgagac ccggtcttgg gtaacgtcgc tgaaaaatc 39

<210> 327
<211> 38
<212> DNA
<213> Artificial Sequence

<220>
<223> Target Specific Oligonucleotide

<400> 327
agtggtctgg atcggtgcgg taacgtcgct gaaaaatc 38

<210> 328
<211> 39
<212> DNA
<213> Artificial Sequence

<220>
<223> Target Specific Oligonucleotide

<400> 328
tccattctgg gtcgagtgga gtaacgtcgc tgaaaaatc 39

<210> 329
<211> 39
<212> DNA
<213> Artificial Sequence

<220>
<223> Target Specific Oligonucleotide

<400> 329
ggaccccaag gtgttccaag gtaacgtcgc tgaaaatc 39

<210> 330
<211> 38
<212> DNA
<213> Artificial Sequence

<220>
<223> Target Specific Oligonucleotide

<400> 330
agcagcttgg ctactccccg taacgtcgct gaaaaatc 38

<210> 331
<211> 42
<212> DNA
<213> Artificial Sequence

<220>
<223> Target Specific Oligonucleotide

<400> 331
tttgggtatg ggtactgtgt agagtaacgt cgctgaaaaa tc 42

<210> 332
<211> 39
<212> DNA
<213> Artificial Sequence

<220>
<223> Target Specific Oligonucleotide

<400> 332
gccgcaggga tagatccagg gtaacgtcgc tgaaaaatc 39

<210> 333
<211> 42
<212> DNA
<213> Artificial Sequence

<220>
<223> Target Specific Oligonucleotide

<400> 333
tgaagacagt cctatggact tccgtaacgt cgctgaaaaa tc 42

<210> 334
<211> 37
<212> DNA
<213> Artificial Sequence

<220>
<223> Target Specific Oligonucleotide

<400> 334
aagctgaagc gcgggtcagt aacgtcgctg aaaaatc 37

<210> 335
<211> 37
<212> DNA
<213> Artificial Sequence

<220>
<223> Target Specific Oligonucleotide

<400> 335
ccacgcagcc cttcgagagt aacgtcgctg aaaaatc 37

<210> 336
<211> 45
<212> DNA
<213> Artificial Sequence

<220>
<223> Target Specific Oligonucleotide

<400> 336
tagtaggtgt cgacaactag agcgtaactc ttcaagatta atacc 45

<210> 337
<211> 44
<212> DNA
<213> Artificial Sequence

<220>
<223> Target Specific Oligonucleotide

<400> 337
gatggtcctg atcgagaaac cagtaactct tcaagattaa tacc 44

<210> 338
<211> 44
<212> DNA
<213> Artificial Sequence

<220>
<223> Target Specific Oligonucleotide

<400> 338
aagaggactt cgctgaattg acgtaactct tcaagattaa tacc 44

<210> 339
<211> 44
<212> DNA
<213> Artificial Sequence

<220>
<223> Target Specific Oligonucleotide

<400> 339
gaacctccga ctgtatgtca gcgtaactct tcaagattaa tacc 44

<210> 340
<211> 45
<212> DNA
<213> Artificial Sequence

<220>
<223> Target Specific Oligonucleotide

<400> 340
tctgaattag agcgatgttg acagtaactc ttcaagatta atacc 45

<210> 341
<211> 41
<212> DNA
<213> Artificial Sequence

<220>
<223> Target Specific Oligonucleotide

<400> 341
gcattcctcc gatcgcacag taactcttca agattaatac c 41

<210> 342
<211> 46
<212> DNA
<213> Artificial Sequence

<220>
<223> Target Specific Oligonucleotide

<400> 342
ctctatattt agctcgctgt tcaagtaact cttcaagatt aatacc 46

<210> 343
<211> 41
<212> DNA
<213> Artificial Sequence

<220>
<223> Target Specific Oligonucleotide

<400> 343
tattcatcac ggcgcgcttg taactcttca agattaatac c 41

<210> 344
<211> 43

<212> DNA
<213> Artificial Sequence

<220>
<223> Target Specific Oligonucleotide

<400> 344
cattggggac cgtgcataaa agtaactctt caagattaat acc 43

<210> 345
<211> 43
<212> DNA
<213> Artificial Sequence

<220>
<223> Target Specific Oligonucleotide

<400> 345
caaggggtgc actatttggg agtaactctt caagattaat acc 43

<210> 346
<211> 43
<212> DNA
<213> Artificial Sequence

<220>
<223> Target Specific Oligonucleotide

<400> 346
ggaccgtatt tcggcgaaat agtaactctt caagattaat acc 43

<210> 347
<211> 43
<212> DNA
<213> Artificial Sequence

<220>
<223> Target Specific Oligonucleotide

<400> 347
atactgcact tgtcggcatg agtaactctt caagattaat acc 43

<210> 348
<211> 43
<212> DNA
<213> Artificial Sequence

<220>
<223> Target Specific Oligonucleotide

<400> 348
atggcagatc gatccattgg tgtaactctt caagattaat acc 43

<210> 349
<211> 43
<212> DNA
<213> Artificial Sequence

<220>
<223> Target Specific Oligonucleotide

<400> 349
tccctttgga ccgtcaagaa ggtaactctt caagattaat acc 43

<210> 350
<211> 42
<212> DNA
<213> Artificial Sequence

<220>
<223> Target Specific Oligonucleotide

<400> 350
aggcttgctg acatacgcag gtaactcttc aagattaata cc 42

<210> 351
<211> 42
<212> DNA
<213> Artificial Sequence

<220>
<223> Target Specific Oligonucleotide

<400> 351
cttgctttgt gcgaacaccc gtaactcttc aagattaata cc 42

<210> 352
<211> 45
<212> DNA
<213> Artificial Sequence

<220>
<223> Target Specific Oligonucleotide

<400> 352
ctgatatcga atgcaatgga tgagtaactc ttcaagatta atacc 45

<210> 353
<211> 43
<212> DNA
<213> Artificial Sequence

<220>
<223> Target Specific Oligonucleotide

<400> 353
taggactggt ccgtcaaaaa cgtaactctt caagattaat acc 43

<210> 354
<211> 44
<212> DNA
<213> Artificial Sequence

<220>
<223> Target Specific Oligonucleotide

<400> 354
tgaccattct cgggacacta acgtaactct tcaagattaa tacc 44

<210> 355
<211> 44
<212> DNA
<213> Artificial Sequence

<220>
<223> Target Specific Oligonucleotide

<400> 355
atgattgggt ccgtaaaaat gcgtaactct tcaagattaa tacc 44

<210> 356
<211> 45
<212> DNA
<213> Artificial Sequence

<220>
<223> Target Specific Oligonucleotide

<400> 356
gtttcatgta tggtaggacc accgtaactc ttcaagatta atacc 45

<210> 357
<211> 43
<212> DNA
<213> Artificial Sequence

<220>
<223> Target Specific Oligonucleotide

<400> 357
ctcttgcatc gtagcgaact agtaactctt caagattaat acc 43

<210> 358
<211> 43
<212> DNA
<213> Artificial Sequence

<220>
<223> Target Specific Oligonucleotide

<400> 358
atgatgattc cctcggtcag agtaactctt caagattaat acc 43

<210> 359
<211> 46
<212> DNA
<213> Artificial Sequence

<220>
<223> Target Specific Oligonucleotide

<400> 359
agtagagaag atcgctgata tccggtaact cttcaagatt aatacc 46

<210> 360
<211> 42
<212> DNA
<213> Artificial Sequence

<220>
<223> Target Specific Oligonucleotide

<400> 360
aacccttgac atcgccagtt tgtaacgtac cgtttgtata tg 42

<210> 361
<211> 45
<212> DNA
<213> Artificial Sequence

<220>
<223> Target Specific Oligonucleotide

<400> 361
gaaggttaaa cgagatttcc aaaggtaacg taccgtttgt atatg 45

<210> 362
<211> 41
<212> DNA
<213> Artificial Sequence

<220>
<223> Target Specific Oligonucleotide

<400> 362
gccactcgac atttctgccg gtaacgtacc gtttgtatat g 41

<210> 363
<211> 42
<212> DNA
<213> Artificial Sequence

<220>

<223> Target Specific Oligonucleotide

<400> 363
tcatactgtc cgcaacatcc ggtaacgtac cgtttgtata tg 42

<210> 364
<211> 41
<212> DNA
<213> Artificial Sequence

<220>
<223> Target Specific Oligonucleotide

<400> 364
gccactcgac atttctgccg gtaacgtacc gtttgtatat g 41

<210> 365
<211> 42
<212> DNA
<213> Artificial Sequence

<220>
<223> Target Specific Oligonucleotide

<400> 365
tcatactgtc cgcaacatcc ggtaacgtac cgtttgtata tg 42

<210> 366
<211> 43
<212> DNA
<213> Artificial Sequence

<220>
<223> Target Specific Oligonucleotide

<400> 366
tgaattttgc ccgaacttca ctgtaacgta ccgtttgtat atg 43

<210> 367
<211> 38
<212> DNA
<213> Artificial Sequence

<220>
<223> Target Specific Oligonucleotide

<400> 367
acacactcgg agcgcgcgta acgtaccgtt tgtatatg 38

<210> 368
<211> 43
<212> DNA
<213> Artificial Sequence

<220>
<223> Target Specific Oligonucleotide

<400> 368
caaacctata tgcccgttga ctgtaacgta ccgtttgtat atg 43

<210> 369
<211> 44
<212> DNA
<213> Artificial Sequence

<220>
<223> Target Specific Oligonucleotide

<400> 369
caggaacctt taccatgttc atggtaacgt accgtttgta tatg 44

<210> 370
<211> 46
<212> DNA
<213> Artificial Sequence

<220>
<223> Target Specific Oligonucleotide

<400> 370
gaaaatctct acggatgaat ttcttgtaac gtaccgtttg tatatg 46

<210> 371
<211> 43
<212> DNA
<213> Artificial Sequence

<220>
<223> Target Specific Oligonucleotide

<400> 371
gctgctaaga tagccttgtg aggtaacgta ccgtttgtat atg 43

<210> 372
<211> 42
<212> DNA
<213> Artificial Sequence

<220>
<223> Target Specific Oligonucleotide

<400> 372
tggcactaaa aaccggagaa cgtaacgtac cgtttgtata tg 42

<210> 373
<211> 39
<212> DNA

<213> Artificial Sequence

<220>
<223> Target Specific Oligonucleotide

<400> 373
tgcgggaccg ccgatacagt aacgtaccgt ttgtatatg 39

<210> 374
<211> 45
<212> DNA
<213> Artificial Sequence

<220>
<223> Target Specific Oligonucleotide

<400> 374
ggaaattaaa cggagtctta caacgtaacg taccgtttgt atatg 45

<210> 375
<211> 40
<212> DNA
<213> Artificial Sequence

<220>
<223> Target Specific Oligonucleotide

<400> 375
agccagagag cggtatgccg taacgtaccg tttgtatatg 40

<210> 376
<211> 41
<212> DNA
<213> Artificial Sequence

<220>
<223> Target Specific Oligonucleotide

<400> 376
ccaccaagtg gggatgtgac gtaacgtacc gtttgtatat g 41

<210> 377
<211> 42
<212> DNA
<213> Artificial Sequence

<220>
<223> Target Specific Oligonucleotide

<400> 377
cacacatgac ctttaagcgc tgtaacgtac cgtttgtata tg 42

<210> 378

<211> 41
<212> DNA
<213> Artificial Sequence

<220>
<223> Target Specific Oligonucleotide

<400> 378
tccacagatt gcgctgtcta gtaacgtacc gtttgtatat g 41

<210> 379
<211> 41
<212> DNA
<213> Artificial Sequence

<220>
<223> Target Specific Oligonucleotide

<400> 379
ctccacggac aggttactgc gtaacgtacc gtttgtatat g 41

<210> 380
<211> 39
<212> DNA
<213> Artificial Sequence

<220>
<223> Target Specific Oligonucleotide

<400> 380
gcgtcatggc gtcagcacgt aacgtaccgt ttgtatatg 39

<210> 381
<211> 39
<212> DNA
<213> Artificial Sequence

<220>
<223> Target Specific Oligonucleotide

<400> 381
tcctggccgg caacacacgt aacgtaccgt ttgtatatg 39

<210> 382
<211> 42
<212> DNA
<213> Artificial Sequence

<220>
<223> Target Specific Oligonucleotide

<400> 382
agccattttg tcgaggtttg ggtaacgtac cgtttgtata tg 42

<210> 383
<211> 44
<212> DNA
<213> Artificial Sequence

<220>
<223> Target Specific Oligonucleotide

<400> 383
catccgaagc atgatagttg atggtaacgt accgtttgta tatg 44

<210> 384
<211> 43
<212> DNA
<213> Artificial Sequence

<220>
<223> Target Specific Oligonucleotide

<400> 384
tagtgtgttg cgtatcaagt atctagacga tgcgaattaa cac 43

<210> 385
<211> 45
<212> DNA
<213> Artificial Sequence

<220>
<223> Target Specific Oligonucleotide

<400> 385
ttattgtgaa ctctagacat gagagtagac gatgcgaatt aacac 45

<210> 386
<211> 46
<212> DNA
<213> Artificial Sequence

<220>
<223> Target Specific Oligonucleotide

<400> 386
acagtgaata ctaagactgt aaaaactaga cgatgcgaat taacac 46

<210> 387
<211> 41
<212> DNA
<213> Artificial Sequence

<220>
<223> Target Specific Oligonucleotide

<400> 387

acacaaggca cgtagaaaca gtagacgatg cgaattaaca c 41

<210> 388
<211> 41
<212> DNA
<213> Artificial Sequence

<220>
<223> Target Specific Oligonucleotide

<400> 388
caatggacag cggttgtgaa atagacgatg cgaattaaca c 41

<210> 389
<211> 42
<212> DNA
<213> Artificial Sequence

<220>
<223> Target Specific Oligonucleotide

<400> 389
caccatcacc tatcgacaga gttagacgat gcgaattaac ac 42

<210> 390
<211> 42
<212> DNA
<213> Artificial Sequence

<220>
<223> Target Specific Oligonucleotide

<400> 390
cattgcatta cgttccacat ggtagacgat gcgaattaac ac 42

<210> 391
<211> 41
<212> DNA
<213> Artificial Sequence

<220>
<223> Target Specific Oligonucleotide

<400> 391
acacagagtg tccgataccc atagacgatg cgaattaaca c 41

<210> 392
<211> 43
<212> DNA
<213> Artificial Sequence

<220>
<223> Target Specific Oligonucleotide

<400> 392
ggtgattaca ttgagtgcta ggatagacga tgcgaattaa cac 43

<210> 393
<211> 41
<212> DNA
<213> Artificial Sequence

<220>
<223> Target Specific Oligonucleotide

<400> 393
tccacataag cgttcaaggt atagacgatg cgaattaaca c 41

<210> 394
<211> 44
<212> DNA
<213> Artificial Sequence

<220>
<223> Target Specific Oligonucleotide

<400> 394
cagaataatc gctattccta gctgtagacg atgcgaatta acac 44

<210> 395
<211> 40
<212> DNA
<213> Artificial Sequence

<220>
<223> Target Specific Oligonucleotide

<400> 395
ttacactcat acgtcgccgg tagacgatgc gaattaacac 40

<210> 396
<211> 43
<212> DNA
<213> Artificial Sequence

<220>
<223> Target Specific Oligonucleotide

<400> 396
tggccataaa ggctacttac aattagacga tgcgaattaa cac 43

<210> 397
<211> 45
<212> DNA
<213> Artificial Sequence

<220>
<223> Target Specific Oligonucleotide

<400> 397
tttcaagtta ttcgatctgc taacctagac gatgcgaatt aacac 45

<210> 398
<211> 45
<212> DNA
<213> Artificial Sequence

<220>
<223> Target Specific Oligonucleotide

<400> 398
agttagtcaa ggactttact aaggttagac gatgcgaatt aacac 45

<210> 399
<211> 42
<212> DNA
<213> Artificial Sequence

<220>
<223> Target Specific Oligonucleotide

<400> 399
ttacctcaaa tacgggctac catagacgat gcgaattaac ac 42

<210> 400
<211> 47
<212> DNA
<213> Artificial Sequence

<220>
<223> Target Specific Oligonucleotide

<400> 400
gagttatatt actctaacta aagccagtag acgatgcgaa ttaacac 47

<210> 401
<211> 43
<212> DNA
<213> Artificial Sequence

<220>
<223> Target Specific Oligonucleotide

<400> 401
ttgtcaacac gcataaaatc tgctagacga tgcgaattaa cac 43

<210> 402
<211> 43
<212> DNA
<213> Artificial Sequence

<220>
<223> Target Specific Oligonucleotide

<400> 402
tcatatacaa tcggggatct gagtagacga tgcgaattaa cac 43

<210> 403
<211> 43
<212> DNA
<213> Artificial Sequence

<220>
<223> Target Specific Oligonucleotide

<400> 403
cccacaaaaa tacccgtaag ttatagacga tgcgaattaa cac 43

<210> 404
<211> 47
<212> DNA
<213> Artificial Sequence

<220>
<223> Target Specific Oligonucleotide

<400> 404
cactaaatta ttacgaattt tgcaaagtag acgatgcgaa ttaacac 47

<210> 405
<211> 45
<212> DNA
<213> Artificial Sequence

<220>
<223> Target Specific Oligonucleotide

<400> 405
tcaaataaaa accgtcaaaa gtttatagac gatgcgaatt aacac 45

<210> 406
<211> 42
<212> DNA
<213> Artificial Sequence

<220>
<223> Target Specific Oligonucleotide

<400> 406
agtgcaatgg tatcacgtac tgtagacgat gcgaattaac ac 42

<210> 407
<211> 43

<212> DNA
<213> Artificial Sequence

<220>
<223> Target Specific Oligonucleotide

<400> 407
atgtgtcttg caattggatt ccctagacga tgcgaattaa cac 43

<210> 408
<211> 38
<212> DNA
<213> Artificial Sequence

<220>
<223> Target Specific Oligonucleotide

<400> 408
ggtgtgcgcg tcgtacacta gacccgttat aagtgttg 38

<210> 409
<211> 40
<212> DNA
<213> Artificial Sequence

<220>
<223> Target Specific Oligonucleotide

<400> 409
gtgtttaggg tcgcggttga tagacccgtt ataagtgttg 40

<210> 410
<211> 42
<212> DNA
<213> Artificial Sequence

<220>
<223> Target Specific Oligonucleotide

<400> 410
gagaatggcg aagtaaatgc cctagacccg ttataagtgt tg 42

<210> 411
<211> 42
<212> DNA
<213> Artificial Sequence

<220>
<223> Target Specific Oligonucleotide

<400> 411
gtagatggaa tagacacggc tgtagacccg ttataagtgt tg 42

<210> 412
<211> 43
<212> DNA
<213> Artificial Sequence

<220>
<223> Target Specific Oligonucleotide

<400> 412
gctcttaatg catggtacaa ctgtagaccc gttataagtg ttg 43

<210> 413
<211> 43
<212> DNA
<213> Artificial Sequence

<220>
<223> Target Specific Oligonucleotide

<400> 413
aaaccagtat ttcgtcacag tgatagaccc gttataagtg ttg 43

<210> 414
<211> 42
<212> DNA
<213> Artificial Sequence

<220>
<223> Target Specific Oligonucleotide

<400> 414
gatgggaacg gtgtagagat gttagacccg ttataagtgt tg 42

<210> 415
<211> 42
<212> DNA
<213> Artificial Sequence

<220>
<223> Target Specific Oligonucleotide

<400> 415
ttccaaatgc cgtcaaaact gttagacccg ttataagtgt tg 42

<210> 416
<211> 39
<212> DNA
<213> Artificial Sequence

<220>
<223> Target Specific Oligonucleotide

<400> 416
tgggtcacag acggtgtggt agacccgtta taagtgttg 39

<210> 417
<211> 46
<212> DNA
<213> Artificial Sequence

<220>
<223> Target Specific Oligonucleotide

<400> 417
aatcaggtat acttctatcc ttgaaataga cccgttataa gtgttg 46

<210> 418
<211> 43
<212> DNA
<213> Artificial Sequence

<220>
<223> Target Specific Oligonucleotide

<400> 418
tgctgtatta gcaacttgga acttagaccc gttataagtg ttg 43

<210> 419
<211> 42
<212> DNA
<213> Artificial Sequence

<220>
<223> Target Specific Oligonucleotide

<400> 419
gccaataaag cgatggttga tctagacccg ttataagtgt tg 42

<210> 420
<211> 43
<212> DNA
<213> Artificial Sequence

<220>
<223> Target Specific Oligonucleotide

<400> 420
gtaaggatca ctggatccta ctgtagaccc gttataagtg ttg 43

<210> 421
<211> 43
<212> DNA
<213> Artificial Sequence

<220>
<223> Target Specific Oligonucleotide

<400> 421
gacctcattc agttgatgag aggtagaccc gttataagtg ttg 43

<210> 422
<211> 41
<212> DNA
<213> Artificial Sequence

<220>
<223> Target Specific Oligonucleotide

<400> 422
gcataacaga cggttgcaag ttagacccgt tataagtgtt g 41

<210> 423
<211> 41
<212> DNA
<213> Artificial Sequence

<220>
<223> Target Specific Oligonucleotide

<400> 423
acctaaagcg agttgctgag ttagacccgt tataagtgtt g 41

<210> 424
<211> 41
<212> DNA
<213> Artificial Sequence

<220>
<223> Target Specific Oligonucleotide

<400> 424
gagtcagaaa cacgcatgga atagacccgt tataagtgtt g 41

<210> 425
<211> 42
<212> DNA
<213> Artificial Sequence

<220>
<223> Target Specific Oligonucleotide

<400> 425
agcaactctg ataggctcac actagacccg ttataagtgt tg 42

<210> 426
<211> 44
<212> DNA
<213> Artificial Sequence

<220>

<223> Target Specific Oligonucleotide

<400> 426
ccatacccac tacggataaa gatgtagacc cgttataagt gttg 44

<210> 427
<211> 42
<212> DNA
<213> Artificial Sequence

<220>
<223> Target Specific Oligonucleotide

<400> 427
gctttctgta cgactcaggt tttagacccg ttataagtgt tg 42

<210> 428
<211> 42
<212> DNA
<213> Artificial Sequence

<220>
<223> Target Specific Oligonucleotide

<400> 428
caatgttgag ccactaaacc actagacccg ttataagtgt tg 42

<210> 429
<211> 42
<212> DNA
<213> Artificial Sequence

<220>
<223> Target Specific Oligonucleotide

<400> 429
atgcttggat taggtccaaa gctagacccg ttataagtgt tg 42

<210> 430
<211> 41
<212> DNA
<213> Artificial Sequence

<220>
<223> Target Specific Oligonucleotide

<400> 430
cccggtgatg gattagtttg gtagacccgt tataagtgtt g 41

<210> 431
<211> 41
<212> DNA
<213> Artificial Sequence

<220>
<223> Target Specific Oligonucleotide

<400> 431
ccatggccat accctggaat ttagacccgt tataagtgtt g 41

<210> 432
<211> 41
<212> DNA
<213> Artificial Sequence

<220>
<223> Target Specific Oligonucleotide

<400> 432
attatactgt ccagcgtagg tggtagacaa ccgtcgttaa g 41

<210> 433
<211> 41
<212> DNA
<213> Artificial Sequence

<220>
<223> Target Specific Oligonucleotide

<400> 433
tcctggtaag gataggtacc atgtagacaa ccgtcgttaa g 41

<210> 434
<211> 41
<212> DNA
<213> Artificial Sequence

<220>
<223> Target Specific Oligonucleotide

<400> 434
cactgaaata ggacggaatc tgctagacaa ccgtcgttaa g 41

<210> 435
<211> 39
<212> DNA
<213> Artificial Sequence

<220>
<223> Target Specific Oligonucleotide

<400> 435
aaagtttatt ggcgcttgcc gtagacaacc gtcgttaag 39

<210> 436
<211> 39
<212> DNA

<213> Artificial Sequence

<220>
<223> Target Specific Oligonucleotide

<400> 436
gcaatgccat tagcgatacg atagacaacc gtcgttaag 39

<210> 437
<211> 38
<212> DNA
<213> Artificial Sequence

<220>
<223> Target Specific Oligonucleotide

<400> 437
tgattgaaca ccacgcgaca tagacaaccg tcgttaag 38

<210> 438
<211> 40
<212> DNA
<213> Artificial Sequence

<220>
<223> Target Specific Oligonucleotide

<400> 438
agtcttcgaa gcactattgc catagacaac cgtcgttaag 40

<210> 439
<211> 39
<212> DNA
<213> Artificial Sequence

<220>
<223> Target Specific Oligonucleotide

<400> 439
tcaatggttg atcggcctct ctagacaacc gtcgttaag 39

<210> 440
<211> 41
<212> DNA
<213> Artificial Sequence

<220>
<223> Target Specific Oligonucleotide

<400> 440
tggcatgatg tctaatagga gtctagacaa ccgtcgttaa g 41

<210> 441

<211> 38
<212> DNA
<213> Artificial Sequence

<220>
<223> Target Specific Oligonucleotide

<400> 441
ctgcggttta gccttgacgt tagacaaccg tcgttaag 38

<210> 442
<211> 38
<212> DNA
<213> Artificial Sequence

<220>
<223> Target Specific Oligonucleotide

<400> 442
cccagaggac gccatcattt tagacaaccg tcgttaag 38

<210> 443
<211> 40
<212> DNA
<213> Artificial Sequence

<220>
<223> Target Specific Oligonucleotide

<400> 443
tttcttgaaa gacgacagca gttagacaac cgtcgttaag 40

<210> 444
<211> 41
<212> DNA
<213> Artificial Sequence

<220>
<223> Target Specific Oligonucleotide

<400> 444
attaaatgtt cggaaggatg acctagacaa ccgtcgttaa g 41

<210> 445
<211> 38
<212> DNA
<213> Artificial Sequence

<220>
<223> Target Specific Oligonucleotide

<400> 445
tcttttgtca gcacggttgc tagacaaccg tcgttaag 38

<210> 446
<211> 38
<212> DNA
<213> Artificial Sequence

<220>
<223> Target Specific Oligonucleotide

<400> 446
ctggatcagc tcgaccagga tagacaaccg tcgttaag 38

<210> 447
<211> 38
<212> DNA
<213> Artificial Sequence

<220>
<223> Target Specific Oligonucleotide

<400> 447
accatggtcc gacttctgcc tagacaaccg tcgttaag 38

<210> 448
<211> 40
<212> DNA
<213> Artificial Sequence

<220>
<223> Target Specific Oligonucleotide

<400> 448
ctggatattc ggttatctgg gctagacaac cgtcgttaag 40

<210> 449
<211> 41
<212> DNA
<213> Artificial Sequence

<220>
<223> Target Specific Oligonucleotide

<400> 449
atccccttta cgatactctt cagtagacaa ccgtcgttaa g 41

<210> 450
<211> 38
<212> DNA
<213> Artificial Sequence

<220>
<223> Target Specific Oligonucleotide

<400> 450

tccagagcgg gaacagtatg tagacaaccg tcgttaag 38

<210> 451
<211> 38
<212> DNA
<213> Artificial Sequence

<220>
<223> Target Specific Oligonucleotide

<400> 451
gttgcctgca taagatgggc tagacaaccg tcgttaag 38

<210> 452
<211> 38
<212> DNA
<213> Artificial Sequence

<220>
<223> Target Specific Oligonucleotide

<400> 452
ccttttgtgc cgatgacccg tagacaaccg tcgttaag 38

<210> 453
<211> 37
<212> DNA
<213> Artificial Sequence

<220>
<223> Target Specific Oligonucleotide

<400> 453
gcttccgtgg actgggacgt agacaaccgt cgttaag 37

<210> 454
<211> 41
<212> DNA
<213> Artificial Sequence

<220>
<223> Target Specific Oligonucleotide

<400> 454
ccatagaagg acgaggtatt tcctagacaa ccgtcgttaa g 41

<210> 455
<211> 39
<212> DNA
<213> Artificial Sequence

<220>
<223> Target Specific Oligonucleotide

<400> 455
agaatgagtc gctgctcgat atagacaacc gtcgttaag 39

<210> 456
<211> 41
<212> DNA
<213> Artificial Sequence

<220>
<223> Target Specific Oligonucleotide

<400> 456
gcttcgacaa acgcaaagcg tagactcaat gatgataaag a 41

<210> 457
<211> 44
<212> DNA
<213> Artificial Sequence

<220>
<223> Target Specific Oligonucleotide

<400> 457
gctagttcac agaaagacca ttttagactc aatgatgata aaga 44

<210> 458
<211> 41
<212> DNA
<213> Artificial Sequence

<220>
<223> Target Specific Oligonucleotide

<400> 458
cagctcatcg aaagcgaccc tagactcaat gatgataaag a 41

<210> 459
<211> 40
<212> DNA
<213> Artificial Sequence

<220>
<223> Target Specific Oligonucleotide

<400> 459
cggctgctac aaacctcggt agactcaatg atgataaaga 40

<210> 460
<211> 42
<212> DNA
<213> Artificial Sequence

<220>
<223> Target Specific Oligonucleotide

<400> 460
aacaatttga aggccccaca atagactcaa tgatgataaa ga 42

<210> 461
<211> 39
<212> DNA
<213> Artificial Sequence

<220>
<223> Target Specific Oligonucleotide

<400> 461
gcgtctgcac cggagactta gactcaatga tgataaaga 39

<210> 462
<211> 39
<212> DNA
<213> Artificial Sequence

<220>
<223> Target Specific Oligonucleotide

<400> 462
gcgctgctca ggcattggta gactcaatga tgataaaga 39

<210> 463
<211> 47
<212> DNA
<213> Artificial Sequence

<220>
<223> Target Specific Oligonucleotide

<400> 463
ttgttgtgta acaacataat ttcaagtaga ctcaatgatg ataaaga 47

<210> 464
<211> 38
<212> DNA
<213> Artificial Sequence

<220>
<223> Target Specific Oligonucleotide

<400> 464
ctccacgtcg gcgtgcatag actcaatgat gataaaga 38

<210> 465
<211> 40
<212> DNA
<213> Artificial Sequence

<220>
<223> Target Specific Oligonucleotide

<400> 465
ctgcggggat tgtagccggt agactcaatg atgataaaga 40

<210> 466
<211> 42
<212> DNA
<213> Artificial Sequence

<220>
<223> Target Specific Oligonucleotide

<400> 466
atcttagcta ctgaccggct atagactcaa tgatgataaa ga 42

<210> 467
<211> 43
<212> DNA
<213> Artificial Sequence

<220>
<223> Target Specific Oligonucleotide

<400> 467
aagagattat acgcctcacg gatagactca atgatgataa aga 43

<210> 468
<211> 44
<212> DNA
<213> Artificial Sequence

<220>
<223> Target Specific Oligonucleotide

<400> 468
tttcgagtaa cgaaattagc tcctagactc aatgatgata aaga 44

<210> 469
<211> 39
<212> DNA
<213> Artificial Sequence

<220>
<223> Target Specific Oligonucleotide

<400> 469
gacccgcaga ttggcacgta gactcaatga tgataaaga 39

<210> 470
<211> 41

<212> DNA
<213> Artificial Sequence

<220>
<223> Target Specific Oligonucleotide

<400> 470
atggccccaa tctcgtttgg tagactcaat gatgataaag a 41

<210> 471
<211> 44
<212> DNA
<213> Artificial Sequence

<220>
<223> Target Specific Oligonucleotide

<400> 471
ccagtagcta taacgaagtc ctctagactc aatgatgata aaga 44

<210> 472
<211> 43
<212> DNA
<213> Artificial Sequence

<220>
<223> Target Specific Oligonucleotide

<400> 472
acaaattccc ggacactatg gatagactca atgatgataa aga 43

<210> 473
<211> 43
<212> DNA
<213> Artificial Sequence

<220>
<223> Target Specific Oligonucleotide

<400> 473
ttcacagagt tgataaggcc actagactca atgatgataa aga 43

<210> 474
<211> 43
<212> DNA
<213> Artificial Sequence

<220>
<223> Target Specific Oligonucleotide

<400> 474
aactttgccg gtctctttac attagactca atgatgataa aga 43

<210> 475
<211> 45
<212> DNA
<213> Artificial Sequence

<220>
<223> Target Specific Oligonucleotide

<400> 475
ttccaatgtg caagaatgat ttcttagact caatgatgat aaaga 45

<210> 476
<211> 43
<212> DNA
<213> Artificial Sequence

<220>
<223> Target Specific Oligonucleotide

<400> 476
ctccttctgg ggtatttcct gctagactca atgatgataa aga 43

<210> 477
<211> 40
<212> DNA
<213> Artificial Sequence

<220>
<223> Target Specific Oligonucleotide

<400> 477
ccacttcagt tggccggtat agactcaatg atgataaaga 40

<210> 478
<211> 39
<212> DNA
<213> Artificial Sequence

<220>
<223> Target Specific Oligonucleotide

<400> 478
tccgtcaacg tccgcagtta gactcaatga tgataaaga 39

<210> 479
<211> 42
<212> DNA
<213> Artificial Sequence

<220>
<223> Target Specific Oligonucleotide

<400> 479
agtgagatcg ccatagtgca atagactcaa tgatgataaa ga 42

<210> 480
<211> 43
<212> DNA
<213> Artificial Sequence

<220>
<223> Target Specific Oligonucleotide

<400> 480
gaaggtgacc ctataaggag tcatagacta caacaaaaga tcg 43

<210> 481
<211> 41
<212> DNA
<213> Artificial Sequence

<220>
<223> Target Specific Oligonucleotide

<400> 481
aacaccgctt gcatagttgt gtagactaca acaaaagatc g 41

<210> 482
<211> 41
<212> DNA
<213> Artificial Sequence

<220>
<223> Target Specific Oligonucleotide

<400> 482
tacagacgcg gaatcattct ctagactaca acaaaagatc g 41

<210> 483
<211> 41
<212> DNA
<213> Artificial Sequence

<220>
<223> Target Specific Oligonucleotide

<400> 483
tggaggaaat gagcatgacc ttagactaca acaaaagatc g 41

<210> 484
<211> 41
<212> DNA
<213> Artificial Sequence

<220>
<223> Target Specific Oligonucleotide

<400> 484
atggtgtaca ctcgaggctg atagactaca acaaaagatc g 41

<210> 485
<211> 41
<212> DNA
<213> Artificial Sequence

<220>
<223> Target Specific Oligonucleotide

<400> 485
ccttgtggta gacgttcagc ttagactaca acaaaagatc g 41

<210> 486
<211> 44
<212> DNA
<213> Artificial Sequence

<220>
<223> Target Specific Oligonucleotide

<400> 486
ggatcaggtt taatggtcac tatgtagact acaacaaaag atcg 44

<210> 487
<211> 40
<212> DNA
<213> Artificial Sequence

<220>
<223> Target Specific Oligonucleotide

<400> 487
cacacggtca acgctgtaca tagactacaa caaaagatcg 40

<210> 488
<211> 40
<212> DNA
<213> Artificial Sequence

<220>
<223> Target Specific Oligonucleotide

<400> 488
caggtgtttg cggaagttcc tagactacaa caaaagatcg 40

<210> 489
<211> 40
<212> DNA
<213> Artificial Sequence

<220>

<223> Target Specific Oligonucleotide

<400> 489
gtgctcatgg tcgtagagga tagactacaa caaaagatcg 40

<210> 490
<211> 43
<212> DNA
<213> Artificial Sequence

<220>
<223> Target Specific Oligonucleotide

<400> 490
aagtcaaagt acgtctcgat cattagacta caacaaaaga tcg 43

<210> 491
<211> 39
<212> DNA
<213> Artificial Sequence

<220>
<223> Target Specific Oligonucleotide

<400> 491
tcgtagcagc cgttggagat agactacaac aaaagatcg 39

<210> 492
<211> 39
<212> DNA
<213> Artificial Sequence

<220>
<223> Target Specific Oligonucleotide

<400> 492
tcgtggccag agtaggcatt agactacaac aaaagatcg 39

<210> 493
<211> 40
<212> DNA
<213> Artificial Sequence

<220>
<223> Target Specific Oligonucleotide

<400> 493
ggtactctcc aaacgctcgg tagactacaa caaaagatcg 40

<210> 494
<211> 42
<212> DNA
<213> Artificial Sequence

<220>
<223> Target Specific Oligonucleotide

<400> 494
aggtacgaat agggatgtcg tctagactac aacaaaagat cg 42

<210> 495
<211> 42
<212> DNA
<213> Artificial Sequence

<220>
<223> Target Specific Oligonucleotide

<400> 495
agggtatcgt acatcgttcc aatagactac aacaaaagat cg 42

<210> 496
<211> 39
<212> DNA
<213> Artificial Sequence

<220>
<223> Target Specific Oligonucleotide

<400> 496
caggtgatct gcgccgttgt agactacaac aaaagatcg 39

<210> 497
<211> 39
<212> DNA
<213> Artificial Sequence

<220>
<223> Target Specific Oligonucleotide

<400> 497
ccacaggcga tacaaccggt agactacaac aaaagatcg 39

<210> 498
<211> 39
<212> DNA
<213> Artificial Sequence

<220>
<223> Target Specific Oligonucleotide

<400> 498
cagcagcttc gagtgctggt agactacaac aaaagatcg 39

<210> 499
<211> 39
<212> DNA

<213> Artificial Sequence

<220>
<223> Target Specific Oligonucleotide

<400> 499
ccctcacagg acgtcgtcat agactacaac aaaagatcg 39

<210> 500
<211> 39
<212> DNA
<213> Artificial Sequence

<220>
<223> Target Specific Oligonucleotide

<400> 500
gcacgaagcc ttcggtgtct agactacaac aaaagatcg 39

<210> 501
<211> 41
<212> DNA
<213> Artificial Sequence

<220>
<223> Target Specific Oligonucleotide

<400> 501
tgggaagtgt cggctttcat gtagactaca acaaaagatc g 41

<210> 502
<211> 41
<212> DNA
<213> Artificial Sequence

<220>
<223> Target Specific Oligonucleotide

<400> 502
tgggaagtgt cggctttcat gtagactaca acaaaagatc g 41

<210> 503
<211> 38
<212> DNA
<213> Artificial Sequence

<220>
<223> Target Specific Oligonucleotide

<400> 503
tgctcgcact ttggccgcta gactacaaca aaagatcg 38

<210> 504

<211> 40
<212> DNA
<213> Artificial Sequence

<220>
<223> Target Specific Oligonucleotide

<400> 504
gtcttttggc acggtttctg ttagactgta ttcaacgtcc 40

<210> 505
<211> 40
<212> DNA
<213> Artificial Sequence

<220>
<223> Target Specific Oligonucleotide

<400> 505
tacttcttca acgcgaagag ctagactgta ttcaacgtcc 40

<210> 506
<211> 40
<212> DNA
<213> Artificial Sequence

<220>
<223> Target Specific Oligonucleotide

<400> 506
tttgaggttg tatccgctgc ttagactgta ttcaacgtcc 40

<210> 507
<211> 44
<212> DNA
<213> Artificial Sequence

<220>
<223> Target Specific Oligonucleotide

<400> 507
caagttgact aaatctcgta ctttctagac tgtattcaac gtcc 44

<210> 508
<211> 43
<212> DNA
<213> Artificial Sequence

<220>
<223> Target Specific Oligonucleotide

<400> 508
gccttattaa cggtatcttc agaatagact gtattcaacg tcc 43

<210> 509
<211> 40
<212> DNA
<213> Artificial Sequence

<220>
<223> Target Specific Oligonucleotide

<400> 509
tgttctggat ttcgcaggtc ctagactgta ttcaacgtcc 40

<210> 510
<211> 42
<212> DNA
<213> Artificial Sequence

<220>
<223> Target Specific Oligonucleotide

<400> 510
gctttatcag gttatgttgc atgtagactg tattcaacgt cc 42

<210> 511
<211> 43
<212> DNA
<213> Artificial Sequence

<220>
<223> Target Specific Oligonucleotide

<400> 511
gattctggct tatagggtat tcactagact gtattcaacg tcc 43

<210> 512
<211> 41
<212> DNA
<213> Artificial Sequence

<220>
<223> Target Specific Oligonucleotide

<400> 512
ttttcctccc gcaattccta gatagactgt attcaacgtc c 41

<210> 513
<211> 40
<212> DNA
<213> Artificial Sequence

<220>
<223> Target Specific Oligonucleotide

<400> 513

tgtttccgtc aaatcgtgtg gtagactgta ttcaacgtcc 40

<210> 514
<211> 41
<212> DNA
<213> Artificial Sequence

<220>
<223> Target Specific Oligonucleotide

<400> 514
ggttccctct agatcttgcc tttagactgt attcaacgtc c 41

<210> 515
<211> 43
<212> DNA
<213> Artificial Sequence

<220>
<223> Target Specific Oligonucleotide

<400> 515
gcatgtacca cctatcatct aatgtagact gtattcaacg tcc 43

<210> 516
<211> 39
<212> DNA
<213> Artificial Sequence

<220>
<223> Target Specific Oligonucleotide

<400> 516
gttgccaaca cgagctgact tagactgtat tcaacgtcc 39

<210> 517
<211> 41
<212> DNA
<213> Artificial Sequence

<220>
<223> Target Specific Oligonucleotide

<400> 517
actgactact agttcaagcg catagactgt attcaacgtc c 41

<210> 518
<211> 39
<212> DNA
<213> Artificial Sequence

<220>
<223> Target Specific Oligonucleotide

<400> 518
tctcttgctc gctttggacc tagactgtat tcaacgtcc 39

<210> 519
<211> 44
<212> DNA
<213> Artificial Sequence

<220>
<223> Target Specific Oligonucleotide

<400> 519
cactgctaga acaactatca atttgtagac tgtattcaac gtcc 44

<210> 520
<211> 41
<212> DNA
<213> Artificial Sequence

<220>
<223> Target Specific Oligonucleotide

<400> 520
ttacatggct taagttgggg agtagactgt attcaacgtc c 41

<210> 521
<211> 40
<212> DNA
<213> Artificial Sequence

<220>
<223> Target Specific Oligonucleotide

<400> 521
gtgaggggac gctcttgtat ttagactgta ttcaacgtcc 40

<210> 522
<211> 40
<212> DNA
<213> Artificial Sequence

<220>
<223> Target Specific Oligonucleotide

<400> 522
aacggtgcta tgcctagtag atagactgta ttcaacgtcc 40

<210> 523
<211> 40
<212> DNA
<213> Artificial Sequence

<220>
<223> Target Specific Oligonucleotide

<400> 523
tttgccttcc ctagagtgct atagactgta ttcaacgtcc 40

<210> 524
<211> 43
<212> DNA
<213> Artificial Sequence

<220>
<223> Target Specific Oligonucleotide

<400> 524
atgtcccaat ggatacttaa agcctagact gtattcaacg tcc 43

<210> 525
<211> 43
<212> DNA
<213> Artificial Sequence

<220>
<223> Target Specific Oligonucleotide

<400> 525
cttccagtaa cgagatactt tccttagact gtattcaacg tcc 43

<210> 526
<211> 42
<212> DNA
<213> Artificial Sequence

<220>
<223> Target Specific Oligonucleotide

<400> 526
caaccatgaa ttagtccctt gggtagactg tattcaacgt cc 42

<210> 527
<211> 44
<212> DNA
<213> Artificial Sequence

<220>
<223> Target Specific Oligonucleotide

<400> 527
cagcattatt agacacttta actgttagac tgtattcaac gtcc 44

<210> 528
<211> 43
<212> DNA
<213> Artificial Sequence

<220>
<223> Target Specific Oligonucleotide

<400> 528
tgcacatatg attgacgctc agtagactct tatattgagt ggt 43

<210> 529
<211> 40
<212> DNA
<213> Artificial Sequence

<220>
<223> Target Specific Oligonucleotide

<400> 529
ttcaccgcct gcaacaagat agactcttat attgagtggt 40

<210> 530
<211> 44
<212> DNA
<213> Artificial Sequence

<220>
<223> Target Specific Oligonucleotide

<400> 530
ttcacttaca ggtaacacca agttagactc ttatattgag tggt 44

<210> 531
<211> 45
<212> DNA
<213> Artificial Sequence

<220>
<223> Target Specific Oligonucleotide

<400> 531
aagcaataga tcgtccataa gttatagact cttatattga gtggt 45

<210> 532
<211> 48
<212> DNA
<213> Artificial Sequence

<220>
<223> Target Specific Oligonucleotide

<400> 532
tacaagaacc ctaattgtaa taatagatag actcttatat tgagtggt 48

<210> 533
<211> 44

<212> DNA
<213> Artificial Sequence

<220>
<223> Target Specific Oligonucleotide

<400> 533
gaataggacc aaagtgtccc ttgtagactc ttatattgag tggt 44

<210> 534
<211> 44
<212> DNA
<213> Artificial Sequence

<220>
<223> Target Specific Oligonucleotide

<400> 534
tccatcagga ctaaatctca cactagactc ttatattgag tggt 44

<210> 535
<211> 44
<212> DNA
<213> Artificial Sequence

<220>
<223> Target Specific Oligonucleotide

<400> 535
aaaggccata cgtttttcct acctagactc ttatattgag tggt 44

<210> 536
<211> 44
<212> DNA
<213> Artificial Sequence

<220>
<223> Target Specific Oligonucleotide

<400> 536
gcagtgtaca atgaacgaga aattagactc ttatattgag tggt 44

<210> 537
<211> 44
<212> DNA
<213> Artificial Sequence

<220>
<223> Target Specific Oligonucleotide

<400> 537
ggcattctgc tttaagatca gaatagactc ttatattgag tggt 44

<210> 538
<211> 40
<212> DNA
<213> Artificial Sequence

<220>
<223> Target Specific Oligonucleotide

<400> 538
ggccagccac gattcacagt agactcttat attgagtggt 40

<210> 539
<211> 39
<212> DNA
<213> Artificial Sequence

<220>
<223> Target Specific Oligonucleotide

<400> 539
gaagccagcg accggactta gactcttata ttgagtggt 39

<210> 540
<211> 41
<212> DNA
<213> Artificial Sequence

<220>
<223> Target Specific Oligonucleotide

<400> 540
ctgagggtcg tcgttgtctt tagactctta tattgagtgg t 41

<210> 541
<211> 43
<212> DNA
<213> Artificial Sequence

<220>
<223> Target Specific Oligonucleotide

<400> 541
ctgaaccact ggcatagagt tctagactct tatattgagt ggt 43

<210> 542
<211> 40
<212> DNA
<213> Artificial Sequence

<220>
<223> Target Specific Oligonucleotide

<400> 542
aaatcaacca cgggtcgcgt agactcttat attgagtggt 40

<210> 543
<211> 40
<212> DNA
<213> Artificial Sequence

<220>
<223> Target Specific Oligonucleotide

<400> 543
agcaacagtc gacgagggat agactcttat attgagtggt 40

<210> 544
<211> 48
<212> DNA
<213> Artificial Sequence

<220>
<223> Target Specific Oligonucleotide

<400> 544
atatattcca tactactaac agacatatag actcttatat tgagtggt 48

<210> 545
<211> 38
<212> DNA
<213> Artificial Sequence

<220>
<223> Target Specific Oligonucleotide

<400> 545
tccacaccgg catcggctag actcttatat tgagtggt 38

<210> 546
<211> 40
<212> DNA
<213> Artificial Sequence

<220>
<223> Target Specific Oligonucleotide

<400> 546
agaagggcaa tccggtggct agactcttat attgagtggt 40

<210> 547
<211> 40
<212> DNA
<213> Artificial Sequence

<220>
<223> Target Specific Oligonucleotide

<400> 547
tcagggagtc tgcgaccagt agactcttat attgagtggt 40

<210> 548
<211> 40
<212> DNA
<213> Artificial Sequence

<220>
<223> Target Specific Oligonucleotide

<400> 548
gctttgccag ctcaccactt agactcttat attgagtggt 40

<210> 549
<211> 43
<212> DNA
<213> Artificial Sequence

<220>
<223> Target Specific Oligonucleotide

<400> 549
aaatacccat aaggcgtgat gctagactct tatattgagt ggt 43

<210> 550
<211> 44
<212> DNA
<213> Artificial Sequence

<220>
<223> Target Specific Oligonucleotide

<400> 550
attactatcc tgcgtgaaat ccatagactc ttatattgag tggt 44

<210> 551
<211> 43
<212> DNA
<213> Artificial Sequence

<220>
<223> Target Specific Oligonucleotide

<400> 551
cccaaagtcg aacagttttg tctagactct tatattgagt ggt 43

<210> 552
<211> 41
<212> DNA
<213> Artificial Sequence

<220>

<223> Target Specific Oligonucleotide

<400> 552
tcctcaaatg tccgaggatg atagacctat tctatgcttc g 41

<210> 553
<211> 42
<212> DNA
<213> Artificial Sequence

<220>
<223> Target Specific Oligonucleotide

<400> 553
gtgaacacaa cgtaagaaca ggtagaccta ttctatgctt cg 42

<210> 554
<211> 42
<212> DNA
<213> Artificial Sequence

<220>
<223> Target Specific Oligonucleotide

<400> 554
actcctctgg ttacgcttca tttagaccta ttctatgctt cg 42

<210> 555
<211> 41
<212> DNA
<213> Artificial Sequence

<220>
<223> Target Specific Oligonucleotide

<400> 555
acctaacata accgtgctgc ctagacctat tctatgcttc g 41

<210> 556
<211> 41
<212> DNA
<213> Artificial Sequence

<220>
<223> Target Specific Oligonucleotide

<400> 556
ttcagttgac cgtctggaca ctagacctat tctatgcttc g 41

<210> 557
<211> 43
<212> DNA
<213> Artificial Sequence

<220>
<223> Target Specific Oligonucleotide

<400> 557
ctctgacaca cgtaacaata acatagacct attctatgct tcg 43

<210> 558
<211> 43
<212> DNA
<213> Artificial Sequence

<220>
<223> Target Specific Oligonucleotide

<400> 558
agtaatgtga ggtacaactg cattagacct attctatgct tcg 43

<210> 559
<211> 41
<212> DNA
<213> Artificial Sequence

<220>
<223> Target Specific Oligonucleotide

<400> 559
gcttctttcc cgcccaaaat atagacctat tctatgcttc g 41

<210> 560
<211> 44
<212> DNA
<213> Artificial Sequence

<220>
<223> Target Specific Oligonucleotide

<400> 560
aaagaaaagc ggtctactag attatagacc tattctatgc ttcg 44

<210> 561
<211> 41
<212> DNA
<213> Artificial Sequence

<220>
<223> Target Specific Oligonucleotide

<400> 561
tgacgtgcgg cgaaatttat gtagacctat tctatgcttc g 41

<210> 562
<211> 41
<212> DNA

<213> Artificial Sequence

<220>
<223> Target Specific Oligonucleotide

<400> 562
gcttattgca gcgatggatg atagacctat tctatgcttc g 41

<210> 563
<211> 42
<212> DNA
<213> Artificial Sequence

<220>
<223> Target Specific Oligonucleotide

<400> 563
tgtcagtaga cgatagagga ggtagaccta ttctatgctt cg 42

<210> 564
<211> 41
<212> DNA
<213> Artificial Sequence

<220>
<223> Target Specific Oligonucleotide

<400> 564
ttggttccga ctacccaaca gtagacctat tctatgcttc g 41

<210> 565
<211> 45
<212> DNA
<213> Artificial Sequence

<220>
<223> Target Specific Oligonucleotide

<400> 565
ctcttaagta cgcaataatt ctctctagac ctattctatg cttcg 45

<210> 566
<211> 43
<212> DNA
<213> Artificial Sequence

<220>
<223> Target Specific Oligonucleotide

<400> 566
gttccgtata tgtcggatct ctctagacct attctatgct tcg 43

<210> 567

<211> 41
<212> DNA
<213> Artificial Sequence

<220>
<223> Target Specific Oligonucleotide

<400> 567
aaattcaccg gacggagtgt ttagacctat tctatgcttc g 41

<210> 568
<211> 42
<212> DNA
<213> Artificial Sequence

<220>
<223> Target Specific Oligonucleotide

<400> 568
tttgtgaaac cgtagtggct cttagaccta ttctatgctt cg 42

<210> 569
<211> 42
<212> DNA
<213> Artificial Sequence

<220>
<223> Target Specific Oligonucleotide

<400> 569
acctctttaa cgaaggtgtc agtagaccta ttctatgctt cg 42

<210> 570
<211> 41
<212> DNA
<213> Artificial Sequence

<220>
<223> Target Specific Oligonucleotide

<400> 570
ggactccggt tctaacttgg ttagacctat tctatgcttc g 41

<210> 571
<211> 43
<212> DNA
<213> Artificial Sequence

<220>
<223> Target Specific Oligonucleotide

<400> 571
atctctatat gacgtgctgt tggtagacct attctatgct tcg 43

<210> 572
<211> 43
<212> DNA
<213> Artificial Sequence

<220>
<223> Target Specific Oligonucleotide

<400> 572
gttggaatta ttcggagact gagtagacct attctatgct tcg 43

<210> 573
<211> 40
<212> DNA
<213> Artificial Sequence

<220>
<223> Target Specific Oligonucleotide

<400> 573
gcacatttga cgacggcttc tagacctatt ctatgcttcg 40

<210> 574
<211> 40
<212> DNA
<213> Artificial Sequence

<220>
<223> Target Specific Oligonucleotide

<400> 574
actccgtgta aacgcagtgg tagacctatt ctatgcttcg 40

<210> 575
<211> 43
<212> DNA
<213> Artificial Sequence

<220>
<223> Target Specific Oligonucleotide

<400> 575
acacataact cgttaacacg ttctagacct attctatgct tcg 43

<210> 576
<211> 42
<212> DNA
<213> Artificial Sequence

<220>
<223> Target Specific Oligonucleotide

<400> 576

cagatctgtt gagctaacag ggtagacatg ttataccatg cg 42

<210> 577
<211> 44
<212> DNA
<213> Artificial Sequence

<220>
<223> Target Specific Oligonucleotide

<400> 577
ttctagacta tgcaaccctc taggtagaca tgttatacca tgcg 44

<210> 578
<211> 43
<212> DNA
<213> Artificial Sequence

<220>
<223> Target Specific Oligonucleotide

<400> 578
cacccatgtg agtaatacac tgctagacat gttataccat gcg 43

<210> 579
<211> 42
<212> DNA
<213> Artificial Sequence

<220>
<223> Target Specific Oligonucleotide

<400> 579
agtcccatat tgccgttcat gctagacatg ttataccatg cg 42

<210> 580
<211> 42
<212> DNA
<213> Artificial Sequence

<220>
<223> Target Specific Oligonucleotide

<400> 580
atttcctcat aacggtcatg gctagacatg ttataccatg cg 42

<210> 581
<211> 42
<212> DNA
<213> Artificial Sequence

<220>
<223> Target Specific Oligonucleotide

<400> 581
ttactgccta gctagcaggt tatagacatg ttataccatg cg 42

<210> 582
<211> 41
<212> DNA
<213> Artificial Sequence

<220>
<223> Target Specific Oligonucleotide

<400> 582
acgtgccctt ggtactatga ctagacatgt tataccatgc g 41

<210> 583
<211> 41
<212> DNA
<213> Artificial Sequence

<220>
<223> Target Specific Oligonucleotide

<400> 583
cttgccaaac cctagcttgg atagacatgt tataccatgc g 41

<210> 584
<211> 45
<212> DNA
<213> Artificial Sequence

<220>
<223> Target Specific Oligonucleotide

<400> 584
attatcactt aacgaaggtc ctttgtagac atgttatacc atgcg 45

<210> 585
<211> 45
<212> DNA
<213> Artificial Sequence

<220>
<223> Target Specific Oligonucleotide

<400> 585
atatacagcc taagcaccaa ttatgtagac atgttatacc atgcg 45

<210> 586
<211> 40
<212> DNA
<213> Artificial Sequence

<220>
<223> Target Specific Oligonucleotide

<400> 586
ggcaacaaaa cgtcatggca tagacatgtt ataccatgcg 40

<210> 587
<211> 43
<212> DNA
<213> Artificial Sequence

<220>
<223> Target Specific Oligonucleotide

<400> 587
gtctgaggac taaccctaaa gggtagacat gttataccat gcg 43

<210> 588
<211> 46
<212> DNA
<213> Artificial Sequence

<220>
<223> Target Specific Oligonucleotide

<400> 588
cttaaaatag atcgaaactc tgtctctaga catgttatac catgcg 46

<210> 589
<211> 44
<212> DNA
<213> Artificial Sequence

<220>
<223> Target Specific Oligonucleotide

<400> 589
tgatctgtac aagctacgat tttatagaca tgttatacca tgcg 44

<210> 590
<211> 43
<212> DNA
<213> Artificial Sequence

<220>
<223> Target Specific Oligonucleotide

<400> 590
aaatcagtta taccaagggg agatagacat gttataccat gcg 43

<210> 591
<211> 45
<212> DNA
<213> Artificial Sequence

<220>
<223> Target Specific Oligonucleotide

<400> 591
taggaaaaca tacgtatact gaatatagac atgttatacc atgcg 45

<210> 592
<211> 40
<212> DNA
<213> Artificial Sequence

<220>
<223> Target Specific Oligonucleotide

<400> 592
agttggcctc acgttgcatt tagacatgtt ataccatgcg 40

<210> 593
<211> 40
<212> DNA
<213> Artificial Sequence

<220>
<223> Target Specific Oligonucleotide

<400> 593
tttggtctcg gcttgcgaat tagacatgtt ataccatgcg 40

<210> 594
<211> 41
<212> DNA
<213> Artificial Sequence

<220>
<223> Target Specific Oligonucleotide

<400> 594
cagtagtcaa cgcagcactc atagacatgt tataccatgc g 41

<210> 595
<211> 44
<212> DNA
<213> Artificial Sequence

<220>
<223> Target Specific Oligonucleotide

<400> 595
caacaggatc acctaatatt cccatagaca tgttatacca tgcg 44

<210> 596
<211> 43

<212> DNA
<213> Artificial Sequence

<220>
<223> Target Specific Oligonucleotide

<400> 596
tttggacaga tatctcctcg aaatagacat gttataccat gcg 43

<210> 597
<211> 43
<212> DNA
<213> Artificial Sequence

<220>
<223> Target Specific Oligonucleotide

<400> 597
aaaggctatc tacactttgg caatagacat gttataccat gcg 43

<210> 598
<211> 40
<212> DNA
<213> Artificial Sequence

<220>
<223> Target Specific Oligonucleotide

<400> 598
tgcctggatc ctatcccgac tagacatgtt ataccatgcg 40

<210> 599
<211> 40
<212> DNA
<213> Artificial Sequence

<220>
<223> Target Specific Oligonucleotide

<400> 599
tgtggtcacc tacacgctgc tagacatgtt ataccatgcg 40

<210> 600
<211> 43
<212> DNA
<213> Artificial Sequence

<220>
<223> Target Specific Oligonucleotide

<400> 600
ggtgagctgg taatctgacc ttagacaatc tatttaccct acg 43

<210> 601
<211> 41
<212> DNA
<213> Artificial Sequence

<220>
<223> Target Specific Oligonucleotide

<400> 601
gttgcaggca tgacgcaagt agacaatcta tttaccctac g 41

<210> 602
<211> 43
<212> DNA
<213> Artificial Sequence

<220>
<223> Target Specific Oligonucleotide

<400> 602
aaaacgccta cgcatcatgt ctagacaatc tatttaccct acg 43

<210> 603
<211> 40
<212> DNA
<213> Artificial Sequence

<220>
<223> Target Specific Oligonucleotide

<400> 603
tggtcctgcg cggatgtcta gacaatctat ttaccctacg 40

<210> 604
<211> 41
<212> DNA
<213> Artificial Sequence

<220>
<223> Target Specific Oligonucleotide

<400> 604
ggtgagcttc tccgtggcgt agacaatcta tttaccctac g 41

<210> 605
<211> 41
<212> DNA
<213> Artificial Sequence

<220>
<223> Target Specific Oligonucleotide

<400> 605
agcgcagggc caattgactt agacaatcta tttaccctac g 41

<210> 606
<211> 43
<212> DNA
<213> Artificial Sequence

<220>
<223> Target Specific Oligonucleotide

<400> 606
ccgggtacac ggttttgatc ttagacaatc tatttaccct acg 43

<210> 607
<211> 40
<212> DNA
<213> Artificial Sequence

<220>
<223> Target Specific Oligonucleotide

<400> 607
ccggctcgat gtcgggtata gacaatctat ttaccctacg 40

<210> 608
<211> 40
<212> DNA
<213> Artificial Sequence

<220>
<223> Target Specific Oligonucleotide

<400> 608
ggcgccttct cgccacatta gacaatctat ttaccctacg 40

<210> 609
<211> 40
<212> DNA
<213> Artificial Sequence

<220>
<223> Target Specific Oligonucleotide

<400> 609
cacgttgaag cgcgggtgta gacaatctat ttaccctacg 40

<210> 610
<211> 44
<212> DNA
<213> Artificial Sequence

<220>
<223> Target Specific Oligonucleotide

<400> 610
gcccacagaa accggtttaa tgtagacaat ctatttaccc tacg 44

<210> 611
<211> 43
<212> DNA
<213> Artificial Sequence

<220>
<223> Target Specific Oligonucleotide

<400> 611
tcagaatgag cgcaatccag gtagacaatc tatttaccct acg 43

<210> 612
<211> 42
<212> DNA
<213> Artificial Sequence

<220>
<223> Target Specific Oligonucleotide

<400> 612
ttcggacaca aagacgctcc tagacaatct atttacccta cg 42

<210> 613
<211> 42
<212> DNA
<213> Artificial Sequence

<220>
<223> Target Specific Oligonucleotide

<400> 613
agcttgacgt aggtgtcggt tagacaatct atttacccta cg 42

<210> 614
<211> 42
<212> DNA
<213> Artificial Sequence

<220>
<223> Target Specific Oligonucleotide

<400> 614
ggttcccgcc aaatttcccg tagacaatct atttacccta cg 42

<210> 615
<211> 45
<212> DNA
<213> Artificial Sequence

<220>

<223> Target Specific Oligonucleotide

<400> 615
cagggctcat gatagtacaa cagtagacaa tctatttacc ctacg 45

<210> 616
<211> 41
<212> DNA
<213> Artificial Sequence

<220>
<223> Target Specific Oligonucleotide

<400> 616
ggactttagc gcggtctcct agacaatcta tttaccctac g 41

<210> 617
<211> 41
<212> DNA
<213> Artificial Sequence

<220>
<223> Target Specific Oligonucleotide

<400> 617
gcagccgttc taagcgctgt agacaatcta tttaccctac g 41

<210> 618
<211> 41
<212> DNA
<213> Artificial Sequence

<220>
<223> Target Specific Oligonucleotide

<400> 618
gctggtaaag gcgcttccct agacaatcta tttaccctac g 41

<210> 619
<211> 41
<212> DNA
<213> Artificial Sequence

<220>
<223> Target Specific Oligonucleotide

<400> 619
gaagaagtcg gtgacgcggt agacaatcta tttaccctac g 41

<210> 620
<211> 41
<212> DNA
<213> Artificial Sequence

<220>
<223> Target Specific Oligonucleotide

<400> 620
ggctggaaac ctcgtccact agacaatcta tttaccctac g 41

<210> 621
<211> 48
<212> DNA
<213> Artificial Sequence

<220>
<223> Target Specific Oligonucleotide

<400> 621
tttattagag tttaggaggt cagaaataga caatctattt accctacg 48

<210> 622
<211> 44
<212> DNA
<213> Artificial Sequence

<220>
<223> Target Specific Oligonucleotide

<400> 622
tgggaggtcc catattcaga ggtagacaat ctatttaccc tacg 44

<210> 623
<211> 44
<212> DNA
<213> Artificial Sequence

<220>
<223> Target Specific Oligonucleotide

<400> 623
tccagggatg tcctacttgt cctagacaat ctatttaccc tacg 44

<210> 624
<211> 40
<212> DNA
<213> Artificial Sequence

<220>
<223> Target Specific Oligonucleotide

<400> 624
ccaggtttcc ggcacagtgg gtaactgaat tgtcacttta 40

<210> 625
<211> 46
<212> DNA

<213> Artificial Sequence

<220>
<223> Target Specific Oligonucleotide

<400> 625
tgttatactt tgtcgctctt agtagagtaa ctgaattgtc acttta 46

<210> 626
<211> 44
<212> DNA
<213> Artificial Sequence

<220>
<223> Target Specific Oligonucleotide

<400> 626
agcttttaac caggtttcga cttcgtaact gaattgtcac ttta 44

<210> 627
<211> 45
<212> DNA
<213> Artificial Sequence

<220>
<223> Target Specific Oligonucleotide

<400> 627
gtatgagaat cccgataaaa ctggtgtaac tgaattgtca cttta 45

<210> 628
<211> 43
<212> DNA
<213> Artificial Sequence

<220>
<223> Target Specific Oligonucleotide

<400> 628
aagaggacat cgaccaatcc tcagtaactg aattgtcact tta 43

<210> 629
<211> 42
<212> DNA
<213> Artificial Sequence

<220>
<223> Target Specific Oligonucleotide

<400> 629
ggtttgtgca agggtcgaaa acgtaactga attgtcactt ta 42

<210> 630

<211> 44
<212> DNA
<213> Artificial Sequence

<220>
<223> Target Specific Oligonucleotide

<400> 630
acacctctcg tagctataga tgtggtaact gaattgtcac ttta 44

<210> 631
<211> 41
<212> DNA
<213> Artificial Sequence

<220>
<223> Target Specific Oligonucleotide

<400> 631
atgagagggc aaccgaggtg agtaactgaa ttgtcacttt a 41

<210> 632
<211> 43
<212> DNA
<213> Artificial Sequence

<220>
<223> Target Specific Oligonucleotide

<400> 632
ccgctgtcag ggtctatcat tccgtaactg aattgtcact tta 43

<210> 633
<211> 41
<212> DNA
<213> Artificial Sequence

<220>
<223> Target Specific Oligonucleotide

<400> 633
gcaatgagca tatcctcggg cgtaactgaa ttgtcacttt a 41

<210> 634
<211> 41
<212> DNA
<213> Artificial Sequence

<220>
<223> Target Specific Oligonucleotide

<400> 634
ggcttgagtg ggttcaccca ggtaactgaa ttgtcacttt a 41

<210> 635
<211> 43
<212> DNA
<213> Artificial Sequence

<220>
<223> Target Specific Oligonucleotide

<400> 635
gcaaggtatg gcaatagctg agtgtaactg aattgtcact tta 43

<210> 636
<211> 45
<212> DNA
<213> Artificial Sequence

<220>
<223> Target Specific Oligonucleotide

<400> 636
cagtaatagt agagacgcca ctacagtaac tgaattgtca cttta 45

<210> 637
<211> 43
<212> DNA
<213> Artificial Sequence

<220>
<223> Target Specific Oligonucleotide

<400> 637
cttccaaagt gtcgcttcag agggtaactg aattgtcact tta 43

<210> 638
<211> 46
<212> DNA
<213> Artificial Sequence

<220>
<223> Target Specific Oligonucleotide

<400> 638
aggcaatttt cccgatactt tttattgtaa ctgaattgtc acttta 46

<210> 639
<211> 46
<212> DNA
<213> Artificial Sequence

<220>
<223> Target Specific Oligonucleotide

<400> 639

tgttagcttc tatagtcact attcgagtaa ctgaattgtc acttta 46

<210> 640
<211> 45
<212> DNA
<213> Artificial Sequence

<220>
<223> Target Specific Oligonucleotide

<400> 640
tcaacaagta atgtatcccg ttcatgtaac tgaattgtca cttta 45

<210> 641
<211> 44
<212> DNA
<213> Artificial Sequence

<220>
<223> Target Specific Oligonucleotide

<400> 641
tttccttggc cgtaagttgt tttcgtaact gaattgtcac ttta 44

<210> 642
<211> 42
<212> DNA
<213> Artificial Sequence

<220>
<223> Target Specific Oligonucleotide

<400> 642
gggtcaaccc agtctgttac ctgtaactga attgtcactt ta 42

<210> 643
<211> 46
<212> DNA
<213> Artificial Sequence

<220>
<223> Target Specific Oligonucleotide

<400> 643
tctgagactg atagggaaac atatgggtaa ctgaattgtc acttta 46

<210> 644
<211> 42
<212> DNA
<213> Artificial Sequence

<220>
<223> Target Specific Oligonucleotide

<400> 644
tctttgccac caatagcttg gagtaactga attgtcactt ta 42

<210> 645
<211> 45
<212> DNA
<213> Artificial Sequence

<220>
<223> Target Specific Oligonucleotide

<400> 645
ctcattggaa tggttagtag agcaagtaac tgaattgtca cttta 45

<210> 646
<211> 44
<212> DNA
<213> Artificial Sequence

<220>
<223> Target Specific Oligonucleotide

<400> 646
ctgagtaaag agcgtgtatt ccaggtaact gaattgtcac ttta 44

<210> 647
<211> 42
<212> DNA
<213> Artificial Sequence

<220>
<223> Target Specific Oligonucleotide

<400> 647
tggaggcata aagctggtag gcgtaactga attgtcactt ta 42

<210> 648
<211> 42
<212> DNA
<213> Artificial Sequence

<220>
<223> Target Specific Oligonucleotide

<400> 648
ggatctcaag cgttgcaggc gtaacgatag atttatacga cg 42

<210> 649
<211> 39
<212> DNA
<213> Artificial Sequence

<220>
<223> Target Specific Oligonucleotide

<400> 649
gcaccgcgag cggactagta acgatagatt tatacgacg 39

<210> 650
<211> 41
<212> DNA
<213> Artificial Sequence

<220>
<223> Target Specific Oligonucleotide

<400> 650
tgctgaggtc gctcacgaag taacgataga tttatacgac g 41

<210> 651
<211> 44
<212> DNA
<213> Artificial Sequence

<220>
<223> Target Specific Oligonucleotide

<400> 651
ccggaccacg tagttaaatc ttgtaacgat agatttatac gacg 44

<210> 652
<211> 43
<212> DNA
<213> Artificial Sequence

<220>
<223> Target Specific Oligonucleotide

<400> 652
aagttctttt gggcgatgcc agtaacgata gatttatacg acg 43

<210> 653
<211> 48
<212> DNA
<213> Artificial Sequence

<220>
<223> Target Specific Oligonucleotide

<400> 653
gttcccttat tagtctaatg ttttgcgtaa cgatagattt atacgacg 48

<210> 654
<211> 45
<212> DNA
<213> Artificial Sequence

<220>
<223> Target Specific Oligonucleotide

<400> 654
ttggaccagt gtacataagg atggtaacga tagatttata cgacg 45

<210> 655
<211> 41
<212> DNA
<213> Artificial Sequence

<220>
<223> Target Specific Oligonucleotide

<400> 655
tggcagtgca cggatggtcg taacgataga tttatacgac g 41

<210> 656
<211> 47
<212> DNA
<213> Artificial Sequence

<220>
<223> Target Specific Oligonucleotide

<400> 656
ataccaattc tagtgagaag ttcacgtaac gatagattta tacgacg 47

<210> 657
<211> 44
<212> DNA
<213> Artificial Sequence

<220>
<223> Target Specific Oligonucleotide

<400> 657
tctctgtagg gtaaggctct gtgtaacgat agatttatac gacg 44

<210> 658
<211> 45
<212> DNA
<213> Artificial Sequence

<220>
<223> Target Specific Oligonucleotide

<400> 658
tttagaggta gaccattgtg tgggtaacga tagatttata cgacg 45

<210> 659
<211> 42

<212> DNA
<213> Artificial Sequence

<220>
<223> Target Specific Oligonucleotide

<400> 659
agagagggca ctctacgctt gtaacgatag atttatacga cg 42

<210> 660
<211> 44
<212> DNA
<213> Artificial Sequence

<220>
<223> Target Specific Oligonucleotide

<400> 660
caaaaagccc tagctcagat gggtaacgat agatttatac gacg 44

<210> 661
<211> 43
<212> DNA
<213> Artificial Sequence

<220>
<223> Target Specific Oligonucleotide

<400> 661
cctctcctcc ctatttggct ggtaacgata gatttatacg acg 43

<210> 662
<211> 46
<212> DNA
<213> Artificial Sequence

<220>
<223> Target Specific Oligonucleotide

<400> 662
ttttacaacg aactcattag gtccgtaacg atagatttat acgacg 46

<210> 663
<211> 45
<212> DNA
<213> Artificial Sequence

<220>
<223> Target Specific Oligonucleotide

<400> 663
ggagtagatc ccccatctat tccgtaacga tagatttata cgacg 45

<210> 664
<211> 44
<212> DNA
<213> Artificial Sequence

<220>
<223> Target Specific Oligonucleotide

<400> 664
gtctcttgcc tcgtttctct ccgtaacgat agatttatac gacg 44

<210> 665
<211> 43
<212> DNA
<213> Artificial Sequence

<220>
<223> Target Specific Oligonucleotide

<400> 665
aattctggcg tagcacaaac agtaacgata gatttatacg acg 43

<210> 666
<211> 45
<212> DNA
<213> Artificial Sequence

<220>
<223> Target Specific Oligonucleotide

<400> 666
ggccatgaac ccctaaatct aaagtaacga tagatttata cgacg 45

<210> 667
<211> 42
<212> DNA
<213> Artificial Sequence

<220>
<223> Target Specific Oligonucleotide

<400> 667
gcccactggt accatcttgg gtaacgatag atttatacga cg 42

<210> 668
<211> 42
<212> DNA
<213> Artificial Sequence

<220>
<223> Target Specific Oligonucleotide

<400> 668
taccacgtgg cactaacaca gtaacgatag atttatacga cg 42

<210> 669
<211> 45
<212> DNA
<213> Artificial Sequence

<220>
<223> Target Specific Oligonucleotide

<400> 669
ttctagcttt tagaccagca gttgtaacga tagatttata cgacg 45

<210> 670
<211> 44
<212> DNA
<213> Artificial Sequence

<220>
<223> Target Specific Oligonucleotide

<400> 670
cccaagtgct agtagaacct gcgtaacgat agatttatac gacg 44

<210> 671
<211> 47
<212> DNA
<213> Artificial Sequence

<220>
<223> Target Specific Oligonucleotide

<400> 671
aaacacatct atactcaaag acagagtaac gatagattta tacgacg 47

<210> 672
<211> 43
<212> DNA
<213> Artificial Sequence

<220>
<223> Target Specific Oligonucleotide

<400> 672
cacagacttg ccgactcttt ggtaacgata gatttatacg acg 43

<210> 673
<211> 44
<212> DNA
<213> Artificial Sequence

<220>
<223> Target Specific Oligonucleotide

<400> 673
cacatgttaa tgttcccgac cagtaacgat agatttatac gacg 44

<210> 674
<211> 44
<212> DNA
<213> Artificial Sequence

<220>
<223> Target Specific Oligonucleotide

<400> 674
attaatcgtt tgcggatctg ccgtaacgat agatttatac gacg 44

<210> 675
<211> 44
<212> DNA
<213> Artificial Sequence

<220>
<223> Target Specific Oligonucleotide

<400> 675
tgggtaacat cggacaagtt tggtaacgat agatttatac gacg 44

<210> 676
<211> 44
<212> DNA
<213> Artificial Sequence

<220>
<223> Target Specific Oligonucleotide

<400> 676
tctgaagttt cgagccaatg ttgtaacgat agatttatac gacg 44

<210> 677
<211> 44
<212> DNA
<213> Artificial Sequence

<220>
<223> Target Specific Oligonucleotide

<400> 677
ccattttcac cgctgaaata gagtaacgat agatttatac gacg 44

<210> 678
<211> 43
<212> DNA
<213> Artificial Sequence

<220>

<223> Target Specific Oligonucleotide

<400> 678
ctggatcaca tgcgttgtca ggtaacgata gatttatacg acg 43

<210> 679
<211> 45
<212> DNA
<213> Artificial Sequence

<220>
<223> Target Specific Oligonucleotide

<400> 679
atatttgcct cgtaagatcc cctgtaacga tagatttata cgacg 45

<210> 680
<211> 45
<212> DNA
<213> Artificial Sequence

<220>
<223> Target Specific Oligonucleotide

<400> 680
ctgaagcggt gttgtatcat agcgtaacga tagatttata cgacg 45

<210> 681
<211> 45
<212> DNA
<213> Artificial Sequence

<220>
<223> Target Specific Oligonucleotide

<400> 681
tagaataatg gttcgtgacc ttcgtaacga tagatttata cgacg 45

<210> 682
<211> 41
<212> DNA
<213> Artificial Sequence

<220>
<223> Target Specific Oligonucleotide

<400> 682
acctgaaggc gacgaggagg taacgataga tttatacgac g 41

<210> 683
<211> 44
<212> DNA
<213> Artificial Sequence

<220>
<223> Target Specific Oligonucleotide

<400> 683
gcctggagtc tatcgtacaa tcgtaacgat agatttatac gacg 44

<210> 684
<211> 47
<212> DNA
<213> Artificial Sequence

<220>
<223> Target Specific Oligonucleotide

<400> 684
acatttaatt agaccgtaac ccttcgtaac gatagattta tacgacg 47

<210> 685
<211> 45
<212> DNA
<213> Artificial Sequence

<220>
<223> Target Specific Oligonucleotide

<400> 685
gctctggcca tacttaacag atagtaacga tagatttata cgacg 45

<210> 686
<211> 46
<212> DNA
<213> Artificial Sequence

<220>
<223> Target Specific Oligonucleotide

<400> 686
cagcattgag tgataatgca atctgtaacg atagatttat acgacg 46

<210> 687
<211> 44
<212> DNA
<213> Artificial Sequence

<220>
<223> Target Specific Oligonucleotide

<400> 687
atacacaccc gctaccttac tggtaacgat agatttatac gacg 44

<210> 688
<211> 44
<212> DNA

<213> Artificial Sequence

<220>
<223> Target Specific Oligonucleotide

<400> 688
acaaagacga gaactatggc aagtaacgat agatttatac gacg 44

<210> 689
<211> 44
<212> DNA
<213> Artificial Sequence

<220>
<223> Target Specific Oligonucleotide

<400> 689
aggcagacgc tctaagtcta aagtaacgat agatttatac gacg 44

<210> 690
<211> 44
<212> DNA
<213> Artificial Sequence

<220>
<223> Target Specific Oligonucleotide

<400> 690
tctcttactc atcgtgaaag gcgtaacgat agatttatac gacg 44

<210> 691
<211> 42
<212> DNA
<213> Artificial Sequence

<220>
<223> Target Specific Oligonucleotide

<400> 691
gcctcaagtt cgcattcagc gtaacgatag atttatacga cg 42

<210> 692
<211> 44
<212> DNA
<213> Artificial Sequence

<220>
<223> Target Specific Oligonucleotide

<400> 692
gaaatggaaa cgttaacagc cagtaacgat agatttatac gacg 44

<210> 693

<211> 43
<212> DNA
<213> Artificial Sequence

<220>
<223> Target Specific Oligonucleotide

<400> 693
cacctgcttc ggaatctcag tgtaacgata gatttatacg acg 43

<210> 694
<211> 46
<212> DNA
<213> Artificial Sequence

<220>
<223> Target Specific Oligonucleotide

<400> 694
gacttgtgct tcgttaatta aacagtaacg atagatttat acgacg 46

<210> 695
<211> 46
<212> DNA
<213> Artificial Sequence

<220>
<223> Target Specific Oligonucleotide

<400> 695
gctgtaatat acgcccactt tagcgtaacg atagatttat acgacg 46

<210> 696
<211> 44
<212> DNA
<213> Artificial Sequence

<220>
<223> Target Specific Oligonucleotide

<400> 696
ttccattgtg cggttgggat tgtaacttat aaaggataga catc 44

<210> 697
<211> 45
<212> DNA
<213> Artificial Sequence

<220>
<223> Target Specific Oligonucleotide

<400> 697
ctccagtaat cgcttatggt ccgtaactta taaaggatag acatc 45

<210> 698
<211> 45
<212> DNA
<213> Artificial Sequence

<220>
<223> Target Specific Oligonucleotide

<400> 698
cctcttctcc gtcaagaagt tcgtaactta taaaggatag acatc 45

<210> 699
<211> 45
<212> DNA
<213> Artificial Sequence

<220>
<223> Target Specific Oligonucleotide

<400> 699
tgattgagat ggcggtattt gagtaactta taaaggatag acatc 45

<210> 700
<211> 45
<212> DNA
<213> Artificial Sequence

<220>
<223> Target Specific Oligonucleotide

<400> 700
gccaattata agtactcggg ctgtaactta taaaggatag acatc 45

<210> 701
<211> 42
<212> DNA
<213> Artificial Sequence

<220>
<223> Target Specific Oligonucleotide

<400> 701
ccaggccagc gtatgttccg taacttataa aggatagaca tc 42

<210> 702
<211> 43
<212> DNA
<213> Artificial Sequence

<220>
<223> Target Specific Oligonucleotide

<400> 702

ctggggcaac caacacttgt gtaacttata aaggatagac atc 43

<210> 703
<211> 43
<212> DNA
<213> Artificial Sequence

<220>
<223> Target Specific Oligonucleotide

<400> 703
ggctggacaa taggctccca gtaacttata aaggatagac atc 43

<210> 704
<211> 44
<212> DNA
<213> Artificial Sequence

<220>
<223> Target Specific Oligonucleotide

<400> 704
aaagctatgg cacgaaagcc ggtaacttat aaaggataga catc 44

<210> 705
<211> 44
<212> DNA
<213> Artificial Sequence

<220>
<223> Target Specific Oligonucleotide

<400> 705
gcagtccatc taaggtggac tgtaacttat aaaggataga catc 44

<210> 706
<211> 46
<212> DNA
<213> Artificial Sequence

<220>
<223> Target Specific Oligonucleotide

<400> 706
tgtatatgga gtaccctctt gctgtaactt ataaaggata gacatc 46

<210> 707
<211> 44
<212> DNA
<213> Artificial Sequence

<220>
<223> Target Specific Oligonucleotide

<400> 707
ctggatcacc aaaacggcaa tgtaacttat aaaggataga catc 44

<210> 708
<211> 45
<212> DNA
<213> Artificial Sequence

<220>
<223> Target Specific Oligonucleotide

<400> 708
ccaggataac ctttacttgc cagtaactta taaaggatag acatc 45

<210> 709
<211> 46
<212> DNA
<213> Artificial Sequence

<220>
<223> Target Specific Oligonucleotide

<400> 709
atttccagct gcgatatcta ctcgtaactt ataaaggata gacatc 46

<210> 710
<211> 47
<212> DNA
<213> Artificial Sequence

<220>
<223> Target Specific Oligonucleotide

<400> 710
tttatagcag ctagtccttt cttggtaact tataaaggat agacatc 47

<210> 711
<211> 46
<212> DNA
<213> Artificial Sequence

<220>
<223> Target Specific Oligonucleotide

<400> 711
tgatatgaga ttttcccgga tgggtaactt ataaaggata gacatc 46

<210> 712
<211> 43
<212> DNA
<213> Artificial Sequence

<220>
<223> Target Specific Oligonucleotide

<400> 712
tgacaacagt tagggcggtg gtaacttata aaggatagac atc 43

<210> 713
<211> 44
<212> DNA
<213> Artificial Sequence

<220>
<223> Target Specific Oligonucleotide

<400> 713
ttggttcccg agtactgtct agtaacttat aaaggataga catc 44

<210> 714
<211> 45
<212> DNA
<213> Artificial Sequence

<220>
<223> Target Specific Oligonucleotide

<400> 714
gtttagttcc aacgccatct gtgtaactta taaaggatag acatc 45

<210> 715
<211> 45
<212> DNA
<213> Artificial Sequence

<220>
<223> Target Specific Oligonucleotide

<400> 715
aagagttgcg acagtatcac ccgtaactta taaaggatag acatc 45

<210> 716
<211> 44
<212> DNA
<213> Artificial Sequence

<220>
<223> Target Specific Oligonucleotide

<400> 716
gctgctggat atccctcaga agtaacttat aaaggataga catc 44

<210> 717
<211> 46
<212> DNA
<213> Artificial Sequence

<220>
<223> Target Specific Oligonucleotide

<400> 717
tgcaagacag actatgtcta tgggtaactt ataaaggata gacatc 46

<210> 718
<211> 46
<212> DNA
<213> Artificial Sequence

<220>
<223> Target Specific Oligonucleotide

<400> 718
cttcaaggac taggtcaatt gcagtaactt ataaaggata gacatc 46

<210> 719
<211> 47
<212> DNA
<213> Artificial Sequence

<220>
<223> Target Specific Oligonucleotide

<400> 719
gatatgctgc actaactagt cttggtaact tataaaggat agacatc 47

<210> 720
<211> 40
<212> DNA
<213> Artificial Sequence

<220>
<223> Target Specific Oligonucleotide

<400> 720
atctcgagca agacgttcag tgtaacggtt atcgtggaaa 40

<210> 721
<211> 43
<212> DNA
<213> Artificial Sequence

<220>
<223> Target Specific Oligonucleotide

<400> 721
ctgtccataa ttagtccatg aggagtaacg gttatcgtgg aaa 43

<210> 722
<211> 43

<212> DNA
<213> Artificial Sequence

<220>
<223> Target Specific Oligonucleotide

<400> 722
atctttggat tatactgcct gaccgtaacg gttatcgtgg aaa 43

<210> 723
<211> 39
<212> DNA
<213> Artificial Sequence

<220>
<223> Target Specific Oligonucleotide

<400> 723
cagcaagctt gcgaccttga gtaacggtta tcgtggaaa 39

<210> 724
<211> 44
<212> DNA
<213> Artificial Sequence

<220>
<223> Target Specific Oligonucleotide

<400> 724
agttaaagtt gagagatcat ctccagtaac ggttatcgtg gaaa 44

<210> 725
<211> 44
<212> DNA
<213> Artificial Sequence

<220>
<223> Target Specific Oligonucleotide

<400> 725
aggaatggat ctatcactat ttctagtaac ggttatcgtg gaaa 44

<210> 726
<211> 44
<212> DNA
<213> Artificial Sequence

<220>
<223> Target Specific Oligonucleotide

<400> 726
cagcataatg attaggtatg caaaagtaac ggttatcgtg gaaa 44

<210> 727
<211> 44
<212> DNA
<213> Artificial Sequence

<220>
<223> Target Specific Oligonucleotide

<400> 727
ttcagtctga taaaatctac agtcagtaac ggttatcgtg gaaa 44

<210> 728
<211> 39
<212> DNA
<213> Artificial Sequence

<220>
<223> Target Specific Oligonucleotide

<400> 728
tccaacactt cgtggggtcc gtaacggtta tcgtggaaa 39

<210> 729
<211> 39
<212> DNA
<213> Artificial Sequence

<220>
<223> Target Specific Oligonucleotide

<400> 729
agggctacaa tgtgatggcc gtaacggtta tcgtggaaa 39

<210> 730
<211> 42
<212> DNA
<213> Artificial Sequence

<220>
<223> Target Specific Oligonucleotide

<400> 730
tcaaggtcat aacctggttc atcgtaacgg ttatcgtgga aa 42

<210> 731
<211> 42
<212> DNA
<213> Artificial Sequence

<220>
<223> Target Specific Oligonucleotide

<400> 731
cctacaacaa acttgtctgg aatgtaacgg ttatcgtgga aa 42

<210> 732
<211> 40
<212> DNA
<213> Artificial Sequence

<220>
<223> Target Specific Oligonucleotide

<400> 732
aaacaacaat ccgcccaaag ggtaacggtt atcgtggaaa 40

<210> 733
<211> 41
<212> DNA
<213> Artificial Sequence

<220>
<223> Target Specific Oligonucleotide

<400> 733
aaagctctac taagcagatg gcgtaacggt tatcgtggaa a 41

<210> 734
<211> 41
<212> DNA
<213> Artificial Sequence

<220>
<223> Target Specific Oligonucleotide

<400> 734
actgctgaca aagattcact gggtaacggt tatcgtggaa a 41

<210> 735
<211> 44
<212> DNA
<213> Artificial Sequence

<220>
<223> Target Specific Oligonucleotide

<400> 735
ctgatagtct ataggctcat agtgcgtaac ggttatcgtg gaaa 44

<210> 736
<211> 40
<212> DNA
<213> Artificial Sequence

<220>
<223> Target Specific Oligonucleotide

<400> 736
atcactaatc acgacgccag ggtaacggtt atcgtggaaa 40

<210> 737
<211> 40
<212> DNA
<213> Artificial Sequence

<220>
<223> Target Specific Oligonucleotide

<400> 737
tggcgatgtc aataggactc cgtaacggtt atcgtggaaa 40

<210> 738
<211> 42
<212> DNA
<213> Artificial Sequence

<220>
<223> Target Specific Oligonucleotide

<400> 738
gaactctcat cttaggcttt gtagtaacgg ttatcgtgga aa 42

<210> 739
<211> 44
<212> DNA
<213> Artificial Sequence

<220>
<223> Target Specific Oligonucleotide

<400> 739
tcaacattac tgaaacacta ctaaagtaac ggttatcgtg gaaa 44

<210> 740
<211> 38
<212> DNA
<213> Artificial Sequence

<220>
<223> Target Specific Oligonucleotide

<400> 740
ggtcgccata acggagccgg taacggttat cgtggaaa 38

<210> 741
<211> 45
<212> DNA
<213> Artificial Sequence

<220>

<223> Target Specific Oligonucleotide

<400> 741
ttcttacaga tatgagttca atgtttgtaa cggttatcgt ggaaa 45

<210> 742
<211> 44
<212> DNA
<213> Artificial Sequence

<220>
<223> Target Specific Oligonucleotide

<400> 742
aggacagaac aaaacttctt agatggtaac ggttatcgtg gaaa 44

<210> 743
<211> 36
<212> DNA
<213> Artificial Sequence

<220>
<223> Target Specific Oligonucleotide

<400> 743
tcgcagccgc ctgaagcgta acggttatcg tggaaa 36

<210> 744
<211> 41
<212> DNA
<213> Artificial Sequence

<220>
<223> Target Specific Oligonucleotide

<400> 744
ggaatttgga accggcttgc gtaacggtac tttatctagc t 41

<210> 745
<211> 46
<212> DNA
<213> Artificial Sequence

<220>
<223> Target Specific Oligonucleotide

<400> 745
agagtcttga gtataatctt ggtaggtaac ggtactttat ctagct 46

<210> 746
<211> 46
<212> DNA
<213> Artificial Sequence

<220>
<223> Target Specific Oligonucleotide

<400> 746
attcaggaag catacactaa ttcttgtaac ggtactttat ctagct 46

<210> 747
<211> 44
<212> DNA
<213> Artificial Sequence

<220>
<223> Target Specific Oligonucleotide

<400> 747
tccagaaata agcgaaaata gcagtaacgg tactttatct agct 44

<210> 748
<211> 47
<212> DNA
<213> Artificial Sequence

<220>
<223> Target Specific Oligonucleotide

<400> 748
atcttttgaa ctactagcta caaaatgtaa cggtacttta tctagct 47

<210> 749
<211> 42
<212> DNA
<213> Artificial Sequence

<220>
<223> Target Specific Oligonucleotide

<400> 749
taacaagctc gtaatggccc agtaacggta ctttatctag ct 42

<210> 750
<211> 42
<212> DNA
<213> Artificial Sequence

<220>
<223> Target Specific Oligonucleotide

<400> 750
catccaaacc gtgtgaaagc tgtaacggta ctttatctag ct 42

<210> 751
<211> 41
<212> DNA

<213> Artificial Sequence

<220>
<223> Target Specific Oligonucleotide

<400> 751
acaacgccat gtgcgatttg gtaacggtac tttatctagc t 41

<210> 752
<211> 45
<212> DNA
<213> Artificial Sequence

<220>
<223> Target Specific Oligonucleotide

<400> 752
ctacctacca ctaggtacct atcagtaacg gtactttatc tagct 45

<210> 753
<211> 43
<212> DNA
<213> Artificial Sequence

<220>
<223> Target Specific Oligonucleotide

<400> 753
aggacattta cgtgtacagc acgtaacggt actttatcta gct 43

<210> 754
<211> 43
<212> DNA
<213> Artificial Sequence

<220>
<223> Target Specific Oligonucleotide

<400> 754
cgcctgaata caacgagtta acgtaacggt actttatcta gct 43

<210> 755
<211> 44
<212> DNA
<213> Artificial Sequence

<220>
<223> Target Specific Oligonucleotide

<400> 755
cttccagatt gtcgccgtta attgtaacgg tactttatct agct 44

<210> 756

<211> 44
<212> DNA
<213> Artificial Sequence

<220>
<223> Target Specific Oligonucleotide

<400> 756
cagatttacc taaaccacca ccagtaacgg tactttatct agct 44

<210> 757
<211> 45
<212> DNA
<213> Artificial Sequence

<220>
<223> Target Specific Oligonucleotide

<400> 757
tttgagctga tctagtaggt cttcgtaacg gtactttatc tagct 45

<210> 758
<211> 41
<212> DNA
<213> Artificial Sequence

<220>
<223> Target Specific Oligonucleotide

<400> 758
tctgtagctc gatccctgga gtaacggtac tttatctagc t 41

<210> 759
<211> 44
<212> DNA
<213> Artificial Sequence

<220>
<223> Target Specific Oligonucleotide

<400> 759
caactgtcat tcggttaaac tgagtaacgg tactttatct agct 44

<210> 760
<211> 44
<212> DNA
<213> Artificial Sequence

<220>
<223> Target Specific Oligonucleotide

<400> 760
tttgcttccg ttcatcatta gtcgtaacgg tactttatct agct 44

<210> 761
<211> 45
<212> DNA
<213> Artificial Sequence

<220>
<223> Target Specific Oligonucleotide

<400> 761
acttccaata gtctggaaat cacagtaacg gtactttatc tagct 45

<210> 762
<211> 45
<212> DNA
<213> Artificial Sequence

<220>
<223> Target Specific Oligonucleotide

<400> 762
tcatctgctg gattaagcta atacgtaacg gtactttatc tagct 45

<210> 763
<211> 41
<212> DNA
<213> Artificial Sequence

<220>
<223> Target Specific Oligonucleotide

<400> 763
agataggctc ccgtatgccc gtaacggtac tttatctagc t 41

<210> 764
<211> 41
<212> DNA
<213> Artificial Sequence

<220>
<223> Target Specific Oligonucleotide

<400> 764
gcttctcggc taccacagac gtaacggtac tttatctagc t 41

<210> 765
<211> 46
<212> DNA
<213> Artificial Sequence

<220>
<223> Target Specific Oligonucleotide

<400> 765

agacttactc ttaagaccat acttcgtaac ggtactttat ctagct 46

<210> 766
<211> 42
<212> DNA
<213> Artificial Sequence

<220>
<223> Target Specific Oligonucleotide

<400> 766
gttgagaggt ggcgtttttg agtaacggta ctttatctag ct 42

<210> 767
<211> 44
<212> DNA
<213> Artificial Sequence

<220>
<223> Target Specific Oligonucleotide

<400> 767
tcaatacgtt tctgcaagag tttgtaacgg tactttatct agct 44

<210> 768
<211> 41
<212> DNA
<213> Artificial Sequence

<220>
<223> Target Specific Oligonucleotide

<400> 768
ttatggcctc tatagcatct cccgtaactc catctagacc g 41

<210> 769
<211> 40
<212> DNA
<213> Artificial Sequence

<220>
<223> Target Specific Oligonucleotide

<400> 769
cagtccgggc aactatctta ttgtaactcc atctagaccg 40

<210> 770
<211> 42
<212> DNA
<213> Artificial Sequence

<220>
<223> Target Specific Oligonucleotide

<400> 770
ttcaagatac gaataatcct ccgagtaact ccatctagac cg 42

<210> 771
<211> 40
<212> DNA
<213> Artificial Sequence

<220>
<223> Target Specific Oligonucleotide

<400> 771
gtaggcgctt agcttgattt tcgtaactcc atctagaccg 40

<210> 772
<211> 38
<212> DNA
<213> Artificial Sequence

<220>
<223> Target Specific Oligonucleotide

<400> 772
cttcaagtcg ggctcttgca gtaactccat ctagaccg 38

<210> 773
<211> 38
<212> DNA
<213> Artificial Sequence

<220>
<223> Target Specific Oligonucleotide

<400> 773
agaatgaggc acgcaaagct gtaactccat ctagaccg 38

<210> 774
<211> 40
<212> DNA
<213> Artificial Sequence

<220>
<223> Target Specific Oligonucleotide

<400> 774
ctcagcatcc gataggactt tcgtaactcc atctagaccg 40

<210> 775
<211> 40
<212> DNA
<213> Artificial Sequence

<220>
<223> Target Specific Oligonucleotide

<400> 775
aagtcactat aacacccgtc ctgtaactcc atctagaccg 40

<210> 776
<211> 39
<212> DNA
<213> Artificial Sequence

<220>
<223> Target Specific Oligonucleotide

<400> 776
ttggcatggt agatcgtcca ggtaactcca tctagaccg 39

<210> 777
<211> 39
<212> DNA
<213> Artificial Sequence

<220>
<223> Target Specific Oligonucleotide

<400> 777
caccttccaa cgactcaatc cgtaactcca tctagaccg 39

<210> 778
<211> 42
<212> DNA
<213> Artificial Sequence

<220>
<223> Target Specific Oligonucleotide

<400> 778
acaacctgta agtgtagttc tctggtaact ccatctagac cg 42

<210> 779
<211> 43
<212> DNA
<213> Artificial Sequence

<220>
<223> Target Specific Oligonucleotide

<400> 779
aaatcagata ctcgtctaca gaatggtaac tccatctaga ccg 43

<210> 780
<211> 38
<212> DNA
<213> Artificial Sequence

<220>
<223> Target Specific Oligonucleotide

<400> 780
agcagcaagt cgcattttcc gtaactccat ctagaccg 38

<210> 781
<211> 39
<212> DNA
<213> Artificial Sequence

<220>
<223> Target Specific Oligonucleotide

<400> 781
actggcatgg cggataaaaa ggtaactcca tctagaccg 39

<210> 782
<211> 38
<212> DNA
<213> Artificial Sequence

<220>
<223> Target Specific Oligonucleotide

<400> 782
cgggtgctct cgtgaacaaa gtaactccat ctagaccg 38

<210> 783
<211> 41
<212> DNA
<213> Artificial Sequence

<220>
<223> Target Specific Oligonucleotide

<400> 783
gatcgaagag tcgagataag gatgtaactc catctagacc g 41

<210> 784
<211> 41
<212> DNA
<213> Artificial Sequence

<220>
<223> Target Specific Oligonucleotide

<400> 784
ttgttgaaga cgcttccata agtgtaactc catctagacc g 41

<210> 785
<211> 37

<212> DNA
<213> Artificial Sequence

<220>
<223> Target Specific Oligonucleotide

<400> 785
gtggtgacgc agaatcccgg taactccatc tagaccg 37

<210> 786
<211> 45
<212> DNA
<213> Artificial Sequence

<220>
<223> Target Specific Oligonucleotide

<400> 786
cattataaag acgtaataat ttagggtgta actccatcta gaccg 45

<210> 787
<211> 39
<212> DNA
<213> Artificial Sequence

<220>
<223> Target Specific Oligonucleotide

<400> 787
gctcccactc ctagacatgt tgtaactcca tctagaccg 39

<210> 788
<211> 40
<212> DNA
<213> Artificial Sequence

<220>
<223> Target Specific Oligonucleotide

<400> 788
tttgttacac ggttaaacag ccgtaactcc atctagaccg 40

<210> 789
<211> 42
<212> DNA
<213> Artificial Sequence

<220>
<223> Target Specific Oligonucleotide

<400> 789
gaggtaaata aacgtcgata ggaagtaact ccatctagac cg 42

<210> 790
<211> 40
<212> DNA
<213> Artificial Sequence

<220>
<223> Target Specific Oligonucleotide

<400> 790
ttaatgtgga gtgcaagttg gggtaactcc atctagaccg 40

<210> 791
<211> 41
<212> DNA
<213> Artificial Sequence

<220>
<223> Target Specific Oligonucleotide

<400> 791
ccttgtatct tagcttggga tgtgtaactc catctagacc g 41

<210> 792
<211> 43
<212> DNA
<213> Artificial Sequence

<220>
<223> Target Specific Oligonucleotide

<400> 792
tcaatgatca gcggatacct attgtaactc agtagatata gcg 43

<210> 793
<211> 43
<212> DNA
<213> Artificial Sequence

<220>
<223> Target Specific Oligonucleotide

<400> 793
cgtgtgatct tacgatcctt atagtaactc agtagatata gcg 43

<210> 794
<211> 43
<212> DNA
<213> Artificial Sequence

<220>
<223> Target Specific Oligonucleotide

<400> 794
tcatcctcta cgagtctatc ttggtaactc agtagatata gcg 43

<210> 795
<211> 40
<212> DNA
<213> Artificial Sequence

<220>
<223> Target Specific Oligonucleotide

<400> 795
tcaaagtaac ccgcgtaggc gtaactcagt agatatagcg 40

<210> 796
<211> 41
<212> DNA
<213> Artificial Sequence

<220>
<223> Target Specific Oligonucleotide

<400> 796
ataggacgca tactgtcgca agtaactcag tagatatagc g 41

<210> 797
<211> 41
<212> DNA
<213> Artificial Sequence

<220>
<223> Target Specific Oligonucleotide

<400> 797
ttatgtccgt cgaccttgca tgtaactcag tagatatagc g 41

<210> 798
<211> 42
<212> DNA
<213> Artificial Sequence

<220>
<223> Target Specific Oligonucleotide

<400> 798
ttccaaggaa cggacttcta gggtaactca gtagatatag cg 42

<210> 799
<211> 42
<212> DNA
<213> Artificial Sequence

<220>
<223> Target Specific Oligonucleotide

<400> 799
ataggtacga acgctctcga tcgtaactca gtagatatag cg 42

<210> 800
<211> 42
<212> DNA
<213> Artificial Sequence

<220>
<223> Target Specific Oligonucleotide

<400> 800
tcttggatga tcgcatgaaa ccgtaactca gtagatatag cg 42

<210> 801
<211> 42
<212> DNA
<213> Artificial Sequence

<220>
<223> Target Specific Oligonucleotide

<400> 801
acaacccaat taacgtgaag ccgtaactca gtagatatag cg 42

<210> 802
<211> 41
<212> DNA
<213> Artificial Sequence

<220>
<223> Target Specific Oligonucleotide

<400> 802
atgcggtata acgcacgttc cgtaactcag tagatatagc g 41

<210> 803
<211> 41
<212> DNA
<213> Artificial Sequence

<220>
<223> Target Specific Oligonucleotide

<400> 803
ctcaagtcac gacgcagttc tgtaactcag tagatatagc g 41

<210> 804
<211> 40
<212> DNA
<213> Artificial Sequence

<220>

<223> Target Specific Oligonucleotide

<400> 804
gcaagtcacg aacgtgactc gtaactcagt agatatagcg 40

<210> 805
<211> 40
<212> DNA
<213> Artificial Sequence

<220>
<223> Target Specific Oligonucleotide

<400> 805
ttggcgttat actgcgccac gtaactcagt agatatagcg 40

<210> 806
<211> 40
<212> DNA
<213> Artificial Sequence

<220>
<223> Target Specific Oligonucleotide

<400> 806
agtggtgcgt atcgtaagcg gtaactcagt agatatagcg 40

<210> 807
<211> 40
<212> DNA
<213> Artificial Sequence

<220>
<223> Target Specific Oligonucleotide

<400> 807
atccaggcga cgaaacgaga gtaactcagt agatatagcg 40

<210> 808
<211> 40
<212> DNA
<213> Artificial Sequence

<220>
<223> Target Specific Oligonucleotide

<400> 808
ttggatgaat cggcaggctg gtaactcagt agatatagcg 40

<210> 809
<211> 43
<212> DNA
<213> Artificial Sequence

<220>
<223> Target Specific Oligonucleotide

<400> 809
tcagtcaatt cgtaatgtac accgtaactc agtagatata gcg 43

<210> 810
<211> 41
<212> DNA
<213> Artificial Sequence

<220>
<223> Target Specific Oligonucleotide

<400> 810
catcgaagac gatccactgg cgtaactcag tagatatagc g 41

<210> 811
<211> 39
<212> DNA
<213> Artificial Sequence

<220>
<223> Target Specific Oligonucleotide

<400> 811
cacggatcaa gcggcacttg taactcagta gatatagcg 39

<210> 812
<211> 39
<212> DNA
<213> Artificial Sequence

<220>
<223> Target Specific Oligonucleotide

<400> 812
tccaccgtat cgcaagctgg taactcagta gatatagcg 39

<210> 813
<211> 41
<212> DNA
<213> Artificial Sequence

<220>
<223> Target Specific Oligonucleotide

<400> 813
atgcaaagtt tcgtagggcg agtaactcag tagatatagc g 41

<210> 814
<211> 38
<212> DNA

<213> Artificial Sequence

<220>
<223> Target Specific Oligonucleotide

<400> 814
gcgttggcac gacgacatgt aactcagtag atatagcg 38

<210> 815
<211> 38
<212> DNA
<213> Artificial Sequence

<220>
<223> Target Specific Oligonucleotide

<400> 815
ttgcagacgg acgcccaagt aactcagtag atatagcg 38

<210> 816
<211> 40
<212> DNA
<213> Artificial Sequence

<220>
<223> Target Specific Oligonucleotide

<400> 816
agctgtatcg tcaaggcact cgtaaccttt tgaaacgcta 40

<210> 817
<211> 39
<212> DNA
<213> Artificial Sequence

<220>
<223> Target Specific Oligonucleotide

<400> 817
ctcgctccca gtccgaaatg gtaacctttt gaaacgcta 39

<210> 818
<211> 44
<212> DNA
<213> Artificial Sequence

<220>
<223> Target Specific Oligonucleotide

<400> 818
acttgtacta gtatgcctta agaaagtaac cttttgaaac gcta 44

<210> 819

<211> 42
<212> DNA
<213> Artificial Sequence

<220>
<223> Target Specific Oligonucleotide

<400> 819
gtgaccatga ctaatagcag tgggtaacct tttgaaacgc ta 42

<210> 820
<211> 41
<212> DNA
<213> Artificial Sequence

<220>
<223> Target Specific Oligonucleotide

<400> 820
gctgtgtcga gaatatccaa gagtaacctt ttgaaacgct a 41

<210> 821
<211> 41
<212> DNA
<213> Artificial Sequence

<220>
<223> Target Specific Oligonucleotide

<400> 821
cctactgtcg ctaatggatt gggtaacctt ttgaaacgct a 41

<210> 822
<211> 41
<212> DNA
<213> Artificial Sequence

<220>
<223> Target Specific Oligonucleotide

<400> 822
acactagcac tacctaagga ccgtaacctt ttgaaacgct a 41

<210> 823
<211> 41
<212> DNA
<213> Artificial Sequence

<220>
<223> Target Specific Oligonucleotide

<400> 823
gtctgtgaac tagttcaggc acgtaacctt ttgaaacgct a 41

<210> 824
<211> 40
<212> DNA
<213> Artificial Sequence

<220>
<223> Target Specific Oligonucleotide

<400> 824
ctaggtcagc gcaaccaaat ggtaaccttt tgaaacgcta 40

<210> 825
<211> 42
<212> DNA
<213> Artificial Sequence

<220>
<223> Target Specific Oligonucleotide

<400> 825
cctaataagc tataactggc ccagtaacct tttgaaacgc ta 42

<210> 826
<211> 43
<212> DNA
<213> Artificial Sequence

<220>
<223> Target Specific Oligonucleotide

<400> 826
ctaaattcct atgcagtgtg actcgtaacc ttttgaaacg cta 43

<210> 827
<211> 42
<212> DNA
<213> Artificial Sequence

<220>
<223> Target Specific Oligonucleotide

<400> 827
atcttgttga gctatccaaa ctggtaacct tttgaaacgc ta 42

<210> 828
<211> 42
<212> DNA
<213> Artificial Sequence

<220>
<223> Target Specific Oligonucleotide

<400> 828

aaatacgcat cgtgttatct ctggtaacct tttgaaacgc ta 42

<210> 829
<211> 42
<212> DNA
<213> Artificial Sequence

<220>
<223> Target Specific Oligonucleotide

<400> 829
tgttaagaga actagccaaa cctgtaacct tttgaaacgc ta 42

<210> 830
<211> 43
<212> DNA
<213> Artificial Sequence

<220>
<223> Target Specific Oligonucleotide

<400> 830
cataaagtag cttgatcgaa gagtgtaacc ttttgaaacg cta 43

<210> 831
<211> 41
<212> DNA
<213> Artificial Sequence

<220>
<223> Target Specific Oligonucleotide

<400> 831
taagcaaaca cgcctttaca tagtaacctt ttgaaacgct a 41

<210> 832
<211> 44
<212> DNA
<213> Artificial Sequence

<220>
<223> Target Specific Oligonucleotide

<400> 832
aaggccttag taagatatta cagacgtaac cttttgaaac gcta 44

<210> 833
<211> 45
<212> DNA
<213> Artificial Sequence

<220>
<223> Target Specific Oligonucleotide

<400> 833
acaatatacg tctgctatat tcttccgtaa ccttttgaaa cgcta 45

<210> 834
<211> 41
<212> DNA
<213> Artificial Sequence

<220>
<223> Target Specific Oligonucleotide

<400> 834
tccactggat agggttctgt ctgtaacctt ttgaaacgct a 41

<210> 835
<211> 44
<212> DNA
<213> Artificial Sequence

<220>
<223> Target Specific Oligonucleotide

<400> 835
aagttatact atgaaagagc agtctgtaac cttttgaaac gcta 44

<210> 836
<211> 44
<212> DNA
<213> Artificial Sequence

<220>
<223> Target Specific Oligonucleotide

<400> 836
acatgttaat gcctaagtct atgtagtaac cttttgaaac gcta 44

<210> 837
<211> 40
<212> DNA
<213> Artificial Sequence

<220>
<223> Target Specific Oligonucleotide

<400> 837
gcctagaatg cctacttggg agtaaccttt tgaaacgcta 40

<210> 838
<211> 41
<212> DNA
<213> Artificial Sequence

<220>
<223> Target Specific Oligonucleotide

<400> 838
tcagtggaat cgtagcaaaa cagtaacctt ttgaaacgct a 41

<210> 839
<211> 45
<212> DNA
<213> Artificial Sequence

<220>
<223> Target Specific Oligonucleotide

<400> 839
acaataatta ggagtagtac agttcagtaa ccttttgaaa cgcta 45

<210> 840
<211> 42
<212> DNA
<213> Artificial Sequence

<220>
<223> Target Specific Oligonucleotide

<400> 840
ttccactcgg ataagatgct gagtaactca gacatgtaga tt 42

<210> 841
<211> 42
<212> DNA
<213> Artificial Sequence

<220>
<223> Target Specific Oligonucleotide

<400> 841
aatactccac acgcaaattt ccgtaactca gacatgtaga tt 42

<210> 842
<211> 40
<212> DNA
<213> Artificial Sequence

<220>
<223> Target Specific Oligonucleotide

<400> 842
ggccagacca tcgctatctg gtaactcaga catgtagatt 40

<210> 843
<211> 42
<212> DNA
<213> Artificial Sequence

<220>
<223> Target Specific Oligonucleotide

<400> 843
caccacacta tgtcgaaaag tggtaactca gacatgtaga tt 42

<210> 844
<211> 42
<212> DNA
<213> Artificial Sequence

<220>
<223> Target Specific Oligonucleotide

<400> 844
ccctttcttg cggagattct ctgtaactca gacatgtaga tt 42

<210> 845
<211> 40
<212> DNA
<213> Artificial Sequence

<220>
<223> Target Specific Oligonucleotide

<400> 845
acaaacacgc acctcaaagc gtaactcaga catgtagatt 40

<210> 846
<211> 42
<212> DNA
<213> Artificial Sequence

<220>
<223> Target Specific Oligonucleotide

<400> 846
cccagtagat taccactgga gtgtaactca gacatgtaga tt 42

<210> 847
<211> 39
<212> DNA
<213> Artificial Sequence

<220>
<223> Target Specific Oligonucleotide

<400> 847
gcagtgctcg cttagtgctg taactcagac atgtagatt 39

<210> 848
<211> 40

<212> DNA
<213> Artificial Sequence

<220>
<223> Target Specific Oligonucleotide

<400> 848
ggctcgacgc taggatctga gtaactcaga catgtagatt 40

<210> 849
<211> 39
<212> DNA
<213> Artificial Sequence

<220>
<223> Target Specific Oligonucleotide

<400> 849
aggatgggcc tccggttcag taactcagac atgtagatt 39

<210> 850
<211> 40
<212> DNA
<213> Artificial Sequence

<220>
<223> Target Specific Oligonucleotide

<400> 850
ggaacatctc gaagcgctca gtaactcaga catgtagatt 40

<210> 851
<211> 40
<212> DNA
<213> Artificial Sequence

<220>
<223> Target Specific Oligonucleotide

<400> 851
agacggaaac cgtagctgcc gtaactcaga catgtagatt 40

<210> 852
<211> 41
<212> DNA
<213> Artificial Sequence

<220>
<223> Target Specific Oligonucleotide

<400> 852
tgttcaatat cgtccgggga cgtaactcag acatgtagat t 41

<210> 853
<211> 40
<212> DNA
<213> Artificial Sequence

<220>
<223> Target Specific Oligonucleotide

<400> 853
cattgcttgg gacggcaagg gtaactcaga catgtagatt 40

<210> 854
<211> 42
<212> DNA
<213> Artificial Sequence

<220>
<223> Target Specific Oligonucleotide

<400> 854
agtggagaat gtcagtctga gtgtaactca gacatgtaga tt 42

<210> 855
<211> 43
<212> DNA
<213> Artificial Sequence

<220>
<223> Target Specific Oligonucleotide

<400> 855
agttttttat ggcgggaggt agagtaactc agacatgtag att 43

<210> 856
<211> 41
<212> DNA
<213> Artificial Sequence

<220>
<223> Target Specific Oligonucleotide

<400> 856
tagaaactac caacccaccg agtaactcag acatgtagat t 41

<210> 857
<211> 42
<212> DNA
<213> Artificial Sequence

<220>
<223> Target Specific Oligonucleotide

<400> 857
ttccctggtt agtacggtga aggtaactca gacatgtaga tt 42

<210> 858
<211> 42
<212> DNA
<213> Artificial Sequence

<220>
<223> Target Specific Oligonucleotide

<400> 858
taagctggta tgtcctactc ccgtaactca gacatgtaga tt 42

<210> 859
<211> 41
<212> DNA
<213> Artificial Sequence

<220>
<223> Target Specific Oligonucleotide

<400> 859
ctgacgcaca cctattgcaa ggtaactcag acatgtagat t 41

<210> 860
<211> 39
<212> DNA
<213> Artificial Sequence

<220>
<223> Target Specific Oligonucleotide

<400> 860
tcaccgtcgt ggaaagcacg taactcagac atgtagatt 39

<210> 861
<211> 44
<212> DNA
<213> Artificial Sequence

<220>
<223> Target Specific Oligonucleotide

<400> 861
gtaaactaac ccttaactgc aagagtaact cagacatgta gatt 44

<210> 862
<211> 44
<212> DNA
<213> Artificial Sequence

<220>
<223> Target Specific Oligonucleotide

<400> 862
tttaggtact aaggttcacc aagagtaact cagacatgta gatt 44

<210> 863
<211> 46
<212> DNA
<213> Artificial Sequence

<220>
<223> Target Specific Oligonucleotide

<400> 863
tcatcatata caagagatga aatcctgtaa ctcagacatg tagatt 46

<210> 864
<211> 41
<212> DNA
<213> Artificial Sequence

<220>
<223> Target Specific Oligonucleotide

<400> 864
ccatggtgac gatcctcacg gtaactaaca taaattcgat g 41

<210> 865
<211> 42
<212> DNA
<213> Artificial Sequence

<220>
<223> Target Specific Oligonucleotide

<400> 865
gtgaacgtct tgggttgctt cgtaactaac ataaattcga tg 42

<210> 866
<211> 39
<212> DNA
<213> Artificial Sequence

<220>
<223> Target Specific Oligonucleotide

<400> 866
ggcctggacg gcgtaagagt aactaacata aattcgatg 39

<210> 867
<211> 40
<212> DNA
<213> Artificial Sequence

<220>

<223> Target Specific Oligonucleotide

<400> 867
gaagctcatg cgggaagcgg taactaacat aaattcgatg 40

<210> 868
<211> 43
<212> DNA
<213> Artificial Sequence

<220>
<223> Target Specific Oligonucleotide

<400> 868
tgtcactgta cgagatgttt cggtaactaa cataaattcg atg 43

<210> 869
<211> 43
<212> DNA
<213> Artificial Sequence

<220>
<223> Target Specific Oligonucleotide

<400> 869
ttgaagcttg gtatgtcagg aggtaactaa cataaattcg atg 43

<210> 870
<211> 42
<212> DNA
<213> Artificial Sequence

<220>
<223> Target Specific Oligonucleotide

<400> 870
ggttcccgat acatagctgg agtaactaac ataaattcga tg 42

<210> 871
<211> 44
<212> DNA
<213> Artificial Sequence

<220>
<223> Target Specific Oligonucleotide

<400> 871
atctttgaag tcgaaagaca ggcgtaacta acataaattc gatg 44

<210> 872
<211> 44
<212> DNA
<213> Artificial Sequence

<220>
<223> Target Specific Oligonucleotide

<400> 872
gcttattaac acgaagcttt gaggtaacta acataaattc gatg 44

<210> 873
<211> 42
<212> DNA
<213> Artificial Sequence

<220>
<223> Target Specific Oligonucleotide

<400> 873
tttcattcca cgggaaggag agtaactaac ataaattcga tg 42

<210> 874
<211> 41
<212> DNA
<213> Artificial Sequence

<220>
<223> Target Specific Oligonucleotide

<400> 874
tcagtgcttc gttggccatt gtaactaaca taaattcgat g 41

<210> 875
<211> 43
<212> DNA
<213> Artificial Sequence

<220>
<223> Target Specific Oligonucleotide

<400> 875
ctctgcgtac caagcagtaa ttgtaactaa cataaattcg atg 43

<210> 876
<211> 43
<212> DNA
<213> Artificial Sequence

<220>
<223> Target Specific Oligonucleotide

<400> 876
tttcaaggcc tcgatttctg tcgtaactaa cataaattcg atg 43

<210> 877
<211> 43
<212> DNA

<213> Artificial Sequence

<220>
<223> Target Specific Oligonucleotide

<400> 877
tagttcacac cgtacatctc cagtaactaa cataaattcg atg 43

<210> 878
<211> 42
<212> DNA
<213> Artificial Sequence

<220>
<223> Target Specific Oligonucleotide

<400> 878
cccacatttc cggagtcatc tgtaactaac ataaattcga tg 42

<210> 879
<211> 45
<212> DNA
<213> Artificial Sequence

<220>
<223> Target Specific Oligonucleotide

<400> 879
ggtcctgagc tatcttcaga tattgtaact aacataaatt cgatg 45

<210> 880
<211> 39
<212> DNA
<213> Artificial Sequence

<220>
<223> Target Specific Oligonucleotide

<400> 880
tcctccgtgc gaatcgctgt aactaacata aattcgatg 39

<210> 881
<211> 43
<212> DNA
<213> Artificial Sequence

<220>
<223> Target Specific Oligonucleotide

<400> 881
gcccttttta ttccggattg cagtaactaa cataaattcg atg 43

<210> 882

<211> 40
<212> DNA
<213> Artificial Sequence

<220>
<223> Target Specific Oligonucleotide

<400> 882
gctgtctcct cagaccgcag taactaacat aaattcgatg 40

<210> 883
<211> 41
<212> DNA
<213> Artificial Sequence

<220>
<223> Target Specific Oligonucleotide

<400> 883
caccccctgtc ggagttctca gtaactaaca taaattcgat g 41

<210> 884
<211> 46
<212> DNA
<213> Artificial Sequence

<220>
<223> Target Specific Oligonucleotide

<400> 884
cagatttatt accctttttg gaagcgtaac taacataaat tcgatg 46

<210> 885
<211> 39
<212> DNA
<213> Artificial Sequence

<220>
<223> Target Specific Oligonucleotide

<400> 885
tcatgggcgg gtacgtgggt aactaacata aattcgatg 39

<210> 886
<211> 42
<212> DNA
<213> Artificial Sequence

<220>
<223> Target Specific Oligonucleotide

<400> 886
caaaaatccc cgcttgtgaa cgtaactaac ataaattcga tg 42

<210> 887
<211> 42
<212> DNA
<213> Artificial Sequence

<220>
<223> Target Specific Oligonucleotide

<400> 887
ctggggtcgt agtcaccata cgtaactaac ataaattcga tg 42

<210> 888
<211> 39
<212> DNA
<213> Artificial Sequence

<220>
<223> Target Specific Oligonucleotide

<400> 888
cacacgatac cggcaaagaa ggtaacgtga cgattaccc 39

<210> 889
<211> 38
<212> DNA
<213> Artificial Sequence

<220>
<223> Target Specific Oligonucleotide

<400> 889
ggtgtcagtc tccgacgtga gtaacgtgac gattaccc 38

<210> 890
<211> 39
<212> DNA
<213> Artificial Sequence

<220>
<223> Target Specific Oligonucleotide

<400> 890
gtgatggtga tcatctgggc cgtaacgtga cgattaccc 39

<210> 891
<211> 36
<212> DNA
<213> Artificial Sequence

<220>
<223> Target Specific Oligonucleotide

<400> 891

ttggcctcgg gaccaagcgt aacgtgacga ttaccc 36

<210> 892
<211> 39
<212> DNA
<213> Artificial Sequence

<220>
<223> Target Specific Oligonucleotide

<400> 892
ctgtcatctt ggtcaggtgg tgtaacgtga cgattaccc 39

<210> 893
<211> 38
<212> DNA
<213> Artificial Sequence

<220>
<223> Target Specific Oligonucleotide

<400> 893
atcaccttgc cgaaagtgcc gtaacgtgac gattaccc 38

<210> 894
<211> 37
<212> DNA
<213> Artificial Sequence

<220>
<223> Target Specific Oligonucleotide

<400> 894
actgcacggc cgtagtcatg taacgtgacg attaccc 37

<210> 895
<211> 37
<212> DNA
<213> Artificial Sequence

<220>
<223> Target Specific Oligonucleotide

<400> 895
aggtactcag gtgtgccgcg taacgtgacg attaccc 37

<210> 896
<211> 38
<212> DNA
<213> Artificial Sequence

<220>
<223> Target Specific Oligonucleotide

<400> 896
gaaggtgcgt tcgatgacag gtaacgtgac gattaccc 38

<210> 897
<211> 39
<212> DNA
<213> Artificial Sequence

<220>
<223> Target Specific Oligonucleotide

<400> 897
ctcctcagga gtctccacat ggtaacgtga cgattaccc 39

<210> 898
<211> 40
<212> DNA
<213> Artificial Sequence

<220>
<223> Target Specific Oligonucleotide

<400> 898
gtctggaaag agtacttcag ggtaacgtg acgattaccc 40

<210> 899
<211> 36
<212> DNA
<213> Artificial Sequence

<220>
<223> Target Specific Oligonucleotide

<400> 899
caggacgcgg ttctcggtgt aacgtgacga ttaccc 36

<210> 900
<211> 38
<212> DNA
<213> Artificial Sequence

<220>
<223> Target Specific Oligonucleotide

<400> 900
atctcagcgc catagaagcg gtaacgtgac gattaccc 38

<210> 901
<211> 37
<212> DNA
<213> Artificial Sequence

<220>
<223> Target Specific Oligonucleotide

<400> 901
tcgttcatgg tcacgcggtg taacgtgacg attaccc 37

<210> 902
<211> 39
<212> DNA
<213> Artificial Sequence

<220>
<223> Target Specific Oligonucleotide

<400> 902
cagcccgaag tctgtgatct tgtaacgtga cgattaccc 39

<210> 903
<211> 37
<212> DNA
<213> Artificial Sequence

<220>
<223> Target Specific Oligonucleotide

<400> 903
tcatggtggc accgtccttg taacgtgacg attaccc 37

<210> 904
<211> 38
<212> DNA
<213> Artificial Sequence

<220>
<223> Target Specific Oligonucleotide

<400> 904
ggtagtccag ggctgacaca gtaacgtgac gattaccc 38

<210> 905
<211> 41
<212> DNA
<213> Artificial Sequence

<220>
<223> Target Specific Oligonucleotide

<400> 905
ccatcattct tgaggaggaa gtagtaacgt gacgattacc c 41

<210> 906
<211> 37
<212> DNA
<213> Artificial Sequence

<220>
<223> Target Specific Oligonucleotide

<400> 906
acaaagcaga ggcggtcgtg taacgtgacg attaccc 37

<210> 907
<211> 38
<212> DNA
<213> Artificial Sequence

<220>
<223> Target Specific Oligonucleotide

<400> 907
cggtcctcgg agaacacacg gtaacgtgac gattaccc 38

<210> 908
<211> 37
<212> DNA
<213> Artificial Sequence

<220>
<223> Target Specific Oligonucleotide

<400> 908
gagcctcacg ttggtccacg taacgtgacg attaccc 37

<210> 909
<211> 40
<212> DNA
<213> Artificial Sequence

<220>
<223> Target Specific Oligonucleotide

<400> 909
ctccttgtag ccaatgaagg tggtaacgtg acgattaccc 40

<210> 910
<211> 38
<212> DNA
<213> Artificial Sequence

<220>
<223> Target Specific Oligonucleotide

<400> 910
ttcacaatag ccacgtcgct gtaacgtgac gattaccc 38

<210> 911
<211> 37

<212> DNA
<213> Artificial Sequence

<220>
<223> Target Specific Oligonucleotide

<400> 911
ccctcgtttg tgcagccaag taacgtgacg attaccc 37

<210> 912
<211> 43
<212> DNA
<213> Artificial Sequence

<220>
<223> Target Specific Oligonucleotide

<400> 912
agctatgtcc gctatcttca tcggtaactc tcttctattg cag 43

<210> 913
<211> 41
<212> DNA
<213> Artificial Sequence

<220>
<223> Target Specific Oligonucleotide

<400> 913
ttggcacatc gagacatggt tgtaactctc ttctattgca g 41

<210> 914
<211> 42
<212> DNA
<213> Artificial Sequence

<220>
<223> Target Specific Oligonucleotide

<400> 914
ccatcttggt cgtactttgt gagtaactct cttctattgc ag 42

<210> 915
<211> 41
<212> DNA
<213> Artificial Sequence

<220>
<223> Target Specific Oligonucleotide

<400> 915
ccaactgagc atacgctagg agtaactctc ttctattgca g 41

<210> 916
<211> 41
<212> DNA
<213> Artificial Sequence

<220>
<223> Target Specific Oligonucleotide

<400> 916
tcacgtacta gccgttccat cgtaactctc ttctattgca g 41

<210> 917
<211> 43
<212> DNA
<213> Artificial Sequence

<220>
<223> Target Specific Oligonucleotide

<400> 917
tttctccaag tcgtctctca tctgtaactc tcttctattg cag 43

<210> 918
<211> 42
<212> DNA
<213> Artificial Sequence

<220>
<223> Target Specific Oligonucleotide

<400> 918
tcgtaagaaa cgccactaga aagtaactct cttctattgc ag 42

<210> 919
<211> 39
<212> DNA
<213> Artificial Sequence

<220>
<223> Target Specific Oligonucleotide

<400> 919
cactggcgtg cctaaaccag taactctctt ctattgcag 39

<210> 920
<211> 45
<212> DNA
<213> Artificial Sequence

<220>
<223> Target Specific Oligonucleotide

<400> 920
acatatcata cgtggacatt agaacgtaac tctcttctat tgcag 45

<210> 921
<211> 40
<212> DNA
<213> Artificial Sequence

<220>
<223> Target Specific Oligonucleotide

<400> 921
gccgatggaa tgctccatcc gtaactctct tctattgcag 40

<210> 922
<211> 43
<212> DNA
<213> Artificial Sequence

<220>
<223> Target Specific Oligonucleotide

<400> 922
gattagcaat cggtcacaaa gacgtaactc tcttctattg cag 43

<210> 923
<211> 43
<212> DNA
<213> Artificial Sequence

<220>
<223> Target Specific Oligonucleotide

<400> 923
ggtgtagtag tacacattgg agcgtaactc tcttctattg cag 43

<210> 924
<211> 42
<212> DNA
<213> Artificial Sequence

<220>
<223> Target Specific Oligonucleotide

<400> 924
gatgaaactt cggttgtcag ctgtaactct cttctattgc ag 42

<210> 925
<211> 42
<212> DNA
<213> Artificial Sequence

<220>
<223> Target Specific Oligonucleotide

<400> 925
ttcttgaggc tagcaacttg tggtaactct cttctattgc ag 42

<210> 926
<211> 43
<212> DNA
<213> Artificial Sequence

<220>
<223> Target Specific Oligonucleotide

<400> 926
gtcccaaaac tcgattagct tccgtaactc tcttctattg cag 43

<210> 927
<211> 42
<212> DNA
<213> Artificial Sequence

<220>
<223> Target Specific Oligonucleotide

<400> 927
tattcgtgga acccctaaca gcgtaactct cttctattgc ag 42

<210> 928
<211> 43
<212> DNA
<213> Artificial Sequence

<220>
<223> Target Specific Oligonucleotide

<400> 928
caaagttgat atcgcccaaa atggtaactc tcttctattg cag 43

<210> 929
<211> 42
<212> DNA
<213> Artificial Sequence

<220>
<223> Target Specific Oligonucleotide

<400> 929
tagatccaag cggttccatt tcgtaactct cttctattgc ag 42

<210> 930
<211> 42
<212> DNA
<213> Artificial Sequence

<220>

<223> Target Specific Oligonucleotide

<400> 930
tgaccagttc ccgtaaaaca ctgtaactct cttctattgc ag 42

<210> 931
<211> 41
<212> DNA
<213> Artificial Sequence

<220>
<223> Target Specific Oligonucleotide

<400> 931
tggcacatgg tcgatacaca tgtaactctc ttctattgca g 41

<210> 932
<211> 41
<212> DNA
<213> Artificial Sequence

<220>
<223> Target Specific Oligonucleotide

<400> 932
tgacagcaca acgatcacaa cgtaactctc ttctattgca g 41

<210> 933
<211> 42
<212> DNA
<213> Artificial Sequence

<220>
<223> Target Specific Oligonucleotide

<400> 933
aagctcatga actatgccag aggtaactct cttctattgc ag 42

<210> 934
<211> 41
<212> DNA
<213> Artificial Sequence

<220>
<223> Target Specific Oligonucleotide

<400> 934
cgtcatacct gaccgagtac tgtaactctc ttctattgca g 41

<210> 935
<211> 43
<212> DNA
<213> Artificial Sequence

<220>
<223> Target Specific Oligonucleotide

<400> 935
tcttgattgt cgagaaagac ttggtaactc tcttctattg cag 43

<210> 936
<211> 42
<212> DNA
<213> Artificial Sequence

<220>
<223> Target Specific Oligonucleotide

<400> 936
tactgagccc tatcggaaag tgtaactgat cttcatatta cg 42

<210> 937
<211> 42
<212> DNA
<213> Artificial Sequence

<220>
<223> Target Specific Oligonucleotide

<400> 937
ccaaatccct tcgcagcaat tgtaactgat cttcatatta cg 42

<210> 938
<211> 42
<212> DNA
<213> Artificial Sequence

<220>
<223> Target Specific Oligonucleotide

<400> 938
gccatgatca atcgcatcac tgtaactgat cttcatatta cg 42

<210> 939
<211> 43
<212> DNA
<213> Artificial Sequence

<220>
<223> Target Specific Oligonucleotide

<400> 939
atcatccaga ttcgaaccgt ccgtaactga tcttcatatt acg 43

<210> 940
<211> 43
<212> DNA

<213> Artificial Sequence

<220>
<223> Target Specific Oligonucleotide

<400> 940
ggactacatt cgtgaagaag ctgtaactga tcttcatatt acg 43

<210> 941
<211> 42
<212> DNA
<213> Artificial Sequence

<220>
<223> Target Specific Oligonucleotide

<400> 941
ggagaggatc cgtccaagaa agtaactgat cttcatatta cg 42

<210> 942
<211> 42
<212> DNA
<213> Artificial Sequence

<220>
<223> Target Specific Oligonucleotide

<400> 942
gtcccagcat tagaacctgt ggtaactgat cttcatatta cg 42

<210> 943
<211> 42
<212> DNA
<213> Artificial Sequence

<220>
<223> Target Specific Oligonucleotide

<400> 943
actctcagcc agacctttag cgtaactgat cttcatatta cg 42

<210> 944
<211> 44
<212> DNA
<213> Artificial Sequence

<220>
<223> Target Specific Oligonucleotide

<400> 944
catagtgtca tgtgcatgta gtcgtaactg atcttcatat tacg 44

<210> 945

<211> 44
<212> DNA
<213> Artificial Sequence

<220>
<223> Target Specific Oligonucleotide

<400> 945
ccctgaccag catattatct acagtaactg atcttcatat tacg 44

<210> 946
<211> 41
<212> DNA
<213> Artificial Sequence

<220>
<223> Target Specific Oligonucleotide

<400> 946
cagcgcctgt tacaagctct gtaactgatc ttcatattac g 41

<210> 947
<211> 41
<212> DNA
<213> Artificial Sequence

<220>
<223> Target Specific Oligonucleotide

<400> 947
cgccagtctt aactgccatg gtaactgatc ttcatattac g 41

<210> 948
<211> 43
<212> DNA
<213> Artificial Sequence

<220>
<223> Target Specific Oligonucleotide

<400> 948
tcttcccaac accgtaatct ttgtaactga tcttcatatt acg 43

<210> 949
<211> 43
<212> DNA
<213> Artificial Sequence

<220>
<223> Target Specific Oligonucleotide

<400> 949
gtgacttatg ttcgggtctc tcgtaactga tcttcatatt acg 43

<210> 950
<211> 43
<212> DNA
<213> Artificial Sequence

<220>
<223> Target Specific Oligonucleotide

<400> 950
tgttaggttc tagggccata gcgtaactga tcttcatatt acg 43

<210> 951
<211> 42
<212> DNA
<213> Artificial Sequence

<220>
<223> Target Specific Oligonucleotide

<400> 951
ctggatagct ttgggcaact cgtaactgat cttcatatta cg 42

<210> 952
<211> 45
<212> DNA
<213> Artificial Sequence

<220>
<223> Target Specific Oligonucleotide

<400> 952
tgcagacagg gtatgtatat ttgggtaact gatcttcata ttacg 45

<210> 953
<211> 44
<212> DNA
<213> Artificial Sequence

<220>
<223> Target Specific Oligonucleotide

<400> 953
aagaaaatat agctgagctc cctgtaactg atcttcatat tacg 44

<210> 954
<211> 40
<212> DNA
<213> Artificial Sequence

<220>
<223> Target Specific Oligonucleotide

<400> 954

gagaccggag aaacccggcg taactgatct tcatattacg 40

<210> 955
<211> 40
<212> DNA
<213> Artificial Sequence

<220>
<223> Target Specific Oligonucleotide

<400> 955
ccgcaggaga ccgaggtctg taactgatct tcatattacg 40

<210> 956
<211> 42
<212> DNA
<213> Artificial Sequence

<220>
<223> Target Specific Oligonucleotide

<400> 956
aggctagggc tcaagacaag ggtaactgat cttcatatta cg 42

<210> 957
<211> 38
<212> DNA
<213> Artificial Sequence

<220>
<223> Target Specific Oligonucleotide

<400> 957
gcgtccggtg tcgccatgta actgatcttc atattacg 38

<210> 958
<211> 41
<212> DNA
<213> Artificial Sequence

<220>
<223> Target Specific Oligonucleotide

<400> 958
actctgggtc ggcttgatcc gtaactgatc ttcatattac g 41

<210> 959
<211> 41
<212> DNA
<213> Artificial Sequence

<220>
<223> Target Specific Oligonucleotide

<400> 959
catgggagga tcgagcctgg gtaactgatc ttcatattac g 41

<210> 960
<211> 39
<212> DNA
<213> Artificial Sequence

<220>
<223> Target Specific Oligonucleotide

<400> 960
ggcactggaa acgattgact tgtaacggca atacatcgt 39

<210> 961
<211> 38
<212> DNA
<213> Artificial Sequence

<220>
<223> Target Specific Oligonucleotide

<400> 961
tcaggcattc gttcacctgt gtaacggcaa tacatcgt 38

<210> 962
<211> 40
<212> DNA
<213> Artificial Sequence

<220>
<223> Target Specific Oligonucleotide

<400> 962
actgtagcga ttaatgccat ccgtaacggc aatacatcgt 40

<210> 963
<211> 38
<212> DNA
<213> Artificial Sequence

<220>
<223> Target Specific Oligonucleotide

<400> 963
atagcctcca ttgcggttgg gtaacggcaa tacatcgt 38

<210> 964
<211> 40
<212> DNA
<213> Artificial Sequence

<220>
<223> Target Specific Oligonucleotide

<400> 964
cattcgggtg atcgatacac ttgtaacggc aatacatcgt 40

<210> 965
<211> 39
<212> DNA
<213> Artificial Sequence

<220>
<223> Target Specific Oligonucleotide

<400> 965
gcacactcat cgagttgctc cgtaacggca atacatcgt 39

<210> 966
<211> 37
<212> DNA
<213> Artificial Sequence

<220>
<223> Target Specific Oligonucleotide

<400> 966
tcccgcttaa tgcgcaggtg taacggcaat acatcgt 37

<210> 967
<211> 37
<212> DNA
<213> Artificial Sequence

<220>
<223> Target Specific Oligonucleotide

<400> 967
ggtcccctgt atggcgtgag taacggcaat acatcgt 37

<210> 968
<211> 38
<212> DNA
<213> Artificial Sequence

<220>
<223> Target Specific Oligonucleotide

<400> 968
aacacactgg cggttgtcaa gtaacggcaa tacatcgt 38

<210> 969
<211> 39
<212> DNA
<213> Artificial Sequence

<220>
<223> Target Specific Oligonucleotide

<400> 969
cacacatcga cgaaggtttc agtaacggca atacatcgt 39

<210> 970
<211> 39
<212> DNA
<213> Artificial Sequence

<220>
<223> Target Specific Oligonucleotide

<400> 970
ggctcttgca cgagttaact tgtaacggca atacatcgt 39

<210> 971
<211> 39
<212> DNA
<213> Artificial Sequence

<220>
<223> Target Specific Oligonucleotide

<400> 971
atgtcaatgg tacaccgctg agtaacggca atacatcgt 39

<210> 972
<211> 38
<212> DNA
<213> Artificial Sequence

<220>
<223> Target Specific Oligonucleotide

<400> 972
gccatgctta cgctttcgtt gtaacggcaa tacatcgt 38

<210> 973
<211> 40
<212> DNA
<213> Artificial Sequence

<220>
<223> Target Specific Oligonucleotide

<400> 973
ggtataccag taacacggac cagtaacggc aatacatcgt 40

<210> 974
<211> 40

<212> DNA
<213> Artificial Sequence

<220>
<223> Target Specific Oligonucleotide

<400> 974
gactctctca cgcataaacc tggtaacggc aatacatcgt 40

<210> 975
<211> 40
<212> DNA
<213> Artificial Sequence

<220>
<223> Target Specific Oligonucleotide

<400> 975
aggagatgcg taggtcaatt cagtaacggc aatacatcgt 40

<210> 976
<211> 39
<212> DNA
<213> Artificial Sequence

<220>
<223> Target Specific Oligonucleotide

<400> 976
ccaacaggac gctagtgtag agtaacggca atacatcgt 39

<210> 977
<211> 42
<212> DNA
<213> Artificial Sequence

<220>
<223> Target Specific Oligonucleotide

<400> 977
cagtaccaat taggttagct tctggtaacg gcaatacatc gt 42

<210> 978
<211> 38
<212> DNA
<213> Artificial Sequence

<220>
<223> Target Specific Oligonucleotide

<400> 978
tccatggccg tcgatcaatg gtaacggcaa tacatcgt 38

<210> 979
<211> 40
<212> DNA
<213> Artificial Sequence

<220>
<223> Target Specific Oligonucleotide

<400> 979
tagtcaggaa tatgcggtca ttgtaacggc aatacatcgt 40

<210> 980
<211> 41
<212> DNA
<213> Artificial Sequence

<220>
<223> Target Specific Oligonucleotide

<400> 980
tatagcaact acttcgcatt tccgtaacgg caatacatcg t 41

<210> 981
<211> 39
<212> DNA
<213> Artificial Sequence

<220>
<223> Target Specific Oligonucleotide

<400> 981
cgatggtgtc ctacggatgt cgtaacggca atacatcgt 39

<210> 982
<211> 44
<212> DNA
<213> Artificial Sequence

<220>
<223> Target Specific Oligonucleotide

<400> 982
gtaaccatag taccacttat tagtgggtaa cggcaataca tcgt 44

<210> 983
<211> 37
<212> DNA
<213> Artificial Sequence

<220>
<223> Target Specific Oligonucleotide

<400> 983
cagcatcagc ccgtgagtag taacggcaat acatcgt 37

<210> 984
<211> 39
<212> DNA
<213> Artificial Sequence

<220>
<223> Target Specific Oligonucleotide

<400> 984
tcagatgcta ctggccgctt agacaagtta agatcggaa 39

<210> 985
<211> 41
<212> DNA
<213> Artificial Sequence

<220>
<223> Target Specific Oligonucleotide

<400> 985
cttgatcaag gcgctgaaca atagacaagt taagatcgga a 41

<210> 986
<211> 41
<212> DNA
<213> Artificial Sequence

<220>
<223> Target Specific Oligonucleotide

<400> 986
tcccttcgta tctcagcgag atagacaagt taagatcgga a 41

<210> 987
<211> 41
<212> DNA
<213> Artificial Sequence

<220>
<223> Target Specific Oligonucleotide

<400> 987
agactgttga ctggcgtgat gtagacaagt taagatcgga a 41

<210> 988
<211> 41
<212> DNA
<213> Artificial Sequence

<220>
<223> Target Specific Oligonucleotide

<400> 988
ccaacatgct cgcatgagtt ctagacaagt taagatcgga a 41

<210> 989
<211> 41
<212> DNA
<213> Artificial Sequence

<220>
<223> Target Specific Oligonucleotide

<400> 989
ttccttcacc gaactgagga gtagacaagt taagatcgga a 41

<210> 990
<211> 40
<212> DNA
<213> Artificial Sequence

<220>
<223> Target Specific Oligonucleotide

<400> 990
agttccaacg agcggcttca tagacaagtt aagatcggaa 40

<210> 991
<211> 40
<212> DNA
<213> Artificial Sequence

<220>
<223> Target Specific Oligonucleotide

<400> 991
ccatggctga cgagatctga tagacaagtt aagatcggaa 40

<210> 992
<211> 40
<212> DNA
<213> Artificial Sequence

<220>
<223> Target Specific Oligonucleotide

<400> 992
tagcctaaga cccggagctt tagacaagtt aagatcggaa 40

<210> 993
<211> 40
<212> DNA
<213> Artificial Sequence

<220>

<223> Target Specific Oligonucleotide

<400> 993
gcagatactc agcggcattg tagacaagtt aagatcggaa 40

<210> 994
<211> 40
<212> DNA
<213> Artificial Sequence

<220>
<223> Target Specific Oligonucleotide

<400> 994
catagctcac gcagaacgtg tagacaagtt aagatcggaa 40

<210> 995
<211> 39
<212> DNA
<213> Artificial Sequence

<220>
<223> Target Specific Oligonucleotide

<400> 995
gtcggccacc gttgaatgat agacaagtta agatcggaa 39

<210> 996
<211> 39
<212> DNA
<213> Artificial Sequence

<220>
<223> Target Specific Oligonucleotide

<400> 996
agaacggtca gcgagcgtat agacaagtta agatcggaa 39

<210> 997
<211> 44
<212> DNA
<213> Artificial Sequence

<220>
<223> Target Specific Oligonucleotide

<400> 997
gttatgctta gagtgttatc tccatagaca agttaagatc ggaa 44

<210> 998
<211> 40
<212> DNA
<213> Artificial Sequence

<220>
<223> Target Specific Oligonucleotide

<400> 998
tggtgatgtc cgtgcgttcc tagacaagtt aagatcggaa 40

<210> 999
<211> 40
<212> DNA
<213> Artificial Sequence

<220>
<223> Target Specific Oligonucleotide

<400> 999
ggtccagagg atcgctctct tagacaagtt aagatcggaa 40

<210> 1000
<211> 39
<212> DNA
<213> Artificial Sequence

<220>
<223> Target Specific Oligonucleotide

<400> 1000
cgtggactgc aagtcccgat agacaagtta agatcggaa 39

<210> 1001
<211> 41
<212> DNA
<213> Artificial Sequence

<220>
<223> Target Specific Oligonucleotide

<400> 1001
acaggtcaat tcccgggtaa gtagacaagt taagatcgga a 41

<210> 1002
<211> 41
<212> DNA
<213> Artificial Sequence

<220>
<223> Target Specific Oligonucleotide

<400> 1002
ctgcaccagg ttagggtgtt ttagacaagt taagatcgga a 41

<210> 1003
<211> 43
<212> DNA

<213> Artificial Sequence

<220>
<223> Target Specific Oligonucleotide

<400> 1003
ggaaatggca tatacgtggg atttagacaa gttaagatcg gaa 43

<210> 1004
<211> 40
<212> DNA
<213> Artificial Sequence

<220>
<223> Target Specific Oligonucleotide

<400> 1004
cttttgtctt tgcgcccagg tagacaagtt aagatcggaa 40

<210> 1005
<211> 38
<212> DNA
<213> Artificial Sequence

<220>
<223> Target Specific Oligonucleotide

<400> 1005
tcctgccggt tgcactccta gacaagttaa gatcggaa 38

<210> 1006
<211> 43
<212> DNA
<213> Artificial Sequence

<220>
<223> Target Specific Oligonucleotide

<400> 1006
atcatacagt gcaacgaaaa ggttagacaa gttaagatcg gaa 43

<210> 1007
<211> 40
<212> DNA
<213> Artificial Sequence

<220>
<223> Target Specific Oligonucleotide

<400> 1007
tcccacttgt cgtagttggg tagacaagtt aagatcggaa 40

<210> 1008

<211> 39
<212> DNA
<213> Artificial Sequence

<220>
<223> Target Specific Oligonucleotide

<400> 1008
aggtggtgtt ccgaacgagc tagacagccc aattaaacg 39

<210> 1009
<211> 39
<212> DNA
<213> Artificial Sequence

<220>
<223> Target Specific Oligonucleotide

<400> 1009
agatccgtga gcggaatgta tagacagccc aattaaacg 39

<210> 1010
<211> 41
<212> DNA
<213> Artificial Sequence

<220>
<223> Target Specific Oligonucleotide

<400> 1010
ctcactcgct ttagtggact cctagacagc ccaattaaac g 41

<210> 1011
<211> 37
<212> DNA
<213> Artificial Sequence

<220>
<223> Target Specific Oligonucleotide

<400> 1011
gccaaagacc gtggcgagta gacagcccaa ttaaacg 37

<210> 1012
<211> 41
<212> DNA
<213> Artificial Sequence

<220>
<223> Target Specific Oligonucleotide

<400> 1012
agagttcttg gtcgttggat cctagacagc ccaattaaac g 41

<210> 1013
<211> 39
<212> DNA
<213> Artificial Sequence

<220>
<223> Target Specific Oligonucleotide

<400> 1013
ctcctttgca accgggtctg tagacagccc aattaaacg 39

<210> 1014
<211> 41
<212> DNA
<213> Artificial Sequence

<220>
<223> Target Specific Oligonucleotide

<400> 1014
cttgttattg acgtcgaagg cttagacagc ccaattaaac g 41

<210> 1015
<211> 40
<212> DNA
<213> Artificial Sequence

<220>
<223> Target Specific Oligonucleotide

<400> 1015
caggaacgtg taactcttgc ctagacagcc caattaaacg 40

<210> 1016
<211> 40
<212> DNA
<213> Artificial Sequence

<220>
<223> Target Specific Oligonucleotide

<400> 1016
ggagtttcac acacgagttg gtagacagcc caattaaacg 40

<210> 1017
<211> 39
<212> DNA
<213> Artificial Sequence

<220>
<223> Target Specific Oligonucleotide

<400> 1017

aggttcccgc tctacggatg tagacagccc aattaaacg 39

<210> 1018
<211> 38
<212> DNA
<213> Artificial Sequence

<220>
<223> Target Specific Oligonucleotide

<400> 1018
accgtcatgg actgccgtct agacagccca attaaacg 38

<210> 1019
<211> 40
<212> DNA
<213> Artificial Sequence

<220>
<223> Target Specific Oligonucleotide

<400> 1019
tgggaccttt agacttcggt gtagacagcc caattaaacg 40

<210> 1020
<211> 37
<212> DNA
<213> Artificial Sequence

<220>
<223> Target Specific Oligonucleotide

<400> 1020
gtccctgtag acgcgcgtta gacagcccaa ttaaacg 37

<210> 1021
<211> 38
<212> DNA
<213> Artificial Sequence

<220>
<223> Target Specific Oligonucleotide

<400> 1021
ccagagaggg cgcatcttgt agacagccca attaaacg 38

<210> 1022
<211> 40
<212> DNA
<213> Artificial Sequence

<220>
<223> Target Specific Oligonucleotide

<400> 1022
tccactctgc acgctcatag ttagacagcc caattaaacg 40

<210> 1023
<211> 40
<212> DNA
<213> Artificial Sequence

<220>
<223> Target Specific Oligonucleotide

<400> 1023
gtgaaactcg acgttcacgt atagacagcc caattaaacg 40

<210> 1024
<211> 40
<212> DNA
<213> Artificial Sequence

<220>
<223> Target Specific Oligonucleotide

<400> 1024
acacctcttt gtcgttgacc ttagacagcc caattaaacg 40

<210> 1025
<211> 39
<212> DNA
<213> Artificial Sequence

<220>
<223> Target Specific Oligonucleotide

<400> 1025
actgttgcga gttctgcgag tagacagccc aattaaacg 39

<210> 1026
<211> 39
<212> DNA
<213> Artificial Sequence

<220>
<223> Target Specific Oligonucleotide

<400> 1026
tggcaactcc gtagttgtcc tagacagccc aattaaacg 39

<210> 1027
<211> 38
<212> DNA
<213> Artificial Sequence

<220>
<223> Target Specific Oligonucleotide

<400> 1027
tcctcggacg ctaagctcat agacagccca attaaacg 38

<210> 1028
<211> 38
<212> DNA
<213> Artificial Sequence

<220>
<223> Target Specific Oligonucleotide

<400> 1028
ttacccggag aaccctcgct agacagccca attaaacg 38

<210> 1029
<211> 38
<212> DNA
<213> Artificial Sequence

<220>
<223> Target Specific Oligonucleotide

<400> 1029
tcccggaaca tgcggtaggt agacagccca attaaacg 38

<210> 1030
<211> 38
<212> DNA
<213> Artificial Sequence

<220>
<223> Target Specific Oligonucleotide

<400> 1030
tgctcgatgt cgcccactgt agacagccca attaaacg 38

<210> 1031
<211> 37
<212> DNA
<213> Artificial Sequence

<220>
<223> Target Specific Oligonucleotide

<400> 1031
tgccggtgcc gcttatggta gacagcccaa ttaaacg 37

<210> 1032
<211> 45
<212> DNA
<213> Artificial Sequence

<220>
<223> Target Specific Oligonucleotide

<400> 1032
ccaaagtctg ctagcttgat ggtagacggt ataatactta tttcc 45

<210> 1033
<211> 48
<212> DNA
<213> Artificial Sequence

<220>
<223> Target Specific Oligonucleotide

<400> 1033
tattaggatg gttaagctcc ttaagtagac ggtataatac ttatttcc 48

<210> 1034
<211> 45
<212> DNA
<213> Artificial Sequence

<220>
<223> Target Specific Oligonucleotide

<400> 1034
catgggtgta agtacgaaca ggtagacggt ataatactta tttcc 45

<210> 1035
<211> 43
<212> DNA
<213> Artificial Sequence

<220>
<223> Target Specific Oligonucleotide

<400> 1035
ggcagaaagc taggccctgg tagacggtat aatacttatt tcc 43

<210> 1036
<211> 43
<212> DNA
<213> Artificial Sequence

<220>
<223> Target Specific Oligonucleotide

<400> 1036
gtacacaact ccgtacgtgc tagacggtat aatacttatt tcc 43

<210> 1037
<211> 45

<212> DNA
<213> Artificial Sequence

<220>
<223> Target Specific Oligonucleotide

<400> 1037
atggaacgca gtatacctct cgtagacggt ataatactta tttcc 45

<210> 1038
<211> 46
<212> DNA
<213> Artificial Sequence

<220>
<223> Target Specific Oligonucleotide

<400> 1038
cttggcttgt aatcaggcat agatagacgg tataatactt atttcc 46

<210> 1039
<211> 41
<212> DNA
<213> Artificial Sequence

<220>
<223> Target Specific Oligonucleotide

<400> 1039
ctccatgaag cgccagcgta gacggtataa tacttatttc c 41

<210> 1040
<211> 43
<212> DNA
<213> Artificial Sequence

<220>
<223> Target Specific Oligonucleotide

<400> 1040
ccgcttgtta gggtcgtagt tagacggtat aatacttatt tcc 43

<210> 1041
<211> 42
<212> DNA
<213> Artificial Sequence

<220>
<223> Target Specific Oligonucleotide

<400> 1041
ctcggtggag gacccgatgt agacggtata atacttattt cc 42

<210> 1042
<211> 44
<212> DNA
<213> Artificial Sequence

<220>
<223> Target Specific Oligonucleotide

<400> 1042
ttactaaaat cttgccgggc ctagacggta taatacttat ttcc 44

<210> 1043
<211> 44
<212> DNA
<213> Artificial Sequence

<220>
<223> Target Specific Oligonucleotide

<400> 1043
gcagcatttg cgataacaag ctagacggta taatacttat ttcc 44

<210> 1044
<211> 45
<212> DNA
<213> Artificial Sequence

<220>
<223> Target Specific Oligonucleotide

<400> 1044
agaaggctat cagagtcgaa gatagacggt ataatactta tttcc 45

<210> 1045
<211> 43
<212> DNA
<213> Artificial Sequence

<220>
<223> Target Specific Oligonucleotide

<400> 1045
ttcaggagct cggtaccaca tagacggtat aatacttatt tcc 43

<210> 1046
<211> 44
<212> DNA
<213> Artificial Sequence

<220>
<223> Target Specific Oligonucleotide

<400> 1046
ccccagagtc cgaaagatcc gtagacggta taatacttat ttcc 44

<210> 1047
<211> 42
<212> DNA
<213> Artificial Sequence

<220>
<223> Target Specific Oligonucleotide

<400> 1047
acagggccca gtaccgtggt agacggtata atacttattt cc 42

<210> 1048
<211> 46
<212> DNA
<213> Artificial Sequence

<220>
<223> Target Specific Oligonucleotide

<400> 1048
tcagcaataa ctaagagaag gggtagacgg tataatactt atttcc 46

<210> 1049
<211> 46
<212> DNA
<213> Artificial Sequence

<220>
<223> Target Specific Oligonucleotide

<400> 1049
gtacggcaaa tctaacgtgt aggtagacgg tataatactt atttcc 46

<210> 1050
<211> 44
<212> DNA
<213> Artificial Sequence

<220>
<223> Target Specific Oligonucleotide

<400> 1050
tgaaacaatg ttgccgcctc ctagacggta taatacttat ttcc 44

<210> 1051
<211> 47
<212> DNA
<213> Artificial Sequence

<220>
<223> Target Specific Oligonucleotide

<400> 1051
taataattat ggggcattca gagatagacg gtataatact tatttcc 47

<210> 1052
<211> 43
<212> DNA
<213> Artificial Sequence

<220>
<223> Target Specific Oligonucleotide

<400> 1052
tctcacctgc ctcataaggc tagacggtat aatacttatt tcc 43

<210> 1053
<211> 47
<212> DNA
<213> Artificial Sequence

<220>
<223> Target Specific Oligonucleotide

<400> 1053
atggtgaagc aatagattgg aatgtagacg gtataatact tatttcc 47

<210> 1054
<211> 49
<212> DNA
<213> Artificial Sequence

<220>
<223> Target Specific Oligonucleotide

<400> 1054
cactaactag ggtataaact gattggtaga cggtataata cttatttcc 49

<210> 1055
<211> 44
<212> DNA
<213> Artificial Sequence

<220>
<223> Target Specific Oligonucleotide

<400> 1055
agaagggaca aacaagggga ctagacggta taatacttat ttcc 44

<210> 1056
<211> 43
<212> DNA
<213> Artificial Sequence

<220>

<223> Target Specific Oligonucleotide

<400> 1056
agtccgatac tcaagcgcaa atagacaata aattctaacg agc 43

<210> 1057
<211> 42
<212> DNA
<213> Artificial Sequence

<220>
<223> Target Specific Oligonucleotide

<400> 1057
cttgttgcgc tcacggtatg tagacaataa attctaacga gc 42

<210> 1058
<211> 43
<212> DNA
<213> Artificial Sequence

<220>
<223> Target Specific Oligonucleotide

<400> 1058
ccagagttgt agacgagcag ctagacaata aattctaacg agc 43

<210> 1059
<211> 41
<212> DNA
<213> Artificial Sequence

<220>
<223> Target Specific Oligonucleotide

<400> 1059
ttagcatggc aacacggcgt agacaataaa ttctaacgag c 41

<210> 1060
<211> 41
<212> DNA
<213> Artificial Sequence

<220>
<223> Target Specific Oligonucleotide

<400> 1060
atgatgcgca cttcacgggt agacaataaa ttctaacgag c 41

<210> 1061
<211> 42
<212> DNA
<213> Artificial Sequence

<220>
<223> Target Specific Oligonucleotide

<400> 1061
cccaccgcta ggagggtagc tagacaataa attctaacga gc 42

<210> 1062
<211> 42
<212> DNA
<213> Artificial Sequence

<220>
<223> Target Specific Oligonucleotide

<400> 1062
gcagtgtggt taggccgatg tagacaataa attctaacga gc 42

<210> 1063
<211> 41
<212> DNA
<213> Artificial Sequence

<220>
<223> Target Specific Oligonucleotide

<400> 1063
gacacggcgg tgcactatct agacaataaa ttctaacgag c 41

<210> 1064
<211> 40
<212> DNA
<213> Artificial Sequence

<220>
<223> Target Specific Oligonucleotide

<400> 1064
gcccaaagcg gcttgcagta gacaataaat tctaacgagc 40

<210> 1065
<211> 44
<212> DNA
<213> Artificial Sequence

<220>
<223> Target Specific Oligonucleotide

<400> 1065
atacacacac ggctaatact gctagacaat aaattctaac gagc 44

<210> 1066
<211> 43
<212> DNA

<213> Artificial Sequence

<220>
<223> Target Specific Oligonucleotide

<400> 1066
gtgaccgtgt acaccaacaa ctagacaata aattctaacg agc 43

<210> 1067
<211> 46
<212> DNA
<213> Artificial Sequence

<220>
<223> Target Specific Oligonucleotide

<400> 1067
ctcttcatac tagcatgact gtcatagaca ataaattcta acgagc 46

<210> 1068
<211> 42
<212> DNA
<213> Artificial Sequence

<220>
<223> Target Specific Oligonucleotide

<400> 1068
agaaggcttt cgtcgttccc tagacaataa attctaacga gc 42

<210> 1069
<211> 42
<212> DNA
<213> Artificial Sequence

<220>
<223> Target Specific Oligonucleotide

<400> 1069
tttgctccgt gcgttcaagg tagacaataa attctaacga gc 42

<210> 1070
<211> 42
<212> DNA
<213> Artificial Sequence

<220>
<223> Target Specific Oligonucleotide

<400> 1070
tccagtgcat ccgtcacatc tagacaataa attctaacga gc 42

<210> 1071

<211> 41
<212> DNA
<213> Artificial Sequence

<220>
<223> Target Specific Oligonucleotide

<400> 1071
gatgaacagc ggtgcgttgt agacaataaa ttctaacgag c 41

<210> 1072
<211> 42
<212> DNA
<213> Artificial Sequence

<220>
<223> Target Specific Oligonucleotide

<400> 1072
cactgatacc ggctctccgt tagacaataa attctaacga gc 42

<210> 1073
<211> 42
<212> DNA
<213> Artificial Sequence

<220>
<223> Target Specific Oligonucleotide

<400> 1073
gaacgcttgc gttccctagc tagacaataa attctaacga gc 42

<210> 1074
<211> 45
<212> DNA
<213> Artificial Sequence

<220>
<223> Target Specific Oligonucleotide

<400> 1074
cagatagatt tccgggatac agctagacaa taaattctaa cgagc 45

<210> 1075
<211> 40
<212> DNA
<213> Artificial Sequence

<220>
<223> Target Specific Oligonucleotide

<400> 1075
tctgctgcgg cgattgtgta gacaataaat tctaacgagc 40

<210> 1076
<211> 41
<212> DNA
<213> Artificial Sequence

<220>
<223> Target Specific Oligonucleotide

<400> 1076
ctcggtgatc atgcgtccct agacaataaa ttctaacgag c 41

<210> 1077
<211> 43
<212> DNA
<213> Artificial Sequence

<220>
<223> Target Specific Oligonucleotide

<400> 1077
tccaatttca gggcgttctc gtagacaata aattctaacg agc 43

<210> 1078
<211> 40
<212> DNA
<213> Artificial Sequence

<220>
<223> Target Specific Oligonucleotide

<400> 1078
tgcgcaggtc gacgtcagta gacaataaat tctaacgagc 40

<210> 1079
<211> 43
<212> DNA
<213> Artificial Sequence

<220>
<223> Target Specific Oligonucleotide

<400> 1079
gtccaggtag tatccggagg gtagacaata aattctaacg agc 43

<210> 1080
<211> 40
<212> DNA
<213> Artificial Sequence

<220>
<223> Target Specific Oligonucleotide

<400> 1080

attcccgaca gactcatcgc tagacgataa attgcgaacg 40

<210> 1081
<211> 40
<212> DNA
<213> Artificial Sequence

<220>
<223> Target Specific Oligonucleotide

<400> 1081
cagttacatg ttcgcgcacg tagacgataa attgcgaacg 40

<210> 1082
<211> 39
<212> DNA
<213> Artificial Sequence

<220>
<223> Target Specific Oligonucleotide

<400> 1082
acgagcacta gggtcgtcgt agacgataaa ttgcgaacg 39

<210> 1083
<211> 46
<212> DNA
<213> Artificial Sequence

<220>
<223> Target Specific Oligonucleotide

<400> 1083
ttttgataat cgaatgaaaa agtcactaga cgataaattg cgaacg 46

<210> 1084
<211> 43
<212> DNA
<213> Artificial Sequence

<220>
<223> Target Specific Oligonucleotide

<400> 1084
ctgaatactg ctagacgaga agctagacga taaattgcga acg 43

<210> 1085
<211> 43
<212> DNA
<213> Artificial Sequence

<220>
<223> Target Specific Oligonucleotide

<400> 1085
ctccttggaa cgaactaatc ctgtagacga taaattgcga acg 43

<210> 1086
<211> 42
<212> DNA
<213> Artificial Sequence

<220>
<223> Target Specific Oligonucleotide

<400> 1086
ttcaggcggt taattggtac cctagacgat aaattgcgaa cg 42

<210> 1087
<211> 42
<212> DNA
<213> Artificial Sequence

<220>
<223> Target Specific Oligonucleotide

<400> 1087
gttaggaatg gcgaacagac agtagacgat aaattgcgaa cg 42

<210> 1088
<211> 42
<212> DNA
<213> Artificial Sequence

<220>
<223> Target Specific Oligonucleotide

<400> 1088
gatggtctta aagcgtgaag cctagacgat aaattgcgaa cg 42

<210> 1089
<211> 42
<212> DNA
<213> Artificial Sequence

<220>
<223> Target Specific Oligonucleotide

<400> 1089
ctgtgcctag ccctaactta tatagacgat aaattgcgaa cg 42

<210> 1090
<211> 42
<212> DNA
<213> Artificial Sequence

<220>
<223> Target Specific Oligonucleotide

<400> 1090
agagaccacc cataacaaga gttagacgat aaattgcgaa cg 42

<210> 1091
<211> 43
<212> DNA
<213> Artificial Sequence

<220>
<223> Target Specific Oligonucleotide

<400> 1091
ttggagcacc gatatagata tggtagacga taaattgcga acg 43

<210> 1092
<211> 41
<212> DNA
<213> Artificial Sequence

<220>
<223> Target Specific Oligonucleotide

<400> 1092
tcaaagacgc aaacgtccaa atagacgata aattgcgaac g 41

<210> 1093
<211> 42
<212> DNA
<213> Artificial Sequence

<220>
<223> Target Specific Oligonucleotide

<400> 1093
gagagctgat taggtgctca ggtagacgat aaattgcgaa cg 42

<210> 1094
<211> 40
<212> DNA
<213> Artificial Sequence

<220>
<223> Target Specific Oligonucleotide

<400> 1094
tgcttgtacg cggagaaggt tagacgataa attgcgaacg 40

<210> 1095
<211> 40
<212> DNA
<213> Artificial Sequence

<220>
<223> Target Specific Oligonucleotide

<400> 1095
ctgcagacac gtgtatgtgc tagacgataa attgcgaacg 40

<210> 1096
<211> 40
<212> DNA
<213> Artificial Sequence

<220>
<223> Target Specific Oligonucleotide

<400> 1096
catccaatgc aagtccggtg tagacgataa attgcgaacg 40

<210> 1097
<211> 42
<212> DNA
<213> Artificial Sequence

<220>
<223> Target Specific Oligonucleotide

<400> 1097
accaagtgat aacgtgttca gatagacgat aaattgcgaa cg 42

<210> 1098
<211> 42
<212> DNA
<213> Artificial Sequence

<220>
<223> Target Specific Oligonucleotide

<400> 1098
aatctgagct ttgcgaaatg gttagacgat aaattgcgaa cg 42

<210> 1099
<211> 44
<212> DNA
<213> Artificial Sequence

<220>
<223> Target Specific Oligonucleotide

<400> 1099
caaagcccac ataacactat cttttagacg ataaattgcg aacg 44

<210> 1100
<211> 44

<212> DNA
<213> Artificial Sequence

<220>
<223> Target Specific Oligonucleotide

<400> 1100
tgtactggac tatagacacc aatttagacg ataaattgcg aacg 44

<210> 1101
<211> 45
<212> DNA
<213> Artificial Sequence

<220>
<223> Target Specific Oligonucleotide

<400> 1101
atcaatatac cacgttagta gtcactagac gataaattgc gaacg 45

<210> 1102
<211> 42
<212> DNA
<213> Artificial Sequence

<220>
<223> Target Specific Oligonucleotide

<400> 1102
tgtattttaa cgccaagcag gctagacgat aaattgcgaa cg 42

<210> 1103
<211> 42
<212> DNA
<213> Artificial Sequence

<220>
<223> Target Specific Oligonucleotide

<400> 1103
cactgaaaac acggttatgt cctagacgat aaattgcgaa cg 42

<210> 1104
<211> 40
<212> DNA
<213> Artificial Sequence

<220>
<223> Target Specific Oligonucleotide

<400> 1104
cgagacagat cggagtagct gtagacgacc gaatttacgg 40

<210> 1105
<211> 41
<212> DNA
<213> Artificial Sequence

<220>
<223> Target Specific Oligonucleotide

<400> 1105
tctctcaatg acgggacaaa gatagacgac cgaatttacg g 41

<210> 1106
<211> 41
<212> DNA
<213> Artificial Sequence

<220>
<223> Target Specific Oligonucleotide

<400> 1106
cagatgtggt gtatggtcca catagacgac cgaatttacg g 41

<210> 1107
<211> 40
<212> DNA
<213> Artificial Sequence

<220>
<223> Target Specific Oligonucleotide

<400> 1107
ttctcctcgg ttacttcgtg atagacgacc gaatttacgg 40

<210> 1108
<211> 39
<212> DNA
<213> Artificial Sequence

<220>
<223> Target Specific Oligonucleotide

<400> 1108
tgtgctaagc cgaaggaggt tagacgaccg aatttacgg 39

<210> 1109
<211> 44
<212> DNA
<213> Artificial Sequence

<220>
<223> Target Specific Oligonucleotide

<400> 1109
gtaaacatag aacgactcat agtcatagac gaccgaattt acgg 44

<210> 1110
<211> 43
<212> DNA
<213> Artificial Sequence

<220>
<223> Target Specific Oligonucleotide

<400> 1110
ttccacttca attagctctt gaattagacg accgaattta cgg 43

<210> 1111
<211> 40
<212> DNA
<213> Artificial Sequence

<220>
<223> Target Specific Oligonucleotide

<400> 1111
ctggggtaga gatagctcgc ttagacgacc gaatttacgg 40

<210> 1112
<211> 41
<212> DNA
<213> Artificial Sequence

<220>
<223> Target Specific Oligonucleotide

<400> 1112
gctataagct cgcctaccat tctagacgac cgaatttacg g 41

<210> 1113
<211> 39
<212> DNA
<213> Artificial Sequence

<220>
<223> Target Specific Oligonucleotide

<400> 1113
ttcttggggc gggtagagtg tagacgaccg aatttacgg 39

<210> 1114
<211> 40
<212> DNA
<213> Artificial Sequence

<220>
<223> Target Specific Oligonucleotide

<400> 1114
aagacctcag accgtcagaa gtagacgacc gaatttacgg 40

<210> 1115
<211> 39
<212> DNA
<213> Artificial Sequence

<220>
<223> Target Specific Oligonucleotide

<400> 1115
tcagatcctg acgacctgca tagacgaccg aatttacgg 39

<210> 1116
<211> 40
<212> DNA
<213> Artificial Sequence

<220>
<223> Target Specific Oligonucleotide

<400> 1116
gcagaaatct cgagaggacg ctagacgacc gaatttacgg 40

<210> 1117
<211> 39
<212> DNA
<213> Artificial Sequence

<220>
<223> Target Specific Oligonucleotide

<400> 1117
ctcgcagtct aggccgaaga tagacgaccg aatttacgg 39

<210> 1118
<211> 39
<212> DNA
<213> Artificial Sequence

<220>
<223> Target Specific Oligonucleotide

<400> 1118
ccggtctccc tcgagaatca tagacgaccg aatttacgg 39

<210> 1119
<211> 39
<212> DNA
<213> Artificial Sequence

<220>

<223> Target Specific Oligonucleotide

<400> 1119
tcctggtgag aacgtcctgc tagacgaccg aatttacgg 39

<210> 1120
<211> 38
<212> DNA
<213> Artificial Sequence

<220>
<223> Target Specific Oligonucleotide

<400> 1120
tgccctttgg taaggcgcct agacgaccga atttacgg 38

<210> 1121
<211> 38
<212> DNA
<213> Artificial Sequence

<220>
<223> Target Specific Oligonucleotide

<400> 1121
cacagacaca gccgaggact agacgaccga atttacgg 38

<210> 1122
<211> 39
<212> DNA
<213> Artificial Sequence

<220>
<223> Target Specific Oligonucleotide

<400> 1122
tcccaaaagc taccgctgag tagacgaccg aatttacgg 39

<210> 1123
<211> 39
<212> DNA
<213> Artificial Sequence

<220>
<223> Target Specific Oligonucleotide

<400> 1123
taaggtagag tcgggcgaag tagacgaccg aatttacgg 39

<210> 1124
<211> 38
<212> DNA
<213> Artificial Sequence

<220>
<223> Target Specific Oligonucleotide

<400> 1124
gagtggccgg aaaaacccgt agacgaccga atttacgg 38

<210> 1125
<211> 37
<212> DNA
<213> Artificial Sequence

<220>
<223> Target Specific Oligonucleotide

<400> 1125
atgcggacat ggtcgcccta gacgaccgaa tttacgg 37

<210> 1126
<211> 37
<212> DNA
<213> Artificial Sequence

<220>
<223> Target Specific Oligonucleotide

<400> 1126
ggctcccaaa cgccctgata gacgaccgaa tttacgg 37

<210> 1127
<211> 40
<212> DNA
<213> Artificial Sequence

<220>
<223> Target Specific Oligonucleotide

<400> 1127
tccatttcac cgtcagaaag gtagacgacc gaatttacgg 40

<210> 1128
<211> 43
<212> DNA
<213> Artificial Sequence

<220>
<223> Target Specific Oligonucleotide

<400> 1128
ttgcctagac agcaccgtaa tgtagacacg aatattccaa ttc 43

<210> 1129
<211> 40
<212> DNA

<213> Artificial Sequence

<220>
<223> Target Specific Oligonucleotide

<400> 1129
ttcccagagc gtggttccat agacacgaat attccaattc 40

<210> 1130
<211> 42
<212> DNA
<213> Artificial Sequence

<220>
<223> Target Specific Oligonucleotide

<400> 1130
taaggacatc gcttttccgg ttagacacga atattccaat tc 42

<210> 1131
<211> 40
<212> DNA
<213> Artificial Sequence

<220>
<223> Target Specific Oligonucleotide

<400> 1131
ttctccacgt cgcggacact agacacgaat attccaattc 40

<210> 1132
<211> 46
<212> DNA
<213> Artificial Sequence

<220>
<223> Target Specific Oligonucleotide

<400> 1132
gttctcatga tagaggttcc ttaagtagac acgaatattc caattc 46

<210> 1133
<211> 38
<212> DNA
<213> Artificial Sequence

<220>
<223> Target Specific Oligonucleotide

<400> 1133
ggccacgtcg gtgcagatag acacgaatat tccaattc 38

<210> 1134

<211> 41
<212> DNA
<213> Artificial Sequence

<220>
<223> Target Specific Oligonucleotide

<400> 1134
gttgtggacg atcaacgggg tagacacgaa tattccaatt c 41

<210> 1135
<211> 42
<212> DNA
<213> Artificial Sequence

<220>
<223> Target Specific Oligonucleotide

<400> 1135
tgtccccttc ggggtcatac ctagacacga atattccaat tc 42

<210> 1136
<211> 44
<212> DNA
<213> Artificial Sequence

<220>
<223> Target Specific Oligonucleotide

<400> 1136
aatagaacct catccggtag tggtagacac gaatattcca attc 44

<210> 1137
<211> 42
<212> DNA
<213> Artificial Sequence

<220>
<223> Target Specific Oligonucleotide

<400> 1137
cagtgcagcg tatgtggtat ttagacacga atattccaat tc 42

<210> 1138
<211> 42
<212> DNA
<213> Artificial Sequence

<220>
<223> Target Specific Oligonucleotide

<400> 1138
aaagcttgtc cgattggatg gtagacacga atattccaat tc 42

<210> 1139
<211> 42
<212> DNA
<213> Artificial Sequence

<220>
<223> Target Specific Oligonucleotide

<400> 1139
aacccttgta gatcggtggt atagacacga atattccaat tc 42

<210> 1140
<211> 42
<212> DNA
<213> Artificial Sequence

<220>
<223> Target Specific Oligonucleotide

<400> 1140
agtagagaac gtttccaccg ttagacacga atattccaat tc 42

<210> 1141
<211> 42
<212> DNA
<213> Artificial Sequence

<220>
<223> Target Specific Oligonucleotide

<400> 1141
actgctcatt gtcgttggtt ctagacacga atattccaat tc 42

<210> 1142
<211> 41
<212> DNA
<213> Artificial Sequence

<220>
<223> Target Specific Oligonucleotide

<400> 1142
aacgtattgc cgagaaccca tagacacgaa tattccaatt c 41

<210> 1143
<211> 43
<212> DNA
<213> Artificial Sequence

<220>
<223> Target Specific Oligonucleotide

<400> 1143

acaaagtgca tatagagtgg cctagacacg aatattccaa ttc 43

<210> 1144
<211> 42
<212> DNA
<213> Artificial Sequence

<220>
<223> Target Specific Oligonucleotide

<400> 1144
agccatccct agacactcgt ttagacacga atattccaat tc 42

<210> 1145
<211> 42
<212> DNA
<213> Artificial Sequence

<220>
<223> Target Specific Oligonucleotide

<400> 1145
gcgatttgga gcataccaga gtagacacga atattccaat tc 42

<210> 1146
<211> 42
<212> DNA
<213> Artificial Sequence

<220>
<223> Target Specific Oligonucleotide

<400> 1146
aggatccgat cgaaactcag ctagacacga atattccaat tc 42

<210> 1147
<211> 44
<212> DNA
<213> Artificial Sequence

<220>
<223> Target Specific Oligonucleotide

<400> 1147
ctggaatata tcgcttgtag ctgtagacac gaatattcca attc 44

<210> 1148
<211> 41
<212> DNA
<213> Artificial Sequence

<220>
<223> Target Specific Oligonucleotide

<400> 1148
cttcaggtca tgcgtggaca tagacacgaa tattccaatt c 41

<210> 1149
<211> 41
<212> DNA
<213> Artificial Sequence

<220>
<223> Target Specific Oligonucleotide

<400> 1149
agagaagcgc acttccgtgt tagacacgaa tattccaatt c 41

<210> 1150
<211> 41
<212> DNA
<213> Artificial Sequence

<220>
<223> Target Specific Oligonucleotide

<400> 1150
tgatgttatc cgtgcgcagg tagacacgaa tattccaatt c 41

<210> 1151
<211> 44
<212> DNA
<213> Artificial Sequence

<220>
<223> Target Specific Oligonucleotide

<400> 1151
aaagaccgta atcgttcaca ttgtagacac gaatattcca attc 44

<210> 1152
<211> 42
<212> DNA
<213> Artificial Sequence

<220>
<223> Target Specific Oligonucleotide

<400> 1152
agagatttgt ctacgtagag ccgtagactc agaaatctca cg 42

<210> 1153
<211> 41
<212> DNA
<213> Artificial Sequence

<220>
<223> Target Specific Oligonucleotide

<400> 1153
tcctttgcat acggtctctt cgtagactca gaaatctcac g 41

<210> 1154
<211> 43
<212> DNA
<213> Artificial Sequence

<220>
<223> Target Specific Oligonucleotide

<400> 1154
aagtttgttt acatccgatg ttactagact cagaaatctc acg 43

<210> 1155
<211> 39
<212> DNA
<213> Artificial Sequence

<220>
<223> Target Specific Oligonucleotide

<400> 1155
ttcccataca gaacgtggcc tagactcaga aatctcacg 39

<210> 1156
<211> 40
<212> DNA
<213> Artificial Sequence

<220>
<223> Target Specific Oligonucleotide

<400> 1156
aacagatcaa cggcaaaagc ctagactcag aaatctcacg 40

<210> 1157
<211> 42
<212> DNA
<213> Artificial Sequence

<220>
<223> Target Specific Oligonucleotide

<400> 1157
catccttggg tagtaggatg aactagactc agaaatctca cg 42

<210> 1158
<211> 40
<212> DNA
<213> Artificial Sequence

<220>
<223> Target Specific Oligonucleotide

<400> 1158
gaatttgcta gttgcagggc atagactcag aaatctcacg 40

<210> 1159
<211> 45
<212> DNA
<213> Artificial Sequence

<220>
<223> Target Specific Oligonucleotide

<400> 1159
tgatcttaca attgatactg tcaatgtaga ctcagaaatc tcacg 45

<210> 1160
<211> 38
<212> DNA
<213> Artificial Sequence

<220>
<223> Target Specific Oligonucleotide

<400> 1160
tggatgtgga ggcgaccgtt agactcagaa atctcacg 38

<210> 1161
<211> 42
<212> DNA
<213> Artificial Sequence

<220>
<223> Target Specific Oligonucleotide

<400> 1161
acttctggac ctatagtagc ctgtagactc agaaatctca cg 42

<210> 1162
<211> 42
<212> DNA
<213> Artificial Sequence

<220>
<223> Target Specific Oligonucleotide

<400> 1162
caacacggat agcttatcca acctagactc agaaatctca cg 42

<210> 1163
<211> 40

<212> DNA
<213> Artificial Sequence

<220>
<223> Target Specific Oligonucleotide

<400> 1163
tgtcaaggta acgtgagcac ttagactcag aaatctcacg 40

<210> 1164
<211> 41
<212> DNA
<213> Artificial Sequence

<220>
<223> Target Specific Oligonucleotide

<400> 1164
tttacagaaa tcggcatcca catagactca gaaatctcac g 41

<210> 1165
<211> 44
<212> DNA
<213> Artificial Sequence

<220>
<223> Target Specific Oligonucleotide

<400> 1165
cagctactag aggtctataa ttccttagac tcagaaatct cacg 44

<210> 1166
<211> 40
<212> DNA
<213> Artificial Sequence

<220>
<223> Target Specific Oligonucleotide

<400> 1166
ctggcacctc tatggaatcc ctagactcag aaatctcacg 40

<210> 1167
<211> 43
<212> DNA
<213> Artificial Sequence

<220>
<223> Target Specific Oligonucleotide

<400> 1167
attctgatat cggctattgt gagatagact cagaaatctc acg 43

<210> 1168
<211> 39
<212> DNA
<213> Artificial Sequence

<220>
<223> Target Specific Oligonucleotide

<400> 1168
gccaccagtc tagtgccaac tagactcaga aatctcacg 39

<210> 1169
<211> 40
<212> DNA
<213> Artificial Sequence

<220>
<223> Target Specific Oligonucleotide

<400> 1169
caactacccc ggacaatttg atagactcag aaatctcacg 40

<210> 1170
<211> 41
<212> DNA
<213> Artificial Sequence

<220>
<223> Target Specific Oligonucleotide

<400> 1170
aggacttaac atgggctatg cctagactca gaaatctcac g 41

<210> 1171
<211> 41
<212> DNA
<213> Artificial Sequence

<220>
<223> Target Specific Oligonucleotide

<400> 1171
ctctgcgaac gaagtgtttt tgtagactca gaaatctcac g 41

<210> 1172
<211> 43
<212> DNA
<213> Artificial Sequence

<220>
<223> Target Specific Oligonucleotide

<400> 1172
tgcagcatat taaggtgttg attttagact cagaaatctc acg 43

<210> 1173
<211> 44
<212> DNA
<213> Artificial Sequence

<220>
<223> Target Specific Oligonucleotide

<400> 1173
aagtaactaa acccatagac tgaaatagac tcagaaatct cacg 44

<210> 1174
<211> 40
<212> DNA
<213> Artificial Sequence

<220>
<223> Target Specific Oligonucleotide

<400> 1174
ctgccagggc ttaacataag gtagactcag aaatctcacg 40

<210> 1175
<211> 42
<212> DNA
<213> Artificial Sequence

<220>
<223> Target Specific Oligonucleotide

<400> 1175
ataacatttg atagggccac tcctagactc agaaatctca cg 42

<210> 1176
<211> 41
<212> DNA
<213> Artificial Sequence

<220>
<223> Target Specific Oligonucleotide

<400> 1176
ctgaagataa tcgcagacac cctagacgtc tcccgtattt t 41

<210> 1177
<211> 41
<212> DNA
<213> Artificial Sequence

<220>
<223> Target Specific Oligonucleotide

<400> 1177
tgactgaaca cgtgttctgt tctagacgtc tcccgtattt t 41

<210> 1178
<211> 39
<212> DNA
<213> Artificial Sequence

<220>
<223> Target Specific Oligonucleotide

<400> 1178
tttcagccat acccggatgg tagacgtctc ccgtatttt 39

<210> 1179
<211> 41
<212> DNA
<213> Artificial Sequence

<220>
<223> Target Specific Oligonucleotide

<400> 1179
tgagaagagt aaccgctgaa tatagacgtc tcccgtattt t 41

<210> 1180
<211> 38
<212> DNA
<213> Artificial Sequence

<220>
<223> Target Specific Oligonucleotide

<400> 1180
acactgcaat tccgccgtct agacgtctcc cgtatttt 38

<210> 1181
<211> 38
<212> DNA
<213> Artificial Sequence

<220>
<223> Target Specific Oligonucleotide

<400> 1181
ttgtcgtggc cggtactgat agacgtctcc cgtatttt 38

<210> 1182
<211> 39
<212> DNA
<213> Artificial Sequence

<220>

<223> Target Specific Oligonucleotide

<400> 1182
ggcaggcgac actaccttca tagacgtctc ccgtatttt 39

<210> 1183
<211> 40
<212> DNA
<213> Artificial Sequence

<220>
<223> Target Specific Oligonucleotide

<400> 1183
ctgggccagt attgaccact gtagacgtct cccgtatttt 40

<210> 1184
<211> 40
<212> DNA
<213> Artificial Sequence

<220>
<223> Target Specific Oligonucleotide

<400> 1184
tctcagatca gtgcggagaa atagacgtct cccgtatttt 40

<210> 1185
<211> 39
<212> DNA
<213> Artificial Sequence

<220>
<223> Target Specific Oligonucleotide

<400> 1185
gcccacaaat atccgtctgc tagacgtctc ccgtatttt 39

<210> 1186
<211> 36
<212> DNA
<213> Artificial Sequence

<220>
<223> Target Specific Oligonucleotide

<400> 1186
ccatgttcgc gcggcgatag acgtctcccg tatttt 36

<210> 1187
<211> 42
<212> DNA
<213> Artificial Sequence

<220>
<223> Target Specific Oligonucleotide

<400> 1187
atgaaaggtc gctagagaag tcctagacgt ctcccgtatt tt 42

<210> 1188
<211> 40
<212> DNA
<213> Artificial Sequence

<220>
<223> Target Specific Oligonucleotide

<400> 1188
aatcacaaac ccggacttgg atagacgtct cccgtatttt 40

<210> 1189
<211> 40
<212> DNA
<213> Artificial Sequence

<220>
<223> Target Specific Oligonucleotide

<400> 1189
actagcctgt cacgtttgtc ttagacgtct cccgtatttt 40

<210> 1190
<211> 41
<212> DNA
<213> Artificial Sequence

<220>
<223> Target Specific Oligonucleotide

<400> 1190
atctttacaa tgcggtcggt cctagacgtc tcccgtattt t 41

<210> 1191
<211> 41
<212> DNA
<213> Artificial Sequence

<220>
<223> Target Specific Oligonucleotide

<400> 1191
tgtcctggta tgatcccaac actagacgtc tcccgtattt t 41

<210> 1192
<211> 41
<212> DNA

<213> Artificial Sequence

<220>
<223> Target Specific Oligonucleotide

<400> 1192
ctgatcattt cgcatcacag tgtagacgtc tcccgtattt t 41

<210> 1193
<211> 41
<212> DNA
<213> Artificial Sequence

<220>
<223> Target Specific Oligonucleotide

<400> 1193
ctttccttac gtgtgtcaca catagacgtc tcccgtattt t 41

<210> 1194
<211> 42
<212> DNA
<213> Artificial Sequence

<220>
<223> Target Specific Oligonucleotide

<400> 1194
tagtttgatc gcctccttaa acctagacgt ctcccgtatt tt 42

<210> 1195
<211> 42
<212> DNA
<213> Artificial Sequence

<220>
<223> Target Specific Oligonucleotide

<400> 1195
aaatccctag gatagcacct ttgtagacgt ctcccgtatt tt 42

<210> 1196
<211> 42
<212> DNA
<213> Artificial Sequence

<220>
<223> Target Specific Oligonucleotide

<400> 1196
cttacactta cgatgagcag atgtagacgt ctcccgtatt tt 42

<210> 1197

<211> 43
<212> DNA
<213> Artificial Sequence

<220>
<223> Target Specific Oligonucleotide

<400> 1197
catttgctac ctagcatatg agagtagacg tctcccgtat ttt 43

<210> 1198
<211> 41
<212> DNA
<213> Artificial Sequence

<220>
<223> Target Specific Oligonucleotide

<400> 1198
acagatgtca aacggtcaca tttagacgtc tcccgtattt t 41

<210> 1199
<211> 43
<212> DNA
<213> Artificial Sequence

<220>
<223> Target Specific Oligonucleotide

<400> 1199
ttctaagata cgtcactcca attctagacg tctcccgtat ttt 43

<210> 1200
<211> 42
<212> DNA
<213> Artificial Sequence

<220>
<223> Target Specific Oligonucleotide

<400> 1200
acagtccgtc gattctttac aatagactct ataaatcagc gt 42

<210> 1201
<211> 41
<212> DNA
<213> Artificial Sequence

<220>
<223> Target Specific Oligonucleotide

<400> 1201
tctccacgta aatcccgatc ttagactcta taaatcagcg t 41

<210> 1202
<211> 40
<212> DNA
<213> Artificial Sequence

<220>
<223> Target Specific Oligonucleotide

<400> 1202
cacgtgctga accaaccact tagactctat aaatcagcgt 40

<210> 1203
<211> 39
<212> DNA
<213> Artificial Sequence

<220>
<223> Target Specific Oligonucleotide

<400> 1203
tcttgccgaa gttggccgtt agactctata aatcagcgt 39

<210> 1204
<211> 41
<212> DNA
<213> Artificial Sequence

<220>
<223> Target Specific Oligonucleotide

<400> 1204
agcagcagtt ggatctacag ttagactcta taaatcagcg t 41

<210> 1205
<211> 43
<212> DNA
<213> Artificial Sequence

<220>
<223> Target Specific Oligonucleotide

<400> 1205
gtctagcagt aaacctgtaa acctagactc tataaatcag cgt 43

<210> 1206
<211> 42
<212> DNA
<213> Artificial Sequence

<220>
<223> Target Specific Oligonucleotide

<400> 1206

gtgcattatt ggacgaacat catagactct ataaatcagc gt 42

<210> 1207
<211> 45
<212> DNA
<213> Artificial Sequence

<220>
<223> Target Specific Oligonucleotide

<400> 1207
gctgttgtta atggtctata atccatagac tctataaatc agcgt 45

<210> 1208
<211> 45
<212> DNA
<213> Artificial Sequence

<220>
<223> Target Specific Oligonucleotide

<400> 1208
cacacatatc ctatgttcat caatatagac tctataaatc agcgt 45

<210> 1209
<211> 43
<212> DNA
<213> Artificial Sequence

<220>
<223> Target Specific Oligonucleotide

<400> 1209
ttgagtggtc gttttcttac acatagactc tataaatcag cgt 43

<210> 1210
<211> 45
<212> DNA
<213> Artificial Sequence

<220>
<223> Target Specific Oligonucleotide

<400> 1210
cataatcaaa acgaaatgtt tggtttagac tctataaatc agcgt 45

<210> 1211
<211> 41
<212> DNA
<213> Artificial Sequence

<220>
<223> Target Specific Oligonucleotide

<400> 1211
tttgtagcat caacgtcctg gtagactcta taaatcagcg t 41

<210> 1212
<211> 41
<212> DNA
<213> Artificial Sequence

<220>
<223> Target Specific Oligonucleotide

<400> 1212
caacacattt gacctcccgt ttagactcta taaatcagcg t 41

<210> 1213
<211> 43
<212> DNA
<213> Artificial Sequence

<220>
<223> Target Specific Oligonucleotide

<400> 1213
ctaaccaccg aatagattcc tggtagactc tataaatcag cgt 43

<210> 1214
<211> 41
<212> DNA
<213> Artificial Sequence

<220>
<223> Target Specific Oligonucleotide

<400> 1214
gcagatgttt gaccggatgt ttagactcta taaatcagcg t 41

<210> 1215
<211> 42
<212> DNA
<213> Artificial Sequence

<220>
<223> Target Specific Oligonucleotide

<400> 1215
tgcttgatgt attcggccat cgtagactct ataaatcagc gt 42

<210> 1216
<211> 42
<212> DNA
<213> Artificial Sequence

<220>
<223> Target Specific Oligonucleotide

<400> 1216
tgagtttact tgcacggaaa ggtagactct ataaatcagc gt 42

<210> 1217
<211> 42
<212> DNA
<213> Artificial Sequence

<220>
<223> Target Specific Oligonucleotide

<400> 1217
cactttatcc cgcagttcag tttagactct ataaatcagc gt 42

<210> 1218
<211> 41
<212> DNA
<213> Artificial Sequence

<220>
<223> Target Specific Oligonucleotide

<400> 1218
gcaaagcatg tagccattcc ctagactcta taaatcagcg t 41

<210> 1219
<211> 43
<212> DNA
<213> Artificial Sequence

<220>
<223> Target Specific Oligonucleotide

<400> 1219
aaagacatct cgagctgcta atgtagactc tataaatcag cgt 43

<210> 1220
<211> 43
<212> DNA
<213> Artificial Sequence

<220>
<223> Target Specific Oligonucleotide

<400> 1220
gtcaccaccc atagtatgag ttttagactc tataaatcag cgt 43

<210> 1221
<211> 43
<212> DNA
<213> Artificial Sequence

<220>
<223> Target Specific Oligonucleotide

<400> 1221
tttctaccta aggctctgat gcatagactc tataaatcag cgt 43

<210> 1222
<211> 41
<212> DNA
<213> Artificial Sequence

<220>
<223> Target Specific Oligonucleotide

<400> 1222
tagagctgca cggaaagatt ttagactcta taaatcagcg t 41

<210> 1223
<211> 43
<212> DNA
<213> Artificial Sequence

<220>
<223> Target Specific Oligonucleotide

<400> 1223
gtgtagcata tgaatcgaca tcatagactc tataaatcag cgt 43

<210> 1224
<211> 43
<212> DNA
<213> Artificial Sequence

<220>
<223> Target Specific Oligonucleotide

<400> 1224
tagccttgta tggcagaatc gtagacctaa attactgtga atg 43

<210> 1225
<211> 45
<212> DNA
<213> Artificial Sequence

<220>
<223> Target Specific Oligonucleotide

<400> 1225
tgaattgccc tgggtataac tcctagacct aaattactgt gaatg 45

<210> 1226
<211> 42

<212> DNA
<213> Artificial Sequence

<220>
<223> Target Specific Oligonucleotide

<400> 1226
ttcctgttgc aacgtgaggg tagacctaaa ttactgtgaa tg 42

<210> 1227
<211> 46
<212> DNA
<213> Artificial Sequence

<220>
<223> Target Specific Oligonucleotide

<400> 1227
atgaaagaca cgatcaaaat tgaatagacc taaattactg tgaatg 46

<210> 1228
<211> 43
<212> DNA
<213> Artificial Sequence

<220>
<223> Target Specific Oligonucleotide

<400> 1228
gtcagcacga agtgagttca atagacctaa attactgtga atg 43

<210> 1229
<211> 44
<212> DNA
<213> Artificial Sequence

<220>
<223> Target Specific Oligonucleotide

<400> 1229
cagtttgcag cgtagaatct cttagaccta aattactgtg aatg 44

<210> 1230
<211> 45
<212> DNA
<213> Artificial Sequence

<220>
<223> Target Specific Oligonucleotide

<400> 1230
gtgcagataa tacgtgtctt tgctagacct aaattactgt gaatg 45

<210> 1231
<211> 45
<212> DNA
<213> Artificial Sequence

<220>
<223> Target Specific Oligonucleotide

<400> 1231
tccaatgagt atgacaagtc cattagacct aaattactgt gaatg 45

<210> 1232
<211> 45
<212> DNA
<213> Artificial Sequence

<220>
<223> Target Specific Oligonucleotide

<400> 1232
tgtaagaaag ctcggtcttc aaatagacct aaattactgt gaatg 45

<210> 1233
<211> 46
<212> DNA
<213> Artificial Sequence

<220>
<223> Target Specific Oligonucleotide

<400> 1233
ccatgtaaga tacacgtaag agaatagacc taaattactg tgaatg 46

<210> 1234
<211> 46
<212> DNA
<213> Artificial Sequence

<220>
<223> Target Specific Oligonucleotide

<400> 1234
gactcaagag catttcgtaa ttgttagacc taaattactg tgaatg 46

<210> 1235
<211> 46
<212> DNA
<213> Artificial Sequence

<220>
<223> Target Specific Oligonucleotide

<400> 1235
atgtcattag tcacctcaag ttcttagacc taaattactg tgaatg 46

<210> 1236
<211> 39
<212> DNA
<213> Artificial Sequence

<220>
<223> Target Specific Oligonucleotide

<400> 1236
ccgcactcgc acgcagatag acctaaatta ctgtgaatg 39

<210> 1237
<211> 47
<212> DNA
<213> Artificial Sequence

<220>
<223> Target Specific Oligonucleotide

<400> 1237
tgttaacagt atcgtgaata tcacttagac ctaaattact gtgaatg 47

<210> 1238
<211> 45
<212> DNA
<213> Artificial Sequence

<220>
<223> Target Specific Oligonucleotide

<400> 1238
actttgccat ctataaggga ggttagacct aaattactgt gaatg 45

<210> 1239
<211> 48
<212> DNA
<213> Artificial Sequence

<220>
<223> Target Specific Oligonucleotide

<400> 1239
cttgctatct cgataattta tgaaactaga cctaaattac tgtgaatg 48

<210> 1240
<211> 48
<212> DNA
<213> Artificial Sequence

<220>
<223> Target Specific Oligonucleotide

<400> 1240
ttaatgtatc aagagtgtta ctgaaataga cctaaattac tgtgaatg 48

<210> 1241
<211> 46
<212> DNA
<213> Artificial Sequence

<220>
<223> Target Specific Oligonucleotide

<400> 1241
tcaaatggga taccttaaca gatctagacc taaattactg tgaatg 46

<210> 1242
<211> 50
<212> DNA
<213> Artificial Sequence

<220>
<223> Target Specific Oligonucleotide

<400> 1242
ataaataagc ttcgatttat attacagcta gacctaaatt actgtgaatg 50

<210> 1243
<211> 45
<212> DNA
<213> Artificial Sequence

<220>
<223> Target Specific Oligonucleotide

<400> 1243
cttgtgtggt aggattaacc tggtagacct aaattactgt gaatg 45

<210> 1244
<211> 43
<212> DNA
<213> Artificial Sequence

<220>
<223> Target Specific Oligonucleotide

<400> 1244
gttggtcaac tcgagcatct ttagacctaa attactgtga atg 43

<210> 1245
<211> 44
<212> DNA
<213> Artificial Sequence

<220>

<223> Target Specific Oligonucleotide

<400> 1245
tttggtttgt aggcctgtag cttagaccta aattactgtg aatg 44

<210> 1246
<211> 45
<212> DNA
<213> Artificial Sequence

<220>
<223> Target Specific Oligonucleotide

<400> 1246
cattaggcag taactcttgt tcctagacct aaattactgt gaatg 45

<210> 1247
<211> 45
<212> DNA
<213> Artificial Sequence

<220>
<223> Target Specific Oligonucleotide

<400> 1247
cttctcagat acgcttcttc ttatagacct aaattactgt gaatg 45

<210> 1248
<211> 31
<212> DNA
<213> Artificial Sequence

<220>
<223> Decoding Oligonucleotide

<400> 1248
ggctaattta ttaccttcta gctataacga t 31

<210> 1249
<211> 31
<212> DNA
<213> Artificial Sequence

<220>
<223> Decoding Oligonucleotide

<400> 1249
accttaagaa tggaattcta gctataacga t 31

<210> 1250
<211> 30
<212> DNA
<213> Artificial Sequence

<220>
<223> Decoding Oligonucleotide

<400> 1250
acttaacttg agtggtctag ctataacgat 30

<210> 1251
<211> 29
<212> DNA
<213> Artificial Sequence

<220>
<223> Decoding Oligonucleotide

<400> 1251
ggctaattta ttacctcggt ctagatgga 29

<210> 1252
<211> 29
<212> DNA
<213> Artificial Sequence

<220>
<223> Decoding Oligonucleotide

<400> 1252
accttaagaa tggaatcggt ctagatgga 29

<210> 1253
<211> 28
<212> DNA
<213> Artificial Sequence

<220>
<223> Decoding Oligonucleotide

<400> 1253
acttaacttg agtggcggtc tagatgga 28

<210> 1254
<211> 32
<212> DNA
<213> Artificial Sequence

<220>
<223> Decoding Oligonucleotide

<400> 1254
ggctaattta ttacctgtta actccgtagt aa 32

<210> 1255
<211> 32
<212> DNA

<213> Artificial Sequence

<220>
<223> Decoding Oligonucleotide

<400> 1255
accttaagaa tggaatgtta actccgtagt aa 32

<210> 1256
<211> 31
<212> DNA
<213> Artificial Sequence

<220>
<223> Decoding Oligonucleotide

<400> 1256
acttaacttg agtgggttaa ctccgtagta a 31

<210> 1257
<211> 31
<212> DNA
<213> Artificial Sequence

<220>
<223> Decoding Oligonucleotide

<400> 1257
ggctaattta ttacctcgct atatctactg a 31

<210> 1258
<211> 31
<212> DNA
<213> Artificial Sequence

<220>
<223> Decoding Oligonucleotide

<400> 1258
accttaagaa tggaatcgct atatctactg a 31

<210> 1259
<211> 30
<212> DNA
<213> Artificial Sequence

<220>
<223> Decoding Oligonucleotide

<400> 1259
acttaacttg agtggcgcta tatctactga 30

<210> 1260

<211> 30
<212> DNA
<213> Artificial Sequence

<220>
<223> Decoding Oligonucleotide

<400> 1260
ggctaattta ttacctgttt ccgtttcgaa 30

<210> 1261
<211> 30
<212> DNA
<213> Artificial Sequence

<220>
<223> Decoding Oligonucleotide

<400> 1261
accttaagaa tggaatgttt ccgtttcgaa 30

<210> 1262
<211> 29
<212> DNA
<213> Artificial Sequence

<220>
<223> Decoding Oligonucleotide

<400> 1262
acttaacttg agtgggtttc cgtttcgaa 29

<210> 1263
<211> 30
<212> DNA
<213> Artificial Sequence

<220>
<223> Decoding Oligonucleotide

<400> 1263
ggctaattta ttaccttagc gtttcaaaag 30

<210> 1264
<211> 30
<212> DNA
<213> Artificial Sequence

<220>
<223> Decoding Oligonucleotide

<400> 1264
accttaagaa tggaattagc gtttcaaaag 30

<210> 1265
<211> 29
<212> DNA
<213> Artificial Sequence

<220>
<223> Decoding Oligonucleotide

<400> 1265
acttaacttg agtggtagcg tttcaaaag 29

<210> 1266
<211> 28
<212> DNA
<213> Artificial Sequence

<220>
<223> Decoding Oligonucleotide

<400> 1266
ggctaattta ttaccttgat ccgacgag 28

<210> 1267
<211> 28
<212> DNA
<213> Artificial Sequence

<220>
<223> Decoding Oligonucleotide

<400> 1267
accttaagaa tggaattgat ccgacgag 28

<210> 1268
<211> 27
<212> DNA
<213> Artificial Sequence

<220>
<223> Decoding Oligonucleotide

<400> 1268
acttaacttg agtggtgatc cgacgag 27

<210> 1269
<211> 31
<212> DNA
<213> Artificial Sequence

<220>
<223> Decoding Oligonucleotide

<400> 1269

ggctaattta ttacctaatc tacatgtctg a 31

<210> 1270
<211> 31
<212> DNA
<213> Artificial Sequence

<220>
<223> Decoding Oligonucleotide

<400> 1270
accttaagaa tggaataatc tacatgtctg a 31

<210> 1271
<211> 30
<212> DNA
<213> Artificial Sequence

<220>
<223> Decoding Oligonucleotide

<400> 1271
acttaacttg agtggaatct acatgtctga 30

<210> 1272
<211> 30
<212> DNA
<213> Artificial Sequence

<220>
<223> Decoding Oligonucleotide

<400> 1272
ggctaattta ttacctggac gttgaataca 30

<210> 1273
<211> 30
<212> DNA
<213> Artificial Sequence

<220>
<223> Decoding Oligonucleotide

<400> 1273
accttaagaa tggaatggac gttgaataca 30

<210> 1274
<211> 29
<212> DNA
<213> Artificial Sequence

<220>
<223> Decoding Oligonucleotide

<400> 1274
acttaacttg agtggggacg ttgaataca 29

<210> 1275
<211> 31
<212> DNA
<213> Artificial Sequence

<220>
<223> Decoding Oligonucleotide

<400> 1275
ggctaattta ttaccttaaa gtgacaattc a 31

<210> 1276
<211> 31
<212> DNA
<213> Artificial Sequence

<220>
<223> Decoding Oligonucleotide

<400> 1276
accttaagaa tggaattaaa gtgacaattc a 31

<210> 1277
<211> 30
<212> DNA
<213> Artificial Sequence

<220>
<223> Decoding Oligonucleotide

<400> 1277
acttaacttg agtggtaaag tgacaattca 30

<210> 1278
<211> 32
<212> DNA
<213> Artificial Sequence

<220>
<223> Decoding Oligonucleotide

<400> 1278
ggctaattta ttacctgatt cccataatta gg 32

<210> 1279
<211> 32
<212> DNA
<213> Artificial Sequence

<220>
<223> Decoding Oligonucleotide

<400> 1279
accttaagaa tggaatgatt cccataatta gg 32

<210> 1280
<211> 31
<212> DNA
<213> Artificial Sequence

<220>
<223> Decoding Oligonucleotide

<400> 1280
acttaacttg agtgggattc ccataattag g 31

<210> 1281
<211> 33
<212> DNA
<213> Artificial Sequence

<220>
<223> Decoding Oligonucleotide

<400> 1281
ggctaattta ttacctcgtc gtataaatct atc 33

<210> 1282
<211> 33
<212> DNA
<213> Artificial Sequence

<220>
<223> Decoding Oligonucleotide

<400> 1282
accttaagaa tggaatcgtc gtataaatct atc 33

<210> 1283
<211> 32
<212> DNA
<213> Artificial Sequence

<220>
<223> Decoding Oligonucleotide

<400> 1283
acttaacttg agtggcgtcg tataaatcta tc 32

<210> 1284
<211> 34
<212> DNA
<213> Artificial Sequence

<220>
<223> Decoding Oligonucleotide

<400> 1284
ggctaattta ttacctgatg tctatccttt ataa 34

<210> 1285
<211> 34
<212> DNA
<213> Artificial Sequence

<220>
<223> Decoding Oligonucleotide

<400> 1285
accttaagaa tggaatgatg tctatccttt ataa 34

<210> 1286
<211> 33
<212> DNA
<213> Artificial Sequence

<220>
<223> Decoding Oligonucleotide

<400> 1286
acttaacttg agtgggatgt ctatccttta taa 33

<210> 1287
<211> 30
<212> DNA
<213> Artificial Sequence

<220>
<223> Decoding Oligonucleotide

<400> 1287
ggctaattta ttacctttc cacgataacc 30

<210> 1288
<211> 30
<212> DNA
<213> Artificial Sequence

<220>
<223> Decoding Oligonucleotide

<400> 1288
accttaagaa tggaattttc cacgataacc 30

<210> 1289
<211> 29

<212> DNA
<213> Artificial Sequence

<220>
<223> Decoding Oligonucleotide

<400> 1289
acttaacttg agtggtttcc acgataacc 29

<210> 1290
<211> 31
<212> DNA
<213> Artificial Sequence

<220>
<223> Decoding Oligonucleotide

<400> 1290
ggctaattta ttaccttagt accgaataag g 31

<210> 1291
<211> 31
<212> DNA
<213> Artificial Sequence

<220>
<223> Decoding Oligonucleotide

<400> 1291
accttaagaa tggaattagt accgaataag g 31

<210> 1292
<211> 30
<212> DNA
<213> Artificial Sequence

<220>
<223> Decoding Oligonucleotide

<400> 1292
acttaacttg agtggtagta ccgaataagg 30

<210> 1293
<211> 32
<212> DNA
<213> Artificial Sequence

<220>
<223> Decoding Oligonucleotide

<400> 1293
ggctaattta ttaccttaaa gttgtaaaat cc 32

<210> 1294
<211> 32
<212> DNA
<213> Artificial Sequence

<220>
<223> Decoding Oligonucleotide

<400> 1294
accttaagaa tggaattaaa gttgtaaaat cc 32

<210> 1295
<211> 31
<212> DNA
<213> Artificial Sequence

<220>
<223> Decoding Oligonucleotide

<400> 1295
acttaacttg agtggtaaag ttgtaaaatc c 31

<210> 1296
<211> 32
<212> DNA
<213> Artificial Sequence

<220>
<223> Decoding Oligonucleotide

<400> 1296
ggctaattta ttacctggat aagtcggtaa tc 32

<210> 1297
<211> 32
<212> DNA
<213> Artificial Sequence

<220>
<223> Decoding Oligonucleotide

<400> 1297
accttaagaa tggaatggat aagtcggtaa tc 32

<210> 1298
<211> 31
<212> DNA
<213> Artificial Sequence

<220>
<223> Decoding Oligonucleotide

<400> 1298
acttaacttg agtggggata agtcggtaat c 31

<210> 1299
<211> 33
<212> DNA
<213> Artificial Sequence

<220>
<223> Decoding Oligonucleotide

<400> 1299
ggctaattta ttacctggta tttcactcta att 33

<210> 1300
<211> 33
<212> DNA
<213> Artificial Sequence

<220>
<223> Decoding Oligonucleotide

<400> 1300
accttaagaa tggaatggta tttcactcta att 33

<210> 1301
<211> 32
<212> DNA
<213> Artificial Sequence

<220>
<223> Decoding Oligonucleotide

<400> 1301
acttaacttg agtggggtat ttcactctaa tt 32

<210> 1302
<211> 32
<212> DNA
<213> Artificial Sequence

<220>
<223> Decoding Oligonucleotide

<400> 1302
ggctaattta ttacctagct agataaagta cc 32

<210> 1303
<211> 32
<212> DNA
<213> Artificial Sequence

<220>
<223> Decoding Oligonucleotide

<400> 1303
accttaagaa tggaatagct agataaagta cc 32

<210> 1304
<211> 31
<212> DNA
<213> Artificial Sequence

<220>
<223> Decoding Oligonucleotide

<400> 1304
acttaacttg agtggagcta gataaagtac c 31

<210> 1305
<211> 32
<212> DNA
<213> Artificial Sequence

<220>
<223> Decoding Oligonucleotide

<400> 1305
ggctaattta ttacctccga aactacgatt at 32

<210> 1306
<211> 32
<212> DNA
<213> Artificial Sequence

<220>
<223> Decoding Oligonucleotide

<400> 1306
accttaagaa tggaatccga aactacgatt at 32

<210> 1307
<211> 31
<212> DNA
<213> Artificial Sequence

<220>
<223> Decoding Oligonucleotide

<400> 1307
acttaacttg agtggccgaa actacgatta t 31

<210> 1308
<211> 32
<212> DNA
<213> Artificial Sequence

<220>

<223> Decoding Oligonucleotide

<400> 1308
ggctaattta ttacctcatc gaatttatgt ta 32

<210> 1309
<211> 32
<212> DNA
<213> Artificial Sequence

<220>
<223> Decoding Oligonucleotide

<400> 1309
accttaagaa tggaatcatc gaatttatgt ta 32

<210> 1310
<211> 31
<212> DNA
<213> Artificial Sequence

<220>
<223> Decoding Oligonucleotide

<400> 1310
acttaacttg agtggcatcg aatttatgtt a 31

<210> 1311
<211> 30
<212> DNA
<213> Artificial Sequence

<220>
<223> Decoding Oligonucleotide

<400> 1311
ggctaattta ttacctccgt aattctaccg 30

<210> 1312
<211> 30
<212> DNA
<213> Artificial Sequence

<220>
<223> Decoding Oligonucleotide

<400> 1312
accttaagaa tggaatccgt aattctaccg 30

<210> 1313
<211> 29
<212> DNA
<213> Artificial Sequence

<220>
<223> Decoding Oligonucleotide

<400> 1313
acttaacttg agtggccgta attctaccg 29

<210> 1314
<211> 29
<212> DNA
<213> Artificial Sequence

<220>
<223> Decoding Oligonucleotide

<400> 1314
ggctaattta ttacctgggt aatcgtcac 29

<210> 1315
<211> 29
<212> DNA
<213> Artificial Sequence

<220>
<223> Decoding Oligonucleotide

<400> 1315
accttaagaa tggaatgggt aatcgtcac 29

<210> 1316
<211> 28
<212> DNA
<213> Artificial Sequence

<220>
<223> Decoding Oligonucleotide

<400> 1316
acttaacttg agtgggggta atcgtcac 28

<210> 1317
<211> 31
<212> DNA
<213> Artificial Sequence

<220>
<223> Decoding Oligonucleotide

<400> 1317
ggctaattta ttacctagtg ttatgagaag c 31

<210> 1318
<211> 31
<212> DNA

<213> Artificial Sequence

<220>
<223> Decoding Oligonucleotide

<400> 1318
accttaagaa tggaatagtg ttatgagaag c 31

<210> 1319
<211> 30
<212> DNA
<213> Artificial Sequence

<220>
<223> Decoding Oligonucleotide

<400> 1319
acttaacttg agtggagtgt tatgagaagc 30

<210> 1320
<211> 31
<212> DNA
<213> Artificial Sequence

<220>
<223> Decoding Oligonucleotide

<400> 1320
ggctaattta ttacctctgc aatagaagag a 31

<210> 1321
<211> 31
<212> DNA
<213> Artificial Sequence

<220>
<223> Decoding Oligonucleotide

<400> 1321
accttaagaa tggaatctgc aatagaagag a 31

<210> 1322
<211> 30
<212> DNA
<213> Artificial Sequence

<220>
<223> Decoding Oligonucleotide

<400> 1322
acttaacttg agtggctgca atagaagaga 30

<210> 1323

<211> 30
<212> DNA
<213> Artificial Sequence

<220>
<223> Decoding Oligonucleotide

<400> 1323
ggctaattta ttacctgatt tttcagcgac 30

<210> 1324
<211> 30
<212> DNA
<213> Artificial Sequence

<220>
<223> Decoding Oligonucleotide

<400> 1324
accttaagaa tggaatgatt tttcagcgac 30

<210> 1325
<211> 29
<212> DNA
<213> Artificial Sequence

<220>
<223> Decoding Oligonucleotide

<400> 1325
acttaacttg agtgggattt ttcagcgac 29

<210> 1326
<211> 32
<212> DNA
<213> Artificial Sequence

<220>
<223> Decoding Oligonucleotide

<400> 1326
ggctaattta ttacctcgta atatgaagat ca 32

<210> 1327
<211> 32
<212> DNA
<213> Artificial Sequence

<220>
<223> Decoding Oligonucleotide

<400> 1327
accttaagaa tggaatcgta atatgaagat ca 32

<210> 1328
<211> 31
<212> DNA
<213> Artificial Sequence

<220>
<223> Decoding Oligonucleotide

<400> 1328
acttaacttg agtggcgtaa tatgaagatc a 31

<210> 1329
<211> 32
<212> DNA
<213> Artificial Sequence

<220>
<223> Decoding Oligonucleotide

<400> 1329
ggctaattta ttacctgaat tggaatattc gt 32

<210> 1330
<211> 32
<212> DNA
<213> Artificial Sequence

<220>
<223> Decoding Oligonucleotide

<400> 1330
accttaagaa tggaatgaat tggaatattc gt 32

<210> 1331
<211> 31
<212> DNA
<213> Artificial Sequence

<220>
<223> Decoding Oligonucleotide

<400> 1331
acttaacttg agtgggaatt ggaatattcg t 31

<210> 1332
<211> 33
<212> DNA
<213> Artificial Sequence

<220>
<223> Decoding Oligonucleotide

<400> 1332

ggctaattta ttacctggta ttaatcttga aga 33

<210> 1333
<211> 33
<212> DNA
<213> Artificial Sequence

<220>
<223> Decoding Oligonucleotide

<400> 1333
accttaagaa tggaatggta ttaatcttga aga 33

<210> 1334
<211> 32
<212> DNA
<213> Artificial Sequence

<220>
<223> Decoding Oligonucleotide

<400> 1334
acttaacttg agtggggtat taatcttgaa ga 32

<210> 1335
<211> 29
<212> DNA
<213> Artificial Sequence

<220>
<223> Decoding Oligonucleotide

<400> 1335
ggctaattta ttacctacga tgtattgcc 29

<210> 1336
<211> 29
<212> DNA
<213> Artificial Sequence

<220>
<223> Decoding Oligonucleotide

<400> 1336
accttaagaa tggaatacga tgtattgcc 29

<210> 1337
<211> 28
<212> DNA
<213> Artificial Sequence

<220>
<223> Decoding Oligonucleotide

<400> 1337
acttaacttg agtggacgat gtattgcc 28

<210> 1338
<211> 32
<212> DNA
<213> Artificial Sequence

<220>
<223> Decoding Oligonucleotide

<400> 1338
ggctaattta ttacctcata tacaaacggt ac 32

<210> 1339
<211> 32
<212> DNA
<213> Artificial Sequence

<220>
<223> Decoding Oligonucleotide

<400> 1339
accttaagaa tggaatcata tacaaacggt ac 32

<210> 1340
<211> 31
<212> DNA
<213> Artificial Sequence

<220>
<223> Decoding Oligonucleotide

<400> 1340
acttaacttg agtggcatat acaaacggta c 31

<210> 1341
<211> 31
<212> DNA
<213> Artificial Sequence

<220>
<223> Decoding Oligonucleotide

<400> 1341
ggctaattta ttacctttcc gatcttaact t 31

<210> 1342
<211> 31
<212> DNA
<213> Artificial Sequence

<220>
<223> Decoding Oligonucleotide

<400> 1342
accttaagaa tggaatttcc gatcttaact t 31

<210> 1343
<211> 30
<212> DNA
<213> Artificial Sequence

<220>
<223> Decoding Oligonucleotide

<400> 1343
acttaacttg agtggttccg atcttaactt 30

<210> 1344
<211> 31
<212> DNA
<213> Artificial Sequence

<220>
<223> Decoding Oligonucleotide

<400> 1344
ggctaattta ttacctgtgt taattcgcat c 31

<210> 1345
<211> 31
<212> DNA
<213> Artificial Sequence

<220>
<223> Decoding Oligonucleotide

<400> 1345
accttaagaa tggaatgtgt taattcgcat c 31

<210> 1346
<211> 30
<212> DNA
<213> Artificial Sequence

<220>
<223> Decoding Oligonucleotide

<400> 1346
acttaacttg agtgggtgtt aattcgcatc 30

<210> 1347
<211> 30
<212> DNA
<213> Artificial Sequence

<220>
<223> Decoding Oligonucleotide

<400> 1347
ggctaattta ttacctcgtt taattgggct 30

<210> 1348
<211> 30
<212> DNA
<213> Artificial Sequence

<220>
<223> Decoding Oligonucleotide

<400> 1348
accttaagaa tggaatcgtt taattgggct 30

<210> 1349
<211> 29
<212> DNA
<213> Artificial Sequence

<220>
<223> Decoding Oligonucleotide

<400> 1349
acttaacttg agtggcgttt aattgggct 29

<210> 1350
<211> 31
<212> DNA
<213> Artificial Sequence

<220>
<223> Decoding Oligonucleotide

<400> 1350
ggctaattta ttacctcaac acttataacg g 31

<210> 1351
<211> 31
<212> DNA
<213> Artificial Sequence

<220>
<223> Decoding Oligonucleotide

<400> 1351
accttaagaa tggaatcaac acttataacg g 31

<210> 1352
<211> 30

<212> DNA
<213> Artificial Sequence

<220>
<223> Decoding Oligonucleotide

<400> 1352
acttaacttg agtggcaaca cttataacgg 30

<210> 1353
<211> 34
<212> DNA
<213> Artificial Sequence

<220>
<223> Decoding Oligonucleotide

<400> 1353
ggctaattta ttacctggaa ataagtatta tacc 34

<210> 1354
<211> 34
<212> DNA
<213> Artificial Sequence

<220>
<223> Decoding Oligonucleotide

<400> 1354
accttaagaa tggaatggaa ataagtatta tacc 34

<210> 1355
<211> 33
<212> DNA
<213> Artificial Sequence

<220>
<223> Decoding Oligonucleotide

<400> 1355
acttaacttg agtggggaaa taagtattat acc 33

<210> 1356
<211> 29
<212> DNA
<213> Artificial Sequence

<220>
<223> Decoding Oligonucleotide

<400> 1356
ggctaattta ttacctctta acgacggtt 29

<210> 1357
<211> 29
<212> DNA
<213> Artificial Sequence

<220>
<223> Decoding Oligonucleotide

<400> 1357
accttaagaa tggaatctta acgacggtt 29

<210> 1358
<211> 28
<212> DNA
<213> Artificial Sequence

<220>
<223> Decoding Oligonucleotide

<400> 1358
acttaacttg agtggcttaa cgacggtt 28

<210> 1359
<211> 33
<212> DNA
<213> Artificial Sequence

<220>
<223> Decoding Oligonucleotide

<400> 1359
ggctaattta ttacctgctc gttagaattt att 33

<210> 1360
<211> 33
<212> DNA
<213> Artificial Sequence

<220>
<223> Decoding Oligonucleotide

<400> 1360
accttaagaa tggaatgctc gttagaattt att 33

<210> 1361
<211> 32
<212> DNA
<213> Artificial Sequence

<220>
<223> Decoding Oligonucleotide

<400> 1361
acttaacttg agtgggctcg ttagaattta tt 32

<210> 1362
<211> 32
<212> DNA
<213> Artificial Sequence

<220>
<223> Decoding Oligonucleotide

<400> 1362
ggctaattta ttaccttctt tatcatcatt ga 32

<210> 1363
<211> 32
<212> DNA
<213> Artificial Sequence

<220>
<223> Decoding Oligonucleotide

<400> 1363
accttaagaa tggaattctt tatcatcatt ga 32

<210> 1364
<211> 31
<212> DNA
<213> Artificial Sequence

<220>
<223> Decoding Oligonucleotide

<400> 1364
acttaacttg agtggtcttt atcatcattg a 31

<210> 1365
<211> 31
<212> DNA
<213> Artificial Sequence

<220>
<223> Decoding Oligonucleotide

<400> 1365
ggctaattta ttacctcgtt cgcaatttat c 31

<210> 1366
<211> 31
<212> DNA
<213> Artificial Sequence

<220>
<223> Decoding Oligonucleotide

<400> 1366
accttaagaa tggaatcgtt cgcaatttat c 31

<210> 1367
<211> 30
<212> DNA
<213> Artificial Sequence

<220>
<223> Decoding Oligonucleotide

<400> 1367
acttaacttg agtggcgttc gcaatttatc 30

<210> 1368
<211> 31
<212> DNA
<213> Artificial Sequence

<220>
<223> Decoding Oligonucleotide

<400> 1368
ggctaattta ttacctcgat cttttgttgt a 31

<210> 1369
<211> 31
<212> DNA
<213> Artificial Sequence

<220>
<223> Decoding Oligonucleotide

<400> 1369
accttaagaa tggaatcgat cttttgttgt a 31

<210> 1370
<211> 30
<212> DNA
<213> Artificial Sequence

<220>
<223> Decoding Oligonucleotide

<400> 1370
acttaacttg agtggcgatc ttttgttgta 30

<210> 1371
<211> 32
<212> DNA
<213> Artificial Sequence

<220>

<223> Decoding Oligonucleotide

<400> 1371
ggctaattta ttacctacca ctcaatataa ga 32

<210> 1372
<211> 32
<212> DNA
<213> Artificial Sequence

<220>
<223> Decoding Oligonucleotide

<400> 1372
accttaagaa tggaatacca ctcaatataa ga 32

<210> 1373
<211> 31
<212> DNA
<213> Artificial Sequence

<220>
<223> Decoding Oligonucleotide

<400> 1373
acttaacttg agtggaccac tcaatataag a 31

<210> 1374
<211> 30
<212> DNA
<213> Artificial Sequence

<220>
<223> Decoding Oligonucleotide

<400> 1374
ggctaattta ttacctccgt aaattcggtc 30

<210> 1375
<211> 30
<212> DNA
<213> Artificial Sequence

<220>
<223> Decoding Oligonucleotide

<400> 1375
accttaagaa tggaatccgt aaattcggtc 30

<210> 1376
<211> 29
<212> DNA
<213> Artificial Sequence

<220>
<223> Decoding Oligonucleotide

<400> 1376
acttaacttg agtggccgta aattcggtc 29

<210> 1377
<211> 30
<212> DNA
<213> Artificial Sequence

<220>
<223> Decoding Oligonucleotide

<400> 1377
ggctaattta ttacctcgtg agatttctga 30

<210> 1378
<211> 30
<212> DNA
<213> Artificial Sequence

<220>
<223> Decoding Oligonucleotide

<400> 1378
accttaagaa tggaatcgtg agatttctga 30

<210> 1379
<211> 29
<212> DNA
<213> Artificial Sequence

<220>
<223> Decoding Oligonucleotide

<400> 1379
acttaacttg agtggcgtga gatttctga 29

<210> 1380
<211> 31
<212> DNA
<213> Artificial Sequence

<220>
<223> Decoding Oligonucleotide

<400> 1380
ggctaattta ttacctcgaa gcatagaata g 31

<210> 1381
<211> 31
<212> DNA

<213> Artificial Sequence

<220>
<223> Decoding Oligonucleotide

<400> 1381
accttaagaa tggaatcgaa gcatagaata g 31

<210> 1382
<211> 30
<212> DNA
<213> Artificial Sequence

<220>
<223> Decoding Oligonucleotide

<400> 1382
acttaacttg agtggcgaag catagaatag 30

<210> 1383
<211> 30
<212> DNA
<213> Artificial Sequence

<220>
<223> Decoding Oligonucleotide

<400> 1383
ggctaattta ttacctaaaa tacgggagac 30

<210> 1384
<211> 30
<212> DNA
<213> Artificial Sequence

<220>
<223> Decoding Oligonucleotide

<400> 1384
accttaagaa tggaataaaa tacgggagac 30

<210> 1385
<211> 29
<212> DNA
<213> Artificial Sequence

<220>
<223> Decoding Oligonucleotide

<400> 1385
acttaacttg agtggaaaat acgggagac 29

<210> 1386

<211> 31
<212> DNA
<213> Artificial Sequence

<220>
<223> Decoding Oligonucleotide

<400> 1386
ggctaattta ttacctcgca tggtataaca t 31

<210> 1387
<211> 31
<212> DNA
<213> Artificial Sequence

<220>
<223> Decoding Oligonucleotide

<400> 1387
accttaagaa tggaatcgca tggtataaca t 31

<210> 1388
<211> 30
<212> DNA
<213> Artificial Sequence

<220>
<223> Decoding Oligonucleotide

<400> 1388
acttaacttg agtggcgcat ggtataacat 30

<210> 1389
<211> 31
<212> DNA
<213> Artificial Sequence

<220>
<223> Decoding Oligonucleotide

<400> 1389
ggctaattta ttacctacgc tgatttatag a 31

<210> 1390
<211> 31
<212> DNA
<213> Artificial Sequence

<220>
<223> Decoding Oligonucleotide

<400> 1390
accttaagaa tggaatacgc tgatttatag a 31

<210> 1391
<211> 30
<212> DNA
<213> Artificial Sequence

<220>
<223> Decoding Oligonucleotide

<400> 1391
acttaacttg agtggacgct gatttataga 30

<210> 1392
<211> 33
<212> DNA
<213> Artificial Sequence

<220>
<223> Decoding Oligonucleotide

<400> 1392
ggctaattta ttacctcgta gggtaaatag att 33

<210> 1393
<211> 33
<212> DNA
<213> Artificial Sequence

<220>
<223> Decoding Oligonucleotide

<400> 1393
accttaagaa tggaatcgta gggtaaatag att 33

<210> 1394
<211> 32
<212> DNA
<213> Artificial Sequence

<220>
<223> Decoding Oligonucleotide

<400> 1394
acttaacttg agtggcgtag ggtaaataga tt 32

<210> 1395
<211> 33
<212> DNA
<213> Artificial Sequence

<220>
<223> Decoding Oligonucleotide

<400> 1395

ggctaattta ttacctcatt cacagtaatt tag 33

<210> 1396
<211> 33
<212> DNA
<213> Artificial Sequence

<220>
<223> Decoding Oligonucleotide

<400> 1396
accttaagaa tggaatcatt cacagtaatt tag 33

<210> 1397
<211> 32
<212> DNA
<213> Artificial Sequence

<220>
<223> Decoding Oligonucleotide

<400> 1397
acttaacttg agtggcattc acagtaattt ag 32

<210> 1398
<211> 15
<212> DNA
<213> Artificial Sequence

<220>
<223> Signal Oligonucleotide

<400> 1398
ccactcaagt taagt 15

<210> 1399
<211> 16
<212> DNA
<213> Artificial Sequence

<220>
<223> Signal Oligonucleotide

<400> 1399
attccattct taaggt 16

<210> 1400
<211> 16
<212> DNA
<213> Artificial Sequence

<220>
<223> Signal Oligonucleotide

```
<400> 1400
aggtaataaa ttagcc                                                       16
```